# EUROPÄISCHE PATENTANMELDUNG

(11) **EP 2 487 245 A2**
(43) Veröffentlichungstag der Anmeldung: **15.08.2012**
(21) Anmeldenummer: 12166996.4
(22) Anmeldetag: 12.10.2007
(51) Int. Cl.: C12N 15/82, A01H 5/00

(54) **Verfahren zur Erhöhung der Pathogenresistenz in transgenen Pflanzen**

(30) Priorität: 12.10.2006 EP 06122217
(62) Teilanmeldung aus: 07821224.8
(71) Anmelder: BASF Plant Science GmbH, 67056 Ludwigshafen (DE)
(72) Erfinder: Frank, Markus, 67434 Neustadt (DE); Schweizer, Patrick, 06493 Ballenstedt (DE); Douchkov, Dimitar, 06458 Hedersleben (DE)
(74) Vertreter: Patzelt, Andrea

(57) **Zusammenfassung**

Die Erfindung betrifft ein Verfahren zur Erhöhung der Resistenz gegenüber einem oder mehreren penetrierenden Pathogen(en) in einer monokotyledonen oder dikotyledonen Pflanze, oder einem Teil einer Pflanze, z.B. in einem Organ, Gewebe, einer Zelle oder einem Teil einer pflanzlichen Zelle, z.B. in einem Organell, dadurch gekennzeichnet, dass eine DNA-Sequenz, die für ein Armadillo Repeat ARM1 Protein kodiert, und eine erhöhte Pathogenresistenz, bevorzugt eine erhöhte Pilzpathogenresistenz, vermittelt, in die Pflanze bzw. Pflanzenzelle eingeführt und dort exprimiert wird; oder dass eine endogene DNA-Sequenz, die für ein ein Armadillo Repeat ARM1 Protein kodiert, und eine erhöhte Pathogenresistenz, bevorzugt eine erhöhte Pilzpathogenresistenz, vermittelt, in der Pflanze bzw. Pflanzenzelle gegenüber der Ausgangs- bzw. Wildtyppflanze bzw. deren Teil erhöht wird, oder dass die endogene Gensequenz oder bevorzugt die 5'-untranslatierte Region (5'UTR) gegenüber der Ausgangssequenz verändert wird. Ebenfalls betrifft die Erfindung Pfanzen, Teile einer Pflanze, z.B. in einem Organ, Gewebe, einer Zelle oder einem Teil einer pflanzlichen Zelle, z.B. in einem Organell, die nach den voranstehenden verfahren erhalten werden, sowie entsprechendendes Vermehrungsgut.

## Beschreibung

Die vorliegende Erfindung betrifft ein Verfahren zur Erhöhung der Pathogenresistenz in transgenen Pflanzen und/oder Pflanzenzellen, wobei eine DNA-Sequenz, die für ein Hordeum vulgare Armadillo-Repeat (HvARM)-Polynukleotid oder ein funktionelles Äquivalent desselben kodiert, in die Pflanze bzw. Pflanzenzelle eingeführt und dort exprimiert wird. Die vorliegende Erfindung betrifft ebenfalls die Verwendung von Nukleinsäuren, die für ein solches Protein kodieren, zur Herstellung von transgenen Pflanzen bzw. Pflanzenzellen mit einer erhöhten Pathogenresistenz. Weiterhin betrifft die vorliegende Erfindung Nukleinsäuresequenzen, die für ein solches Protein kodieren, das eine erhöhte Pathogenresistenz in Pflanzen vermittelt. Ferner betrifft die Erfindung homologe Sequenzen (ARM1) davon, sowie deren Verwendung in Verfahren zum Erreichen einer Pathogenresistenz in Pflanzen, sowie Nukleinsäurekonstrukte, Expressionskassetten und Vektoren, die diese Sequenzen umfassen, und die geeigntet sind, eine Pilzresistenz in Pflanzen zu vermitteln. Die Erfindung betrifft ferner mit diesen Expressionskassetten oder Vektoren transformierte transgene Organismen, insbesondere Pflanzen, davon abgeleitete Kulturen, Teile oder transgenes Vermehrungsgut.

Pflanzenkrankheiten, die durch verschiedene Pathogene wie z.B. Viren, Bakterien und Pilze verursacht werden, können beim Anbau von Kulturpflanzen zu erheblichen Emteausfällen führen, was zum einen wirtschaftliche Folgen hat, aber auch die Sicherheit der menschlichen Ernährung bedroht. Seit dem letzten Jahrhundert werden zur Kontrolle von Pilzerkrankungen chemische Fungizide eingesetzt. Durch den Einsatz dieser Stoffe konnte zwar das Ausmaß von Pflanzenerkrankungen reduziert werden, es ist allerdings bis heute nicht auszuschließen, dass diese Verbindungen eine schädliche Wirkung auf Mensch, Tier und Umwelt ausüben. Um langfristig den Verbrauch von herkömmlichen Pflanzenschutzmitteln auf ein Minimum zu reduzieren, ist es daher von Bedeutung, die natürliche Pathogenabwehr von verschiedenen Pflanzen gegenüber unterschiedlichen Erregern zu untersuchen und sie sich durch gentechnologische Manipulation, z.B. durch das Einführen externer Resistenzgene oder durch die Manipulation der endogenen Genexpression in den Pflanzen, zur Herstellung von pathogenresistenten Pflanzen gezielt nutzbar zu machen.

Es gibt nur wenige Ansätze, die Pflanzen eine Resistenz gegenüber Pathogenen, vor allem Pilzpathogenen, verleihen. Dieser Mangel ist zum Teil in der Komplexität der betrachteten biologischen Systeme begründet. Dem Erreichen von Resistenzen gegenüber Pathogenen steht auch entgegen, dass über die Wechselwirkungen zwischen Pathogen und Pflanze wenig bekannt ist. Die große Anzahl unterschiedlicher Pathogene, die von ihnen entwickelten Infektionsmechanismen und die von den Pflanzen-familien, -gattungen und -arten entwickelten Abwehrmechanismen stehen in vielfältigen Wechselwirkungen miteinander.

Pilzpathogene haben im Wesentlichen zwei Infektionsstrategien entwickelt. Manche Pilze dringen über die Spaltöffnungen in das Wirtsgewebe ein (z.B. Rostpilze, Septoria-, Fusarium-Arten) und penetrieren das Mesophyllgewebe, während andere über die Cuticula die darunterliegenden Epidermiszellen penetrieren (z.B. Blumeria Arten).

Die durch die Pilzpathogene verursachten Infektionen führen in den befallen Pflanzen zur Aktivierung der pflanzlichen Abwehrmechanismen. So konnte gezeigt werden, dass Abwehrreaktionen gegen Epidermis penetrierende Pilze häufig mit der Ausbildung einer Penetrationsresistenz (Papillenbildung, Zellwandverdickungen mit Kallose als Hauptbestandteil) unterhalb der pilzlichen Penetrationshyphe beginnen (Elliott et al., Mol. Plant Microbe Interact. 15, 1069 (2002)).

Die pflanzlichen Abwehrmechanismen vermitteln jedoch in vielen Fällen nur einen unzureichenden Schutzmechnismus gegen Pathogenbefall.

Die Ausbildung einer Penetrationsresistenz gegenüber Pathogenen, deren Infektionsmechanismus eine Penetration der Epidermis- oder Mesophyllzellen beinhaltet, ist sowohl für monokotyledone als auch dikotyledone Pflanzen von grosser Bedeutung. Sie kann vermutlich im Gegensatz zur beschriebenen mlo-vermittelten Resistenz die Ausbildung einer BreitspektrumResistenz gegen obligat-biotrophe, hemibiotrophe und nekrotrophe Pilze ermöglichen.

Bisher wurden zur Erzeugung pilzresistenter Pflanzen häufig quantitative Resistenzmerkmale (Resistenz-QTLs) eingekreuzt. Dieses Verfahren hat aber den Nachteil, dass oft unerwünschte Merkmale mit eingekreuzt werden. Darüber hinaus sind die dazu benötigten Züchtungsmethoden sehr kompliziert und zeitaufwendig.

Daher lag der vorliegenden Erfindung die Aufgabe zugrunde, ein Verfahren zur Erzeugung einer Resistenz von Pflanzen gegen penetrierende Pathogene bereitzustellen.

Die Lösung der Aufgabe wird durch die in den Ansprüchen charakterisierten Ausführungsformen gelöst.

Folglich betrifft die Erfindung ein Verfahren zur Erhöhung der Resistenz gegenüber einem oder mehreren penetrierenden Pathogen(en) in einer monokotyledonen oder dikotyledonen Pflanze, oder einem Teil einer Pflanze, z.B. in einem Organ, Gewebe, einer Zelle oder einem Teil einer pflanzlichen Zelle, z.B. in einem Organell, dadurch gekennzeichnet, dass eine DNA-Sequenz, die für ein ein Armadillo Repeat ARM1 Protein kodiert, und eine erhöhte Pathogenresistenz, bevorzugt eine erhöhte Pilzpathogenresistenz, vermittelt, in die Pflanze bzw. Pflanzenzelle eingeführt und dort exprimiert wird.

In einer weiteren Ausführungsform betrifft die Erfindung ein Verfahren zur Erhöhung der Resistenz gegenüber einem oder mehreren penetrierenden Pathogen(en) in einer monokotyledonen oder dikotyledonen Pflanze, oder einem Teil einer Pflanze, z.B. in einem Organ, Gewebe, einer Zelle oder einem Teil einer pflanzlichen Zelle, z.B. in einem Organell, dadurch gekennzeichnet, dass eine endogene DNA-Sequenz, die für ein ein Armadillo Repeat ARM1 Protein kodiert, und eine erhöhte Pathogenresistenz, bevorzugt eine erhöhte Pilzpathogenresistenz, vermittelt, in der Pflanze, Pflanzenteil bzw. Pflanzenzelle gegenüber der Ausgangs- bzw. Wildtyppflanze, bzw. deren Teil erhöht wird, oder dass die endogene Gensequenz oder bevorzugt die 5'-untranslatierte Region (5'UTR) gegenüber der Ausgangssequenz verändert wird.

Ursprünglich wurde im Rahmen einer TIGS (=Transient Induced Gene Silencing)-Analyse in Gerste nach der Methode von Schweizer et al. (2001) gefunden, dass durch ein dsRNAivermitteltes Silencing eines Gens codierend für ein Armadillo-Repeat die Empfänglichkeit der Pflanze für das Pilzpathogen Blumeria graminis herabgesetzt ist und daher das Gen eine Rolle bei der Vermittlung der Pathogenresistenz von Pflanzen spielen könnte.

In diesem Zusammenhang muss jedoch angemerkt werden, dass es aufgrund der hohen Zahl konservierter Reste und der Homologie zwischen den einzelnen Armadillo-Repeat ARM1 Polypeptiden und ihren funktionellen Äquivalenten möglich ist, mit einer einzigen dsRNA-Sequenz, die ausgehend von einer bestimmten Armadillo-Repeat ARM1 Proteinsequenz eines Organismus generiert wurde, die Expression weiterer homologer Polypeptide und/oder deren funktioneller Äquivalente des gleichen Organismus zu unterdrücken.

Überraschenderweise wurde jedoch nun festgestellt, dass das Einführen des Gens aus Gerste bzw. dessen Expression oder Überexpression in von Gerste verschiedenen Pflanzen, bevorzugt monokotyle Pflanzen, insbesondere Weizen ebenfalls eine Erhöhung der Resistenz hervorrufen kann.

Vorzugsweise wird in dem erfindungsgemäßen Verfahren eine rasseunspezifische Resistenz erreicht. So kann z.B. durch das erfindungsgemäße Verfahren eine Breitspektrumresistenz gegen obligat biotrophe und/oder hemibiotrohpe und/oder nekrotrophe Pilze von Pflanzen, insbesondere gegen Mesophyll und/ oder Epidermis-penetrierende Pathogene erreicht werden.

Das Armadillo Repeat-Motiv war ursprünglich in dem Segment-Polaritäts-Gen Armadillo aus Drosophila melanogaster entdeckt worden. Es kodiert für ein beta-Catenin, welches in der Zell-Zell-Adhäsion sowie für die Zelldifferenzierung eine wichtige Rolle spielt. Armadillo (Arm) Repeat Proteine enthalten tandemartig angeordnete Kopien einer degenerierten Sequenz von ca. 42 Aminosäuren, welche eine drei-dimensionale Struktur zur Vermittlung von Protein-Protein-Interaktionen kodiert (Azevedo et al., Trends Plant Sci. 6, 354 (2001)). Die meisten dieser Proteine sind in die intrazelluläre Signaltransduktion oder in die Regulation der Genexpression im Rahmen zellulärer Entwicklungsprozesse involviert. Im Gegensatz zu Tieren sind erst zwei pflanzliche Armadillo-Repeat-Proteine funktionell charakterisiert worden: Bei einem Gen handelt es sich um PHOR1 (photoperiod-responsive 1) aus Kartoffel, für welches eine Rolle in der Gibberellinsäure-Signaltransduktion demonstriert werden konnte (Amador V., Cell 106 (3), 343 (2001)) Bei dem zweiten Armadillo-Repeat Protein handelt es sich um ARC1 (Armadillo-Repeat Containing Protein 1) aus Raps, das mit der Rezeptor-Kinase SRK1 interagiert (Gu et al., Proc. Natl. Acad. Sci. USA 95, 382 (1998)). Damit spielt es bei der Regulation der Selbstinkompatibilität von Raps eine große Rolle. Transgene Pflanzen, in denen die Expression von ARC1 durch Silencing herabgesetzt ist, zeigen eine reduzierte Selbstinkomptibilität. Interessanterweise zählt ARC1 zu der U-Box enthaltenden Subklasse der Armadillo-Repeat Proteine, zu der in Arabidopsis 18 Gene zählen (Azevedo et al., Trends Plant Sci. 6, 354 (2001)). Die U-Box ist ein Motiv bestehend aus ca. 70 Aminosäure-Resten. Neben den HECT- und den RING-Finger-Proteinen bilden sie vermutlich eine dritte Klasse an Ubiquitin E3-Ligasen, deren primäre Funktion darin besteht, die Substratspezifität der Ubiquitinierungsmaschinerie festzulegen (Hatakeyama et al., J. Biol. Chem. 76, 33111 (2001)).

Gene mit einer hohen Homologie zu HvArm vermitteln vermutlich ähnliche Funktionen. Vorzugsweise haben die Gene oder die verwendeten Nukleinsäuren oder die exprimierten Proteine eine Identität von 40% oder mehr, vorzugweise 50%, 60%, 70%, 80%, 85%, 90%, 95%, 97%,98%, 99% oder mehr verglichen mit der jeweiligen Sequenz von HvARM (SEQ ID NO: 1 oder SEQ ID NO: 63 [cDNA-Sequenz mit UTR] bzw. der Proteinsequenz SEQ ID NO: 2). Die Gene mit den höchsten Homolgien zu HvArm aus Reis (Acc. Nr.: XM_479734.1, XM_463544, AP003561, oder XM_506432), Tabak (AY219234) und Arabidopsis (Acc.-No. NM_127878, AC004401, BT020206, AB007645, NM_115336, AK118613, AL138650, AL133314, AC010870, AY125543, AY087360, AB016888, AK175585, AL049655, AY096530 und AK118730) nehmen somit vermutlich ähnliche Funktionen wie HvARM in der Pflanze wahr. Im folgenden werden die homologen Sequenzen mit dem Begriff "Armadillo Repeat ARM1" oder "ARM1" benannt. HvARM bzw. HvARM1 beziehen sich hingegen auf eine solche Sequenz aus Gerste usw.

In der Beschreibung wird aus Gründen der Vereinfachung der Begriff der "erfindungsgemäßen Sequenz(en)" verwendet, was sich je nach Zusammenhang auf die hierin offenbarten Nukleinsäure- und/oder Aminosäuresequenzen bezieht. Dem Fachmann wird aus dem Zusammenhang der Bezug ersichtlich sein.

Kürzlich wurde in Mais mit Spl11 ein weiteres pflanzliches Armadillo-Repeat Protein beschrieben, für das eine Regulation der pflanzlichen Zelltodantwort im Rahmen der abiotischen Stressantwort nachgewiesen wurde. Der Funktionsverlust des entsprechenden Gens führt zu einem sog. Lesion Mimic-Phänotyp, der die agronomische Leistungsfähigkeit der Pflanze beeinträchtigt (Zeng L.R., Plant Cell. 16 (10), 2795 (2004)). Interessanterweise beträgt die Sequenzhomologie von Spl11 zu HvARM lediglich 23,4% auf Aminosäure-Ebene. Ohne durch Theorie eine Festlegung oder Einschränkung herbeizuführen deutet neben den unterschiedlichen Funktionen auch die geringe Sequenzhomologie auf die Zugehörigkeit von HvARM und Spl11 zu verschiedenen Subklassen von Armadillo-Repeat Proteinen hin.

Es war folglich überraschend, dass das Einführen und Exprimieren von erfindungsgemäßen HvArm-Sequenzen zu einer Erhöhung der Resistenz von Weizen gegen Weizenmehltau führt. In einer bevorzugten Ausführungsform besitzt das erfindungsgemäße Polypeptid, das von einer erfindungsgemäßen Sequenz kodiert wird, keine U box im 5'-UTR.

In einer weitere Ausführungsform betrifft die Erfindung folglich ein Verfahren zur Herstellung einer Pflanze mit erhöhter Resistenz gegen ein oder mehrere pflanzliche Pathogen(e), vorzugsweise mit einer Breitspektrumresistenz, insbesondere gegen Pilzpathogene, beispielsweise aus der Klasse der Ascomycetes, Basidiomycetes, Chytridiomycetes oder Oomycetes, bevorzugt von Mehltaupilzen der Familie Erysiphaceae, und besonders bevorzugt der Gattung Blumeria, und zwar durch Einführen und Expression einer erfindungsgemäßen Sequenz, die dadurch charakterisiert ist, dass sie für ein Protein kodiert, das mindestens ein Armadillo-Repeat enthält. Vorzugsweise enthält das Protein zwei, besonders bevorzugt mehr als zwei Armadillo-Repeats.

In einer weiteren Ausführungsform umfasst die das Protein codierende cDNA (bzw. die mRNA einschließlich der UTR-Sequenz(en)) im Wesentlichen keine U-Box, d.h. entweder keine U-Box oder keine funktionelle U-Box.

Die erfindungsgemäße Nukleinsäuresequenz, d.h. jene die für ein ein Armadillo Repeat ARM1 Protein kodiert, die eine erhöhte Pathogenresistenz, bevorzugt eine erhöhte Pilzpathogenresistenz, vermittelt, und die in den erfindungsgemäßen Verfahren in die Pflanze bzw. Pflanzenzelle bzw. einen Teil von dieser eingeführt und dort exprimiert wird, oder die ebensolche endogene DNA-Sequenz, die in der Pflanze bzw. Pflanzenzelle gegenüber der Ausgangs- oder Wildtyppflanze bzw. deren Teil erhöht wird, oder wobei die endogene Gensequenz oder bevorzugt die 5'-untranslatierte Region (5'UTR) gegenüber der Ausgangssequenz verändert wird, ist ausgewählt aus der Gruppe bestehend aus:
(a) Nukleinsäuremolekül, das mindestens ein Polypeptid kodiert, umfassend die in SEQ ID No: 2, 4, 6, 8, 10, 12, 14, 16, 18, 20, 22, 24, 26, 28, 30, 32, 34, 36, 38, 40, 42, 44, 60, 61 oder 62 gezeigte Sequenz;
(b) Nukleinsäuremolekül, das zumindest ein Polynukleotid der Sequenz gezeigt in SEQ ID No: 1, 3, 5, 7, 9, 11, 13, 15, 17, 19, 21, 23, 25, 27, 29, 31, 33, 35, 37, 39, 41, 43 oder 63 umfasst;
(c) Nukleinsäuremolekül, das ein Polypeptid kodiert, dessen Sequenz eine Identität von mindestens 50%, vorzugweise mindestens 60%, 70%, 80%, 85%, 90%, 95%, 97% ,98%, oder 99%, zu den Sequenzen SEQ ID No: 2 aufweist;
(d) Nukleinsäuremolekül nach (a) bis (c), das für ein Fragment oder ein Epitop der Sequenzen gemäß SEQ. ID No.: 2, 4, 6, 8, 10, 12, 14, 16, 18, 20, 22, 24, 26, 28, 30, 32, 34, 36, 38, 40, 42, 44, 60, 61 oder 62 kodiert;
(e) Nukleinsäuremolekül, das ein Polypeptid kodiert, welches von einem monoklonalen Antikörper, gerichtet gegen ein Polypeptid welches durch die Nukleinsäuremoleküle gemäß (a) bis (c) kodiert wird, erkannt wird;
(f) Nukleinsäuremolekül, das unter stringenten Bedingungen mit einem Nukleinsäuremolekül gemäß (a) bis (c) hybridisiert; und
(g) Nukleinsäuremolekül, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann;
oder eine komplementäre Sequenz davon umfasst.

Vorzugsweise wird in dem erfindungsgemäßen Verfahren insbesondere die Resistenz gegen Mesophyll- und/ oder Epidermiszellen penetrierende Pathogene erhöht.

In einer Ausführungsform wird die Resistenz dadurch erreicht, dass eine erfindungsgemäße Nukleinsäuresequenz, zum Beispiel die eines ARM1 aus Reis (Acc. Nr.: XM_479734.1, XM_463544, AP003561, oder XM_506432), Tabak (AY219234) und Arabidopsis (Acc.-No. NM_127878, AC004401, BT020206, AB007645, NM_115336, AK118613, AL138650, AL133314, AC010870, AY125543, AY087360, AB016888, AK175585, AL049655, AY096530 und AK118730) eingeführt und exprimiert wird.

Anderseits kann durch dem Fachmann bekannte Verfahren auch die endogene Expression oder Aktivität einer dieser Sequenzen erhöht werden, z.B. durch Mutation einer UTR-Region, bevorzugt der 5'-UTR, einer Promotorregion, einer genomischen codierenden Region für das aktive Zentrum, für Bindungsstellen, für Lokalisationssignalen, für Domainen, Cluster, etc. wie z.B. von codierenden Regionen für coiled coil, HEAT, FBOX, LRR, IBIB, C2, WD40, Beach, U-Box, oder UND-Domänen. Die Aktivität kann erfindungsgemäß erhöht werden durch Mutationen, die die Sekundär-, Tertiär-, oder Quartärstruktur des Proteins beinflussen.

Mutationen können z.B. durch eine EMS-Mutagenese eingeführt werden. Domänen können durch geeignete Computerprogramme identifiziert werden, wie z.B. SMART, oder InterPRO, z.B. wie in Andersen P., The Journal of Biol. Chemistry, 279 (38) 40053 (2004) oder Mudgil Y., Plant Physiology, 134, 59 (2004), und darin genannten Schriften beschrieben. Die geeigneten Mutanten können dann z.B. durch Tilling (bspw. nach Henikoff et al., Plant Physiol. 135 (2), 630 (2004)) identifiziert werden.

In einer anderen Ausführungsform wird die Einführung und Expression einer erfindungsgemäßen Sequenz in eine Pflanze bzw. das Erhöhen oder Verändern bzw. Mutieren einer eindungsgemäßen endogenen Sequenz, ggf. eines oder beider untranslatierten Bereiche, in einer Pflanze kombiniert mit einer Erhöhung der Polypeptidmenge, Aktivität oder Funktion anderer Resistenzfaktoren, bevorzugt eines Bax Inhibitor 1-Proteins (BI-1), bevorzugt des Bax Inhibitor 1-Proteins aus Hordeum vulgare (GenBank Acc.-No.: AJ290421), aus Nicotiana tabacum (GenBank Acc.-No.: AF390556), Reis (GenBank Acc.-No.: AB025926), Arabidopsis (GenBank Acc.-No.: AB025927) bzw. Tabak und Raps (GenBank Acc.-No.: AF390555, Bolduc N. et al., Planta 216, 377 (2003)) oder von ROR2 (z.B. aus Gerste (GenBank Acc.-No.: AY246906), SnAP34 (z.B. aus Gerste (GenBank Acc.-No.: AY247208) und/oder des Lumenal Binding Protein BiP z.B. aus Reis (GenBank Acc.-No. AF006825). Eine Erhöhung kann z.B. u.a. durch Mutagenese oder Überexpression eines Transgens erreicht werden.

In einer weiteren Ausführungsform wird eine Verminderung der Proteinmenge oder Aktivität oder Funktion der Proteine RacB (z.B. aus Gerste (GenBank Acc.-No.: AJ344223)), CSL1 (z.B. aus Arabidopsis (GenBank Acc.-No.: NM116593), HvNaOX (z.B. aus Gerste (GenBank Acc.-No.: AJ251717), MLO (z.B. aus Gerste (GenBank Acc.-No. Z83834) mit den erfindungsgemäßen Verfahren kombiniert.

Die Aktivität oder Funktion von MLO, BI-1 und/oder NaOX kann analog wie für MLO in WO 98/04586; WO 00/01722; WO 99/47552 und den weiteren unten genannten Schriften beschrieben reduziert oder inhibiert werden, deren Inhalt hiermit ausdrücklich und expressis verbis mit aufgenommen gilt, insbesondere um die Aktivität und Inhibierung von MLO zu beschreiben. Die Beschreibung der genannten Schriften beschreibt Verfahren, Methoden und besonders bevorzugte Ausführungsformen zur Verminderung oder Inhibierung der Aktivität oder Funktion von MLO, die Beispiele geben konkret an, wie dies ausgeführt werden kann.

Die Reduzierung der Aktivität oder Funktion und ggf. der Expression von BI-1 ist in der WO 2003/020939 ausführlich beschrieben, die hiermit ausdrücklich und expressis verbis mit als in die vorliegenden Beschreibung aufgenommen gilt. Die Beschreibung der genannten Schrift beschreibt Verfahren und Methoden zur Verminderung oder Inhibierung der Aktivität oder Funktion von BI-1, die Beispiele geben konkret an, wie dies ausgeführt werden kann. Besonders bevorzugt wird die Reduktion oder Inhibierung der Aktivität oder Funktion von BI-1 gemäß der in der WO 2003/020939 besonders bevorzugten Ausführungsformen und der Beispiele und in den dort als besonders bevorzugt dargestellten Organismen durchgeführt, insbesondere in einer Pflanze, z.B. konstitutiv, oder in einem Teil davon, z.B. in einem Gewebe, besonders jedoch mindestens in der Epidermis oder einem wesentlichen Teil der Epidermiszellen. Die Reduzierung der Aktivität oder Funktion und ggf. der Expression von BI-1 ist in der WO 2003/020939 ausführlich beschrieben. Der Fachmann findet in der WO 2003/020939 die Sequenzen, die für BI-1 Proteine codieren, und kann mit dem in der WO 2003/020939 zur Verfügung gestellten Verfahren auch BI-1 identifizieren.

Die Reduzierung der Aktivität oder Funktion und ggf. der Expression von NaOX ist in der WO 2004/09820 (PCT/EP/03/07589) ausführlich beschrieben, die hiermit ausdrücklich und expressis verbis als in die vorliegenden Beschreibung mit aufgenommen gilt. Die Beschreibung der genannten Schrift beschreibt Verfahren und Methoden zur Erniedrigung oder Inhibierung der Aktivität oder Funktion von NaOX, die Beispiele geben konkret an, wie dies ausgeführt werden kann. Besonders bevorzugt wird die Reduktion oder Inhibierung der Aktivität oder Funktion von NaOX gemäß der in der WO 2004/09820 (PCT/EP/03/07589) besonders bevorzugten Ausführungsformen und der Beispiele und in den dort als besonders bevorzugt dargestellten Organismen durchgeführt insbesondere in einer Pflanze, z.B. konstitutiv, oder einem Teil davon, z.B. in einem Gewebe, besonders vorteilhaft jedoch mindestens in der Epidermis oder einem wesentlichen Teil der Epidermiszellen. Der Fachmann findet in der WO 2004/09820 (PCT/EP/03/07589) die Sequenzen, die für NaOX Proteine codieren und kann mit dem in der WO 2004/09820 (PCT/EP/03/07589) zur Verfügung gestellten Verfahren auch NaOX identifizieren.

Die Begriffe "vermindern", "reduzieren" oder "repremieren" oder deren Substantive werden hierein synonym verwendet.

In einer weiteren Ausführungsform erfolgt die Erhöhung der Polypeptidmenge, Aktivität oder Funktion des Bax Inhibitor 1-Proteins aus Hordeum vulgare (GenBank Acc.-No.: AJ290421), aus Nicotiana tabacum (GenBank Acc.-No.: AF390556), Reis (GenBank Acc.-No.: AB025926), Arabidopsis (GenBank Acc.-No.: AB025927) bzw. Tabak und Raps (GenBank Acc.-No.: AF390555, Bolduc N. et al., Planta 216, 377 (2003)) oder von ROR2 (z.B. aus Gerste (GenBank Acc.-No.: AY246906), SnAP34 (z.B. aus Gerste (GenBank Acc.-No.: AY247208) und/oder des Lumenal Binding Protein BiP z.B. aus Reis (GenBank Acc.-No. AF006825) kombiniert mit der Verminderung der Proteinmenge oder Aktivität oder Funktion der Proteine RacB (z.B. aus Gerste (GenBank Acc.-No.: AJ344223), CSL1 (z.B. aus Arabidopsis (GenBank Acc.-No.: NM116593), HvNaOx (z.B. aus Gerste (GenBank Acc.-No.: AJ251717), und/oder MLO (z.B. aus Gerste (GenBank Acc.-No. Z83834). Folglich wird in einer Ausführungsform mindestens eins der oben genannten zur Überexpression oder erhöhten Aktivität geeigneten Gene aktiviert oder überexprimiert und/oder mindestens eins der oben genannten zur Verminderung geeigneten Gene vermindert.

Eine Erhöhung der Expression kann wie hierin beschrieben erreicht werden. Unter Erhöhung der Expression oder Funktion wird hierein sowohl die Aktivierung oder Steigerung der Expression oder Funktion des endogenen Proteins einschließlich einer de novo Expression als auch eine Erhöhung oder Steigerung durch die Expression eines transgenen Proteins oder Faktors verstanden.

"Organismus" im Rahmen der Erfindung meint "nicht humane Organismen" solange sich der Begriff auf einen lebensfähigen vielzelligen Organismus bezieht, vorzugsweise Pflanzen. "Pflanzen" im Rahmen der Erfindung meint alle dicotyledonen oder monokyledonen Pflanzen. Bevorzugt sind Pflanzen die unter der Klasse der Liliatae (Monocotyledoneae bzw. einkeimblättrige Pflanzen) subsumiert werden können. Eingeschlossen unter dem Begriff sind die reifen Pflanzen, Saatgut, Sprosse und Keimlinge, sowie davon abgeleitete Teile, Vermehrungsgut, Pflanzenorgane, Gewebe, Protoplasten, Kallus und andere Kulturen, zum Beispiel Zellkulturen, sowie alle anderen Arten von Gruppierungen von Pflanzenzellen zu funktionellen oder strukturellen Einheiten. Reife Pflanzen meint Pflanzen zu jedem beliebigen Entwicklungsstadium jenseits des Keimlings. Keimling meint eine junge, unreife Pflanze in einem frühen Entwicklungsstadium.

Ebenfalls bevorzugt sind zweikeimblättrige Pflanzen. Eingeschlossen unter dem Begriff sind die reifen Pflanzen, Saatgut, Sprosse und Keimlinge, sowie davon abgeleitete Teile, Vermehrungsgut, Pflanzenorgane, Gewebe, Protoplasten, Kallus und andere Kulturen, zum Beispiel Zellkulturen, sowie alle anderen Arten von Gruppierungen von Pflanzenzellen zu funktionellen oder strukturellen Einheiten. Reife Pflanzen meint Pflanzen zu jedem beliebigen Entwicklungsstadium jenseits des Keimlings. Keimling meint eine junge, unreife Pflanze in einem frühen Entwicklungsstadium.

"Pflanze" umfasst auch einjährige und mehrjährige dicotyledone oder monokotyledone Pflanzen und schließt, beispielhaft, jedoch nicht einschränkend, solche der Gattungen, Bromus, Asparagus, Pennisetum, Lolium, Oryza, Zea, Avena, Hordeum, Secale, Triticum, Sorghum und Saccharum ein.

In einer bevorzugten Ausführungsform wird das Verfahren auf monokotyle Pflanzen, beispielsweise aus der Familie der Poaceae, besonders bevorzugt auf die Gattungen Oryza, Zea, Avena, Hordeum, Secale, Triticum, Sorghum, und Saccharum, ganz besonders bevorzugt auf Pflanzen mit landwirtschaftlicher Bedeutung, wie z.B. Hordeum vulgare (Gerste), Triticum aestivum (Weizen), Triticum aestivum subsp.spelta (Dinkel), Triticale, Avena sative (Hafer), Secale cereale (Roggen), Sorghum bicolor (Hirse), Zea mays (Mais), Saccharum officinarum (Zuckerrohr) oder Oryza sative (Reis) angewendet.

"Epidermisgewebe" oder Epidermis meint die äußeren Gewebeschichten der Pflanzen. Sie kann ein- bis mehrschichtig sein; es gibt Epidermis-"angereicherte" Genexpression, wie z.B. Cer3, die als Marker dienen kann (Hannoufa A. Plant J. 10 (3), 459 (1996)).

Unter "Epidermis" versteht der Fachmann vorzugsweise das vorherrschende Abschlussgewebe primärer oberirdischer Pflanzenteile, so des Sprosses, der Blätter, Blüten, Früchte und Samen. Nach außen scheiden die Epidermiszellen eine wasserabstoßende Schicht, die Kutikula ab. Die Wurzeln sind von der Rhizodermis umgeben, welche der Epidermis in vieler Hinsicht ähnelt, jedoch auch markante Unterschiede zu ihr aufweist. Die Epidermis entsteht aus der äußersten Schicht des Apikalmeristems. Die Ableitung der Rhizodermis hingegen ist weniger klar. Je nach Art kann sie entwicklungsgeschichtlich entweder der Wurzelhaube oder der primären Rinde zugerechnet werden. Der Epidermis können zahlreiche Funktionen zugeschrieben werden: Sie bietet der Pflanze Schutz vor Austrocknung und regelt die Transpirationsrate Sie schützt die Pflanze vor den verschiedensten chemischen und physikalischen Fremdeinflüssen sowie vor Tierfraß und Befall durch Parasiten. Sie ist am Gasaustausch, an der Sekretion bestimmter Stoffwechselprodukte und an der Absorption von Wasser beteiligt. In ihr sind Rezeptoren für Licht und mechanische Reize enthalten. Sie wirkt damit als ein Signalwandler zwischen Umwelt und Pflanze. Entsprechend den verschiedenen Funktionen enthält die Epidermis eine Anzahl unterschiedlich differenzierter Zellen. Hinzu kommen artspezifische Varianten und unterschiedliche Organisation der Epidermen in den einzelnen Teilen einer Pflanze. Im Wesentlichen besteht sie aus drei Kategorien von Zellen: den "eigentlichen" Epidermiszellen, den Zellen der Stomata (Spaltöffnungen) und den Trichomen (griech.: Trichoma, Haar), epidermalen Anhangsgebilden verschiedener Form, Struktur und Funktion.

Die "eigentlichen", d.h., die am wenigsten spezialisierten Epidermiszellen machen die Hauptmasse der Zellen des Abschlußgewebes aus. Sie sind in der Aufsicht entweder polygonal (von platten- oder tafelförmiger Gestalt) oder gestreckt. Die zwischen ihnen ausgebildeten Wände sind vielfach gewellt oder gebuchtet. Wodurch diese Form während der Entwicklung induziert wird, ist unbekannt, die vorliegenden Hypothesen erklären den Sachverhalt nur unbefriedigend. Gestreckte Epidermiszellen findet man an Organen oder Organteilen, die selbst gestreckt sind, so z.B. an Stengeln, Blattstielen und Blattrippen sowie an den Blättern der meisten Monokotyledonen. Ober- und Unterseite von Blattspreiten können von unterschiedlich strukturierten Epidermen bedeckt sein wobei sowohl die Form der Zellen, die Dicke der Wände als auch die Verteilung und Zahl spezialisierter Zellen (Stomata und/oder Trichome) pro Flächeneinheit variieren kann. Große Variationen findet man auch innerhalb einzelner Familien, z. B. bei den Crassulaceen. Meist ist die Epidermis einschichtig, jedoch sind bei Arten aus mehreren Familien (Moraceae: hier die meisten Ficus-Arten, Piperaceae: Peperonia [Peperonie], Begoniaceae, Malvaceae u.a.) mehrschichtige wasserspeichernde Epidermen nachgewiesen worden. Epidermiszellen sondern nach außen eine Cutinschicht (Kutikula) ab, die als ein ununterbrochener Film alle epidermalen Oberflächen überzieht. Sie kann entweder glatt oder durch Vorwölbungen, Leisten, Falten und Furchen strukturiert sein. Doch nicht immer beruht eine durch Betrachtung der Oberfläche sichtbare Faltung der Kutikula auf der Ausbildung von Kutikularleisten. Es gibt durchaus Fälle, wo eine Kutikulafaltung nur der Ausdruck der darunterliegenden Ausstülpungen der Zellwand ist. Epidermale Anhangsgebilde verschiedener Form, Struktur und Funktion werden als Trichome bezeichnet und hierin ebenfalls unter dem Begriff "Epidermis" verstanden. Sie treten als Schutz-, Stütz- und Drüsenhaare in Form von Schuppen, verschiedenen Papillen und bei Wurzeln als absorbierende Haare auf. An ihrer Bildung sind allein Epidermiszellen beteiligt. Oft entsteht ein Trichom aus nur einer solchen Zelle, manchmal sind an der Entstehung mehrere beteiligt.

Ebenfalls umfasst unter dem Begriff "Epidermis" sind Papillen. Papillen sind Ausstülpungen der Epidermisoberfläche. Das Lehrbuchbeispiel hierfür sind die Papillen auf Blütenoberflächen des Stiefmütterchens (Viola tricolor) sowie die Blattoberflächen vieler Arten im tropischen Regenwald. Sie verleihen der Oberfläche eine samtartige Konsistenz. Einige Zellen von Epidermen können als Wasserspeicher ausgebildet sein. Ein typisches Beispiel stellen die Blasenzellen an Oberflächen vieler Mittagsblumenarten und anderer Sukkulenten dar. Bei manchen Pflanzen, z.B. bei der Glockenblume (Campanula persicifolia) sind die Außenwände der Epidermis linsenförmig verdickt.

Die Hauptmasse aller Gewebe bildet das Grundgewebe oder Parenchym. Zu den parenchymatischen Geweben gehört das Mesophyll, das in Blättern in Palisadenparenchym und Schwammparenchym differenziert sein kann. Folglich versteht der Fachmann unter Mesophyll ein parenchymatisches Gewebe. Parenchymatische Zellen sind durchweg lebend, meist isodiametrisch, seltener gestreckt. Das Mark der Sprosse, die Speichergewebe der Früchte, Samen, der Wurzel und anderer unterirdischer Organe sind ebenso als Parenchyme zu betrachten wie das Mesophyll. "Mesophyllgewebe" meint das zwischen den Epidermisschichten liegende Blattgewebe, bestehend aus dem Palisadengewebe, dem Schwammgewebe und den Blattadern.

Das Mesophyll ist in den Blättern der meisten Farne und Phanerogamen, besonders ausgeprägt bei den Dikotyledonen und vielen Monokotyledonen, in Palisaden- und Schwammparenchym untergliedert. Ein "typisches" Blatt ist dorsiventral gebaut. Das Palisadenparenchym liegt dabei meist an der Blattoberseite unmittelbar unter der Epidermis. Das Schwammparenchym füllt den darunterliegenden Raum aus. Es ist von einem voluminösen Interzellularsystem durchsetzt, dessen Gasraum über die Spaltöffnungen in direktem Kontakt zur Außenwelt steht.

Das Palisadenparenchym besteht aus langgestreckten, zylindrischen Zellen. Bei einigen Arten sind die Zellen irregulär, gelegentlich sind sie gegabelt (Y-förmig: Armpalisadenparenchym). Solche Varianten kommen bei Farnen, Coniferen und einigen wenigen Angiospermen (z.B. bei einigen Ranunculaceen- und Caprifoliaceenarten [Beispiel: Holunder]) vor. Neben der eben beschriebenen, am weitesten verbreiteten Organisationsform sind die folgenden Varianten nachgewiesen worden: Palisadenparenchym an der Blattunterseite. Besonders auffällig bei Schuppenblättern. Beispiel: Lebensbaum (Thuja), sowie bei den Blättern des Bärlauchs (Allium ursinum)

Palisadenparenchym an beiden Blattseiten (Ober- und Unterseite) wird häufig bei Pflanzen trockener Standorte (Xerophyten) angetroffen, z.B. bei der Kompaßpflanze (Lactuca serriola).

Ringförmig geschlossenes Palisadenparenchym findet sich beispielsweise in zylindrisch organisierten Blättern und in Nadeln der Koniferen.

Die Variabilität der Schwammparenchymzellen und die Ausbildung des Schwammparenchyms selbst sind noch vielgestaltiger als die des Palisadenparenchyms. Es wird meist als Durchlüftungsgewebe bezeichnet, denn es enthält eine Vielzahl untereinander verbundener Interzellularen.

Das Mesophyll kann das so genannte Assimilationsgewebe umfassen, jedoch sind die Begriffe Mesophyll und Assimilationsgewebe nicht als Synonyme zu verwenden. Es gibt chloroplastenfreie Blätter, die sich in ihrem Aufbau nur unwesentlich von vergleichbaren, grünen Blättern unterscheiden. Folglich enthalten sie Mesophyll, doch eine Assimilation unterbleibt; umgekehrt findet eine Assimilation z.B. auch in Sproßabschnitten statt. Weitere Hilfsmittel zur Charakterisierung von Epidermis und Mesophyll findet der Fachmann z.B. in v. Guttenberg H. "Lehrbuch der Allgemeinen Botanik", Berlin, Akademie-Verlag 1955 (5. Aufl.); Haberlandt G., "Physiologische Pflanzenanatomie", Leipzig, W. Engelmann 1924 (6. Aufl.); Troll W. "Morphologie der Pflanzen", Band 1: Vegetationsorgane, Berlin, Gebr. Borntraeger, 1937; Troll W., "Praktische Einführung in die Pflanzenmorphologie", Jena, VEB G. Thieme Verlag 1954/1957; Troll W., Höhn K., "Allgemeine Botanik", Stuttgart, F. Enke Verlag, 1973 (4. Aufl.).

Folglich können Epidermis oder Epidmermiszellen histologisch oder biochemisch, einschließlich molekularbiologisch, charakterisiert werden. In einer Ausführungsform wird die Epidermis biochemisch charakterisiert. Die Epidermis kann in einer Ausführungsform durch die Aktivität eines oder mehrer der folgenden Promotoren gekennzeichnet werden:
WIR5 (=GstA1), acc. X56012, Dudler & Schweizer, unveröff.
GLP4, acc. AJ310534; Wei,Y., Plant Molecular Biology 36, 101 (1998).
GLP2a, acc. AJ237942, Schweizer P., Plant J. 20, 541 (1999).
Prx7, acc. AJ003141, Kristensen B.K., Molecular Plant Pathology 2 (6), 311 (2001).
GerA, acc. AF250933 ; Wu S., Plant Phys. Biochem. 38, 685 (2000).
OsROC1, acc. AP004656
RTBV, acc. AAV62708, AAV62707 ; Klöti A., PMB 40, 249 (1999).
Cer3; Hannoufa A., Plant J. 10 (3), 459 (1996).

In einer anderen Ausführungsform wird die Epidermis dadurch gekennzeichnet, dass nur ein Teil der Promotoren aktiv ist, z.B. 2, 3, 5 oder 7 oder mehr, mindestens jedoch ist einer der oben aufgezählten aktiv. In einer Ausführungsform wird die Epidermis dadurch gekennzeichnet, dass alle genannten Promoter in dem Gewebe oder der Zelle aktiv sind.

Folglich können Mesophyll oder Mesophyllzellen biochemisch einschließlich molekularbiologisch oder histologisch charakterisiert werden. In einer Ausführungsform wird das Mesophyll biochemisch charakterisiert. Das Mesophyll kann in einer Ausführungsform durch die Aktivität eines oder mehrer der folgenden Promotoren gekennzeichnet werden:
PPCZm1 (=PEPC); Kausch A.P., Plant Mol. Biol. 45, 1 (2001).
OsrbcS, Kyozuka J. et al., Plant Phys. 102 (3), 991 (1993).
OsPPDK, acc. AC099041.
TaGF-2.8, acc. M63223; Schweizer P., Plant J. 20, 541 (1999).
TaFBPase, acc. X53957.
TaWIS1, acc. AF467542; US 200220115849.
HvBIS1, acc. AF467539; US 200220115849.
ZmMIS1, acc. AF467514; US 200220115849.
HvPR1a, acc. X74939 ; Bryngelsson et al., Mol. Plant Microbe Interact. 7 (2), 267 (1994).
HvPR1b, acc. X74940; Bryngelsson et al. Mol. Plant Microbe Interact. 7 (2),267 (1994). HvB1,3gluc; acc. AF479647.
HvPrx8, acc. AJ276227; Kristensen B.K., Molecular Plant Pathology 2 (6), 311 (2001 ).
HvPAL, acc. X97313 ; Wei,Y. Plant Molecular Biology 36, 101 (1998).

In einer anderen Ausführungsform wird das Mesophyll dadurch gekennzeichnet, dass nur ein Teil der Promotoren aktiv ist, z.B. 2, 3, 5 oder 7 oder mehr, mindestens jedoch einer der oben aufgezählten. In einer Ausführungsform wird das Mesophyll dadurch gekennzeichnet, dass alle genannten Promotoren in dem Gewebe oder der Zelle aktiv sind.

In einer Ausführungsform sind in der Epidermis einer erfindungsgemäß verwendeten oder hergestellten Pflanze oder einer erfindungsgemäßen Pflanze in der Epidermis und im Mesophyll alle genannten Promotoren aktiv. In einer Ausführungsform sind nur ein Teil der genannten Promotoren aktiv, z.B. 2, 5, 7 oder mehr, mindestens ist jedoch einer der oben aufgezählten Promotoren jeweils aktiv.

"Nukleinsäuren" meint Biopolymere aus Nukleotiden, die über Phosphodiesterbindungen miteinander verknüpft sind (Polynukleotide, Polynukleinsäuren). Je nach dem Zuckertyp in den Nukleotiden (Ribose oder Desoxyribose), unterscheidet man zwischen den beiden Klassen der Ribonukleinsäuren (RNA) und den Desoxyribonukleinsäuren (DNA).

Der Begriff "Ernte" meint alle durch landwirtschaftlichen Anbau von Pflanzen gewonnenen und im Rahmen des Erntevorgangs gesammelten Pflanzenteile.

"Resistenz" bedeutet das Verhindern, das Reprimieren, das Vermindern oder Abschwächen von Krankheitssymptomen einer Pflanze, die infolge eines Befalls durch ein Pathogen auftreten. Die Symptome können vielfältiger Art sein, umfassen aber bevorzugt solche, die direkt oder indirekt zu einer Beeinträchtigung der Qualität der Pflanze, der Quantität des Ertrages, der Eignung zur Verwendung als Futter- oder Nahrungsmittel führen oder aber auch Aussaat, Anbau, Ernte oder Prozessierung des Erntegutes erschweren.

In einer bevorzugten Ausführungsform werden die folgenden Krankheitssymptome abgeschwächt, vermindert oder verhindert: Bildung von Pusteln und Sporienlagern auf den Oberflächen der befallenen Gewebe, Mazeration der Gewebe, spreitende Nekrosen des Gewebes, Akkumulation von Mykotoxinen z.B. aus Fusarium graminearum oder F. culmorum, Penetration der Epidermis und/oder des Mesophylls, usw.

Eine "erhöhte Pathogenresistenz" bedeutet, dass die Abwehrmechanismen einer bestimmten Pflanze oder in einem Teil einer Pflanze, z.B. in einem Organ, einem Gewebe, einer Zelle oder einem Organell, durch Anwendung des erfindungsgemäßen Verfahrens im Vergleich zu einer geeigneten Kontrolle, z.B. dem Wildtyp der Pflanze ("Kontrollpflanze", "Ausgangspflanze"), auf den das erfindungsgemäße Verfahren nicht angewendet wurde, unter ansonsten gleichen Bedingungen (wie beispielsweise Klima- oder Anbaubedingungen, Pathogenart etc.) eine erhöhte Resistenz gegen ein oder mehr Pathogene aufweist. Vorzugsweise weisen in einer Pflanze mindestens die Epidermis und/oder Mesophyll-Gewebe oder die Organe, die ein Epidermis und/oder Mesophyll-Gewebe besitzen, eine erhöhte Resistenz gegen die Pathogene auf. Beispielsweise ist die Resistenz in den Blättern erhöht.

In einer Ausführungsform wird die Resistenz in Lemma (Deckspelze), Palea (Vorspelze), und/oder Glume (Antherenanlage) erhöht.

Die erhöhte Resistenz äußert sich bevorzugt in einer verminderten Ausprägung der Krankheitssymptome, wobei die Krankheitssymptome - neben den oben erwähnten Beeinträchtigungen - auch beispielsweise die Penetrationseffizienz eines Pathogens in die Pflanze oder pflanzliche Zelle oder die Proliferationseffizienz des Pathogens in oder auf denselben umfassen. Dabei sind die Krankheitssymptome bevorzugt um mindestens 10 % oder mindestens 20 %, besonders bevorzugt um mindestens 40 % oder 60 %, insbesondere bevorzugt um mindestens 70 % oder 80 %, am meisten bevorzugt um mindestens 90 % oder 95 % gegenüber der Kontrollpflanze vermindert.

Dabei äußert sich die erhöhte Resistenz bevorzugt in einer verminderten Ausprägung der Krankheitssymptome, wobei Krankheitssymptome - neben den oben erwähnten Beeinträchtigungen - auch beispielsweise die Penetrationseffizienz eines Pathogens in die Pflanze oder pflanzliche Zelle oder die Proliferationseffizienz in oder auf denselben umfasst. Veränderungen in der Zellwandstruktur können beispielsweise einen grundlegenden Mechanismus der Pathogenresistenz darstellen, wie z.B. gezeigt in Jacobs A.K. et al., Plant Cell 15 (11), 2503 (2003).

"Pathogen" meint im Rahmen der Erfindung Organismen, deren Interaktionen mit einer Pflanze zu den oben beschriebenen Kranheitssymptomen führen, insbesondere meint Pathogene Organismen aus dem Reich der Pilze. Vorzugsweise wird unter Pathogen ein Epidermis- bzw. Mesophyllzellen- penetrierendes Pathogen verstanden, besonders bevorzugt Pathogene, die über Spaltöffnungen in Pflanzen eindringen und anschließend Mesophyllzellen penetrieren. Bevorzugt sind dabei Organismen der Stämme Ascomycota und Basidomycota genannt. Besonders bevorzugt sind dabei die Familien Blumeriaceae, Pucciniaceae, Mycosphaerellaceae und Hypocreaceae.

Besonders bevorzugt sind Organismen dieser Familien, die zu den Gattungen Blumeria, Puccinia, Fusarium oder Mycosphaerella zählen.

Ganz besonders bevorzugt sind die Arten Blumeria graminis, Puccinia triticina, Puccinia striiformis, Mycosphaerella graminicola, Stagonospora nodorum, Fusarium graminearum, Fusarium culmorum, Fusarium avenaceum, Fusarium poae und Microdochium nivale.

Es ist jedoch anzunehmen, dass die erfindungsgemäßen Verfahren auch eine Resistenz gegen weitere Pathogene bewirken.

Besonders bevorzugt sind Ascomycota wie z.B. Fusarium oxysporum (Fusarium-Welke an Tomate), Septoria nodorum und Septoria tritici (Spelzenbräune an Weizen), Basidiomyceten wie z.B. Puccinia graminis (Schwarzrost an Weizen, Gerste, Roggen, Hafer), Puccinia recondita (Braunrost an Weizen), Puccinia dispersa (Braunrost an Roggen), Puccinia hordei (Braunrost an Gerste), Puccinia coronata (Kronenrost an Hafer).

In bevorzugten Ausführungsformen führt das erfindungsgemäße Verfahren zu einer Resistenz bei
- Gerste gegen die Pathogene:
   Puccinia graminis f.sp. hordei (barley stem rust), Blumeria graminis f.sp. hordei (Gerstenmehltau), und/oder
- bei Weizen gegen die Pathogene:
   Blumeria graminis f.sp. tritici (Weizenmehltau), Fusarium graminearum, Fusarium avenaceum, Fusarium culmorum, Puccinia graminis f.sp. tritici, Puccinia recondita f.sp. tritici, Puccinia striiformis, Septoria nodorum, Septoria tritici, Septoria avenae oder Puccinia graminis f.sp. tritici (Wheat stem rust), und/oder
- bei Mais gegen die Pathogene:
   Fusarium moniliforme var. subglutinans, Puccinia sorghi oder Puccinia polysora, und/oder
- bei Sorghum gegen die Pathogene:
   Puccinia purpurea, Fusarium monilifonne, Fusarium graminearum oder Fusarium oxysporum, und/oder
- bei Soja gegen die Pathogene:
   Phakopsora pachyrrhizi und Phakopsora meibomiae.

Der Begriff "Nukleinsäure(molekül)", wie hier verwendet, umfasst in einer bevorzugten Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs.

Besonders bevorzugt sind in der vorliegenden Erfindung zudem isolierte Nukleinsäuresequenzen. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden; hiervon bleiben jedoch die oben genannten Ausführungsformen umfassend 5'- und 3'-UTR-Bereiche unberührt). Bei verschiedenen Ausführungsformen kann das isolierte Molekül z.B. weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt, flankieren. Bei allen hier erwähnten Nukleinsäuremolekülen kann es sich z.B. um RNA, DNA oder cDNA handeln.

Die erfindungsgemäßen Nukleinsäuremoleküle können unter Verwendung molekularbiologischer Standardtechniken und der hier bereit gestellten Sequenzinformation isoliert werden. Auch können mit Hilfe von Vergleichsalgorithmen, wie sie z.B. auf der NCBI-Homepage unter http://www.ncbi.nlm.nih.gov zu finden sind, beispielsweise eine homologe Sequenz oder homologe, konservierte Sequenzbereiche auf DNA- oder Aminosäureebene identifiziert werden. Wesentliche Teile dieser Sequenz oder die gesamte homologe Sequenz können als Hybridisierungssonde unter Verwendung von Standardhybridisierungstechniken (wie z.B. beschrieben in Sambrook et al., vide supra) zur Isolierung weiterer im Verfahren nützlicher Nukleinsäuresequenzen aus anderen Organismen durch das Screening von cDNA- und/oder genomischen Banken verwendet werden.

Überdies lässt sich ein erfindungsgemäßes Nukleinsäuremolekül bzw. ein Teil von dieser durch Polymerasekettenreaktion isolieren, wobei Oligonukleotidprimer auf der Basis der hier angegebenen Sequenzen oder von Teilen davon verwendet werden (z.B. kann ein Nukleinsäuremolekül, umfassend die vollständige Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al., Biochemistry 18, 5294 (1979)) und daraus mittels reverser Transkriptase (z.B. Moloney-MLV-Reverse Transkriptase, erhältlich von Gibco/BRL, Bethesda, MD oder AMV-Reverse-Transkriptase, erhältlich von Seikagaku Amerika, Inc., St. Petersburg, FL) cDNA herstellen. Synthetische Oligonukleotidprimer zur Amplifizierung mittels Polymerasekettenreaktion lassen sich auf der Basis der hierin offenbarten Sequenzen erstellen. Eine erfindungsgemäße Nukleinsäure kann unter Verwendung von cDNA oder alternativ von genomischer DNA als Matrize und geeigneten Oligonukleotidprimern mittels Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Nukleotidsequenz entsprechen, welche für ein erfindungsgemäßes Protein kodiert, können durch Standardsyntheseverfahren, beispielsweise mit einem automatischen DNA-Synthesegerät, hergestellt werden.

Unter dem Begriff "Fragmente der DNA", wie er hier verwendet wird, versteht man Teilstücke der DNA, die für ein erfindungsgemäßes Protein kodieren, dessen biologische Aktivität darin besteht, dass es eine Erhöhung der Pathogenresistenz (bevorzugt der Pilzpathogenresistenz) vermittelt.

Der Begriff "Fragmente des Proteins", wie er hier verwendet wird, bezeichnet Teilstücke des Proteins, dessen biologische Aktivität darin besteht, dass es eine Erhöhung der Pathogenresistenz (bevorzugt der Pilzpathogenresistenz) in Pflanzen vermittelt.

"Armadillo Repeat Arm1 Polypeptid" oder "Armadillo Repeat ARM1 Protein" oder "Arm" oder "Arm1" und deren Abwandlungen meint im Rahmen der Erfindung ein Protein mit einem oder mehreren Armadillo repeats.

In einer besonders bevorzugten Ausführungsform betrifft die Erfindung ein Armadillo-Repeat ARM1 Polypeptid, das die in den Beispielen gezeigte Aktivität hat. In einer Ausführungsform wird unter einem Armadillo Repeat ARM1 Protein ein Protein verstanden mit einer Homologie zu einer der in SEQ ID No: 2, 4, 6, 8, 10, 12, 14, 16, 18, 20, 22, 24, 26, 28, 30, 32, 34, 36, 38, 42, 44, 60, 61 or 62 oder in den Figuren gezeigten Aminosäuresequenzen, z.B ein Armadillo-Repeat ARM1 Polypeptid aus Gerste (HvARM) gemäß SEQ ID NO: 2 und/oder aus Reis (Oryza sative) gemäß SEQ ID NO: 4, 6, 8, und/oder 10, und/oder aus Tabak (Nicotiana tabacum) gemäß SEQ ID NO.: 12 und/oder aus A.thaliana gemäß SEQ ID NO: 14, 16, 18, 20, 22, 24, 26, 28, 30, 32, 34, 36, 38, 42, und/oder 44, oder gemäß einer der Consensussequenzen gemäß SEQ ID NO.: 60, 61 oder 62 oder ein funktionelles Fragment davon. In einer Ausführungsform betrifft die Erfindung funktionelle Äquivalente der vorgenannten Polypeptidsequenzen.

"Polypeptidmenge" meint beispielsweise Molekülzahl oder Mol an Armadillo-Repeat ARM1 Polypeptidmolekülen in einem Organismus, einem Gewebe, einer Zelle oder einem Zellkompartiment. "Erhöhung" der Polypeptidmenge meint die molare Erhöhung der Anzahl an jeweiligen Polypeptiden in einem Organismus, einem Gewebe, einer Zelle oder einem Zellkompartiment - beispielsweise durch eines der unten beschriebenen Verfahren - im Vergleich zu einer geeigneten Kontrolle, z.B. dem Wildtyp (Kontrollpflanze) derselben Gattung und Art auf den dieses Verfahren nicht angewendet wurde, unter ansonst gleichen Rahmenbedingungen (wie beispielsweise Kulturbedingungen, Alter der Pflanzen etc.). Die Erhöhung beträgt dabei mindestens 5 %, bevorzugt mindestens 10 % oder mindestens 20 %, besonders bevorzugt mindestens 40 % oder 60 %, ganz besonders bevorzugt mindestens 70 % oder 80 %, am meisten bevorzugt mindestens 90 %, 95 % oder 99 %, insbesondere 100 %, insbersondere bevorzugt mehr als 100%, vorzugsweise mehr als 150 %, 200 % oder 300 %.

Unter Homologie zwischen zwei Nukleinsäuresequenzen wird die Identität der Nukleinsäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA; Altschul et al., Nucleic Acids Res. 25, 3389 (1997)) unter Einstellung folgender Parameter berechnet wird:

| | |
|---|---|
| Gap Weight: 50 | Length Weight: 3 |
| Average Match: 10 | Average Mismatch: 0 |

Beispielhaft wird unter einer Sequenz, die eine Homologie von mindestens 80 % auf Nukleinsäurebasis mit der Sequenz SEQ ID NO: 1 aufweist, eine Sequenz verstanden, die bei einem Vergleich mit der Sequenz SEQ ID NO: 1 nach obigem Programmalgorithmus mit obigem Parametersatz eine Homologie von mindestens 80 % aufweist.

Unter Homologie zwischen zwei Polypeptiden wird die Identität der Aminosäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 8 | Length Weight: 2 |
| Average Match: 2,912 | Average Mismatch:-2,003 |

Beispielhaft wird unter einer Sequenz, die eine Homologie von mindestens 80 % auf Polypeptidbasis mit der Sequenz SEQ ID NO: 2 aufweist, eine Sequenz verstanden, die bei einem Vergleich mit der Sequenz SEQ ID NO: 2 nach obigem Programmalgorithmus mit obigem Parametersatz eine Homologie von mindestens 80 % aufweist.

Unter "Armadillo repeat" wird eine Sequenz verstanden die tandemartig angeordnete Kopien einer degenerierten Sequenz von ca. 42 Aminosäuren enthält, welche eine drei-dimensionale Struktur zur Vermittlung von Protein-Protein-Interaktionen kodiert (Azevedo et al. Trends Plant Sci. 6, 354 (2001)). Beispielsweise hat das Polypeptid, das im erfindungsgemäßen Verfahren eingesetzt wird, oder das erfindungsgemäße Polypeptid eine Aktivität, die in die intrazelluläre Signaltransduktion oder in die Regulation der Genexpression im Rahmen zellulärer Entwicklungsprozesse involviert ist.

Das Armadillo-Repeat ARM1 Protein wird beispielsweise kodiert von einem Nukleinsäuremolekül umfassend ein Nukleinsäuremolekül ausgewählt aus der Gruppe bestehend aus:
(a) Nukleinsäuremolekül, das ein Polypeptid kodiert, das die in SEQ ID No: 2, 4, 6, 8, 10, 12, 14, 16, 18, 20, 22, 24, 26, 28, 30, 32, 34, 36, 38, 42, 44, 60, 61 oder 62 gezeigte Sequenz umfasst;
(b) Nukleinsäuremolekül, das zumindest ein Polynukleotid der Sequenz nach der SEQ ID No: 1, 3, 5, 7, 9, 11, 13, 15, 17, 19, 21, 23, 25, 27, 29, 31, 33, 35, 37, 39, 41, 43 oder 63 umfaßt;
(c) Nukleinsäuremolekül, das ein Polypeptid kodiert, dessen Sequenz eine Identität von 40%, 50%, 60%, 70%, 80%, 90%, 95%, 97%, 98%, 99% oder mehr zu den Sequenzen SEQ ID No: 2, 4, 6, 8, 10, 12, 14, 16, 18, 20, 22, 24, 26, 28, 30, 32, 34, 36, 38, 42, 44, 60, 61 oder 62 aufweist;
(d) Nukleinsäuremolekül nach (a) bis (c), das für ein funktionelle Fragment oder ein Epitop der Sequenzen gemäß SEQ ID No: 2, 4, 6, 8, 10, 12, 14, 16, 18, 20, 22, 24, 26, 28, 30, 32, 34, 36, 38, 42, 44, 60, 61 oder 62 kodiert;
(e) Nukleinsäuremolekül, das ein Polypeptid kodiert, welches von einem monoklonalen Antikörper, gerichtet gegen ein Polypeptid welches durch die Nukleinsäuremoleküle gemäß (a) bis (c) kodiert wird, erkannt wird; und
(f) Nukleinsäuremolekül, das unter stringenten Bedingungen mit einem Nukleinsäuremolekül gemäß (a) bis (c) oder deren Teilfragmente bestehend aus mindestens 15 Nukleotiden (nt), vorzugsweise 20 nt, 30 nt, 50 nt, 100 nt, 200 nt oder 500 nt hybridisiert;
(g) Nukleinsäuremolekül, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann;
oder eine komplementäre Sequenz davon umfasst, oder ein funktionelles Äquivalent davon darstellt.

In einer bevorzugten Ausführungsform besitzt die das erfindungsgemäße Polypeptid kodierende Sequenz keine U box im 5'-UTR.

Erfindungsgemäß wird die Aktivität der genannten Polypeptide in eine Pflanze oder einen Teil einer Pflanze, vorzugsweise in den Epidermis- und/oder Mesophyllzellen einer Pflanze, wie oben erläutert, eingeführt und exprimiert, bzw. entsprechend die Expression des endogenen Polypeptids erhöht.

In einer Ausführungsform wird die Aktivität von ARM1 in Lemma, Palea und/oder Glume erhöht.

"Einführung" bzw. "Einbringen" umfasst im Rahmen der Erfindung alle Verfahren, die dazu geeignet sind, eine erfindungsgemäße Nukleinsäuresequenz, direkt oder indirekt, in eine Pflanze oder eine Zelle, Kompartiment, Gewebe, Organ oder Samen derselben einzuführen oder dort zu generieren. Die Einführung kann zu einer vorübergehenden (transienten) oder zu einer dauerhaften (stabilen) Präsenz einer erfindungsgemäßen Nukleinsäuresequenz führen.

"Einführung" bzw. "Einbringen" umfasst beispielsweise Verfahren wie Transfektion, Transduktion oder Transformation.

Die Einbringung einer erfindungsgemäßen Expressionskassette in einen Organismus oder Zellen, Gewebe, Organe, Teile bzw. Samen desselben (bevorzugt in Pflanzen bzw. pflanzliche Zellen, Gewebe, Organe, Teile oder Samen), kann vorteilhaft unter Verwendung von Vektoren realisiert werden, in denen die Expressionskassetten enthalten sind. Die Expressionskassette kann in den Vektor (zum Beispiel ein Plasmid) über eine geeignete Restriktionsschnittstelle eingeführt werden. Das entstandene Plasmid wird zunächst in E.coli-Zellen eingeführt. Korrekt transformierte E.coli-Zellen werden selektioniert, gezüchtet und das rekombinante Plasmid mit dem Fachmann geläufigen Methoden gewonnen. Restriktionsanalyse und Sequenzierung können dazu dienen, den Klonierungsschritt zu überprüfen.

Bei den Vektoren kann es sich beispielsweise um Plasmide, Cosmide, Phagen, Viren oder auch Agrobakterien handeln. In einer vorteilhaften Ausführungsform wird die Einführung der Expressionskassette mittels Plasmidvektoren realisiert. Bevorzugt sind solche Vektoren, die eine stabile Integration der Expressionskassette in das Wirtsgenom ermöglichen.

Unter "Epitop" versteht man die die Spezifität der Antikörper bestimmenden Bereiche eines Antigens (die antigene Determinante). Ein Epitop ist demnach der Teil eines Antigens, der tatsächlich mit dem Antikörper in Kontakt tritt.

Solche antigenischen Determinaten sind die Bereiche eines Anitgens, auf welches die T-Zell Rezeptoren reagieren und in der Folge Antikörper produzieren, die spezifisch die Antigene- Determinante/ das Epitop eines Antigens binden. Antigene bzw. deren Epitope sind demnach in der Lage, die Immunantwort eines Organismus mit der Folge der Bildung spezifischer - gegen das Epitop gerichteter - Antikörper zu induzieren. Epitope bestehen z.B. aus linearen Abfolgen von Aminosäuren in der Primärstruktur von Proteinen, oder aus komplexen sekundären oder tertiären Proteinstrukturen. Unter einem Hapten versteht man ein aus dem Kontext der Antigenumgebung herausgelöstes Epitop. Obwohl Haptene per Definition einen Antikörper gegen sich gerichtet haben, sind Haptene unter Umständen nicht in der Lage nach z.B. Injektion in einen Organismus eine Immunantwort zu induzieren. Zu diesem Zweck werden Haptene an Trägermoleküle gekoppelt. Als Beispiel sei Dinitrophenol (DNP) genannt, welches nach Kopplung an BSA (bovine serum albumine) zur Herstellung von Antikörpern gerichtet gegen DNP verwendet wurde (Bohn A., König W., Immunology 47 (2), 297 (1982)).

Haptene sind daher insbesondere (oft niedermolekulare bzw. kleine) Substanzen, die selbst keine Immunreaktion auslösen, aber sehr wohl, wenn sie an einen großmolekularen Träger gekoppelt wurden.

Unter den so erzeugten Antikörpern finden sich auch solche, die das Hapten alleine binden können.

In einer Ausführungsform betrifft die vorliegende Erfindung einen Antikörper gegen ein hierin charakterisiertes Polypeptid, insbesondere einen monoklonalen Antikörper der ein Polypeptid bindet, das eine AA-Sequenz umfasst oder daraus besteht gemäß den Sequenzen, die in SEQ ID No: 2, 4, 6, 8, 10, 12, 14, 16, 18, 20, 22, 24, 26, 28, 30, 32, 34, 36, 38, 40, 42, 44, 60, 61 oder 62 gezeigt sind.

Antikörper im Rahmen der vorliegenden Erfindung können zur Identifikation und Isolierung von erfindungsgemäß offenbarten Polypeptiden aus Organismen, bevorzugt Pflanzen, besonders bevorzugt monokotyledonen Pflanzen, oder weiterhin bevorzugt dikotyledonen Pflanzen, verwendet werden. Die Antikörper können sowohl monoklonal, polyklonal, als auch synthetischer Natur sein, oder aus Antikörperfragmenten wie Fab, Fv oder scFv Fragmenten bestehen, die durch proteolytischen Abbau entstehen. "Single chain" Fv (scFv) Fragmente sind einkettige Fragmente, die verbunden über eine flexible Linkersequenz nur die variablen Bereiche der schweren und leichten Antikörperketten enthalten. Solche scFv Fragmente können auch als rekombinante Antikörperderivate produziert werden. Eine Präsentation solcher Antikörperfragmente auf der Oberfläche filamentöser Phagen ermöglicht die direkte Selektion hochaffin bindender scFv-Moleküle aus kombinatorischen Phagenbibliotheken.

Monoklonale Antikörper können gemäß der von Köhler und Milstein beschriebenen Methode gewonnen werden (Nature 256, 495 (1975)).

"Funktionelle Äquivalente" eines Armadillo-Repeat ARM1 Proteins meint bevorzugt solche Polypeptide, die zu den durch die Sequenzen SEQ ID No: 2, 4, 6, 8, 10, 12, 14, 16, 18, 20, 22, 24, 26, 28, 30, 32, 34, 36, 38, 42, 44, 60, 61 or 62 beschriebenen Polypeptiden eine Homologie von mindestens 40% aufweisen und im Wesentlichen die gleichen Eigenschaften aufweisen oder Funktionen besitzen. Vorzugsweise beträgt die Homologie 50%, 60%, 70%, 80%, 90%, besonders bevorzugt 95%, 97%, 98%, 99% oder mehr.

Funktionelle Äquivalenz kann zum Beispiel durch Vergleich der Phänotypen von Testorganismen nach Expression der jeweiligen Polypeptide unter möglichst identischen Bedingungen bestimmt werden.

"Im Wesentlichen gleiche Eigenschaften" eines funktionellen Äquivalentes meint vor allem die Verleihung eines pathogenresistenten Phänotypes oder die Verleihung oder Steigerung der Pathogenresistenz gegen zumindest ein Pathogen bei Erhöhen der Polypeptidmenge, Aktivität oder Funktion des besagten funktionellen Armadillo-Repeat ARM1 Protein Äquivalentes in einer Pflanze, Organ, Gewebe, Teil oder Zellen, insbesondere in Epidermis- und/oder Mesophyllzellen derselben, vorzugsweise gemessen an der Penetrationseffizienz eines Pathogens wie in den Beispielen gezeigt.

"Analoge Bedingungen" bedeutet, dass alle Rahmenbedingungen wie beispielsweise Kultur- oder Zuchtbedingungen, Assaybedingungen (wie Puffer, Temperatur, Substrate, Pathogenkonzentration etc.) zwischen den zu vergleichenden Versuchen im Wesentlichen identisch gehalten werden und die Ansätze sich allein durch die Sequenz der zu vergleichenden Armadillo-Repeat ARM1 Polypeptide, ihrem Ursprungs-Organismus und gegebenenfalls dem Pathogen unterscheiden.

"Funktionelle Äquivalente" meint auch natürliche oder künstliche Mutationsvarianten der Armadillo-Repeat ARM1 Polypeptide gemäß SEQ ID No: 2, 4, 6, 8, 10, 12, 14, 16, 18, 20, 22, 24, 26, 28, 30, 32, 34, 36, 38, 42, 44, 60, 61 or 62 sowie homologe Polypeptide aus anderen monokotyledonen und dikotyledonen Pflanzen, welche weiterhin im Wesentlichen gleiche Eigenschaften aufweisen. Bevorzugt sind homologe Polypeptide aus hierin beschriebenen bevorzugten Pflanzen. Die zu den im Rahmen dieser Erfindung offenbarten Armadillo-Repeat ARM1 Proteinsequenzen homologen Sequenzen aus anderen Pflanzen können z.B. durch Datenbanksuche oder Durchmustern von Gen-Banken - unter Verwendung der Armadillo-Repeat ARM1 ProteinSequenzen als Suchsequenz bzw. Sonde - leicht aufgefunden werden.

Funktionelle Äquivalente können z.B. auch von einem der erfindungsgemäßen Polypeptide gemäß SEQ ID No: 2, 4, 6, 8, 10, 12, 14, 16, 18, 20, 22, 24, 26, 28, 30, 32, 34, 36, 38, 42, 44, 60, 61 or 62 durch Substitution, Insertion oder Deletion abgeleitet sein und zu diesen Polypeptiden eine Homologie von mindestens 40 %, 50, 60 %, bevorzugt mindestens 80 %, vorzugsweise mindestens 90 %, besonders bevorzugt mindestens 95 %, ganz besonders bevorzugt mindestens 98 % aufweisen und zeichnen sich durch im Wesentlichen gleiche funktionelle Eigenschaften wie die Polypeptide gemäß SEQ ID No: 2, 4, 6, 8, 10, 12, 14, 16, 18, 20, 22, 24, 26, 28, 30, 32, 34, 36, 38, 42, 44, 60, 61 oder 62 aus.

Funktionelle Äquivalente sind auch jene von den erfindungsgemäßen Nukleinsäuresequenzen gemäß SEQ ID No: 1, 3, 5, 7, 9, 11, 13, 15, 17, 19, 21, 23, 25, 27, 29, 31, 33, 35, 37, 39, 41, oder 43 durch Substitution, Insertion oder Deletion abgeleitete Nukleinsäuremoleküle, und haben eine Homologie von mindestens 40 %, 50 , 60 %, bevorzugt 80 %, vorzugsweise mindestens 90 %, besonders bevorzugt mindestens 95 %, ganz besonders bevorzugt mindestens 98 % zu einem der erfindungsgemäßen Polynukleotide gemäß SEQ ID No: 1, 3, 5, 7, 9, 11, 13, 15, 17, 19, 21, 23, 25, 27, 29, 31, 33, 35, 37, 39, 41, oder 43 und kodieren für Polypeptide mit im Wesentlichen identischen funktionellen Eigenschaften wie Polypeptide gemäß SEQ ID No: 2, 4, 6, 8, 10, 12, 14, 16, 18, 20, 22, 24, 26, 28, 30, 32, 34, 36, 38, 42, 44, 60, 61 or 62.

Beispiele für die in dem erfindungsgemäßen Verfahren zu erhöhenden funktionellen Äquivalente der Armadillo-Repeat ARM1 Proteine gemäß SEQ ID No: 2, 4, 6, 8, 10, 12, 14, 16, 18, 20, 22, 24, 26, 28, 30, 32, 34, 36, 38, 42, 44, 60, 61 or 62 lassen sich beispielsweise aus Organismen, deren genomische Sequenz bekannt ist, durch Homologievergleiche aus Datenbanken auffinden.

Auch die Durchmusterung von cDNA- oder genomischen Bibliotheken anderer Organismen, bevorzugt von den weiter unten genannten als Wirt zur Transformation geeigneten Pflanzenarten, unter Verwendung der unter SEQ ID No: 1, 3, 5, 7, 9, 11, 13, 15, 17, 19, 21, 23, 25, 27, 29, 31, 33, 35, 37, 39, 41, oder 43 beschriebene Nukleinsäuresequenzen oder Teilen derselben als Sonde, ist ein dem Fachmann geläufiges Verfahren, um Homologe in anderen Arten zu identifizieren. Dabei haben die von der Nukleinsäuresequenz gemäß SEQ ID No: 1, 3, 5, 7, 9, 11, 13, 15, 17, 19, 21, 23, 25, 27, 29, 31, 33, 35, 37, 39, 41, oder 43 abgeleiteten Sonden eine Länge von mindestens 20 bp, bevorzugt mindestens 50 bp, besonders bevorzugt mindestens 100 bp, ganz besonders bevorzugt mindestens 200 bp, am meisten bevorzugt mindestens 400 bp. Die Sonde kann auch ein oder mehrere Kilobasen lang sein, z.b. 1 Kb, 1,5 Kb oder 3 Kb. Für die Durchmusterung der Bibliotheken kann auch ein zu den unter SEQ ID No: 1, 3, 5, 7, 9, 11, 13, 15, 17, 19, 21, 23, 25, 27, 29, 31, 33, 35, 37, 39, 41, oder 43 beschriebenen Sequenzen komplementärer DNA-Strang, oder ein Fragment desselben mit einer Länge zwischen 20 bp und mehreren Kilobasen eingesetzt werden.

Im erfindungsgemäßen Verfahren können auch solche DNA Moleküle verwendet werden, die unter Standardbedingungen mit den durch SEQ ID No: 1, 3, 5, 7, 9, 11, 13, 15, 17, 19, 21, 23, 25, 27, 29, 31, 33, 35, 37, 39, 41, 43 oder 63 beschriebenen und für Armadillo-Repeat ARM1 Proteine kodierenden Nukleinsäuremoleküle, der zu diesen komplementären Nukleinsäuremolekülen oder Teilen der vorgenannten hybridisieren und als vollständige Sequenzen für Polypeptide kodieren, die im Wesentlichen über die gleichen Eigenschaften, bevorzugt funktionellen Eigenschaften, wie die unter SEQ ID No: 2, 4, 6, 8, 10, 12, 14, 16, 18, 20, 22, 24, 26, 28, 30, 32, 34, 36, 38, 42, 44, 60, 61 or 62 beschriebenen Polypeptide verfügen.

"Standardhybridisierungsbedingungen" ist breit zu verstehen und meint je nach Anwendung stringente als auch weniger stringente Hybridisierungsbedingungen. Solche Hybridisierungsbedingungen sind unter anderem bei Sambrook J., Fritsch E.F., Maniatis T. et al., in "Molecular Cloning (A Laboratory Manual)", 2. Auflage, Cold Spring Harbor Laboratory Press, 1989, Seiten 9.31-9.57) oder in "Current Protocols in Molecular Biology", John Wiley BSons, N.Y. (1989), 6.3.1-6.3.6. beschrieben.

Der Fachmann wird aus seinem Fachwissen heraus Hybridisierungsbedingungen auswählen, die es ihm ermöglichen, spezifische von unspezifischen Hybridisierungen zu unterscheiden.

Beispielhaft können die Bedingungen während des Waschschrittes ausgewählt sein aus Bedingungen mit geringer Stringenz (mit ungefähr 2X SSC bei 50°C) und solchen mit hoher Stringenz (mit ungefähr 0.2X SSC bei 50°C bevorzugt bei 65°C) (20X SSC: 0,3 M Natriumcitrat, 3 M NaCl, pH 7.0). Darüberhinaus kann die Temperatur während des Waschschrittes von niedrig stringenten Bedingungen bei Raumtemperatur, ungefähr 22°C, bis zu stärker stringenten Bedingungen bei ungefähr 65°C angehoben werden. Beide Parameter, Salzkonzentration und Temperatur, können gleichzeitig oder auch einzeln variiert werden, wobei der jeweils andere Parameter konstant gehalten wird. Während der Hybridisierung können auch denaturierende Agenzien wie zum Beispiel Formamid oder SDS eingesetzt werden. In Gegenwart von 50% Formamid wird die Hybridisierung bevorzugt bei 42°C ausgeführt. Einige bevorzugte Bedingungen für Hybridisierung und Waschschritt sind nachstehend angegeben:
(1) Hybridisierungbedingungen können zum Beispiel aus nachfolgenden Bedingungen ausgewählt sein:
   a) 4X SSC bei 65°C,
   b) 6X SSC bei 45°C,
   c) 6X SSC, 100 µg/ml denaturierter, fragmentierte Fischsperma-DNA bei 68°C,
   d) 6X SSC, 0,5 % SDS, 100 µg/ml denaturierter, Lachssperma-DNA bei 68°C,
   e) 6X SSC, 0,5 % SDS, 100 µg/ml denaturierter, fragmentierte Lachssperma-DNA, 50 % Formamid bei 42°C,
   f) 50 % Formamid, 4X SSC bei 42°C,
   g) 50 % (vol/vol) Formamid, 0, 1 % Rinderserumalbumin, 0,1 % Ficoll, 0,1 % Polyvinylpyrrolidon, 50 mM Natriumphosphatpuffer pH 6,5, 750 mM NaCl, 75 mM Natriumcitrat bei 42°C,
   h) 2X oder 4X SSC bei 50°C (schwach stringente Bedingung),
   i) 30 bis 40 % Formamid, 2X oder 4X SSC bei 42°C (schwach stringente Bedingung), oder
   j) 500 mN Natriumphosphatpuffer pH 7,2, 7% SDS (g/V), 1mM EDTA, 10µg/ml single stranded DNA, 0,5% BSA (g/V) (Church und Gilbert, Proc. Natl. Acad.Sci. U.S.A. 81, 1991 (1984))
(2) Waschschritte können zum Beispiel aus nachfolgenden Bedingungen ausgewählt sein:
   a) 0,015 M NaCl/0,0015 M Natriumcitrat/0,1 % SDS bei 50°C,
   b) 0.1X SSC bei 65°C,
   c) 0,1X SSC, 0,5 % SDS bei 68°C,
   d) 0,1X SSC, 0,5 % SDS, 50 % Formamid bei 42°C,
   e) 0,2X SSC, 0,1 % SDS bei 42°C, oder
   f) 2X SSC bei 65°C (schwach stringente Bedingung),

In einer Ausführungsform werden die Hybridisierungsbedingungen wie folgt gewählt:
Es wird ein Hybridisierungspuffer gewählt, der Formamid, NaCl und PEG 6000 enthält. Die Anwesenheit von Formamid im Hybridisierungspuffer destabilisiert Doppelstrang-Nukleinsäuremoleküle, wodurch die Hybridisierungstemperatur auf 42°C gesenkt werden kann, ohne dadurch die Stringenz zu erniedrigen. Die Verwendung von Salz im Hybridisierungspuffer erhöht die Renaturierungsrate einer Duplex-DNA, bzw. die Hybridisierungseffizienz. Obwohl PEG die Viskosität der Lösung erhöht, was einen negativen Einfluß auf Renaturierungsraten besitzt, wird durch die Anwesenheit des Polymers in der Lösung die Konzentration der Sonde im verbleibenden Medium erhöht, was die Hybridisierungsrate steigert. Die Zusammensetzung des Puffers ist:

| Hybridisierungspuffer |
|---|
| 250 mM Natriumphosphat-Puffer pH 7,2 |
| 1 mM EDTA |
| 7 % SDS (g/v) |
| 250 mM NaCl |
| 10 µg/ml ssDNA |
| 5 % Polyethylenglykol (PEG) 6000 |
| 40 % Formamid |

Die Hybridisierungen werden bei 42°C etwa 12 Stunden, beispielsweise über Nacht, durchgeführt. Die Filter werden anschliessend 3x mit 2× SSC + 0,1 % SDS für jeweils ca. 10 min. gewaschen.

"Genexpression" und "Expression" sind synomym zu verwenden und meinen das Realisieren der Information, die in einem Nukleinsäuremolekül gespeichert ist.

Das erfindungsgemäße "Verändern" von Nukleotid- bzw. Aminosäuresequenzen umfasst bevorzugt deren Mutieren bzw. Mutationen. "Mutationen" im Sinne der vorliegenden Erfindung meint die Veränderung der Nukleinsäureabfolge einer Genvariante in einem Plasmid oder im Genom eines Organismus. Mutationen können z.B als Folge von Fehlern bei der Replikation entstehen oder durch Mutagene hervorgerufen werden. Die Rate der Spontanmutationen im Zellgenom von Organismen ist sehr gering, allerdings sind dem kundigen Fachmann eine Vielzahl von biologischen, chemischen oder physikalischen Mutagenen und Verfahren zum zufälligen oder gezielten Mutieren von Nukleotidsequenzen, und damit letzlich potentiell auch zum Verändern der von diesen kodierten Aminosäuresequenzen, bekannt.

Mutationen umfassen Substitutionen, Additionen, Deletionen eines oder mehrerer Nukleinsäurereste. Unter Substitutionen versteht man den Austausch von einzelnen Nukleinsäurebasen, dabei unterscheidet man zwischen Transitionen (Substitution einer Purin- gegen eine Purinbase bzw. einer Pyrimidin- gegen eine Pyrimidinbase) und Transversionen (Substitution einer Puringegen eine Pyrimidinbase (oder umgekehrt).

Unter Addition bzw. Insertion versteht man den Einbau von zusätzlichen Nukleinsäureresten in die DNA, wobei es zu Verschiebungen des Leserahmens kommen kann. Bei derartigen Leserahmenverschiebungen unterscheidet man zwischen "in frame" Insertionen/Additionen und "out of frame" Insertionen. Bei den "in-frame" Insertionen/Additionen bleibt der Leserahmen erhalten und ein um die Anzahl der von den insertierten Nukleinsäuren kodierten Aminosäuren vergrößertes Polypeptid entsteht. Bei "out of frame" Insertionen/Additionen geht der ursprüngliche Leserahmen verloren und die Bildung eines vollständigen und funktionstüchtigen Polypeptids ist in vielen Fällen, natürlich abhängig vom Ort der Mutation, nicht mehr möglich.

Deletionen beschreiben den Verlust von einem oder mehreren Basenpaaren, die ebenfalls zu "in frame" oder "out of frame" Verschiebungen des Leserahmens und den damit verbundenen Folgen bezüglich der Bildung eines intakten Proteins führen.

Die zur Erzeugung von zufälligen oder gezielten Mutationen verwendbaren mutagenen Agenzien (Mutagene) und die anwendbaren Methoden und Techniken sind dem Fachmann bekannt. Deratige Methoden und Mutagene sind z.B. beschrieben bei van Harten A.M. ("Mutation breeding: theory and practical applications", Cambridge University Press, Cambridge, UK (1998)), Friedberg E., Walker G., Siede W. ("DNA Repair and Mutagenesis", Blackwell Publishing (1995)), oder Sankaranarayanan K., Gentile J. M., Ferguson L. R. ("Protocols in Mutagenesis", Elsevier Health Sciences(2000)).

Für die Einführung von gezielten Mutationen können geläufige molekulabiologische Methoden und Verfahren wie z.B der vitro Mutagenese Kits, "LA PCR in vitro Mutagenesis Kit" (Takara Shuzo, Kyoto), oder PCR Mutagenesen unter Verwendung geeigenter Primer verwendet werden.

Wie bereits oben aufgeführt, gibt es eine Vielzahl von chemischen physikalischen und biologischen Mutagenen.

Die im folgenden aufgeführten seien beispielhaft aber nicht einschränkend genannt.

Chemische Mutagene können gemäß ihres Wirkmechanismus unterteilt werden. So gibt es Basenanaloga (z.B.5-Bromuracil, 2-Aminopurin), mono- und bifunktionale alkylierende Agenzien (z.B. monfunktionale wie Ethylmethylsulfonat, Dimethylsulfat, oder bifunktionale wie Dichlorethylsulfit, Mitomycin, Nitrosoguanidin - dialkylnitrosamin, N-Nitrosoguanidin-Derivate) oder interkalierende Substanzen (z.B. Acridin, Ethidiumbromid).

Physikalische Mutagene sind zum Beispiel ionisierende Strahlungen. Ionisierende Strahlungen sind elektromagnetische Wellen oder Teilchenstrahlungen die in der Lage sind, Moleküle zu ionisieren, d.h. aus diesen Elektronen zu entfernen. Die zurückbleibenden Ionen sind meist sehr reaktiv, so dass sie, falls sie in lebendem Gewebe entstehen, großen Schaden z.B an der DNA anrichten können und (bei geringer Intensität) dadurch Mutationen induzieren. Ionisierende Strahlungen sind z.B. Gammastrahlung (Photonenenergie von etwa einem Megaelektronenvolt MeV), Röntgenstrahlung (Photonenenergie von mehreren oder vielen Kiloelektronenvolt keV) oder auch Ultraviolettes Licht (UV-Licht, Photonenenergie von über 3,1 eV). UV Licht bewirkt die Bildung von Dimeren zwischen Basen, am häufigsten sind Thymidindimere, durch die Mutationen entstehen.

Die klassische Erzeugung von Mutanten durch Behandlung der Samen mit mutagenisierenden Agentien wie z.B. Ethylmethylsulfonat (EMS) (Birchler J.A., Schwartz D., Biochem. Genet. 17 (11-12), 1173 (1979); Hoffmann G.R., Mutat. Res. 75 (1), 63 (1980)) oder ionosierender Strahlung ist durch die Verwendung biologischer Mutagene z.B. Transposons (z.B. Tn5, Tn903, Tn916, Tn1000, Balcells et a1., 1991, May B.P. et al., Proc. Natl. Acad. Sci U S A. 100 (20), 11541 (2003)) oder molekularbiologischer Methoden wie der Mutagense durch T-DNA Insertion (Feldman K.A., Plant J. 1, 71 (1991), Koncz et al., Plant Mol. Biol. 20 (5), 963 (1992)) erweitert worden.

Bevorzugt ist die Verwendung von chemischen oder biologischen Mutagenen zur Erzeugung von mutierten Genvarianten. Bei den chemischen Agenzien ist besonders bevorzugt die Erzeugung von Mutanten durch Verwendung der EMS (Ethylmethylsulfonat) Mutagenese genannt. Bei der Erzeugung von Mutanten unter Verwendung biologischer Mutagene sei bevorzugt die T-DNA-Mutagenese oder Transposonmutagenese genannt.

Somit können beispielsweise auch solche Polypeptide für das erfindungsgemäße Verfahren eingesetzt werden, welche man in Folge einer Mutation einer Nukleotidsequenz kodierend für ein erfindungsgemäßes Polypeptid z.B. gemäß SEQ ID No: 2, 4, 6, 8, 10, 12, 14, 16, 18, 20, 22, 24, 26, 28, 30, 32, 34, 36, 38, 42, 44, 60, 61 or 62 erhält.

"Transgen" meint zum Beispiel bezüglich einer Nukleinsäuresequenz, einer Expressionskassette oder einem Vektor enthaltend besagte Nukleinsäuresequenz oder einem Organismus transformiert mit besagter Nukleinsäuresequenz, Expressionskassette oder Vektor, alle solche durch gentechnische Methoden zustande gekommenen Konstruktionen oder Organismen, in denen sich entweder
(a) die Armadillo-Repeat ARM1 Protein Nukleinsäuresequenz, oder
(b) eine mit der Armadillo-Repeat ARM1 Protein Nukleinsäuresequenz funktionell verknüpfte genetische Kontrollsequenz, zum Beispiel ein Promotor, oder
(c) (a) und (b)
nicht in ihrer natürlichen, genetischen Umgebung befinden oder durch gentechnische Methoden modifiziert wurden, wobei die Modifikation beispielhaft eine Substitution, Addition, Deletion, oder Insertion eines oder mehrerer Nukleotidreste(s) sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des Armadillo-Repeat ARM1 Protein-Promotors mit dem entsprechenden Armadillo-Repeat ARM1 Protein-Gen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beschrieben (US 5,565,350; WO 00/15815).

Die erfindungsgemäße Erhöhung der Pathogenresistenz kann auch dadurch erreicht werden, dass die Expression des Pflanzen-eigenen, d.h. endogenen Proteins, welches dem erfindungsgemäßen Protein entspricht, bzw. einer endogenen Nukleotidsequenz, welche eine erfindungsgemäße Sequenz darstellt, und welche auch den 5'- und/oder 3'-UTR-Bereich umfassen kann, manipuliert wird. Es handelt sich also um eine pflanzeneigene Nukleotid- oder Peptidsequenz, die eine Erhöhung der Pathogenresistenz vermittelt und die bevorzugt ein oder mehrere Armadillo-Repeat-Sequenzen aufweist, oder um eine erfindungsgemäße Aminosäuresequenz, die für ein solches Protein kodiert. Diese Manipulation kann durch irgendeine Veränderung der Sequenz, bevorzugt eine Mutation, erreicht werden, aber auch beispielsweise durch eine Veränderung der Promotor-DNA-Sequenz des Protein-kodierenden Gens erreicht werden. Eine solche Veränderung, die eine veränderte, vorzugsweise erhöhte Expressionsrate des endogenen erfindungsgemäßen Gens zur Folge hat, kann durch Deletion oder Insertion von DNA-Sequenzen erfolgen. Eine Veränderung der 5'-UTR-Region insgesamt und/oder der Promotor-Sequenz von endogenen erfindungsgemäßen Genen führt in der Regel zu einer Veränderung der exprimierten Menge des Gens und/ oder der Funktion des exprimierten Gens bzw. Genprodukts, und damit vorzugsweise auch zu einer Veränderung der in der Zelle bzw. den Pflanzen nachweisbaren Aktivität. Die Veränderung der 5'-UTR-Region insgesamt und/oder der Promotor-Sequenz des erfindungsgemäßen endogenen Gens kann auch zu einer Veränderung der Menge an und/oder Funktion eines erfindungsgemäßen Proteins in der Zelle führen.

Eine weitere Möglichkeit zur Erhöhung der Aktivität und des Gehalts des erfindungsgemäßen endogenen Proteins besteht darin, Transkriptionsfaktoren, die in die Transkription des entsprechenden endogenen Genes involviert sind, z.B. durch Überexpression hochzuregulieren. Die Maßnahmen zur Überexpression von Transkriptionsfaktoren sind dem Fachmann bekannt und werden ebenfalls im Rahmen der vorliegenden Erfindung für erfindungsgemäße Proteine offenbart.

Des Weiteren kann eine erhöhte Expression eines endogenen erfindungsgemäßen Gens dadurch erzielt werden, dass ein im nicht-transformierten Organismus nicht vorkommendes Regulatorprotein mit dem Promotor dieser Gene in Wechselwirkung tritt. Bei einem solchen Regulator kann es sich um ein chimäres Protein handeln, welches aus einer DNA-Bindedomäne und einer Transkriptionsaktivator-Domäne besteht, wie beispielsweise in WO 96/06166 beschrieben.

Die Herstellung eines transformierten Organismus (bzw. einer transformierten Zelle) erfordert, dass das entsprechende DNA-Moleküle in die entsprechende Wirtszelle eingebracht wird und anschließend durch die Genexpression die entsprechenden RNAs und Proteine gebildet werden.

Für diesen Vorgang, der als Transformation (oder Transduktion bzw. Transfektion) bezeichnet wird, steht eine Vielzahl von Methoden zur Verfügung (Keown et al., Methods in Enzymology 185, 527(1990)). So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglykol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen in die Zelle eingeführt werden. Elektroporation ist eine weitere geeignete Methode zur Einführung von DNA, bei der die Zellen reversibel durch einen elektrischen Impuls permeabilisert werden. Entsprechende Verfahren sind beschrieben (beispielsweise bei Bilang et al., Gene 100, 247 (1991); Scheid et al., Mol. Gen. Genet. 228, 104 (1991); Guerche et al., Plant Science 52, 111 (1987); Neuhause et al., Theor. Appl. Genet. 75, 30 (1987); Klein et al., Nature 327, 70(1987); Howell et al., Science 208, 1265 (1980); Horsch et al., Science 227, 1229 (1985); DeBlock et al., Plant Physiology 91, 694 (1989); "Methods for Plant Molecular Biology" (Weissbach and Weissbach, eds.) Academic Press Inc. (1988); und "Methods in Plant Molecular Biology" (Schuler and Zielinski, eds.) Academic Press Inc. (1989)).

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, das biolistische Verfahren mit der Genkanone, die sogenannte "particle bombardment" Methode, die Elektroporation, die Inkubation trockener Embryonen in DNA-haltiger Lösung und die Mikroinjektion.

Neben diesen "direkten" Transformationstechniken kann eine Transformation auch durch bakterielle Infektion mittels Agrobacterium tumefaciens oder Agrobacterium rhizogenes durchgeführt werden. Die Verfahren sind beispielsweise beschrieben bei Horsch et al. Science 225, 1229 (1985).

Werden Agrobakterien verwendet, so ist die Expressionskassette in spezielle Plasmide zu integrieren, bei denen es sich entweder um einen Zwischenvektor (englisch: shuttle or intermediate vector) oder um einen binären Vektor handeln kann. Wird ein Ti- oder Ri-Plasmid zur Transformation verwendet, ist zumindest die rechte Begrenzung, meistens jedoch sowohl die rechte als auch die linke Begrenzung der Ti- oder Ri-Plasmid-T-DNA als flankierende Region mit der einzuführenden Expressionskassette verbunden.

Bevorzugt werden binäre Vektoren verwendet. Binäre Vektoren können sowohl in E.coli als auch in Agrobacterium replizieren. Sie enthalten in der Regel ein Selektionsmarkergen und einen Linker oder Polylinker flankiert von der rechten und linken T-DNA-Begrenzungssequenz. Sie können direkt in Agrobacterium transformiert werden (Holsters et al., Mol. Gen. Genet. 163, 181 (1978)). Das Selektionsmarkergen, zum Beispiel das nptll Gen, das eine Resistenz gegen Kanamycin verleiht, erlaubt eine Selektion transformierter Agrobakterien. Das in diesem Fall als Wirtsorganismus fungierende Agrobacterium sollte bereits ein Helfer-Ti-Plasmid mit der vir-Region enthalten, die für die Übertragung der T-DNA auf die pflanzliche Zelle erforderlich ist. Ein so transformiertes Agrobacterium kann zur Transformation pflanzlicher Zellen verwendet werden. Die Verwendung von T-DNA zur Transformation pflanzlicher Zellen ist intensiv untersucht und beschrieben (EP 120 516; Hoekema, in "The Binary Plant Vector System", Offsetdrukkerij Kanters B.V., Alblasserdam, Chapter V; An et al. EMBO J. 4, 277 (1985)). Verschiedene binäre Vektoren sind bekannt und teilweise kommerziell erhältlich wie zum Beispiel pB1101.2 oder pBIN19 (Clontech Laboratories, Inc. USA).

Im Falle der Injektion oder Elektroporation von DNA bzw. RNA in pflanzliche Zellen werden keine besonderen Anforderungen an das verwendete Plasmid gestellt. Einfache Plasmide wie die der pUC-Reihe können verwendet werden. Sollen aus den transformierten Zellen vollständige Pflanzen regeneriert werden, so ist er erforderlich, das sich auf dem Plasmid ein zusätzliches selektionierbares Markergen befindet.

Stabil transformierte Zellen, d.h. solche, die die eingeführte DNA integriert in die DNA der Wirtszelle enthalten, können von untransformierten unterschieden werden, wenn ein selektionierbarer Marker Bestandteil der eingeführten DNA ist (McCormick et al., Plant Cell Reports 5, 81 (1986)). Als Marker kann beispielhaft jedes Gen fungieren, dass eine Resistenz gegen Antibiotika oder Herbizide (wie Kanamycin, G 418, Bleomycin, Hygromycin oder Phosphinotricin) zu verleihen vermag. Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiele umfassen das bar-Gen, das Resistenz gegen das Herbizid Phosphinotricin verleiht (Rathore et al., Plant Mol. Biol. 21 (5), 871 (1993)), das nptll-Gen, das Resistenz gegen Kanamycin verleiht, das hpt-Gen, das Resistenz gegen Hygromycin verleiht, oder das EPSP-Gen, das Resistenz gegen das Herbizid Glyphosat verleiht. Die erhaltenen Pflanzen können in üblicher Weise gezüchtet und gekreuzt werden. Es sollten zwei oder mehr Generationen kultiviert werden, um sicherzustellen, dass die genomische Integration stabil und vererblich ist.

Die oben genannten Verfahren sind beispielsweise beschrieben in Jenes et al. ("Techniques for Gene Transfer", in "Transgenic Plants", Vol. 1, Engineering and Utilization, herausgegeben von Kung S.D. und Wu R., Academic Press, S. 128-143 (1993), sowie in Potrykus, Annu. Rev. Plant Physiol. Plant Molec. Biol. 42, 205 (1991)). Vorzugsweise wird das zu exprimierende Konstrukt in einen Vektor kloniert, der geeignet ist, Agrobacterium tumefaciens zu transformieren, beispielsweise in pBin19 (Bevan et al., Nucl. Acids Res. 12, 8711 (1984)).

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Dem Fachmann sind auch Verfahren bekannt, um aus Pflanzenzellen Pflanzenteile und ganze Pflanzen zu regenerieren. Beispielsweise werden hierzu Verfahren beschrieben von Fennell et al., Plant Cell Rep. 11, 567 (1992); Stoeger et al., Plant Cell Rep. 14, 273 (1995); Jahne et al., Theor. Appl. Genet. 89, 525 (1994), verwendet.

Ein weiterer Gegenstand der vorliegenden Erfindung ist ein rekombinantes Nukleinsäuremolekül, umfassend die folgenden Elemente in 5'-3'-Orientierung:
- regulatorische Sequenzen eines in Pflanzenzellen aktiven Promotors,
- operativ daran gebunden eine erfindungsgemäße DNA-Sequenz,
- ggf. operativ daran gebunden regulatorische Sequenzen, die in der Pflanzenzelle als Transkriptions-, Terminations- und/oder Polyadenylierungssignale dienen können.

In besagten Expressionskonstrukten/Expressionskassetten steht ein Nukleinsäuremolekül, dessen Expression (Transkription und ggf. Translation) eine "Armadillo-Repeat ARM1 Protein Verbindung" generiert, bevorzugt in funktioneller Verknüpfung mit mindestens einem genetischen Kontrollelement (beispielsweise einem Promotor), das eine Expression in Pflanzen gewährleistet. Soll das Expressionskonstrukt direkt in die Pflanze eingeführt und die " Armadillo-Repeat ARM1 Protein Verbindung" dort in planta erzeugt werden, so sind pflanzenspezifische genetische Kontrollelemente (beispielsweise Promotoren) bevorzugt. Die "Armadillo-Repeat ARM1 Protein Verbindung" kann jedoch auch in anderen Organismen oder in vitro erzeugt und dann in die Pflanze eingebracht werden. In diesem sind alle prokaryotischen oder eukaryotischen genetischen Kontrollelemente (beispielsweise Promotoren) bevorzugt, die die Expression in der jeweils für die Herstellung gewählte Pflanze erlauben.

Unter "Operativ (daran) gebunden" bzw. "funktionell (damit) verknüpft" versteht man zum Beispiel die sequentielle Anordnung eines Promotors mit der zu exprimierenden Nukleinsäuresequenz (zum Beispiel einer "Armadillo-Repeat ARM1 Protein Verbindung") und ggf. weiterer regulativer Elemente wie zum Beispiel einem Terminator derart, dass jedes der regulativen (bzw. regulatorischen) Elemente seine Funktion bei der transgenen Expression der Nukleinsäuresequenz, je nach Anordnung der Nukleinsäuresequenzen zu sense oder anti-sense RNA, erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierende Nukleinsäuresequenz hinter der als Promotor fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer funktionellen Verknüpfung als auch die Herstellung einer Expressionskassette kann mittels gängiger Rekombinations- und Klonierungstechniken realisiert werden, wie sie beispielsweise in Maniatis T., Fritsch E.F. und Sambrook J., "Molecular Cloning: A Laboratory Manual", Cold Spring Harbor Laboratory, Cold Spring Harbor (NY) (1989), in Silhavy T.J., Berman M.L. und Enquist L.W. "Experiments with Gene Fusions", Cold Spring Harbor Laboratory, Cold Spring Harbor (NY) (1984), in Ausubel F.M. et al., "Current Protocols in Molecular Biology", Greene Publishing Assoc. and Wiley Interscience (1987) und bei Gelvin et al., in "Plant Molecular Biology Manual" (1990) beschrieben sind. Zwischen beiden Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann die Expressionskassette, bestehend aus einer Verknüpfung von Promotor und zu exprimierender Nukleinsäuresequenz, integriert in einem Vektor vorliegen und durch zum Beispiel Transformation in ein pflanzliches Genom insertiert werden.

Das erfindungsgemäße Verfahren kann vorteilhaft mit weiteren Verfahren, die eine Pathogenresistenz (beispielsweise gegen Insekten, Pilze, Bakterien, Nematoden etc.), Stressresistenz oder eine andere Verbesserung der pflanzlichen Eigenschaften bewirken, kombiniert werden. Beispiele sind u.a. genannt bei Dunwell J.M., J. Exp. Bot. 51, (Spec No) 487 (2000).

In einer bevorzugten Ausführungsform erfolgt die Erzeugung oder Erhöhung der Funktion eines Armadillo-Repeat ARM1 Proteins in einer Pflanze kombiniert mit einer Erhöhung der Aktivität eines Bax Inhibitor 1-Proteins. Dies kann beispielsweise durch Expression einer für ein Bax-Inhibitor-1 Protein kodierenden Nukleinsäuresequenz, z.B. im Mesophyllgewebe und/oder Wurzelgewebe erfolgen.

Im erfindungsgemäßen Verfahren sind die Bax-Inhibitor-1 Proteine aus Hordeum vulgare oder Nicotiana tabacum besonders bevorzugt.

Ein weiterer Gegenstand der Erfindung betrifft Nukleinsäuremoleküle, die Nukleinsäuremoleküle kodierend für Armadillo-Repeat ARM1 Proteine aus Gerste gemäß den Polynukleotiden SEQ. ID No: 1 umfassen, sowie die dazu komplementären Nukleinsäuresequenzen, und die durch Entartung (Degeneration) des genetischen Codes abgeleiteten Sequenzen und die für funktionelle Äquivalente der Polypeptide gemäß SEQ. ID No.: 1 kodierenden Nukleinsäuremoleküle, wobei die Nukleinsäuremoleküle nicht aus der SEQ ID NO: 3, 5, 7, 9, 11, 13, 15, 17, 19, 21, 23, 25, 27, 29, 31, 33, 35, 37, 39, 41, oder 43 bestehen.

Ein weiterer Gegenstand der Erfindung betrifft das Armadillo-Repeat ARM1 Protein aus Gerste gemäß SEQ. ID No.: 2 oder eines, das diese Sequenzen umfasst, sowie funktionelle Äquivalente davon, die nicht einer der Sequenzen der SEQ ID NO: 4, 6, 8, 10, 12, 14, 16, 18, 20, 22, 24, 26, 28, 30, 32, 34, 36, 38, 42, oder 44, entsprechen.

Ein weiterer Gegenstand der Erfindung betrifft transgene Expressionskassetten, die eine der erfindungsgemäßen Nukleinsäuresequenzen umfassen. In den erfindungsgemäßen transgenen Expressionskassetten ist die Nukleinsäuresequenz kodierend für die Armadillo-Repeat ARM1 Proteine aus Gerste, Weizen und Mais mit mindestens einem genetischen Kontrollelement nach obiger Definition derart verknüpft, dass die Expression (Transkription und ggf. Translation) in einem beliebigen Organismus - bevorzugt in monokotyledonen Pflanzen - realisiert werden kann. Dazu geeignete genetische Kontrollelemente sind oben beschrieben. Die transgenen Expressionskassetten können auch weitere Funktionselemente gemäß obiger Definition enthalten.

Derartige Expressionskassetten enthalten beispielsweise eine erfindungsgemäße Nukleinsäuresequenz, z.B. eine die im Wesentlichen identisch ist zu einem Nukleinsäuremolekül nach SEQ ID No: 1, 3, 5, 7, 9, 11, 13, 15, 17, 19, 21, 23, 25, 27, 29, 31, 33, 35, 37, 39, 41, oder 43 oder einem erfindungsgemäßen Fragment davon, wobei besagte Nukleinsäuresequenz bevorzugt in Sense-Orientierung oder in Antisense-Orientrierung zu einem Promotor vorliegt und damit zur Expression von sense- oder antisense-RNA führen kann, wobei es sich bei dem besagten Promotor um einen in Pflanzen aktiven, bevorzugt um einen durch Pathogenbefall induzierbaren Promotor handelt. Erfindungsgemäß sind auch transgene Vektoren umfaßt, die die besagten transgenen Expressionskassetten beinhalten.

Pflanzenspezifische Promotoren meint grundsätzlich jeden Promotor, der die Expression von Genen, insbesondere Fremdgenen, in Pflanzen oder Pflanzenteilen, -zellen, -geweben, - kulturen steuern kann. Dabei kann die Expression beispielsweise konstitutiv, induzierbar oder entwicklungsabhängig sein.

Bevorzugt sind:

### a) Konstitutive Promotoren

"Konstitutiver" Promotor meint solche Promotoren, die eine Expression in zahlreichen, bevorzugt allen, Geweben über einen größeren Zeitraum der Pflanzenentwicklung, bevorzugt zu allen Zeitpunkten der Pflanzenentwicklung, gewährleisten. Vorzugsweise verwendet man insbesondere einen pflanzlichen Promotor oder einen Promotor, der einem Pflanzenvirus entstammt. Insbesondere bevorzugt ist der Promotor des 35S-Transkriptes des CaMV (Blumenkohlmosaikvirus) (Franck et al., Cell 21, 285 (1980); Odell et al., Nature 313, 810 (1985); Shewmaker et al., Virology 140, 281 (1985); Gardner et al., Plant Mol. Biol. 6, 221 (1986)) oder der 19S CaMV-Promotor (US 5,352,605; WO 84/02913; Benfey et al., EMBO J. 8, 2195-2202 (1989)). Ein weiterer geeigneter konstitutiver Promotor ist der "Rubisco small subunit (SSU)"-Promotor (US 4,962,028), der Promotor der Nopalinsynthase aus Agrobacterium, der TR-Doppelpromotor, der OCS (Octopin Synthase)-Promotor aus Agrobacterium, der Ubiquitin-Promotor (Holtorf S. et al., Plant Mol. Biol. 29, 637 (1995)), der Ubiquitin 1-Promotor (Christensen et al., Plant Mol. Biol. 18, 675 (1992); Bruce et al., Proc. Natl. Acad. Sci. USA 86, 9692 (1989)), der Smas-Promotor, der Cinnamylalkoholdehydrogenase-Promotor (US 5,683,439), die Promotoren der vakuolärer ATPase Untereinheiten oder der Promotor eines prolinreichen Proteins aus Weizen (WO 91/13991), sowie weitere Promotoren von Genen, deren konstitutive Expression in Pflanzen dem Fachmann bekannt ist. Als konstitutiver Promotor insbesondere bevorzugt ist der Promotor des Nitrilase-1 (nit1) Gens aus A. thaliana (GenBank Acc.-No.: Y07648.2, Nukleotide 2456-4340, Hillebrand et al., Gene 170,197 (1996)).

### b) Gewebespezifische Promotoren

In einer Ausführungsform werden Promotoren mit Spezifitäten für die Antheren, Ovarien, Blüten, Blätter, Stengel, Wurzeln und Samen verwendet.

Samenspezifische Promotoren sind zum Beispiel der Promotor des Phaseolins (US 5,504,200; Bustos et al., Plant Cell 1 (9), 839 (1989)), des 2S-Albumingens (Joseffson et al., J. Biol. Chem. 262, 12196 (1987)), des Legumins (Shirsat et al., Mol. Gen. Genet. 215 (2), 326 (1989)), des USP (unknown seed protein; Bäumlein et al., Mol. Gen. Genet. 225 (3), 459 (1991)), des Napin-Gens (US 5,608,152; Stalberg et al., L. Planta 199, 515 (1996)), des Gens kodierend für das Saccharosebindeprotein (WO 00/26388) oder der Legumin B4-Promotor (LeB4; Bäumlein et al., Mol. Gen. Genet. 225, 121 (1991); Bäumlein et al, Plant Journal 2 (2), 233 (1992); Fiedler et al., Biotechnology (NY) 13 (10), 1090 (1995)), der Oleosin-Promotor aus Arabidopsis (WO 98/45461), der Bce4-Promotor aus Brassica (WO 91/13980). Weitere geeignete samenspezifische Promotoren sind die der Gene kodierend für das "High Molecular Weight Glutenin" (HMWG), Gliadin, Verzweigungsenzym, ADP-Glucose-Pyrophosphatase (AGPase) oder die Stärkesynthase. Bevorzugt sind ferner Promotoren, die eine samenspezifische Expression in Monokotyledonen wie Mais, Gerste, Weizen, Roggen, Reis etc. erlauben. Vorteilhaft eingesetzt werden können der Promotor des Ipt2- oder des Ipt1-Gens (WO 95/15389, WO 95/23230) oder die in WO 99/16890 beschriebenen Promotoren (Promotoren des Hordein-Gens, des Glutelin-Gens, des Oryzin-Gens, des Prolamin-Gens, des Gliadin-Gens, des Zein-Gens, des Kasirin-Gens oder des Secalin-Gens).

Knollen-, Speicherwurzel- oder Wurzel-spezifische Promotoren sind beispielsweise der Patatin-Promotor Klasse I (B33) und der Promotor des Cathepsin D-Inhibitors aus Kartoffel.

Blattspezifische Promotoren sind beispielsweise der Promotor der cytosolischen FBPase aus Kartoffel (WO 97/05900), der SSU Promotor (small subunit) der Rubisco (Ribulose-1,5-bisphosphatcarboxylase) oder der ST-LSI Promotor aus Kartoffel (Stockhaus et al., EMBO J. 8, 2445 (1989)). Epidermis-spezifische Promotoren sind beispielsweise der Promotor des OXLP-Gens ("Oxalat-Oxidase like protein"; Wei et al., Plant Mol. Biol. 36, 101 (1998)), ein Promotor bestehend aus dem GSTA1-Promotor und dem WIR1a-Intron (WO 2005/035766) und der GLP4-Promotor (WO2006/128882 = PCT/EP 2006/062747).

Andere gewebespezifische Promotoren sind z.B. blütenspezifische Promotoren wie beispielsweise der Phytoen-Synthase-Promotor (WO 92/16635) oder der Promotor des Prr-Gens (WO 98/22593) und Antheren-spezifische Promotoren wie der 5126-Promotor (US 5,689,049, US 5,689,051), der globl-Promotor und der γ-Zein Promotor.

### c) Chemisch induzierbare Promotoren

Die Expressionskassetten können auch einen chemisch induzierbaren Promotor enthalten (Übersichtsartikel: Gatz et al., Annu. Rev. Plant Physiol. Plant Mol. Biol. 48, 89 (1997)), durch den die Expression des exogenen Gens in der Pflanze zu einem bestimmten Zeitpunkt gesteuert werden kann. Derartige Promotoren, wie z.B. der PRP1 Promotor (Ward et al., Plant Mol. Biol. 22, 361 (1993)), ein durch Salicylsäure induzierbarer Promotor (WO 95/19443), ein durch Benzolsulfonamid induzierbarer Promotor (EP 0 388 186), ein durch Tetrazyklin induzierbarer Promotor (Gatz et al., Plant J. 2, 397 (1992)), ein durch Abscisinsäure induzierbarer Promotor (EP 0 335 528) bzw. ein durch Ethanol oder Cyclohexanon induzierbarer Promotor (WO 93/21334) können ebenfalls verwendet werden.

### d) Stress- oder Pathogen-induzierbare Promotoren

Ganz besonders vorteilhaft ist die Verwendung von pathogen-induzierbaren Promotoren, da diese eine Expression nur im Bedarfsfall (d.h. Pathogenbefall) ermöglichen.

Im erfindungsgemäßen Verfahren werden daher in einer Ausführungsform in Pflanzen aktive Promotoren verwendet, bei denen es sich Pathogen-induzierbare Promotor handelt.

Pathogen-induzierbare Promotoren umfassen die Promotoren von Genen, die infolge eines Pathogenbefalls induziert werden, wie beispielsweise Gene von PR-Proteinen, SAR-Proteinen, β-1,3-Glucanase, Chitinase usw. (beispielsweise Redolfi et al., Neth. J. Plant Pathol. 89, 245 (1983); Uknes et al., Plant Cell 4, 645 (1992); Van Loon Plant Mol. Virol. 4, 111 (1985); Marineau et al., Plant Mol. Biol. 9, 335 (1987); Matton et al. Molecular Plant-Microbe Interactions 2, 325 (1987); Somssich et al., Proc. Natl. Acad. Sci. USA 83, 2427 (1986); Somssich et al., Mol. Gen. Genetics 2, 93 (1988); Chen et al., Plant J. 10, 955 (1996); Zhang and Sing, Proc. Natl. Acad. Sci. USA 91, 2507 (1994); Warner et al., Plant J. 3, 191 (1993); Siebertz et al., Plant Cell 1, 961-968 (1989)). Umfasst sind auch durch Verwundung induzierbare Promotoren wie der des pinll-Gens (Ryan, Ann. Rev. Phytopath. 28, 425 (1990); Duan et al., Nat. Biotech. 14, 494 (1996)), des wun1- und wun2-Gens (US 5,428,148), des win1- und win2-Gens (Stanford et al., Mol. Gen. Genet. 215, 200 (1989)), des Systemin-Gens (McGurl et al., Science 225, 1570 (1992)), des WIP1-Gens (Rohmeier et al., Plant Mol. Biol. 22, 783 (1993); Eckelkamp et al., FEBS Letters 323, 73 (1993)), des MPI-Gens (Corderok et al., Plant J. 6 (2), 141(1994)) und dergleichen.

Eine Quelle für weitere pathogen-induzierbare Promotoren stellt die PR-Genfamilie dar. Eine Reihe von Elementen in diesen Promotoren haben sich als vorteilhaft erwiesen. So vermittelt der Nukleotidbereich von Nukleotid -364 bis Nukleotid -288 im Promotor von PR-2d Salicylat-Spezifität (Buchel et al., Plant Mol. Biol. 30, 493 (1996)). Die Sequenz 5'-TCATCTTCTT-3' taucht im Promotor der Gersten β-1,3-Glucanase und in mehr als 30 weiteren Stress-induzierten Genen wiederholt auf. Diese Region bindet in Tabak ein nukleäres Protein, dessen Menge durch Salicylat erhöht wird. Die PR-1-Promotoren aus Tabak und Arabidopsis (EP-A 0 332 104, WO 98/03536) eignen sich ebenfalls als pathogen-induzierbare Promotoren. Bevorzugt, da besonders spezifisch durch Pathogen induziert, sind die "acidic PR-5"-(aPR5)-Promotoren aus Gerste (Schweizer et al., Plant Physiol. 114, 79 (1997)) und Weizen (Rebmann et al., Plant Mol. Biol. 16, 329 (1991)). aPR5-Proteine akkumulieren in ca. 4 bis 6 Stunden nach Pathogenbefall und zeigen nur eine sehr geringe Hintergrundexpression (WO 99/66057). Ein Ansatz, um eine erhöhte pathogen-induzierte Spezifität zu erreichen, bildet die Herstellung synthetischer Promotoren aus Kombinationen von bekannten pathogen-responsiven Elementen (Rushton et al., Plant Cell 14, 749 (2002); WO 00/01830; WO 99/66057). Weitere pathogen-induzierbare Promotoren aus verschiedenen Arten sind dem Fachmann bekannt (EP-A 1 165 794; EP-A 1 062 356; EP-A 1 041 148; EP-A 1 032 684).

Weitere pathogen-induzierbare Promotoren umfassen den Flachs Fis1-Promotor (WO 96/34949), den Vst1-Promotor (Schubert et al., Plant Mol. Biol. 34, 417 (1997)) sowie den EAS4 Sesquiterpen-Cyclase-Promotor aus Tabak (US 6,100,451).

Ferner sind Promotoren bevorzugt, die durch biotischen oder abiotischen Stress induziert werden, wie beispielsweise der pathogen-induzierbare Promotor des PRP1-Gens (bzw. gst1 Promotor) z.B. aus Kartoffel (WO 96/28561; Ward et al., Plant Mol. Biol. 22, 361 (1993)), der hitzeinduzierbare hsp70- oder hsp80-Promotor aus Tomate (US 5,187,267), der kälteinduzierbare alpha-Amylase-Promotor aus der Kartoffel (WO 96/12814), der lichtinduzierbare PPDK-Promotor oder der verwundungsinduzierte pinll-Promotor (EP-A 0 375 091).

### e) Mesophyllgewebe-spezifische Promotoren

"Mesophyllgewebe" meint das zwischen den Epidermisschichten liegende Blattgewebe, bestehend aus dem Palisadengewebe, dem Schwammgewebe und den Blattadern.

Im erfindungsgemäßen Verfahren werden in einer Ausführungsform Mesophyllgewebe-spezifische Promotoren wie beispielsweise der Promotor des Weizen Germin 9f-3.8 Gens (GenBank Acc.-No.: M63224) oder der Gerste GerA-Promotor (WO 02/057412) verwendet. Besagte Promotoren sind besonders vorteilhaft, da sie sowohl Mesophyllgewebespezifisch als auch pathogen-induzierbar sind. Ferner geeignet ist der Mesophyllgewebe-spezifische Arabidopsis CAB-2 Promotor (GenBank Acc.-No.: X15222), sowie der Zea mays PPCZm1-Promotor (GenBank Acc.-No.: X63869) oder Homologe davon. Mesophyll-gewebe-spezifisch meint eine durch die spezifische Wechselwirkung von in der Promotor-sequenz vorhandenen Cis-Elementen und an diese bindende Transkriptionsfaktoren hervorgerufene Beschränkung der Transkription eines Gens auf möglichst wenige, das Mesophyllgewebe enthaltende Pflanzengewebe, vorzugsweise ist eine auf das Mesophyllgewebe beschränkte Transkription gemeint.

Weitere Mesophyll-spezifische Promotoren sind PPCZm1 (= PEPC; Kausch, Plant Mol. Biol. 45, 1 (2001)); OsrbcS (Kyozuka et al., Plant Phys. 102, 991- (1993)); OsPPDK, acc. AC099041; TaGF-2.8, acc. M63223 (Schweizer, Plant J. 20, 541 (1999)); TaFBPase, acc. X53957;.TaWIS1, acc. AF467542 (US 2002/115849); HvBIS1, acc. AF467539 (US 2002/115849); ZmMIS1, acc. AF467514 (US 2002/115849); HvPR1a, acc. X74939 (Bryngelsson et al., Molecular Plant-Microbe Interactions 7 (2), 267 (1994); HvPR1b, acc. X74940 (Bryngelsson et al., Molecular Plant-Microbe Interactions 7 (2), 267 (1994)); HvB1,3gluc; acc. AF479647; HvPrx8, acc. AJ276227 (Kristensen et al., Molecular Plant Pathology 2 (6), 311(2001)); und HvPAL, acc. X97313 (Wei, Plant Molecular Biology 36, 101 (1998)).

### f) Epidermis-spezifische Promotoren

"Epidermisgewebe" oder Epidermis meint die äußeren Gewebeschichten der Pflanzen. Die Epidermis kann ein- bis mehrschichtig sein; es gibt epidermis-"angereicherte" Genexpression, wie z.B. von Cer3 , die als Marker dienen kann (Hannoufa, Plant J. 10 (3), 459 (1996)).

Unter "Epidermis" versteht der Fachmann vorzugsweise das vorherrschende Abschlussgewebe primärer oberirdischer Pflanzenteile, z.B. des Sprosses, der Blätter, Blüten, Früchte und Samen.

Epidermisspezifische Promotoren sind beispielsweise WIR5 (=GstA1), acc. X56012 (Dudler & Schweizer, unveröff.); GLP4, acc. AJ310534 (Wei, Plant Molecular Biology 36, 101 (1998)); GLP2a, acc. AJ237942 (Schweizer, Plant J. 20, 541 (1999).); Prx7, acc. AJ003141 (Kristensen, Molecular Plant Pathology 2 (6), 311(2001)); GerA, acc. AF250933 (Wu, Plant Phys. Biochem. 38, 685 (2000)); OsROC1, acc. AP004656; RTBV, acc. AAV62708, AAV62707 (Klöti, PMB 40, 249 (1999)) und Cer3 (Hannoufa, Plant J. 10 (3), 459 (1996)).

### g) Entwicklungsabhängige Promotoren

Weitere geeignete Promotoren sind beispielsweise fruchtreifungs-spezifische Promotoren, wie beispielsweise der fruchtreifungs-spezifische Promotor aus Tomate (WO 94/21794, EP 409 625). Entwicklungsabhängige Promotoren schließen zum Teil die gewebespezifischen Promotoren ein, da die Ausbildung einzelner Gewebe naturgemäß entwicklungsabhängig erfolgt.

Besonders bevorzugt sind konstitutive, sowie Blatt- und/oder Stengel-spezifische, pathogen-induzierbare, Wurzel-spezifische, Mesophyllgewebe-spezifische Promotoren, wobei konstitutive, pathogen-induzierbare, Mesophyllgewebe-spezifische und Wurzel-spezifische Promotoren am meisten bevorzugt sind.

Es können ferner weitere Promotoren operativ mit der zu exprimierenden Nukleinsäuresequenz verknüpft sein, die eine Expression in weiteren Pflanzengeweben oder in anderen Organismen, wie zum Beispiel E.coli-Bakterien ermöglichen. Als Pflanzen Promotoren kommen im Prinzip alle oben beschriebenen Promotoren in Frage.

Weitere zur Expression in Pflanzen geeignete Promotoren sind beschrieben (Rogers et al., Meth. in Enzymol. 153, 253 (1987); Schardl et al., Gene 61, 1 (1987); Berger et al., Proc. Natl. Acad. Sci. USA 86, 8402 (1989)).

Darüber hinaus ist der Durchschnittsfachmann in der Lage, mittels Routinemethoden weitere geeignete Promotoren zu isolieren. So kann der Fachmann mit Hilfe gängiger molekularbiologischer Methoden, z.B. Hybridisierungsexperimenten oder DNA-Protein-Bindungsstudien z. B. weitere epidermis-spezifische regulatorische Nukleinsäureelemente identifizieren. Dabei wird z.B. in einem ersten Schritt das gewünschte Gewebe aus dem gewünschten Organismus, aus dem die regulatorischen Sequenzen isoliert werden sollen, isoliert und daraus die gesamte poly(A)+-RNA isoliert und eine cDNA-Bank angelegt. In einem zweiten Schritt werden mit Hilfe von cDNA-Klonen, die auf poly(A)+-RNA-Molekülen aus einem anderen Gewebe basieren, aus der ersten Bank mittels Hybridisierung diejenigen Klone identifiziert, deren korrespondierende poly(A)+-RNA-Moleküle lediglich im gewünschten Gewebe akkumulieren. Anschließend werden mit Hilfe dieser so identifizierten cDNAs Promotoren isoliert, die über Gewebe-spezifische regulatorische Elemente verfügen. Dem Fachmann stehen darüber hinaus weitere auf PCR basierende Methoden für die Isolierung geeigneter Gewebe-spezifischer Promotoren zur Verfügung.

Die in den erfindungsgemäßen Expressionskassetten oder Vektoren enthaltenen Nukleinsäuresequenzen können mit weiteren genetischen Kontrollsequenzen neben einem Promotor operativ verknüpft sein. Der Begriff der genetischen Kontrollsequenzen ist breit zu verstehen und meint all solche Sequenzen, die einen Einfluss auf das Zustandekommen oder die Funktion des erfindungsgemäßen rekombinanten Nukleinsäuremoleküls haben. Genetische Kontrollsequenzen modifizieren zum Beispiel die Transkription und Translation in prokaryotischen oder eukaryotischen Organismen. Vorzugsweise umfassen die erfindungsgemäßen Expressionskassetten 5'-stromaufinrärts von der jeweiligen transgen zu exprimierenden Nukleinsäuresequenz einen Promotor mit einer vorab beschriebenenen Spezifität und 3'-stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Genetische Kontrollsequenzen umfassen auch weitere Promotoren, Promotorelemente oder Minimalpromotoren, die die expressionssteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E. und Chua N.H., J. Biol. Chem. 266 (26), 17131 (1991)) und Hitzestress (Schoffl F. et al., Mol. Gen. Genet. 217 (2-3), 246 (1989)) beschrieben.

Prinzipiell können alle natürlichen Promotoren mit ihren Regulationssequenzen wie die oben genannten für das erfindungsgemäße Verfahren verwendet werden. Darüberhinaus können auch synthetische Promotoren vorteilhaft verwendet werden.

Genetische Kontrollsequenzen umfassen ferner auch die 5'-untranslatierten Regionen (5'-UTR), Introns oder die nicht-kodierende 3'-Region von Genen wie beipielsweise das Actin-1 Intron, oder die Adh1-S Introns 1, 2 und 6 (allgemein: "The Maize Handbook", Chapter 116, Freeling and Walbot, Eds., Springer, New York (1994)). Es ist gezeigt worden, dass diese eine signifikante Rolle bei der Regulation der Genexpression spielen können. So wurde gezeigt, dass 5'-untranslatierte Sequenzen die transiente Expression heterologer Gene verstärken können. Beispielhaft für Translationsverstärker sei die 5'-Leadersequenz aus dem Tabak-Mosaik-Virus zu nennen (Gallie et al., Nucl. Acids Res. 15, 8693 (1987)) und dergleichen. Sie können ferner die Gewebespezifität fördern (Rouster J. et al., Plant J. 15, 435 (1998)). Besonders bevorzugt ist die natürliche 5'-UTR des HvArm-Gens, und insbesondere die mit der Sequenz entsprechend SEQ ID No: 64 oder einer dazu zu mindestens 50%, 60%, 70%, 80%, 90%, 95%, 97%, oder insbesondere 99% oder mehr identischen Sequenz.

Das rekombinante Nukleinsäuremolekül kann vorteilhafterweise eine oder mehrere sogenannte "enhancer"-Sequenzen funktionell verknüpft mit dem Promotor enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien im Genkonstrukt enthalten sein.

Als Kontrollsequenzen geeignete Polyadenylierungssignale sind pflanzliche Polyadenylierungssignale, vorzugsweise solche, die im wesentlichen T-DNA Polyadenylierungssignalen aus Agrobacterium tumefaciens, insbesondere des Gens 3 der T-DNA (Octopin Synthase) des Ti-Plasmids pTiACHS entsprechen (Gielen et al., EMBO J. 3, 835 (1984)) oder funktionelle Äquivalente davon. Beispiele für besonders geeignete Terminatorsequenzen sind der OCS (Octopin-Synthase)-Terminator und der NOS (Nopalin-Synthase)-Terminator.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel der natürliche Promotor eines bestimmten Gens gegen einen Promotor mit Spezifität für die embryonale Epidermis und/oder die Blüte ausgetauscht werden.

Ein rekombinantes Nukleinsäuremolekül und ein von ihr abgeleiteter Vektor können weitere Funktionselemente enthalten. Der Begriff Funktionselement ist breit zu verstehen und meint all solche Elemente, die einen Einfluss auf Herstellung, Vermehrung oder Funktion der erfindungsgemäßen Nukleinsäuremoleküle, Vektoren oder transgenen Organismen haben. Beispielhaft aber nicht einschränkend seien zu nennen:
a) Selektionsmarker, die eine Resistenz gegen einen Metabolismusinhibitor wie 2-Desoxyglucose-6-phosphat (WO 98/45456), Antibiotika oder Biozide, bevorzugt Herbizide, wie zum Beispiel Kanamycin, G 418, Bleomycin, Hygromycin, oder Phosphinotricin verleihen. Besonders bevorzugte Selektionsmarker sind solche, die eine Resistenz gegen Herbizide verleihen. Beispielhaft seien genannt: DNA-Sequenzen, die für Phosphinothricinacetyltransferasen (PAT) kodieren und Glutaminsynthaseinhibitoren inaktivieren (bar und pat Gen), 5-Enolpyruvylshikimat-3-phosphatsynthasegene (EPSP-Synthasegene), die eine Resistenz gegen Glyphosat® (N-(Phosphonomethyl)glycin) verleihen, das für Glyphosat® degradierende Enzyme kodierende gox-Gen (Glyphosatoxidoreduktase), das deh-Gen (kodierend für eine Dehalogenase, die Dalapon inaktiviert), sowie bxn-Gene, die für Bromoxynil degradierende Nitrilaseenzyme kodieren, das aasa-Gen, das eine Resistenz gegen das Antibiotikum Apectinomycin verleiht, das Streptomycinphosphotransferase (SPT)-Gen, das eine Resistenz gegen Streptomycin vermittelt, das Neomycinphosphotransferase (NPTII)-Gen, das eine Resistenz gegen Kanamycin oder Geneticidin verleiht, das Hygromycinphosphotransferase (HPT)-Gen, das eine Resistenz gegen Hygromycin vermittelt, das Acetolactatsynthase-Gen (ALS), das eine Resistenz gegen Sulfonylharnstoff-Herbizide verleiht (z.B. mutierte ALS-Varianten mit z.B. der S4 und/oder Hra Mutation), sowie das Acetolactatsynthase-Gen (ALS), das eine Resistenz gegen Imidazolinon-Herbizide verleiht.
b) Reportergene, die für leicht quantifizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (Schenborn und Groskreutz, Mol. Biotechnol. 13 (1), 29 (1999)) wie das "green fluorescence protein" (GFP) (Sheen et al., Plant Journal 8 (5), 777 (1995); Haseloff et al., Proc. Natl. Acad. Sci. USA 94 (6), 2122 (1997); Reichel et al., Proc. Natl. Acad. Sci. USA 93 (12), 5888 (1996); Tian et al., Plant Cell Rep. 16, 267 (1997); WO 97/41228; Chui et al., Curr Biol 6, 325 (1996); Leffel et al., Biotechniques 23(5): 912-8(1997)), die Chloramphenicoltransferase, eine Luziferase (Ow et al., Science 234, 856 (1986); Millar et al., Plant Mol. Biol. Rep. 10, 324 (1992)), das Aequoringen (Prasher et al., Biochem. Biophys. Res. Commun. 126 (3), 1259 (1985)), die ß-Galaktosidase, das R-Locus Gen (kodiert ein Protein, das die Produktion von Anthocyaninpigmenten (rote Färbung) in pflanzlichen Gewebe reguliert und so eine direkte Analyse der Promotoraktivität ohne Zugabe zusätzlicher Hilfstoffe oder chromogener Substrate ermöglicht; Dellaporta et al., in "Chromosome Structure and Function: Impact of New Concepts", 18th Stadler Genetics Symposium, 11, 263 (1988), ganz besonders bevorzugt ist die ß-Glukuronidase (Jefferson et al., EMBO J. 6, 3901 (1987)).
c) Replikationsursprünge, die eine Vermehrung der erfindungsgemäßen Expressionskassetten oder Vektoren in zum Beispiel E.coli gewährleisten. Beispielhaft seien genannt ORI (origin of DNA replication), der pBR322 ori oder der P15A ori (Sambrook Sambrook J. et al., in "Molecular Cloning (A Laboratory Manual)", 2. Auflage, Cold Spring Harbor Laboratory Press, Cold Spring Harbor, NY, 1989).
d) Elemente, die für eine Agrobakterium-vermittelte Pflanzentransformation erforderlich sind, wie zum Beispiel die rechte oder linke Begrenzung der T-DNA oder die vir-Region.

Zur Selektion erfolgreich transformierter Zellen ist es in der Regel erforderlich, einen selektionierbaren Marker zusätzlich einzuführen, der den erfolgreich transformierten Zellen eine Resistenz gegen ein Biozid (zum Beispiel ein Herbizid), einen Metabolismusinhibitor wie 2-Desoxyglucose-6-phosphat (WO 98/45456) oder ein Antibiotikum verleiht, und damit die Selektion der transformierten Zellen gegenüber untransformierten erlaubt (McCormick et al., Plant Cell Reports 5, 81 (1986)).

Die vorliegende Erfindung betrifft weiterhin transgene Pflanzenzellen und transgene Pflanzen, die eine erfindungsgemäße Nukleinsäuresequenz oder ein erfindungsgemäßes rekombinantes Nukleinsäuremolekül umfassen, sowie Teile der Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanze.

Ein anderer Gegenstand der Erfindung betrifft Pflanzen, die durch natürliche Vorgänge oder künstlich induziert eine oder mehrere Mutationen in einem Nukleinsäuremolekül enthalten, das die Nukleinsäuresequenz gemäß SEQ ID No: 1, 3, 5, 7, 9, 11, 13, 15, 17, 19, 21, 23, 25, 27, 29, 31, 33, 35, 37, 39, 41, oder 43 umfaßt, wobei besagte Mutation eine Erhöhung der Aktivität, Funktion oder Polypeptidmenge eines der durch die Nukleinsäuremoleküle gemäß SEQ ID No: 1, 3, 5, 7, 9, 11, 13, 15, 17, 19, 21, 23, 25, 27, 29, 31, 33, 35, 37, 39, 41, oder 43 kodierten Polypeptids bewirkt. Beispielsweise eine durch Tilling hergestellte und identifizierte Mutation.

Bevorzugt sind dabei monokotyledone Pflanzen, insbesondere solche die zu der Familie der Poaceae gehören, besonders bevorzugt sind Pflanzen ausgewählt aus den Pflanzengattungen Hordeum, Avena, Secale, Triticum, Sorghum, Zea, Saccharum und Oryza, ganz besonders bevorzugt Pflanzen ausgewählt aus den Arten Hordeum vulgare (Gerste), Triticum aestivum (Weizen), Triticum aestivum subsp.spelta (Dinkel), Triticale, Avena sative (Hafer), Secale cereale (Roggen), Sorghum bicolor (Hirse), Zea mays (Mais), Saccharum officinarum (Zuckerrohr) und Oryza sative (Reis).

Folglich betrifft die Erfindung in einer Ausführungsform eine Pflanze, insbesondere eine monokotyledone Pflanze, enthaltend eine erfindungsgemäße Nukleinsäuresequenz, welche eine Mutation enthält, die eine Erhöhung der Aktivität eines der durch die erfindungsgemäßen Nukleinsäuremoleküle kodierten Proteine in der Pflanze oder Teilen davon bewirkt. Beispielsweise betrifft die Mutation einen oder mehr Aminosäurereste, die in der in den Figuren gezeigten Consensussequenz als konserviert oder hochkonserviert markiert ist.

Ein weiterer Gegenstand der Erfindung betrifft folglich transgene Pflanzen, transformiert mit wenigstens
a) einer Nukleinsäuresequenz, die Nukleinsäuremoleküle gemäß SEQ ID No: 1, 3, 5, 7, 9, 11, 13, 15, 17, 19, 21, 23, 25, 27, 29, 31, 33, 35, 37, 39, 41, oder 43 umfasst, enthalten, die dazu komplementären Nukleinsäuresequenzen, oder die für funktionelle Äquivalente der Polypeptide gemäß SEQ ID No: 2, 4, 6, 8, 10, 12, 14, 16, 18, 20, 22, 24, 26, 28, 30, 32, 34, 36, 38, 40, 42, 44, 60, 61 oder 62 kodierenden Nukleinsäuremoleküle;
b) einer transgenen Expressionskassette, die eine der erfindungsgemäßen Nukleinsäuresequenzen umfaßt, oder einem erfindungsgemäßen Vektor, sowie Zellen, Zellkulturen, Gewebe, Teile - wie zum Beispiel bei pflanzlichen Organismen Blätter, Wurzeln usw.- oder Vermehrungsgut abgeleitet von solchen Organismen.

In einer Ausführungsform ist die erfindungsgemäße Pflanze oder die erfindungsgemäß verwendete Pflanze keine Arabidopsis thaliana.

Als "transgene Organismen" bevorzugte Wirts- oder Ausgangs-organismen sind vor allem Pflanzen gemäß der oben genannten Definition. In einer Ausführungsform ist der transgene Organismus eine reife Pflanze, Saatgut, Spross und Keimling, sowie davon abgeleitete Teile, Vermehrungsgut und Kulturen, zum Beispiel Zellkulturen. "Reife Pflanze" meint Pflanzen zu jedem beliebigen Entwicklungsstadium jenseits des Keimlings. "Keimling" meint eine junge, unreife Pflanze in einem frühen Entwicklungsstadium. Insbesondere als Wirtsorganismen bevorzugte Pflanzen sind Pflanzen, auf die der erfindungsgemäße Verfahren zum Erzielen einer Pathogenresistenz gemäß oben genannten Kriterien angewendet werden kann. In einer Ausführungsform ist die Pflanze eine monokotyle Pflanze wie z.B. Weizen, Hafer, Hirse, Gerste, Roggen, Mais, Reis, Buchweizen, Sorghum, Triticale, Dinkel oder Zuckerrohr, insbesondere ausgewählt aus den Arten Hordeum vulgare (Gerste), Triticum aestivum (Weizen), Triticum aestivum subsp.spelta (Dinkel), Triticale, Avena sative (Hafer), Secale cereale (Roggen), Sorghum bicolor (Hirse), Zea mays (Mais), Saccharum officinarum (Zuckerrohr) oder Oryza sativa (Reis).

Die Herstellung der transgenen Organismen kann mit den oben beschriebenen Verfahren zur Transformation oder Transfektion von Organismen realisiert werden.

Ein weiterer Gegenstand der Erfindung betrifft die erfindungsgemäß beschriebenen transgenen Pflanzen, die zusätzlich über eine erhöhte Bax Inhibitor 1 Aktivität verfügen, bevorzugt sind dabei Pflanzen, die eine erhöhte Bax Inhibitor 1 Aktivität in Mesophyllzellen oder Wurzelzellen aufweisen, besonders bevorzugt sind transgene Pflanzen die zu der Familie der Poaceae gehören und eine erhöhte Bax Inhibitor 1 Aktivität in Mesophyllzellen oder Wurzelzellen aufweisen, am meisten bevorzugt sind transgene Pflanzen ausgewählt aus den Pflanzengattungen Hordeum, Avena, Secale, Triticum, Sorghum, Zea, Saccharum und Oryza, am allermeisten bevorzugt sind die Pflanzenarten Hordeum vulgare (Gerste), Triticum aestivum (Weizen), Triticum aestivum subsp.spelta (Dinkel), Triticale, Avena sative (Hafer), Secale cereale (Roggen), Sorghum bicolor (Hirse), Zea mays (Mais), Saccharum officinarum (Zuckerrohr) und Oryza sative (Reis).

Ein weiterer Gegenstand der Erfindung betrifft die Verwendung der erfindungsgemäßen, transgenen Organismen und der von ihnen abgeleitete Zellen, Zellkulturen, Teile - wie zum Beispiel bei transgenen pflanzlichen Organismen Wurzeln, Blätter etc.-, und transgenes Vermehrungsgut wie Saaten oder Früchte, zur Herstellung von Nahrungs- oder Futtermitteln, Pharmazeutika oder Feinchemikalien.

In einer Ausführungsform betrifft die Erfindung zudem ein Verfahren zur rekombinanten Herstellung von Pharmazeutika oder Feinchemikalien in Wirtsorganismen wobei ein Wirtsorganismus oder ein Teil davon mit einer der oben beschriebenen Nukleinsäuremoleküle Expressionskassetten transformiert wird und diese Expressionskassette ein oder mehrere Strukturgene enthält, die für die gewünschte Feinchemikalie kodieren oder die Biosynthese der gewünschten Feinchemikalie katalysieren, der transformierte Wirtsorganismus gezüchtet wird und die gewünschte Feinchemikalie aus dem Züchtungsmedium isoliert wird. Dieses Verfahren ist für Feinchemikalien wie Enzyme, Vitamine, Aminosäuren, Zucker, Fettsäuren, natürliche und synthetische Geschmacks-, Aroma- und Farbstoffe breit anwendbar. Besonders bevorzugt ist die Produktion von Tocopherolen und Tocotrienolen sowie Carotinoiden. Die Züchtung der transformierten Wirtsorganismen sowie die Isolierung aus den Wirtsorganismen bzw. aus dem Züchtungsmedium erfolgt mit dem Fachmann bekannten Verfahren. Die Produktion von Pharmazeutika, wie zum Beispiel Antikörpern oder Vakkzinen ist beschrieben bei Hood E.E., Jilka J.M., Curr Opin Biotechnol. 10 (4), 382 (1999).; Ma J.K., Vine N.D., Curr. Top. Microbiol. Immunol. 236, 275 (1999).

Erfindungsgemäß kann die Expression eines Strukturgens natürlich auch unabhängig von der Ausführung des erfindungsgemäßen Verfahrens oder der Verwendung der erfindungsgemäßen Gegenstände erfolgen oder beeinflusst werden.

Die folgenden Beispiele dienen stellen Ausführungsformen der Erfindung dar und es ist nicht beabsichtigt, dass sie den Umfang der Erfindung, wie er im Rest der Beschreibung, der Figuren und Ansprüche enthalten ist, einschränken sollen.

### Sequenzen:

- 1.: SEQ ID NO:1 und 2: HvArm
- 2.: SEQ ID NO: 3 und 4: OS_1_XM_479734.1
- 3.: SEQ ID NO: 5 und 6 Os_2_XM_463544
- 4.: SEQ ID NO: 7 und 8 Os_3_AP003561
- 5.: SEQ ID NO:9 und 10 Os_4_XM_506432
- 6.: SEQ ID NO:11 und 12 NT_1_AY219234
- 7.: SEQ ID NO: 13 und 14 At_1_NM_127878
- 8.: SEQ ID NO: 15 und 16 At_2_AC004401
- 9.: SEQ ID NO: 17 und 18 At_3_BT020206
- 10.: SEQ ID NO:19 und 20 At_4_AB007645
- 11.: SEQ ID NO: 21 und 22 At_5_NM_115336 (At3g54790)
- 12.: SEQ ID NO:23 und 24 At_6_AK118613
- 13.: SEQ ID NO: 25 und 26 At_7_AL138650
- 14.: SEQ ID NO: 27 und 28 At_8_AL133314
- 15.: SEQ ID NO: 29 und 30 At_9_AC010870
- 16.: SEQ ID NO: 31 und 32 At_10_AY125543 (At3g01400)
- 17.: SEQ ID NO:33 und 34 At_11_AY087360
- 18.: SEQ ID NO: 35 und 36 At_12_AB016888
- 19.: SEQ ID NO: 37 und 38 At_13_AK175585
- 20.: SEQ ID NO: 39 und 40 At_14_AL049655
- 21.: SEQ ID NO: 41 und 42 At_15_AY096530 (At3g54850)
- 22.: SEQ ID NO:43 und 44 At_16_AK118730 (At4g16490)
- 23.: SEQ ID NO: 45 bis 59: Primer
- 24.: SEQ ID NO: 60, 61, 62: Consensussequenzen der Polynukleotid SEQ ID NO. aus 1. bis 22.
- 25.: SEQ ID NO: 63: 5'UTR in Kombination mit der Sequenz von HvArm
- 26.: SEQ ID NO: 64: 5'UTR von HvArm

Die Figuren zeigen:

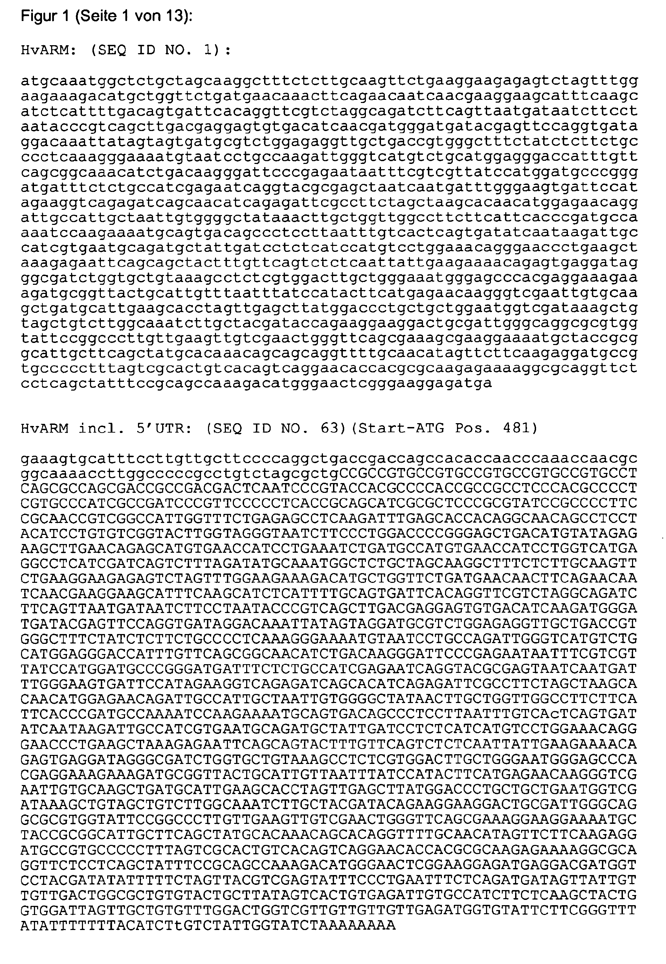

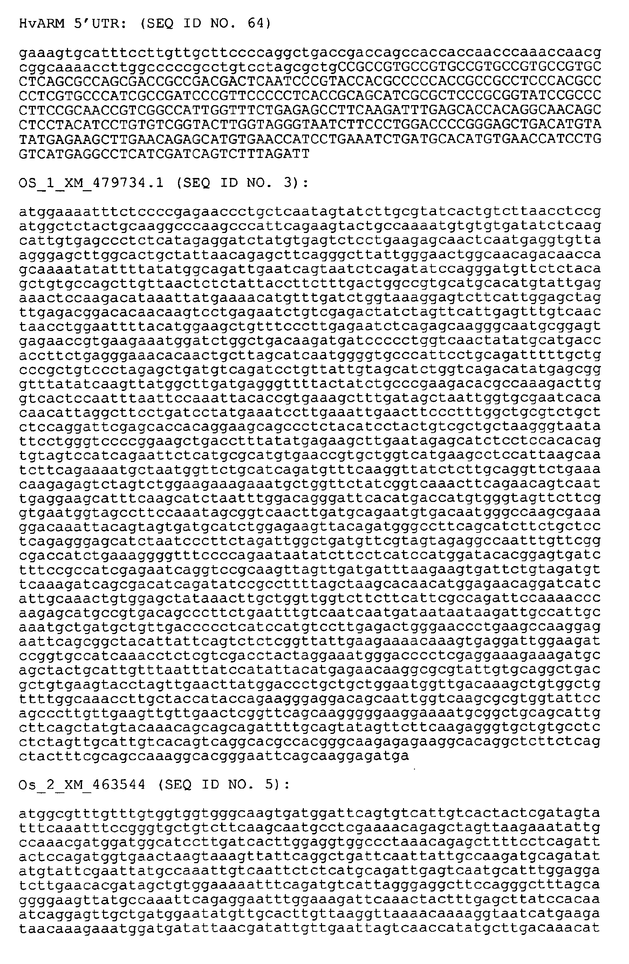

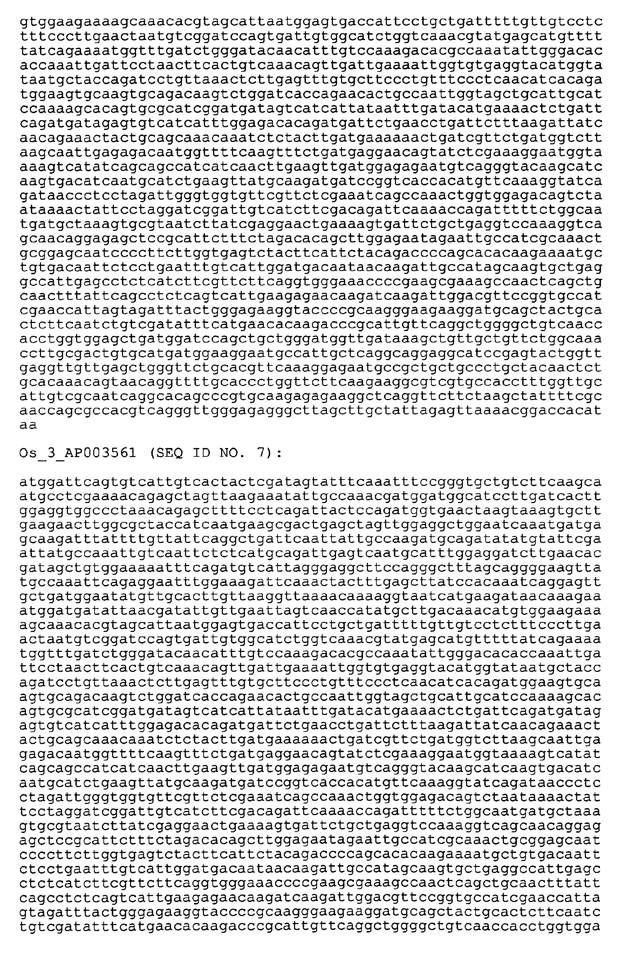

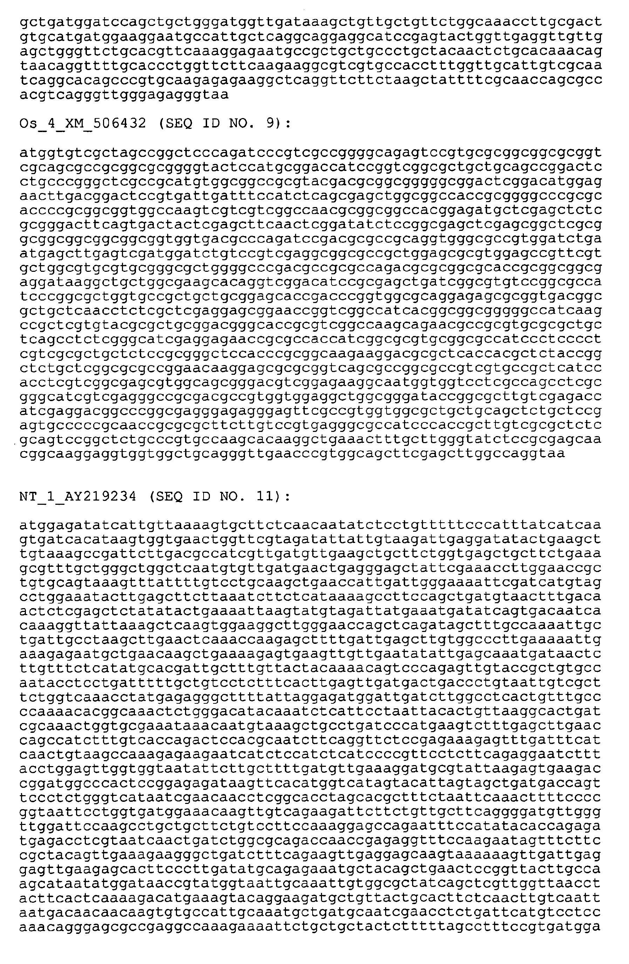

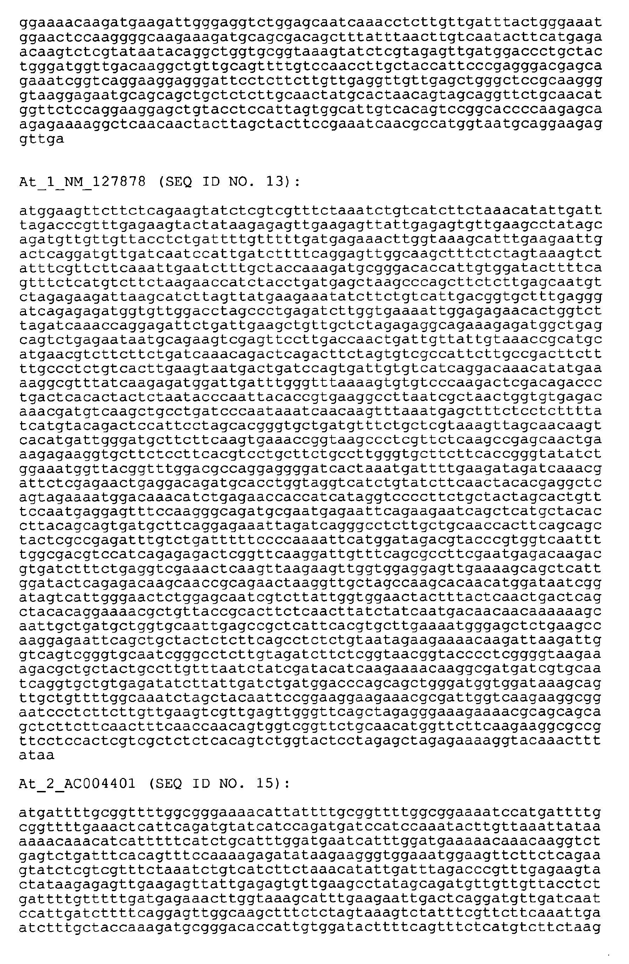

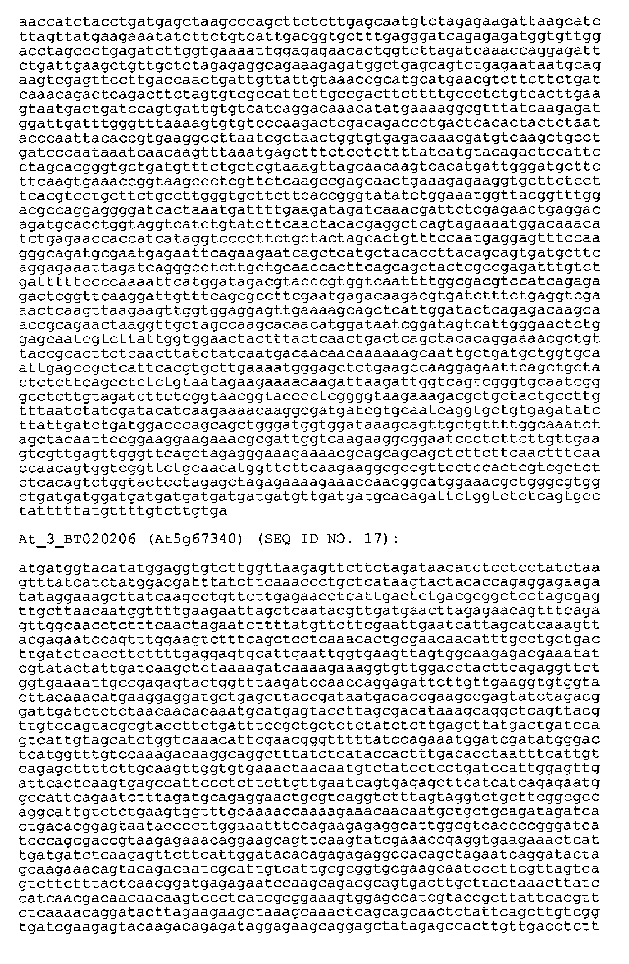

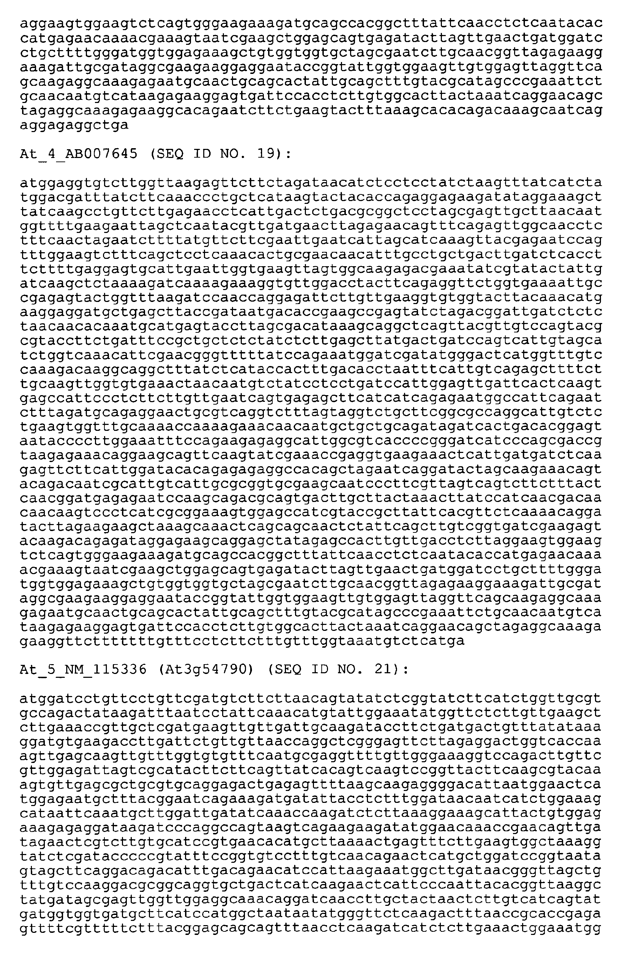

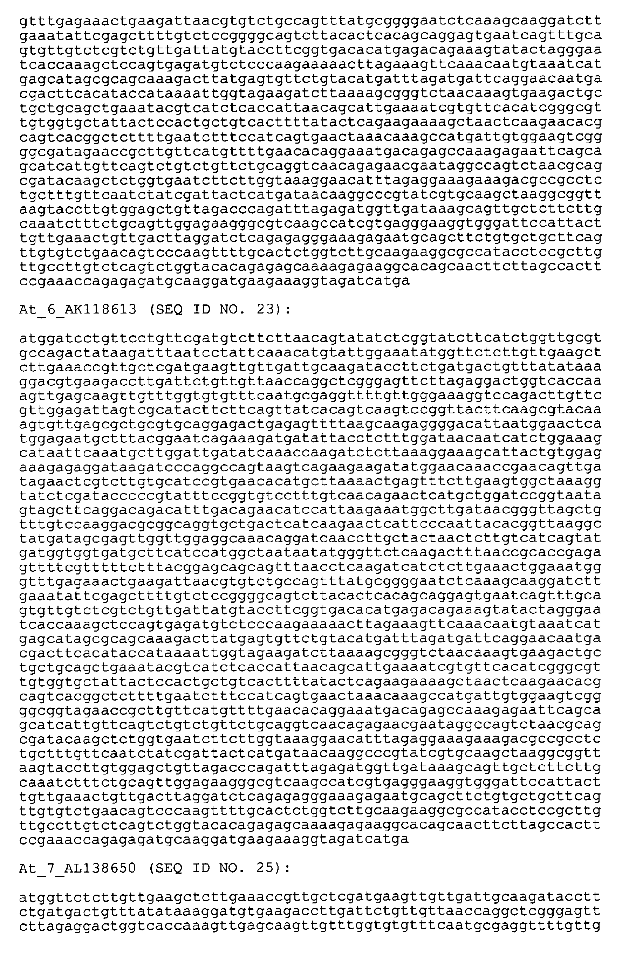

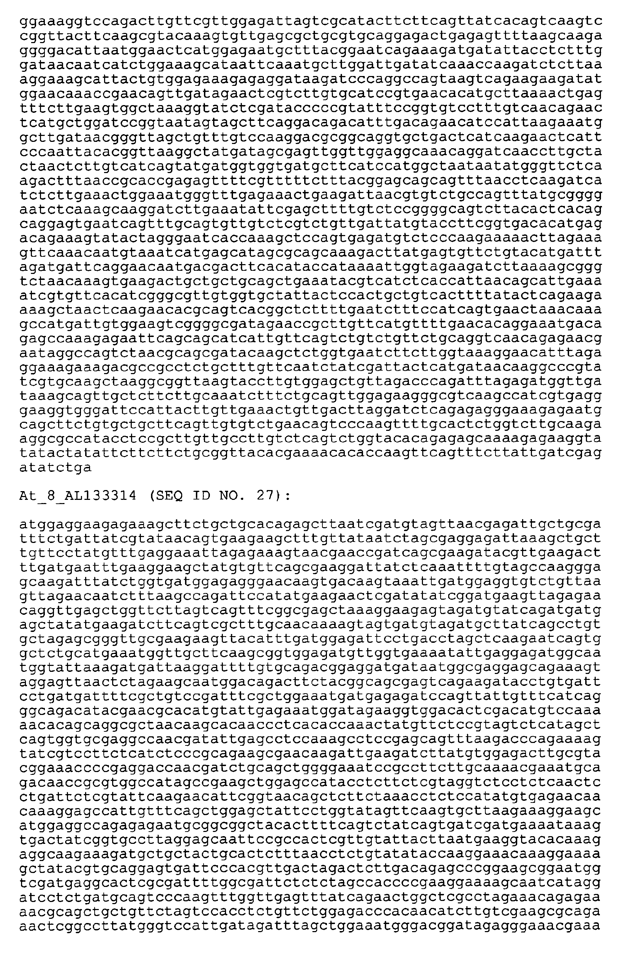

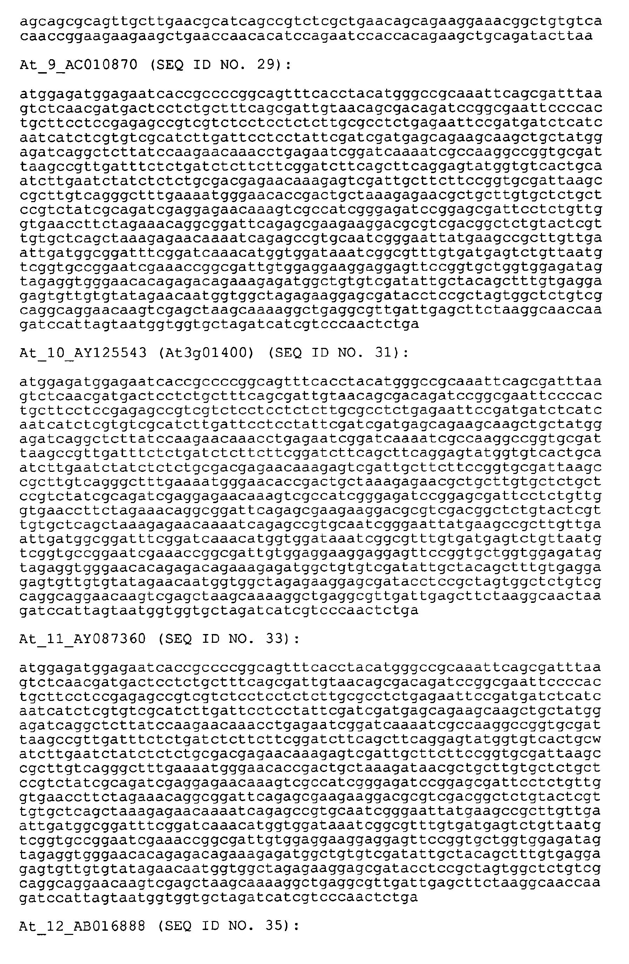

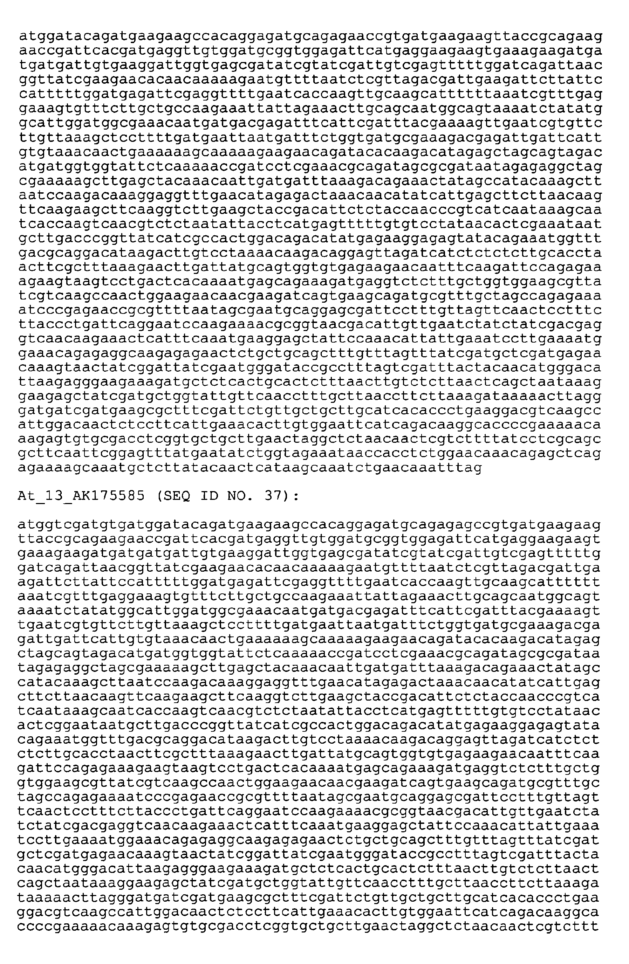

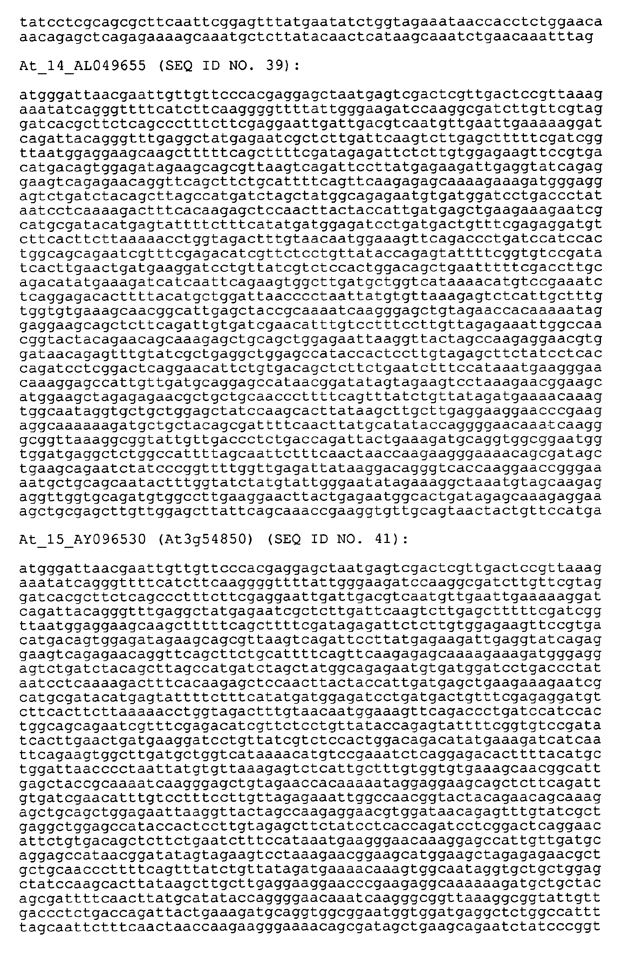

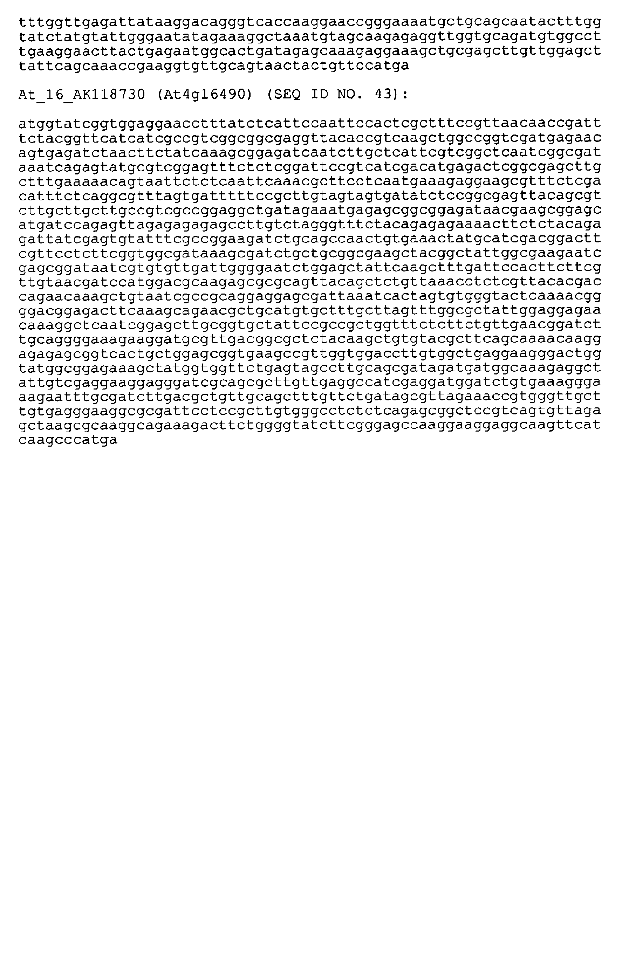

- Figur 1:: Nukleinsäuresequenzen von ARM1 aus Gerste, Reis, und Arabidopsis thaliana.

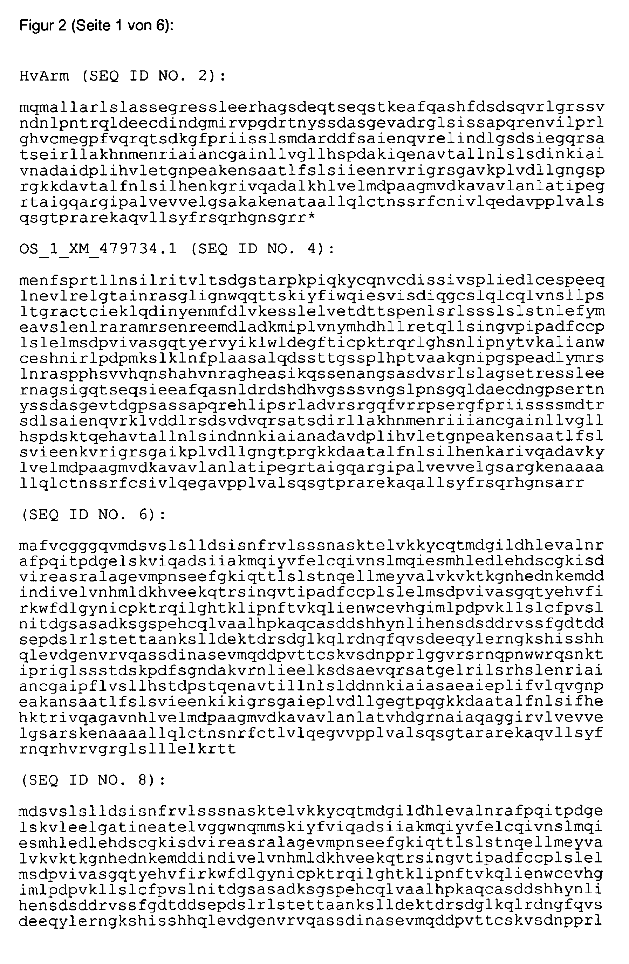

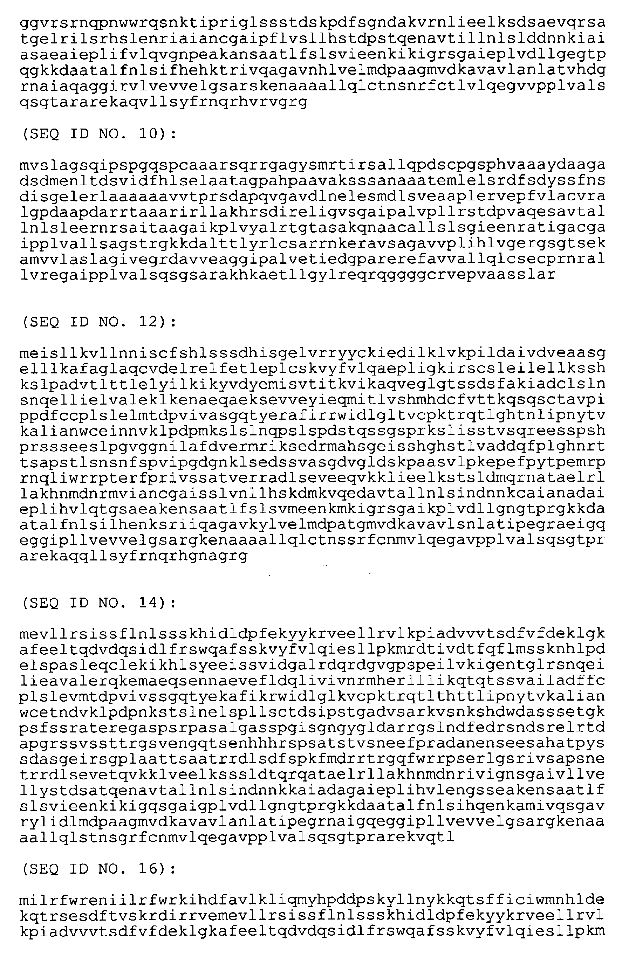

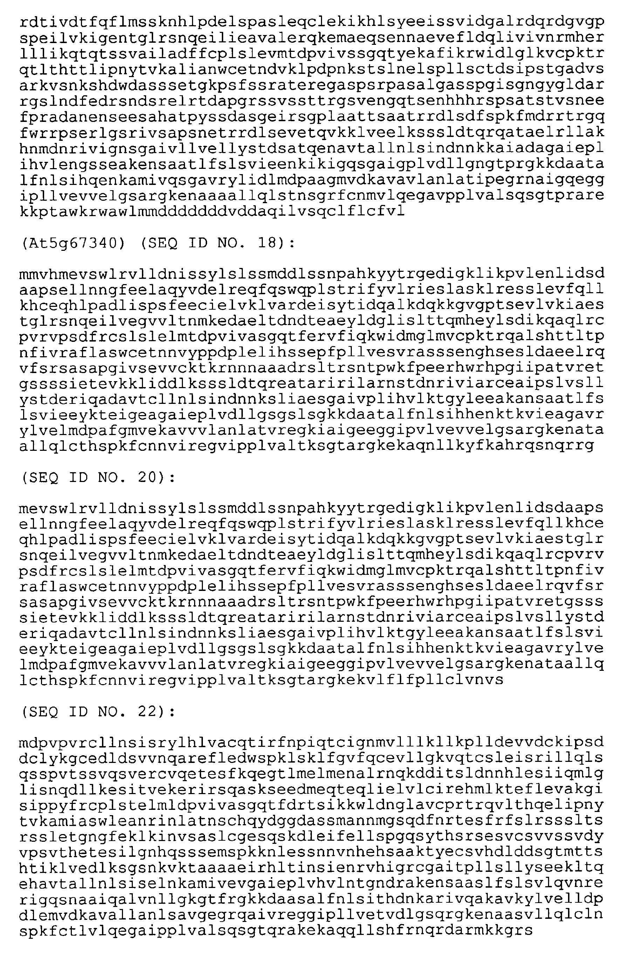

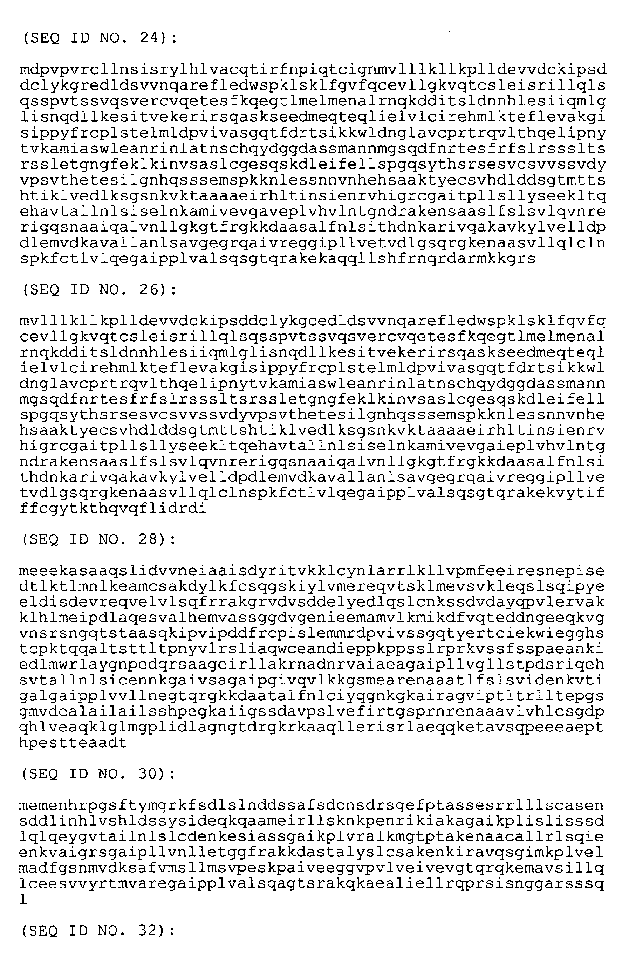

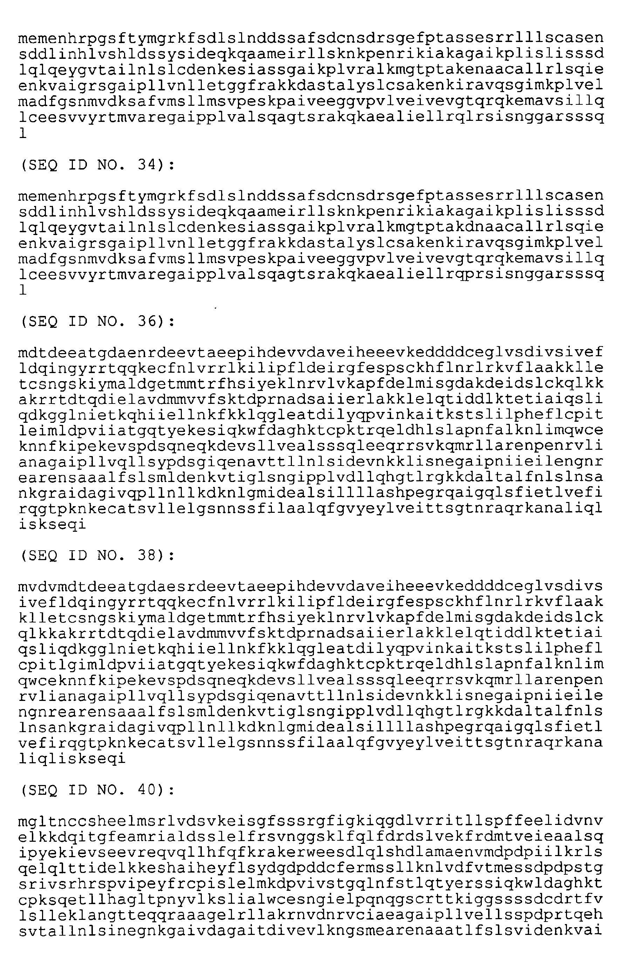

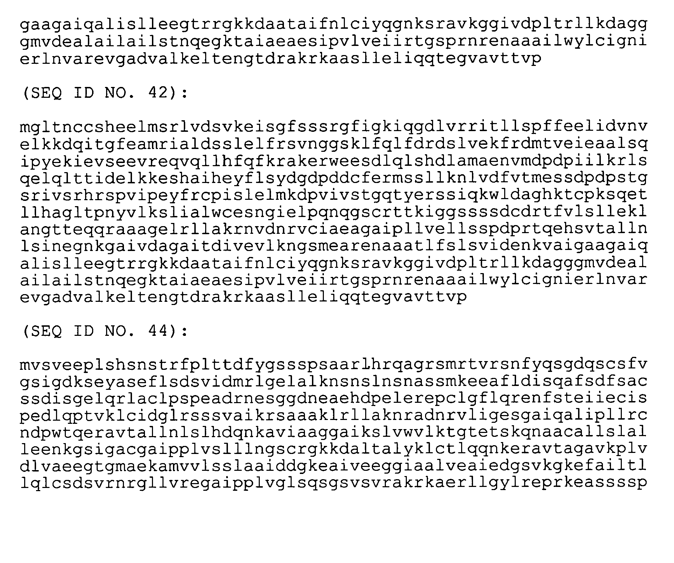

- Figur 2:: Polypeptidsequenzen von ARM1 aus Gerste, Reis, und Arabidopsis thaliana.

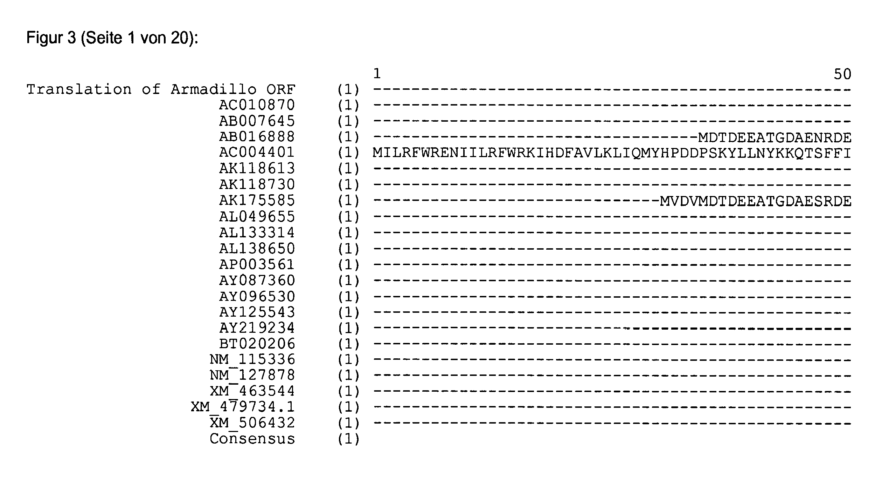

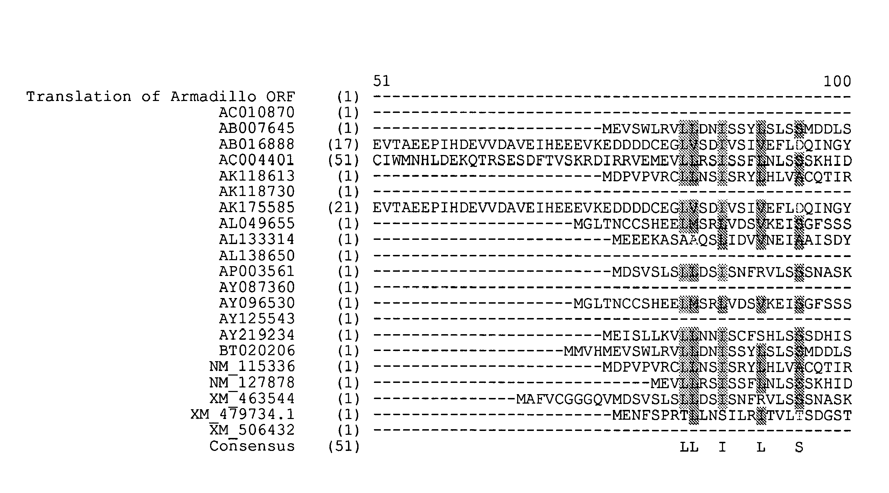

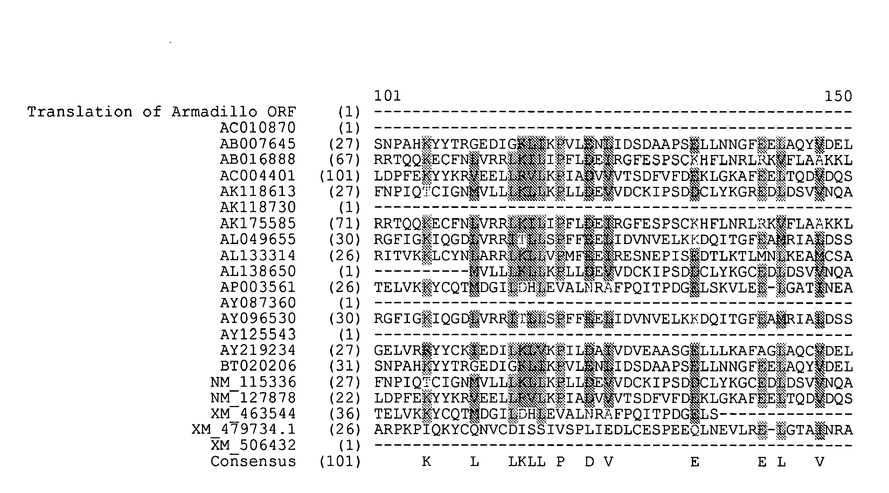

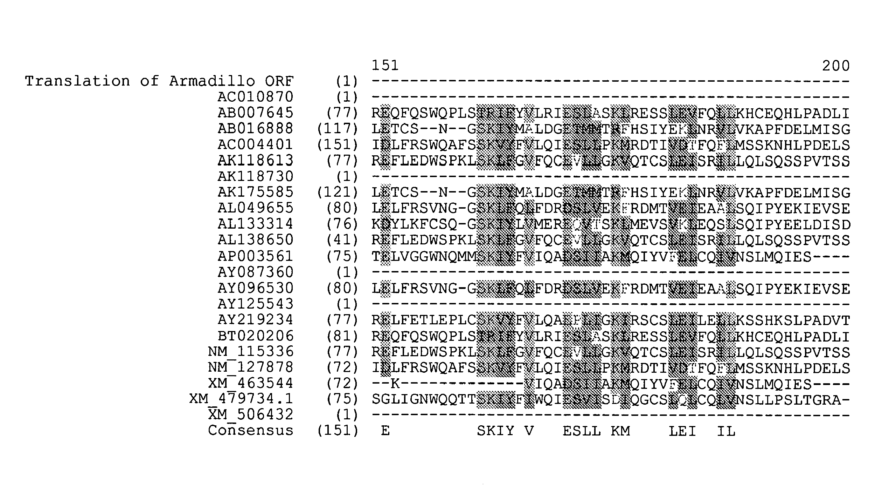

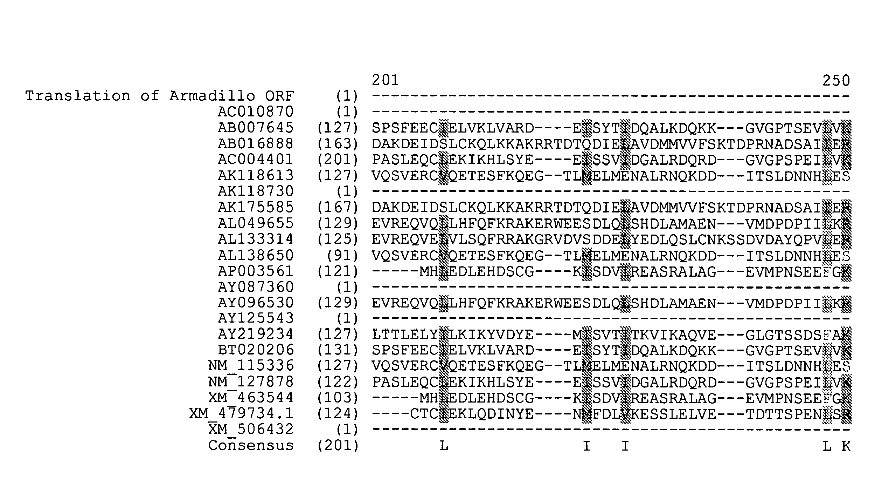

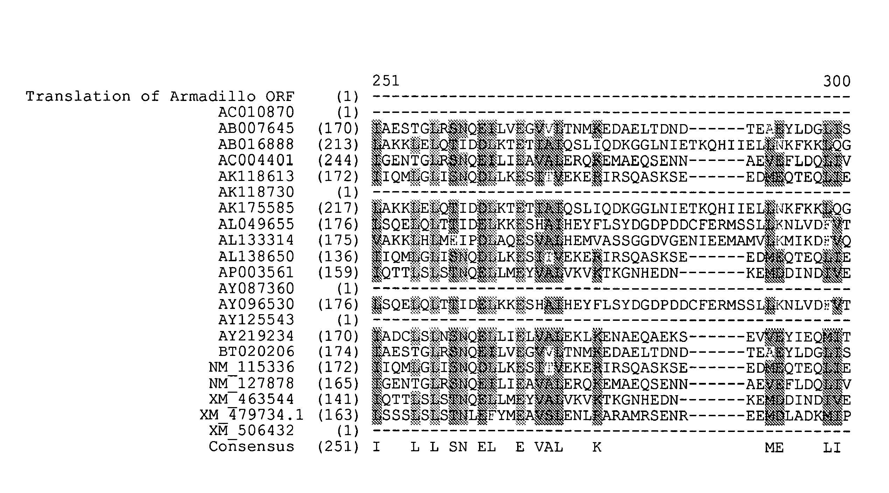

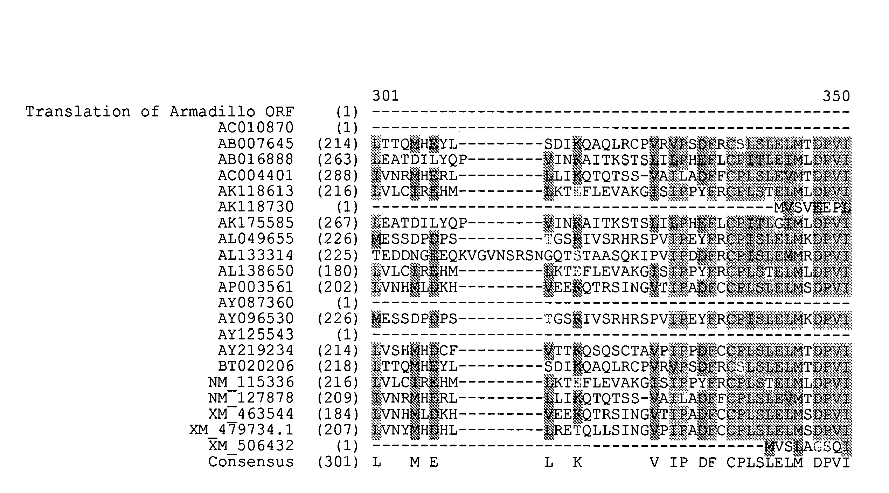

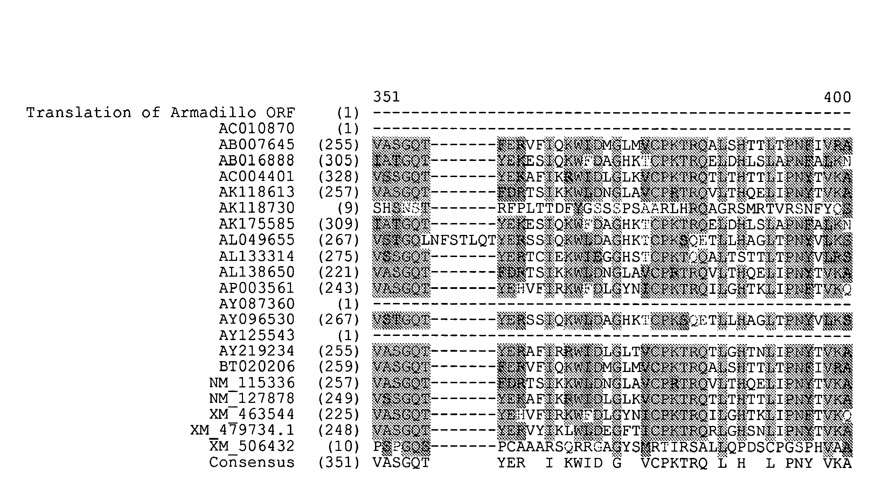

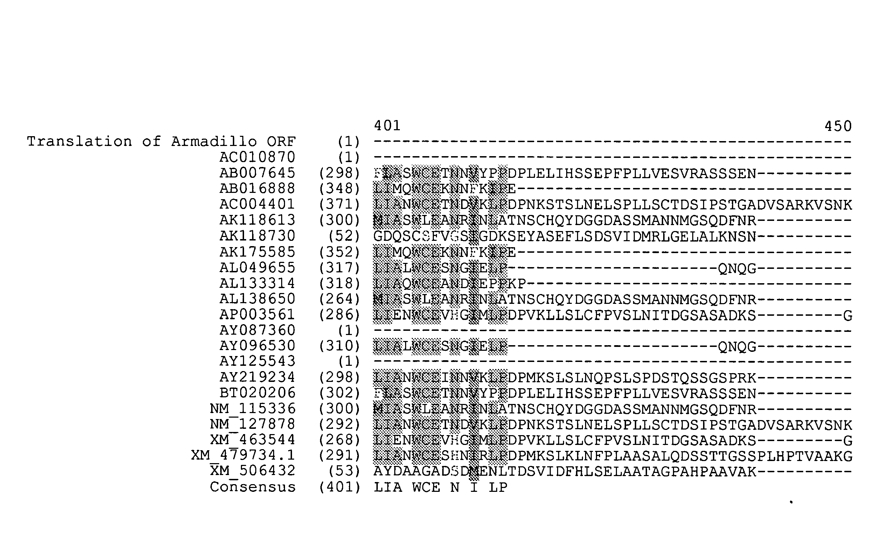

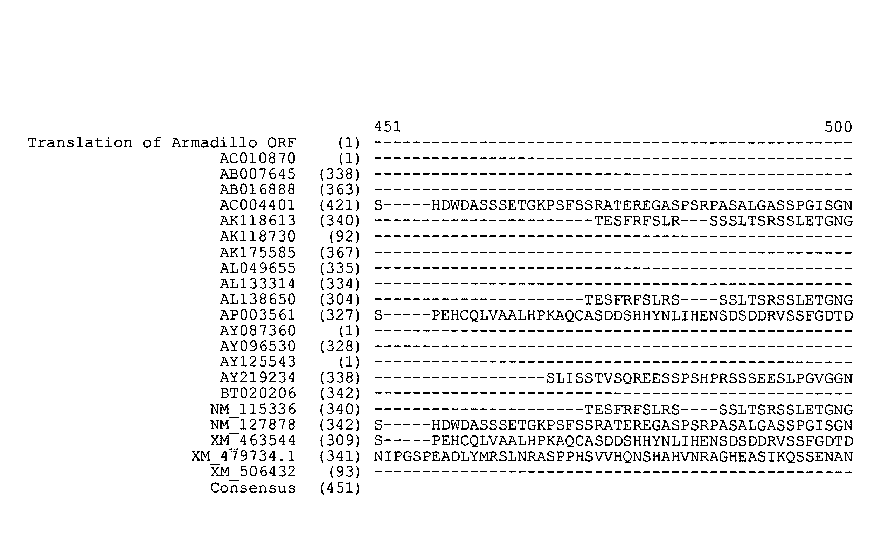

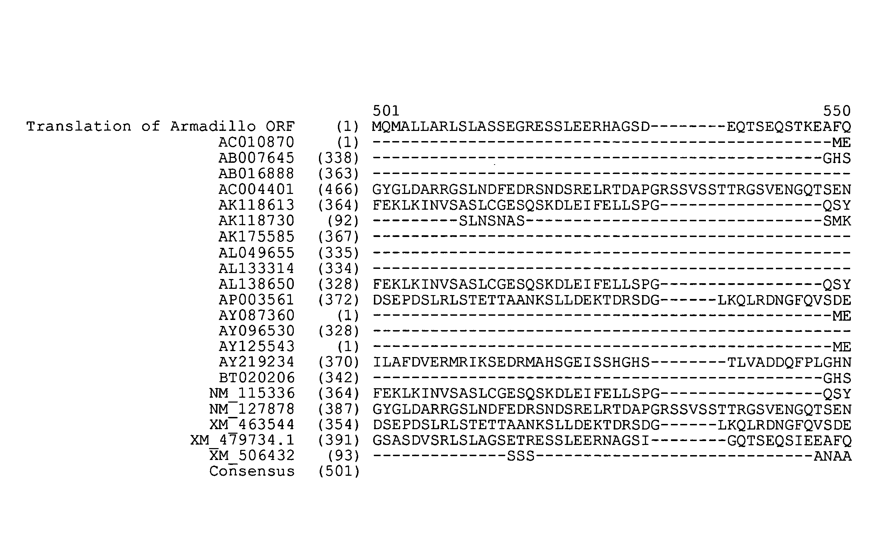

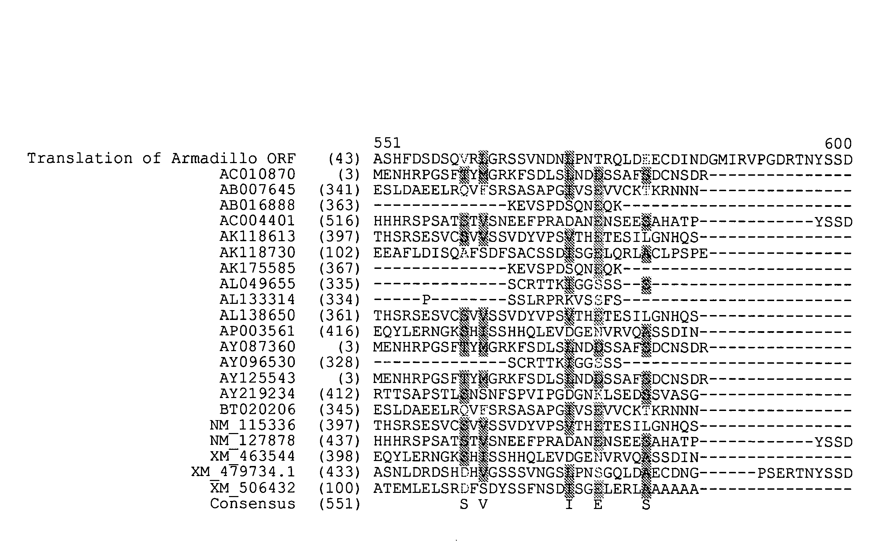

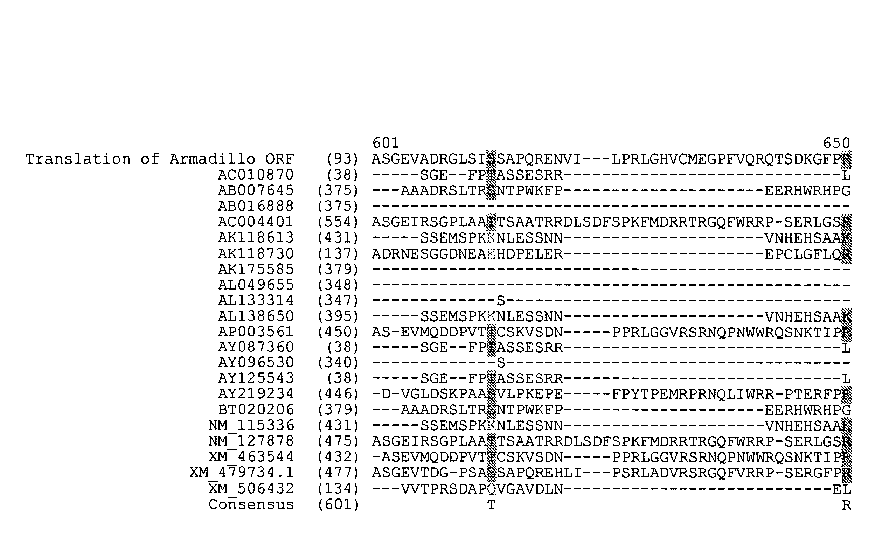

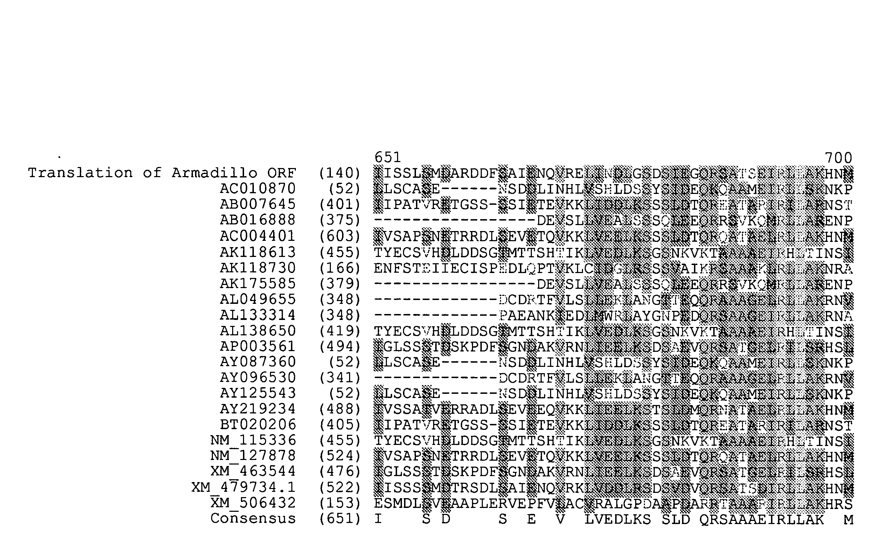

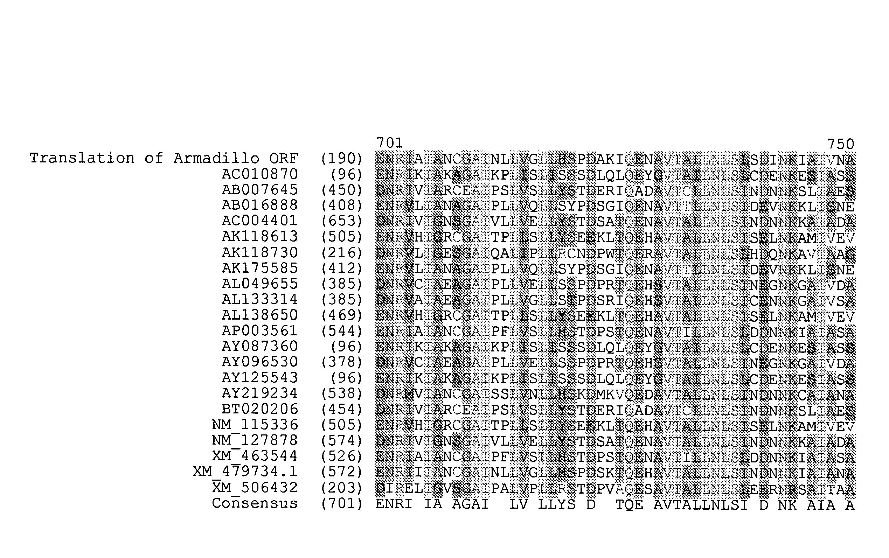

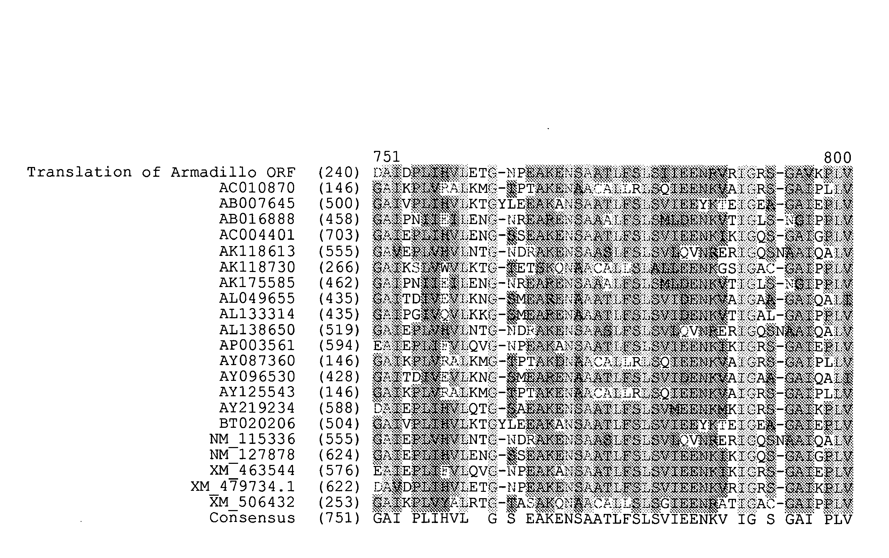

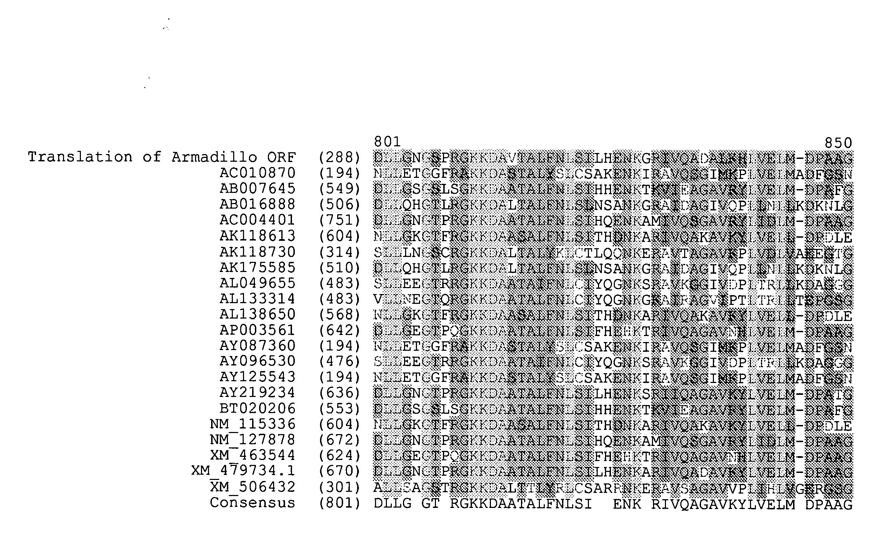

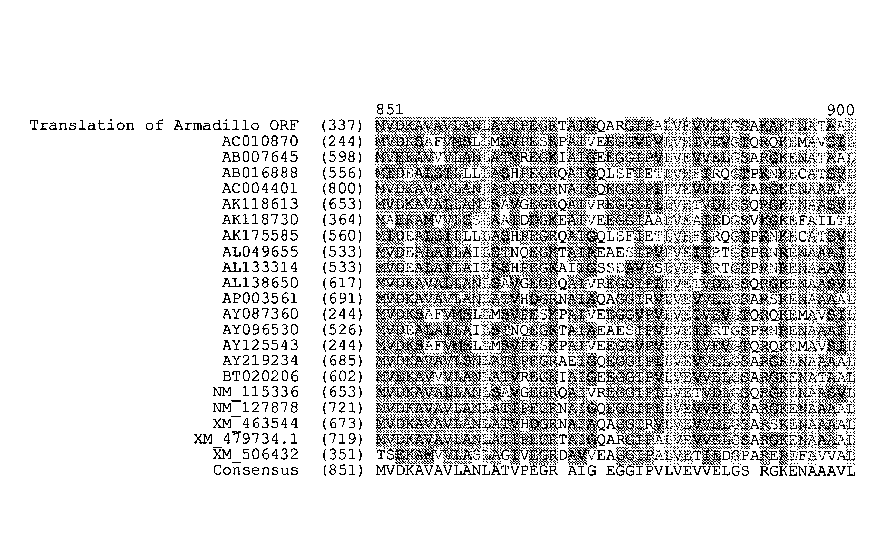

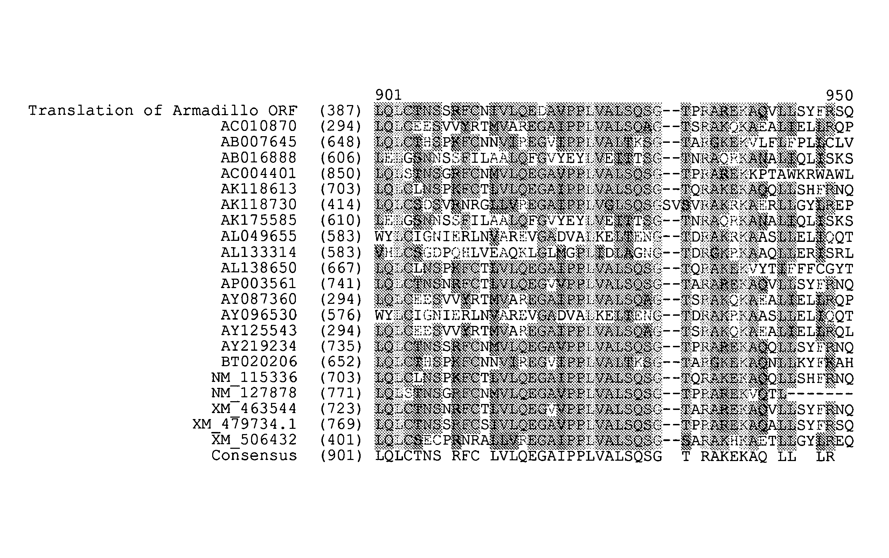

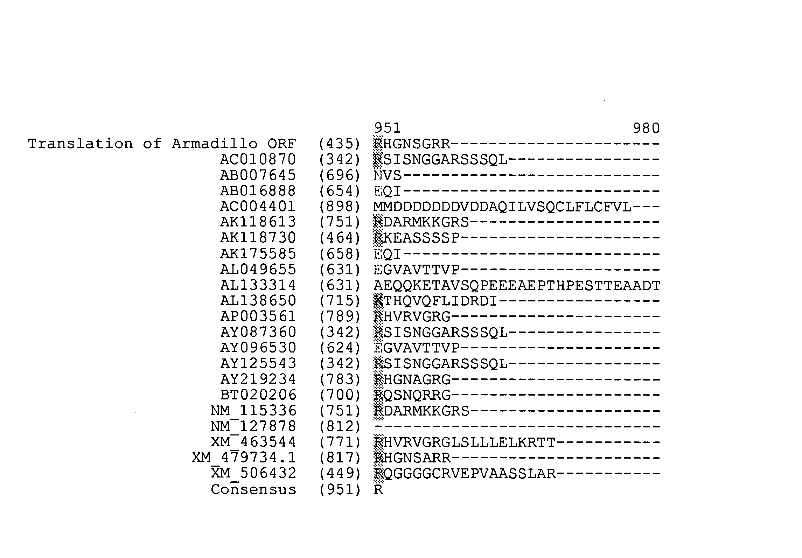

- Figur 3:: Sequenzvergleich von ARM1 Proteinsequenzen Polypeptiden aus Gerste, Reis, und Arabidopsis thaliana.
- Figur 4:: Erhöhung der Mehltauresistenz von Weizen durch Einbringen und Exprimieren von ARM-Repeat-Sequenzen

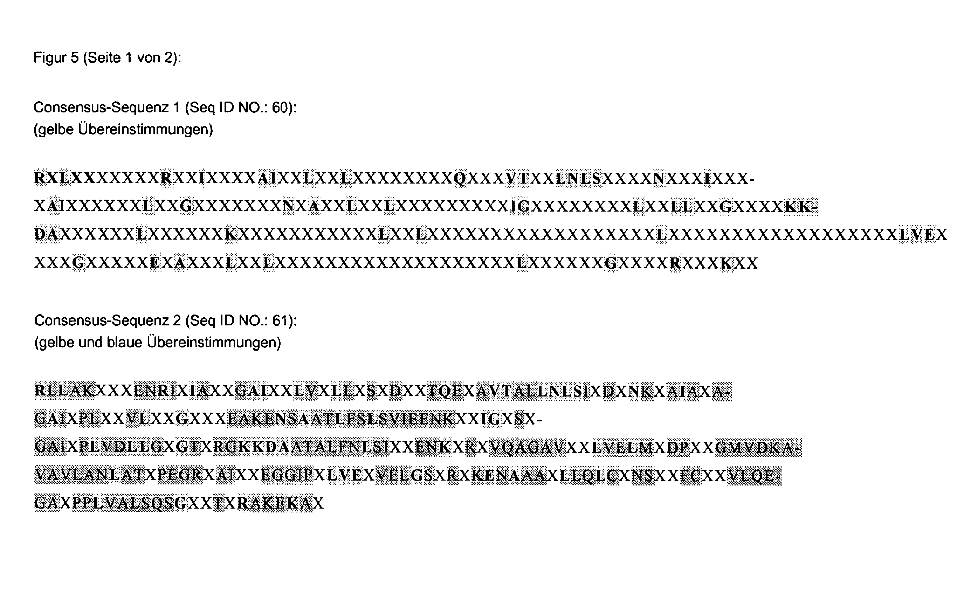

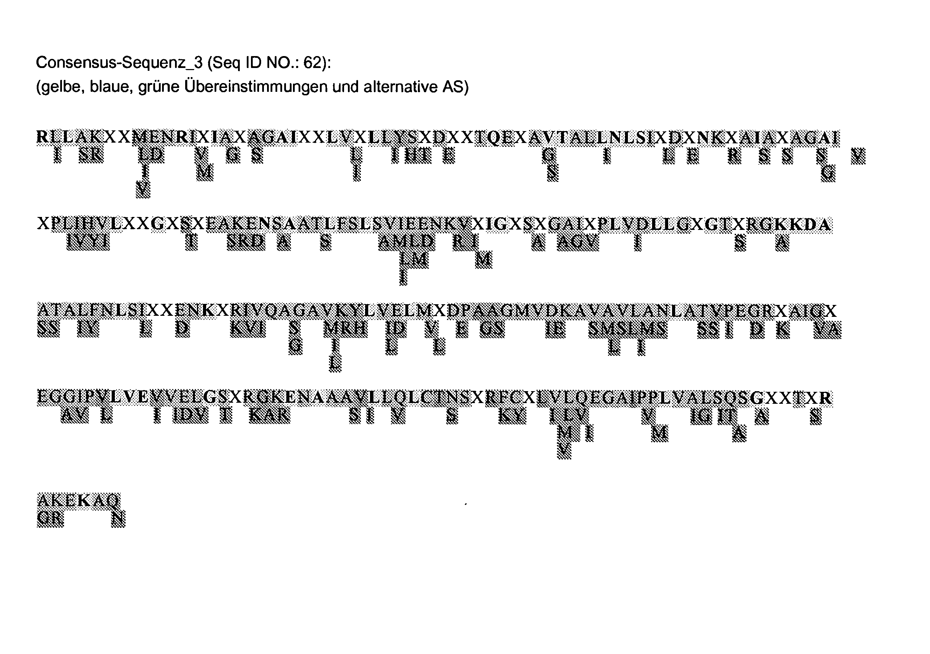

- Figur 5:: Consensussequenzen des Sequenzvergleich von ARM1 Proteinsequenzen Polypeptiden aus Gerste, Reis, und Arabidopsis thaliana.

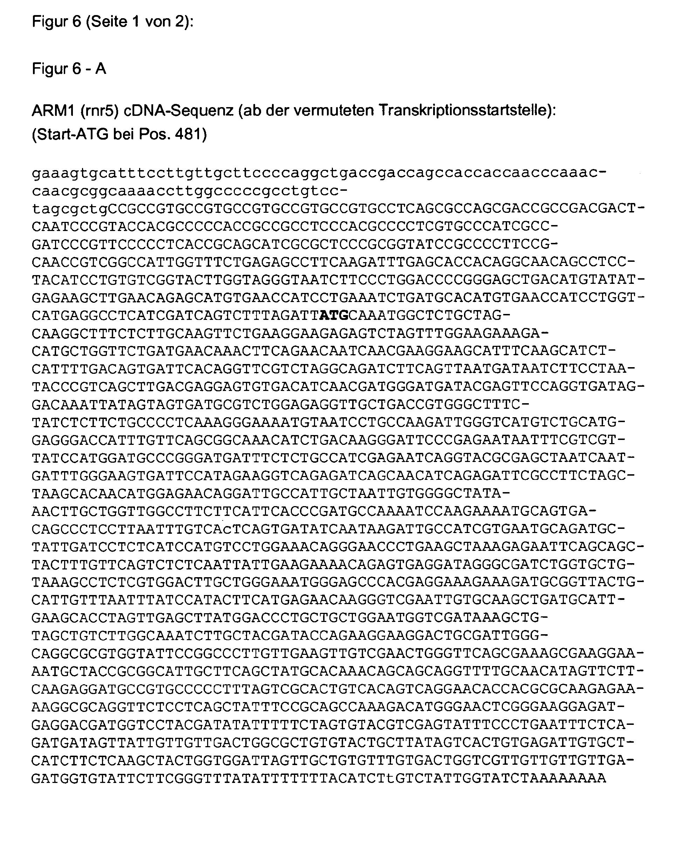

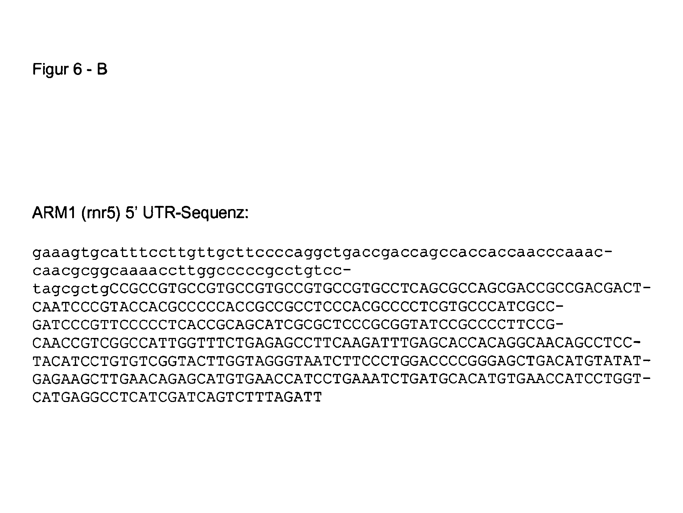

- Figur 6:: Nukleinsäuresequenz von ARM1 aus Gerste einschließlich der 5'-UTR-Region

### Beispiele

### Allgemeine Methoden:

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophorese, Reinigung von DNA-Fragmenten, Transfer von Nukleinsäuren auf Nitrozellulose und Nylonmembranen, Verknüpfen von DNA-Fragmenten, Transformation von E.coli Zellen, Anzucht von Bakterien, Vermehrung von Phagen und Sequenzanalyse rekombinanter DNA werden wie bei Sambrook et al., Cold Spring Harbor Laboratory Press (1989); ISBN 0-87969-309-6 beschrieben durchgeführt. Die Sequenzierung rekombinanter DNA-Moleküle erfolgt mit einem Laserfluoreszenz-DNA-Sequenzierer der Firma MWG-Licor nach der Methode von Sanger (Sanger et al., Proc Natl Acad Sci U.S.A. 74, 5463 (1977)).

### Beispiel 1: Pflanzen, Pathogene und Inokulation

Die Gerstensorte Golden Promise stammt von Patrick Schweizer, Institut für Pflanzengenetik und Kulturpflanzenforschung Gatersleben. Die Sorte Pallas und die rückgekreuzte Linie BClngrid-mlo5 wurden von Lisa Munk, Department of Plant Pathology, Royal Veterinary and Agriculturai University, Kopenhagen, Dänemark zur Verfügung gestellt. Ihre Herstellung ist beschrieben (Kølster P. et al., Crop Sci. 26, 903 (1986)).

Die Weizensorte Kanzler stammt ebenfalls von Patrick Schweizer, Institut für Pflanzengenetik und Kulturpflanzenforschung Gatersleben.

Das 12 bis 36 h im Dunkeln auf feuchtem Filterpapier vorgekeimte Gerste- oder Weizensaatgut wird, wenn nicht anders beschrieben, zu je 5 Körnern an den Rand eines Vierkanttopfes (8x8cm) in Fruhstorfer Erde vom Typ P ausgelegt, mit Erde bedeckt und regelmäßig mit Leitungswasser gegossen. Alle Pflanzen werden in Klimaschränken oder -kammern bei 16 bis 18°C, 50 bis 60 % relativer Luftfeuchte und einem 16-stündigen Licht / 8-stündigen Dunkelheitszyklus mit 3000 bzw. 5000 lux (50 bzw. 60 µmols⁻¹m⁻² Photonenflussdichte) 5 bis 8 Tage lang kultiviert und im Keimlingsstadium in den Versuchen verwendet. Bei Experimenten, in denen Applikationen an Primärblättern durchgeführt werden, sind diese vollständig entwickelt.

### Vor Durchführung der transienten Transfektionsexperimente werden die Pflanzen in Klimaschränken oder -kammern bei tagsüber 24°C, nachts 20°C, 50 bis 60 % relativer Luftfeuchte und einem 16stündigen Licht / 8stündigen Dunkelheitszyklus mit 30000 lux kultiviert.

Für die Inokulation von Gerstenpflanzen wird Echte Gerstenmehltau Blumeria graminis (DC) Speer f.sp. hordei Em. Marchal der Rasse A6 (Wiberg A. Hereditas 77, 89 (1974)) (BghA6) verwendet. Dieser wurde vom Institut für Biometrie, JLU Gießen bereitgestellt. Die Nachzucht des Inokulums erfolgt in Klimakammern zu den gleichen Bedingungen, wie sie oben für die Pflanzen beschrieben sind, durch Übertragung der Konidien von befallenem Pflanzenmaterial auf regelmäßig angezogene, 7 Tage alte Gerstenpflanzen cv. Golden Promise bei einer Dichte von 100 Konidia/mm².

Für die Inokulation von Weizenpflanzen wird der Echte Weizenmehltau Blumeria graminis (DC) Speer f. sp. tritici verwendet. Dieser Mehltau wurde von Feldpflanzen isoliert. Die Nachzucht des Inokulums erfolgt in Klimakammern zu den gleichen Bedingungen, wie sie oben für die Gerste- und Weizenpflanzen beschrieben sind, durch Übertragung der Konidien von befallenem Pflanzenmaterial auf regelmäßig angezogene, 7 Tage alte Pflanzen bei einer Dichte von 100 Konidia/mm².

### Beispiel 2: RNA-Extraktion

Gesamt RNA wird aus 8 bis 10 primären Blattsegmenten (Länge 5 cm) mittels "RNA Extraction Buffer" (AGS, Heidelberg, Germany) extrahiert.

Dazu werden zentrale Primärblattsegmente von 5 cm Länge geerntet und in flüssigem Stickstoff in Mörsern homogenisiert. Das Homogenisat wird bis zur RNA-Extraktion bei -70°C gelagert.

Aus dem tiefgefrorenen Blattmaterial wird mit Hilfe eines RNA-Extraktions-Kits (AGS, Heidelberg) Gesamt-RNA extrahiert. Dazu wird 200 mg des tiefgefrorenen Blattmaterials in einem Mikrozentrifugenröhrchen (2 mL) mit 1,7 mL RNA-Extraktionspuffer (AGS) überschichtet und sofort gut durchmischt. Nach Zugabe von 200 µL Chloroform wird erneut gut gemischt und bei Raumtemperatur 45 min auf einem Horizontalschüttler bei 200 U/min geschüttelt. Anschließend wird zur Phasentrennung 15 min bei 20000 g und 4°C zentrifugiert, die obere (wässrige) Phase in ein neues Mikrozentrifugenröhrchen überführt und die untere verworfen. Die wässrige Phase wird erneut mit 900 µL Chloroform gereinigt, indem 3-mal für 10 sec homogenisiert und erneut zentrifugiert (s.o.) und abgehoben wird. Zur Fällung der RNA wird dann 850 µL 2-Propanol hinzugegeben, homogenisiert und für 30 bis 60 min auf Eis gestellt. Im Anschluss daran wird für 20 min zentrifugiert (s.o), vorsichtig der Überstand dekantiert, 2 mL 70 %iges Ethanol (-20°C) hinzu pipettiert, durchmischt und erneut 10 min zentrifugiert. Der Überstand wird dann wiederum dekantiert und das Pelet vorsichtig mit einer Pipette von Flüssigkeitsresten befreit, bevor es an einem Reinluftarbeitsplatz im Reinluftstrom getrocknet wird. Danach wird die RNA in 50 µL DEPC-Wasser auf Eis gelöst, durchmischt und 5 min zentrifugiert (s.o.). 40 µl des Überstandes werden als RNA-Lösung in ein neues Mikrozentrifugenröhrchen überführt und bei -70°C gelagert.

Die Konzentration der RNA wird photometrisch bestimmt. Dazu wird die RNA-Lösung 1:99 (v/v) mit destilliertem Wasser verdünnt und die Extinktion (Photometer DU 7400, Beckman) bei 260 nm gemessen (E₂₆₀ₙₘ = 1 bei 40 µg RNA/mL). Gemäß der errechneten RNA-Gehalte werden die Konzentrationen der RNA-Lösungen anschließend mit DEPC-Wasser auf 1 µg/µL angeglichen und im Agarosegel überprüft.

Zur Überprüfung der RNA-Konzentrationen im horizontalen Agarosegel (1 % Agarose in 1 x MOPS-Puffer mit 0,2 µg/mL Ethidiumbromid) wird 1 µL RNA-Lösung mit 1 µL 10 x MOPS, 1 µL Farbmarker und 7 µL DEPC-Wasser versetzt, nach Ihrer Größe bei 120 V Spannung im Gel in 1 x MOPS-Laufpuffer über 1,5 h aufgetrennt und unter UV-Licht fotographiert. Eventuelle Konzentrationsunterschiede der RNA-Extrakte werden mit DEPC-Wasser ausgeglichen und die Anpassung erneut im Gel überprüft.

### Beispiel 3: Klonierung der HvARM-cDNA-Sequenz aus Gerste

Die zur Isolation der Armadillo cDNA, ihrer Klonierung, Sequenzierung benötigten cDNA Fragmente wurden mittles RT-PCR unter Verwendung des GeneRacer Kit (Fa. Invitrogen Life Technologies) erhalten. Dazu wurde Gesamt-RNA aus Gerstenepidermis als Matrize verwendet. Die RNA wurde aus Epidermiszellen von Gerste Ingrid + Bgt 12h und 24h nach Infektion isoliert.

Die cDNA-Sequenz von HvArm wurde mittels der RACE-Technologie unter Verwendung des "GeneRacer Kit" (INVITROGENE Life Technologies) verlängert. Dazu wurden 4000 ng GesamtmRNA, 1 µL 10xClP-Puffer, 10 Units RNAse-Inhibitor, 10 Units CIP ("calf intestinal phosphatase") und DEPC-behandeltes Wasser bis zu einem Gesamtvolumen von 10 µL für 1 h bei 50°C behandelt. Zur Präzipitation der RNA wurden weitere 90 µL DEPC-Wasser und 100 µL Phenol:Chloroform hinzugegeben und intensiv für ca. 30 sek durchmischt. Nach 5 min Zentrifugation bei 20.000 g wurde die obere Phase mit 2 µl 10 mg/ml Mussel Glycogen, 10 µl 3 M Natriumacetat (pH 5,2) in einem neuen Mikroreaktionsgefäß versetzt. 220 µl 95 % Ethanol wurden hinzugegeben und die Mischung auf Eis inkubiert. Anschließend wurde die RNA durch Zentrifugation für 20 min bei 20.000 g und 4°C präzipitiert. Der Überstand wurde verworfen, 500 µl 75 % Ethanol hinzugegeben, kurz gevortext und wieder für 2 min zentrifugiert (20000 g). Der Überstand wurde wiederum verworfen, das Präzipitat für 2 min bei Raumtemperatur an der Luft getrocknet und anschließend in 6 µl DEPC-Wasser suspendiert. mRNA CAP-Strukturen wurden durch Zugage von 1 µl 10xTAP Puffer, 10 Units RNAsin und 1 Unit TAP ("tobacco acid pyrophosphatase") entfernt. Die Mischung wurde für 1 h bei 37°C inkubiert und anschließend auf Eis gekühlt. Die RNA wurde wiederum - wie oben beschrieben - präzipitiert und in ein Reaktionsgefäß mit 0,25 µg GeneRacer Oligonukleotid-Primer überführt. Der Oligonukleotid-Primer wurde in der RNA-Lösung resuspendiert, die Mischung für 5 min bei 70°C inkubiert und dann auf Eis gekühlt. 1 µl 10xLigasepuffer, 10 mM ATP, 1 Unit RNAsin und 5 Units T4 RNA-Ligase wurden hinzugegeben und der Reaktionsansatz für 1 h bei 37°C inkubiert. Die RNA wurde wiederum - wie oben beschrieben - präzipitiert und in 7 µl DEPC-Wasser resuspendiert. 10 pMol GeneRacer Oligo-dT Primer und 2 µL jeder dNTP-Lösung (25 mM) wurden zu der RNA gegeben, für 10 min auf 70°C erhitzt und wieder auf Eis gekühlt. Anschließend wurde ein Mix aus 2 µL 10xRT buffer, 4µl 25mM MgCl₂, 2µl 0,1 M DTT, 5U (1 µl) Superscriptlll Transkriptase (200 U/µl) und 1µl RNAse Out (40 U/µl) hinzugegeben, die Reaktionslösung für 50 min bei 50°C inkubiert und anschließend für 5 min bei 85°C inaktiviert. Nach einer Inkubation von 20 min bei 37°C mit 1µl RNAse H (2 U/µl) wurde die so hergestellte Erststrang-cDNA bei -20°C gelagert.

Für die RT-PCR wurden folgende Primer verwendet:
GeneRacer Oligo-dT-Primer (Fa. Invitrogen Life Technologies): GCTGTCAACGATACGCTACGTAACGGCATGACAGTG (T)18 (Seq ID No.: 45)

Für die Reaktion (20 µL-Ansatz) wurden je 4000 ng Gesamt-RNA, 10 mM dNTPs, 50µM GeneRacer Oligo-dT-Primer (Fa. Invitrogen Life Technologies), 1 µl RNase-Inhibitor und 1 µl Enzymmix in 1 x RT-Puffer (GeneRacer Kit Invitrogen) eingesetzt.

Die Reaktion wurde für 50 Minuten bei 50°C inkubiert.

Zur Amplifikation der 5'-cDNA-Enden wurden nachfolgende Primer verwendet:
MWG 1:
   5'GCAGACATGACCCAATCTTGGCAGG 3' (Seq ID No.: 46)
GR 5'Primer (Invitrogen):
   5'cgactggagcacgaggacactga 3' (Seq ID No.: 47)
MWG 2:
   5'CCACGGTCAGCAACCTCTCCAGACG 3' (Seq ID No.: 48)
GeneRacer 5'nested Primer (Invitrogen):
   5'ggacactgacatggactgaaggagta 3' (Seq ID No.: 49)
MWG 3:
   5' cagatgatagttattgttgttgactgg 3' (Seq ID No.: 50)
GR 3'Primer (Invitrogen):
   5' GCTGTCAACGATACGCTACGTAACG 3' (Seq ID No.: 51)
MWG 4:
   5'ctcatcttctcaagctactggtgg 3' (Seq ID No.: 52)
GeneRacer 3'nested Primer (Invitrogen):
   5'CGCTACGTAACGGCATGACAGTG 3' (Seq ID No.: 53)

Der Ansatz (Gesamtvolumen 50 µL) hatte nachfolgende Zusammensetzung:
4 µl MWG1 (10 pmol/µL)
4,5 µl 5'Gene Racer (10 pmol/µL)
5 µl 10x Puffer Roche
1,5 µl 10 mM dNTPs
1 µl cDNA
1 µl Taq (Roche)
33 µl H₂O

Folgendes Temperaturprogramm wurde verwendet (GeneAmp PCR System 9700; Applied Bio-systems):

| | |
|---|---|
| | 94°C 2 min Denaturierung |
| 5 Zyklen mit | 94°C 30 sek (Denaturierung) |
| | 72°C 2 min (Extension) |
| 5 Zyklen mit | 94°C 30 sek (Denaturierung) |
| | 70°C 2 min (Extension) |
| 30 Zyklen mit | 94°C 30 sek (Denaturierung) |
| | 65°C 30 sek (Annealing) |
| | 68°C 2 min (Extension) |
| | 68°C 7 min abschließende Extension |
| | 4°C Kühlung bis zu Weiterverarbeitung |

Die PCR ergab kein Produkt. Davon ausgehend wurde eine "nested"-RACE mit MWG2, dem Armadillo-spezifischen Oligonukleotidprimer und dem "GeneRacer Nested 5'Primer" durchgeführt:

| | |
|---|---|
| | 94°C 2 min Denaturierung |
| 30 Zyklen mit | 94°C 30 sek (Denaturierung) |
| | 65°C 30 sek (Annealing) |
| | 68°C 2 min (Extension) |
| | 68°C 7 min abschließende Extension |
| | 4°C Kühlung bis zur Weiterverarbeitung |

Die PCR ergab ein Produkt von ca. 850bp. Das erhaltene PCR-Produkt wurde über ein 1 % Agarosegel isoliert, aus dem Gel extrahiert und in pCR4-Topo (Fa. Invitrogen Life Technologies) mittels T-Überhang-Ligation kloniert und sequenziert. Die unter SEQ ID NO: wiedergegebene Sequenz ist also identisch mit der Armadillo-Sequenz aus Gerste.

Zur Amplifikation der Volllängensequenz von HvArm wurden nachfolgende Primer verwendet:
MWG 29:
   5'atatgcaaatggctctgctag 3' (Seq ID No.: 54)
MWG 30:
   5'TATCATCTCCTTCCCGAGTTC 3' (Seq ID No.: 55)

Der Ansatz (Gesamtvolumen 50µL) hatte nachfolgende Zusammensetzung:
4 µl MWG29 (10 pmol/µL)
4 µl MWG30 (10 pmol/µL)
5 µl 10x Pfu Ultra Puffer (Stratagene)
1,5 µl 10mM dNTPs
1 µl cDNA
1 µl Pfu Ultra (Stratagene)
33 µl H₂O

Folgendes Temperaturprogramm wurde verwendet (GeneAmp PCR System 9700; Applied Biosystems):

| | |
|---|---|
| | 94°C 2 min Denaturierung |
| 30 Zyklen mit | 94°C 30 sek (Denaturierung) |
| | 55°C 30 sek (Annealing) |
| | 72°C 1,5 min (Extension) |
| | 72°C 7min abschließende Extension |
| | 4°C Kühlung bis zu Weiterverarbeitung |

Die PCR ergab ein Produkt von 1326bp. Das erhaltene PCR-Produkt wurde über ein 1 % Agarosegel isoliert, aus dem Gel extrahiert und in pCR4-Topo (Fa. Invitrogen Life Technologies) mittels T-Überhang-Ligation kloniert und sequenziert. Die unter SEQ ID NO: wiedergegebene Sequenz ist also identisch mit der Armadillo-Sequenz aus Gerste.

### Beispiel 4: Klonierung der genomischen Volllängensequenz von HvARM:

### 1) BAC-Screening zur Identifizierung des Klones, der die erfindungsmässige Sequenz enthält.

Für die Identifizierung des Genes kodierend für die erfindungsmässige Sequenz in Gerste wurden DNA-pools aus einer Gersten-BAC-Bibliothek (Yu et al., TAG 101,1093 (2000)) verwendet. BAC Klone, welche die erfindungsmässige Sequenz tragen, wurden mittels PCR unter Verwendung der Primer 5' TAA TGA TAA TCT TCC TAA TAC CCG TCA G und 5' CCT TTG AGG GGC AGA AGA GAT AG identifiziert. Dabei wurden zwei überlappende BAC-Klone Nr. 705A01 sowie Nr. 581 D24 identifiziert, welche das identitische HvARM-Gen enthielten.

Identifizierte Einzelklone wurden für die Gen- und Promotoridentifizierung in zwei Stufen subkloniert. Zunächst wurde die BAC-DNA eines Einzelklones mittels Qiagen-Säule isoliert (Maxi-Kit; Qiagen; Isolierung nach Herstellerprotokoll). Durch Scherung (Hydroshear: Genomic Solutions) wurden von dieser BAC-DNA 5 - 10 kbp Fragmente erzeugt und die entstandenen Enden mit Klenow zu glatten Enden aufgefüllt (Reaktion nach Protokoll des Herstellers). Die Selektion der Fragmentlängen erfolgte über ein 0,8%iges Agarosegel in 0,5 % TBE. Aus dem Gel wurde der entsprechende Fragmentlängenbereich herausgeschnitten und die DNA aus dem Agarosegel mit Hilfe von Qiagen Gel Extraction Kit eluiert (Elution nach Protokoll des Herstellers). Die eluierten 5-10 kbp Fragmente werden in einen mit EcoRV linearisierten pBluescript II SK(-) Vektor mit glatten dephosphorylierten Enden ligiert (Restriktion und Dephosphorylierung entsprechend den Herstellerangaben) und in hochkompetente E.coli Zellen chemisch-thermisch transformiert. Anschließend werden die Transformanten mit Hilfe eines Picking-Roboters (Qpick, Genetix) willkürlich geordnet und in Mikrotiterplatten mit LB-Medium überführt.

Mittels PCR wurde dasjenige Subfragment ausgewählt, welche das interessierende Gen trägt und die Länge des potenziellen 5'-upstream Bereichs maximiert. Das gewählte Subfragment wurde erneut in 1-2 kbp Fragmente geschert, ligiert, transformiert und die Klone in Microtiterplatten gelagert (s.o.). Von den gepickten Klonen wurden 96 Kolonien willkürlich ausgewählt und mit dem TempliPhi Protokoll nach Herstellerprotokoll sequenziert. Die Sequenzen wurden assembliert. Die erhaltene Sequenzinformation wurde für die Annotation der kodierenden Exons im Vergleich zu bereits bekannten Sequenzen anderer Organismen genutzt, um die erfindungsmässige Sequenz und ihre potenziellen Promotoren zu bestimmen.

### PCR:

Die PCR wurde aufgrund ihrer hohen Sensitivität zum Nachweis der gesuchten DNA-Sequenz gewählt. Die Analyse wurde in 20 µl Reaktionsvolumen durchgeführt. Der Reaktionsansatz bestand aus 10 mM Tris-HCl, pH 9,0; 50 mM KCI; 0,1 % Triton X-100, 0,2 mM dNTP; 2 mM MgCl2, je 0,6 µM Oligonukleotide und Taq-Polymerase (Konzentration im Reaktionsansatz: - 1 U µl⁻¹). Pro Reaktionsansatz wurden entweder 10 ng BAC-Pool-DNA oder 2 µl Bakterienkultur (für Kolonie-PCR) verwendet. Vorhandene cDNA-Sequenzen dienten als Grundlage zur Ableitung der Oligonukleotide 5' CAG CCA CCA CCA ACC CAA AC und 5' GCC CAC GGT CAG CAA CCT CTC C.

Die zu amplifizierende BAC-DNA und die Primer wurden vorgelegt und anschließend mit dem PCR-Reaktionsansatz vermischt. Zum Abtöten und Aufschließen der Bakterien in einer Kolonie-PCR wurde die Vorlage vor der Zugabe des PCR-Reaktionsgemisches für 5 min auf 95°C erhitzt. Zur Denaturierung der doppelsträngigen DNA wurde ein initialer Schritt von 5 min bei 95°C benutzt. Die Touch-Down PCR-Reaktion erfolgte in den Abschnitten 30 sek 95°C; 30 sek Temperatur T und 60 sek 72°C für die ersten 10 Zyklen wobei T im ersten Zyklus 60°C beträgt und bei jedem Zyklus um 0,5 °C gesenkt wird. Weitere 30 Zyklen erfolgten in den Abschnitten 30 sek 95°C; 30 sek 55°C und 60 sek 72°C. Zur abschließenden Kettenverlängerung wurde 5 min bei 72°C inkubiert, bevor die Reaktion auf eine Temperatur von 20°C abgekühlt und konstant gehalten wurde. Die Analyse der PCR-Experimente erfolgte aufgrund des erwartet kurzen Reaktionsproduktes von 1831 bp mit 2,5 %igen Agarosegelen in 0,5x TBE-Puffer.

### 2) Klonierung der genomischen Volllängensequenz

Über die Verwendung der Primerpaare 5' ACC GAC TAG TCA CCA CCA ACC CAA ACC (enthaltend eine Spel site) und 5' GCA GGG CCC AGC GCC AGT CAA CAA CAA TAA C (enthält eine Apal Schnittstelle) sowie BAC 705A01 als Template wurde via PCR ein 3,0 Kbp-Fragment isoliert, welches die Volllängensequenz von HvARM enthielt.

### Die PCR-Reaktion wurde unter folgenden Bedingungen durchgeführt:

Reaktionspuffer- 20 mM Tris-HCl (pH 8,8 at 25°C), 10 mM (NH₄)₂SO₄, 10 mM KCl, 1 % (v/v) Triton X-10, 0,1 mg/ml BSA, 1 mM MgSO4, jeweils dNTPs ä 0.2 mM, jeweils 1 µM Primer, 2,5 U Pfu proofreading DNA polymerase (Fermentas, Burlington, Canada). Das Temerpaturprogramm lautete: 94° für 2 min; dann 5 Zyklen bei 94°, 30 sek 50°C, von wo die Temperatur mit jedem Zyklus um 2°C (touchup) auf 60°C erhöht wurde; darauf 30 Zyklen bei 94°C für 30 sek, 60°C für 30 sek, 72°C für 6 min; schließlich wurde eine Extensionsreaktion bei 72°C für 7 min hinzugefügt. Das 3,0 Kbp PCR-Amplicon wurde mit Apal and Spel verdaut, die DNA aus einem Agarosegel eluiert und in mit Apal/Spel-verdauten pIPKTA9-Vektor für die transiente Überexpression kloniert.

### Beispiel 5: Klonierung der Volllängen-cDNA-Sequenz von AtARM (At2g23140) aus Arabidopsis thaliana.

Zur Amplifikation der Volllängensequenz von AtArm wurden nachfolgende Primer verwendet:
MWG 31:
   5' cccgggatgattttgcggttttggcgg 3' (Seq ID No.: 56)
MWG 32:
   5' CCCGGGTCACAAGACAAAACATAAAAATAGG 3' (Seq ID No.: 57)
MWG 32b:
   5'gactcacactactctaatacc 3' (Seq ID No.: 58)
MWG 33:
   5'GACATCGTTTGTCTCACACC 3' (Seq ID No.: 59)

Der Ansatz (Gesamtvolumen 50 µL) hatte nachfolgende Zusammensetzung (das Gen wurde aufgrund seiner Größe von 2775 bp für die PCR in zwei Teile geteilt):
4 µl MWG31 (10 pmol/µL)
4 µl MWG34 (10 pmol/µL)
5 µl 10x Pfu Ultra Puffer (Stratagene)
1,5 µl 10mM dNTPs
1 µl cDNA
1 µl Pfu Ultra (Stratagene)
33 µl H₂O
   bzw.
4 µl MWG32 (10 pmol/µL)
4 µl MWG33 (10 pmol/µL)
5 µl 10x Pfu Ultra Puffer (Stratagene)
1,5 µl 10mM dNTPs
1 µl cDNA
1 µl Pfu Ultra (Stratagene)
33 µl H₂O

Folgendes Temperaturprogramm wurde verwendet (GeneAmp PCR System 9700; Applied Biosystems):

| | |
|---|---|
| 94°C | 2 min Denaturierung |
| 30 Zyklen mit | 94°C 30sek (Denaturierung) |
| | 59°C 30 sek (Annealing) |
| | 72°C 1,5 min (Extension) |
| | 72°C 7 min abschließende Extension |
| | 4°C Kühlung bis zu Weiterverarbeitung |

Die PCR ergibt ein Produkt von 1143bp bzw. 1705bp. Das erhaltene PCR-Produkt wird über ein 1 % Agarosegel isoliert, aus dem Gel extrahiert und in pCR4-Topo (Fa. Invitrogen Life Technologies) mittels T-Überhang-Ligation kloniert und sequenziert. Die unter SEQ ID NO: wiedergegebene Sequenz ist also identisch mit der Armadillo-Sequenz aus Arabidopsis thaliana.

Um das Gen zusammenzufügen, wird das PCR-Produkt von 1705bp in pUC18 kloniert. Hiernach erfolgt die Klonierung von AtArm 1143bp in pUC18-AtArm 1705bp.

### Beispiel 6: Transiente Expression in Gerste und Weizen durch Partikelbeschuß

### 1) Folgendes Gemisch von Konstrukten wurde mit einer "Genkanone" (Bio-Rad, Modell PDS-1000/He, Hepta-Adapter) mittels biolistischer Transformation in Weizenblätter eingebracht, gemäss Douchkov et al., Mol. Plant-Microbe Interact. 18, 755 (2005):

| Plasmid | Ansatz 1 (µg/Schuss) | Ansatz 2 (µg/Schuss) | Ansatz 3 (µg/Schuss) | Ansatz 4 (µg/Schuss) | Ansatz 5 (µg/Schuss) |
|---|---|---|---|---|---|
| pUbiGUS (Reportergenkonstrukt) | 7 | 7 | 7 | 7 | 7 |
| pIPKTA9 (leerer Überexpressionsvektor) | 7 | - | - | - | - |
| plPKTA9_Rnr5 (Überexpressionskonstrukt) | - | - | - | - | 7 |
| pIPKTA9_TaPERO (Positivkontrolle) | - | 7 | - | - | - |
| BAC 632F23 (Kontroll BAC (leer)) | - | - | 14 | - | - |
| BAC 581 D24 (Rnr5) | - | - | - | 14 | - |

Verwendete Vektoren und Kontrollen:
pUbiGUS - GUS-Überepxressions-Reportergen-Konstrukt (Schweizer et al., Molecular Plant-Microbe Interactions 12 (8), 647 (1999)).
pIPKTA9 - Leere Vektorkontrolle (Zimmermann et al., Plant Physiology 142, 181 (2006)), pIPKTA9_ARM40F - pIPKTA9-basiertes HvARM-Überexpresssionskonstrukt,
BAC 581 D24 - HvARM enthaltender BAC-Klon,
BAC 632F23 - "leere" BAC-Kontrolle (BAC hybridisiert nicht mit irgendeinem bekannten EST).

Für die Beschichtung mit DNA wurden pro Schuß 2.18 mg Goldpartikel (1.0 µm Durchmesser, Partikeldichte 25 mg ml⁻¹ in 50% (v/v) Glyzerin) mit 14-21 µg "supercoiled" DNA gemischt und mit 1 M Ca(NO₃)₂ pH 10 versetzt, sodass die finale Konzentration von Ca(NO₃)₂ 0.5 M war. Nach Zentrifugieren und Waschen mit 70 % (v/v) Ethanol wurden die Partikel in 96 % (v/v) Ethanol resuspendiert und auf den 7 Makrocarriern verteilt. Die Partikel wurden in einem Vakuum (3.6x10³ Pa) durch eine Heliumdruckwelle von 7.6x10^{s} Pa auf jeweils 7 Blattsegmente von 7 Tage alten Gersten- oder Weizenpflanzen (Gerste: Sorte Golden Promise; Weizen: Sorte Kanzler) eingebracht. Zum Beschuß wurden die Blattsegmente in eine Petrischale auf 0.5% (w/v) Phytoagar gelegt, der mit 20 µg ml⁻¹ Benzimidazol versetzt war. Die Blätter wurden anschließend bei +20°C und indirektem Tageslicht für 4 h inkubiert.

### 2) Inokulation der Blattsegmente

Die bombardierten Blätter wurden auf 1 % (w/v) Phytoagar mit 20 µg ml⁻¹ Benzimidazol in 20 x 20 cm großen Polycarbonat-Schalen umgelegt. In einem Inokulationsturm erfolgte die Infektion der Gerstenblätter mit Gerstenmehltau-Sporen und der Weizenblätter mit Weizenmehltau-Sporen, indem Sporen von stark infizierten Gersten- oder Weizenblättern in den Turm geschüttelt wurden. Die Inokulumdichte lag bei rund 200 Sporen/mm². Nach 5 min wurden die Schalen entfernt, geschlossen und bei +20°C und indirektem Tageslicht für 40-48 h inkubiert.

### 3) Histochemischer GUS-Nachweis

Die Blätter wurden unter Vakuum mit der GUS-Nachweislösung (10 mM EDTA, 1.4 mM K₃[Fe(CN)₆], 1.4 mM K₄[Fe(CN)₆], 0.1 % (v/v) Triton X-1 00, 20 % (v/v) Methanol, 1 mg/ml 5-Bromo-4-chloro-3-indolyl-β-D-glucuronsäure, 100 mM Na-Phosphatpuffer, pH 7.0) infiltriert und über Nacht bei +37°C inkubiert. Nach Entfernen der Nachweislösung wurden die Blätter mit einer Lösung aus 7.5 % (w/v) Trichloressigsäure und 50 % (v/v) Methanol für 15 min bei +20°C entfärbt.

### 4) Bestimmung der Suszeptibilität bombardierter Epidermiszellen

Die Lichtmikroskopie erfolgte mit einem Zeiss Axiolab bei 200-facher Vergrösserung. Zellen mit GUS-Expression besitzen einen blau gefärbten Zellinhalt. Mittels quantitativer Mikroskopie wurden pro Schuss die Anzahl GUS-gefärbter Zellen und die Anzahl der GUS-gefärbten Zellen, die mindestens 1 Haustorium des Weizenmehltaupilzes tragen, gezählt. Der Suszeptibilitätsindex berechnete sich aus der Anzahl der haustoriuntragenden GUS-pos. Zellen/alle GUS-positiven Zellen.

### Ergebnisse: Erhöhung der Mehltauresistenz von Weizen durch Expression von HvARM-Repeat Protein

**Tabelle 1: Komplementationsdaten HvARM (= 'Rnr5')**

| | | Relative Suszeptibilität (%) | Relative Suszeptibilität (%) |
|---|---|---|---|
| | | Mittelwert | Standardabweichung |
| Gerste | pIPKTA9 | 100,00 | |
| Gerste | pGY1_TaPERO | 36,71 | 5,04136 |
| Gerste | BAC_632F23 | 149,60 | 13,9 |
| Gerste | BAC_581D24 | 147,69 | 19,04006 |
| Gerste | pIPKTA9_Rnr5 | 104,97 | 15,22278 |
| | | | |
| Weizen | pIPKTA9 | 100 | |
| Weizen | pGY1_TaPERO | 25,48284139 | 2,973353 |
| Weizen | BAC_632F23 | 107,8383963 | 11,40531 |
| Weizen | BAC_581D24 | 48,48787766 | 4,162226 |
| Weizen | pIPKTA9_Rnr5 | 66,78504562 | 3,821332 |

Die Expression der TaPERO (WO 2005/035766) dient als positive Kontrolle für eine erfolgreiche Melhtauresistenz im transienten Assay.

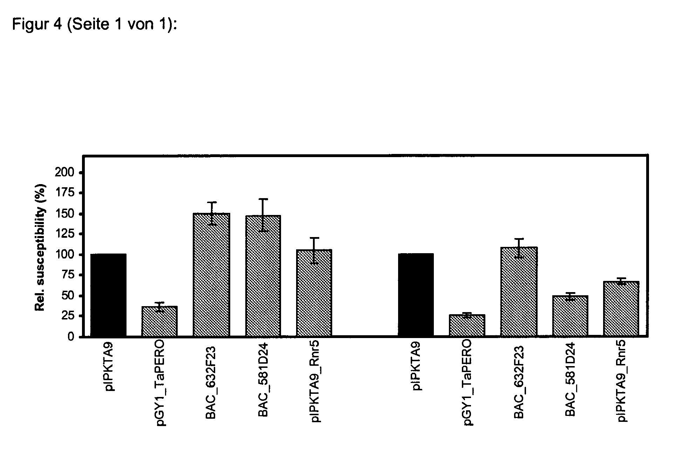

Abbildung 4 fasst die Daten aus Tabelle 1 zusammen.

### Beispiel 7: Expression in Weizen durch Partikelbeschuß

Nach dem Fachmann bekannten Verfahren wurden transgener Weizen (Triticum aestivum) mittels Partikelbeschuß generiert. Zur Expression wurden die Armadillo-Repeat Gene mit dem Weizen RbcS-Promtor (ribose-1,5-bisphosphate carboxylase small unit) fusioniert. Nach den üblichen Methoden erhielt man hieraus Setzlinge der T1 Generation, welche bzgl. ihrer Resistent gegen Blumeria graminis f.sp. tritici getestet wurden. Hierzu wurden die Setzlinge in Erde gepflanzt und nach sieben Tagen wie voranstehend bereits beschrieben mit Sporen von Blumeria graminis f.sp. tritici inoculiert. Die so infizierten Pfanzen wurden dann nach weiteren sieben bis zehn Tagen in Hinblick auf ihren Befall mit Blumeria graminis f.sp. tritici untersucht. Drei der so erhaltenenen transgenen Weizenlinien zeigten nahezu keinen Pilzbefall (5 % Pilzbefall im Vergleich zu der Wildtypkontrolle).

## Patentansprüche

1. Verfahren zur Erzeugung oder Erhöhung der Resistenz gegenüber einem oder mehreren Pathogen(en) in einer Pflanze, oder einem Teil einer Pflanze, z.B. in einem Organ, Gewebe, einer Zelle oder einem Teil einer pflanzlichen Zelle, z.B. in einem Organell, bei dem eine Nukleotid-Sequenz, die für ein Armadillo Repeat ARM1 Protein oder einen Teil von diesem kodiert, in die Pflanze bzw. Pflanzenzelle oder deren Teil eingeführt und dort exprimiert wird, wobei die Nukleotidsequenz mindestens ein Nukleinsäuremolekül umfasst, ausgewählt aus der Gruppe bestehend aus:
a) Nukleinsäuremolekül, das mindestens ein Polypeptid kodiert umfassend die in SEQ ID No: 2 gezeigte Sequenz;
b) Nukleinsäuremolekül, das zumindest ein Polynukleotid der Sequenz gezeigt in SEQ ID No: 1 umfasst;
c) Nukleinsäuremolekül, das ein Polypeptid kodiert, dessen Sequenz eine Identität von mindestens 50% zu der Sequenz SEQ ID No: 2 aufweist;
d) Nukleinsäuremolekül dessen Sequenz eine Identität von mindestens 50% zu der Sequenz SEQ ID No: 1 aufweist;
e) Nukleinsäuremolekül nach (a) bis (d), das für ein Fragment oder ein Epitop der Sequenzen gemäß SEQ. ID No.: 2 kodiert;
f) Nukleinsäuremolekül, das ein Polypeptid kodiert, welches von einem monoklonalen Antikörper, gerichtet gegen ein Polypeptid welches durch die Nukleinsäuremoleküle gemäß (a) bis (d) kodiert wird, erkannt wird;
g) Nukleinsäuremolekül, das unter stringenten Bedingungen mit einem Nukleinsäuremolekül gemäß (a) bis (d) hybridisiert; und
h) Nukleinsäuremolekül, das aus einer DNA-Bank unter Verwendung eines Nukleinsäuremoleküls gemäß (a) bis (d) oder deren Teilfragmenten von mindestens 15 nt, vorzugsweise 20 nt, 30 nt, 50 nt, 100 nt, 200 nt oder 500 nt als Sonde unter stringenten Hybridisierungsbedingungen isoliert werden kann;
oder eine komplementäre Sequenz davon umfasst, wobei die Nukleotidsequenz so gestaltet ist, dass dadurch eine Pathogenresistenz erzeugt oder erhöht wird.

2. Verfahren nach Anspruch 1, bei dem eine Pilzpathogenresistenz erzeugt oder erhöht wird, wobei es sich bevorzugt um ein penetrierendes Pilzpathogen handelt.

3. Verfahren nach Anspruch 1 oder 2, bei dem der Teil einer Pflanze Mesophyll- und/ oder Epidermiszellen sind.

4. Verfahren nach einem der Ansprüche 1 bis 3, bei dem der Teil einer Pflanze Lemma (Deckspelze), Palea (Vorspelze) und/ oder Glume (Antherenanlage) ist.

5. Verfahren nach einem der vorstehenden Ansprüche, bei dem das Armadillo Repeat ARM1 Protein zwei oder mehr Armadillo Repeats umfasst.

6. Verfahren nach einem der vorstehenden Ansprüche, umfassend
a) die Einbringung einer rekombinanten Expressionskassette enthaltend in funktioneller Verknüpfung mit einem in Pflanzen aktiven Promotor eine Nukleinsäuresequenz wie charakterisiert in Anspruch 1 (a-h) in eine pflanzliche Zelle;
b) Regeneration der Pflanze aus der pflanzlichen Zelle, und
c) Expression besagter Nukleinsäuresequenz in einer Menge und für eine Zeit, hinreichend um eine Pathogenresistenz in besagter Pflanze zu erzeugen oder zu erhöhen.

7. Verfahren nach Anspruch 6, **dadurch gekennzeichnet, dass** es sich bei dem in Pflanzen aktiven Promotor um einen Pathogen-induzierbaren Promotor oder einen Epidermis- oder Mesophyll-spezifischen Promotor handelt.

8. Verfahren nach einem der Ansprüche 1 bis 7, **dadurch gekennzeichnet, dass** in der Pflanze, dem pflanzlichen Organ, pflanzlichen Gewebe oder der pflanzlichen Zelle zusätzlich die Aktivität eines Polypeptids codierend für Bax Inhibitor 1, ROR2, SnAP34, und/oder Lumenal Binding Protein BiP erhöht ist.

9. Verfahren nach einem der Ansprüche 1 bis 8, **dadurch gekennzeichnet, dass** in der Pflanze, dem pflanzlichen Organ, pflanzlichen Gewebe oder der pflanzlichen Zelle die Aktivität eine Polypeptides codierend für RacB, CSL1, HvNaOX und/oder MLO vermindert wird.

10. Verfahren gemäß Anspruch 8, **dadurch gekennzeichnet, dass** der Bax-Inhibitor 1 unter Kontrolle eines Mesophyll und/oder Wurzel spezifischen Promotors exprimiert wird.

11. Verfahren nach einem der Ansprüche 1 bis 10, **dadurch gekennzeichnet, dass** das Pathogen ausgewählt ist aus den Arten Blumeria graminis, Puccinia triticina, Puccinia striiformis, Mycosphaerella graminicola, Stagonospora nodorum, Fusarium graminearum, Fusarium culmorum, Fusarium avenaceum, Fusarium poae oder Microdochium nivale, wobei bevorzugt das Pathogen Blumeria graminis ist.

12. Verfahren nach Anspruch 11, bei dem das Pathogen Blumeria graminis (DC) Speer f.sp. hordei oder Blumeria graminis (DC) Speer f.sp. tritici ist.

13. Verfahren nach einem der vorstehenden Ansprüche, bei dem das Pathogen Blumeria graminis (DC) Speer f.sp. hordei ist und die Pflanze aus der Gattung Hordeum ausgewählt ist.

14. Verfahren nach einem der vorstehenden Ansprüche, bei dem das Pathogen Blumeria graminis (DC) Speer f.sp. tritici ist und die Pflanze aus der Gattung Triticum, bevorzugt Triticum aestivum, ausgewählt ist.

15. Verfahren nach Anspruch 14, bei dem die Armadillo Repeat ARM1 Nukleotidsequenz aus der Gerste stammt.

16. Nukleinsäuremolekül kodierend für ein Armadillo-repeat ARM1 Protein, umfassend mindestens ein Nukleinsäuremolekül ausgewählt aus der Gruppe bestehend aus:
a) Nukleinsäuremolekül, das mindestens ein Polypeptid kodiert umfassend die in SEQ ID No: 2 gezeigte Sequenz;
b) Nukleinsäuremolekül, das zumindest ein Polynukleotid der Sequenz gezeigt in SEQ ID No: 1 umfasst;
c) Nukleinsäuremolekül, das ein Polypeptid kodiert, dessen Sequenz eine Identität von mindestens 50% zu der Sequenz SEQ ID No: 2 aufweist;
d) Nukleinsäuremolekül dessen Sequenz eine Identität von mindestens 50% zu der Sequenz SEQ ID No: 1 aufweist;
e) Nukleinsäuremolekül nach (a) bis (d), das für ein Fragment oder ein Epitop der Sequenzen gemäß SEQ. ID No.: 2 kodiert;
f) Nukleinsäuremolekül, das ein Polypeptid kodiert, welches von einem monoklonalen Antikörper, gerichtet gegen ein Polypeptid welches durch die Nukleinsäuremoleküle gemäß (a) bis (d) kodiert wird, erkannt wird;
g) Nukleinsäuremolekül, das unter stringenten Bedingungen mit einem Nukleinsäuremolekül gemäß (a) bis (d) hybridisiert; und
h) Nukleinsäuremolekül, das aus einer DNA-Bank unter Verwendung eines Nukleinsäuremoleküls gemäß (a) bis (d) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann;
oder eine komplementäre Sequenz davon umfasst;
oder ein Polypeptid umfassend eine Aminosäuresequenz kodiert von dem Nukleinsäuremolekül;
wobei das Nukleinsäuremolekül nicht aus der Sequenz gezeigt in SEQ ID NO.: 3, 5, 7, 9, 11, 13, 15, 17, 19, 21, 23, 25, 27, 29, 31, 33, 35, 37, 39, 41, oder 43 besteht.

17. Nukleinsäure-Konstrukt enthaltend ein Nukleinsäuremolekül enthaltend mindestens ein Fragment eines Nukleinsäuremoleküls kodierend für ein Armadillo repeat ARM1 Polypeptid funktionell verknüpft mit einem pathogen-induzierbaren Promoter oder einem Epidermis- und/oder Mesophyll-spezifischen Promoter, wobei das Armadillo repeat ARM1 Polypeptid kodiert wird von einem Nukleinsäuremolekül umfassend ein Nukleinsäuremolekül ausgewählt aus der Gruppe bestehend aus
a) Nukleinsäuremolekül, das ein Polypeptid kodiert, umfassend die in SEQ ID No: 2 gezeigte Sequenz;
b) Nukleinsäuremolekül, das zumindest ein Polynukleotid der Sequenz nach der SEQ ID No: 1 umfasst;
c) Nukleinsäuremolekül, das ein Polypeptid kodiert, dessen Sequenz eine Identität von mindestens 40 % zu der Sequenz SEQ ID No: 2 aufweist;
d) Nukleinsäuremolekül dessen Sequenz eine Identität von mindestens 50% zu der Sequenz SEQ ID No: 1 aufweist;
e) Nukleinsäuremolekül nach (a) bis (d), das für ein Fragment oder ein Epitop der Sequenzen gemäß SEQ. ID No: 2 kodiert;
f) Nukleinsäuremolekül das ein Polypeptid kodiert, welches von einem monoklonalen Antikörper, gerichtet gegen ein Polypeptid welches durch die Nukleinsäuremoleküle gemäß (a) bis (d) kodiert wird, erkannt wird;
g) Nukleinsäuremolekül, das unter stringenten Bedingungen mit einem Nukleinsäuremolekül gemäß (a) bis (d) hybridisiert; oder
h) Nukleinsäuremolekül, das aus einer DNA-Bank unter Verwendung eines Nukleinsäuremoleküls gemäß (a) bis (d) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann;
oder komplementär dazu ist.

18. DNA Expressionskassette umfassend ein Nukleinsäuremolekül, das im wesentlichen identisch ist zu einem Nukleinsäuremolekül wie in Anspruch 1 charakterisiert.

19. DNA Expressionskassette nach Anspruch 18, wobei besagte Nukleinsäuresequenz mit einem Promotor funktionell verknüpft ist.

20. DNA Expressionskassette nach Anspruch 19, wobei der Promotor ein in Pflanzen funktioneller Promotor ist, wobei der in Pflanzen funktionelle Promotor bevorzugt ein pathogeninduzierbarer oder einem Epidermis- und/oder Mesophyll-spezifischer Promotor ist.

21. Vektor umfassend eine Expressionskassette gemäß einem der Ansprüche 18 bis 20.

22. Zelle, umfassend ein Nukleinsäuremolekül wie in Anspruch 1 charakterisiert, eine DNA-Expressionskassette gemäß einem der Ansprüche 18 bis 20, ein Nukleinsäuremolekül oder ein Polypeptid nach Anspruch 16, oder einen Vektor gemäß Anspruch 21, oder bei der eine Resistenz durch ein Verfahren nach einem der Ansprüche 1 bis 15 erzeugt oder erhöht wurde.

23. Transgener nicht-humaner Organismus, bevorzugt Pflanze, umfassend ein Nukleinsäuremolekül wie in Anspruch 1 charakterisiert, eine DNA-Expressionskassette gemäß einem der Ansprüche 18 bis 20, ein Nukleinsäuremolekül oder ein Polypeptid nach Anspruch 16 oder einen Vektor gemäß Anspruch 21, oder bei dem eine Resistenz durch ein Verfahren nach einem der Ansprüche 1 bis 15 erzeugt oder erhöht wurde, oder umfassend eine Zelle nach Anspruch 22, wobei der transgene nicht-humane Organismus bevorzugt ein monokotyledoner Organismus ist.

24. Transgener nicht-humaner Organismus nach Anspruch 23, der zudem über eine erhöhte Bax Inhibitor 1-Protein-, ein ROR2-, und/oder SnAP34- Aktivität verfügt und/oder eine verminderte RacB-, CSL1-, und/oder HvRBOHF-Aktivität aufweist.

25. Transgener nicht-humaner Organismus nach einem der Ansprüche 23 oder 24, der eine erhöhte Bax Inhibitor 1-, ein ROR2-, und/oder SnAP34- Aktivität und/oder eine verminderte RacB-, CSL1-, und/oder HvRBOHF-Aktivität in Mesophyllzellen und/oder Wurzelzellen besitzt.

26. Verwendung eines Nukleinsäuremoleküls wie in Anspruch 1 charakterisiert, einer DNA-Expressionskassette gemäß einem der Ansprüche 18 bis 20, eines Nukleinsäuremoleküls oder ein Polypeptids nach Anspruch 16, oder eines Vektors gemäß Anspruch 21, oder einer Zelle nach Anspruch 22 zur Herstellung einer Pflanze, die resistent gegen Mesophyllzellen penetrierende Pathogene ist.

27. Ernte, Vermehrungsmaterial oder Zusammensetzung umfassend ein Nukleinsäuremolekül wie in Anspruch 1 charakterisiert, eine DNA-Expressionskassette gemäß einem der Ansprüche 18 bis 20, ein Nukleinsäuremolekül oder ein Polypeptid nach Anspruch 16, oder einen Vektor gemäß Anspruch 21, oder bei dem/ der eine Resistenz durch ein Verfahren nach einem der Ansprüche 1 bis 15 erzeugt oder erhöht wurde, oder umfassend eine Zelle nach Anspruch 22.


SEQUENCE LISTING

<110>  BASF Plant Science GmbH

<120>  Verfahren zur Erhöhung der Patogenresistenz in transgenen
       Pflanzen

<130>  PF 58471

<150>  EP 06122217.0
<151>  2006-10-12

<160>  64

<170>  PatentIn version 3.4

<210>  1
<211>  1329
<212>  DNA
<213>  Hordeum vulgare


<220>
<221>  CDS
<222>  (1)..(1329)

<400>  1
atg caa atg gct ctg cta gca agg ctt tct ctt gca agt tct gaa gga        48
Met Gln Met Ala Leu Leu Ala Arg Leu Ser Leu Ala Ser Ser Glu Gly
1               5                   10                  15

aga gag tct agt ttg gaa gaa aga cat gct ggt tct gat gaa caa act        96
Arg Glu Ser Ser Leu Glu Glu Arg His Ala Gly Ser Asp Glu Gln Thr
            20                  25                  30

tca gaa caa tca acg aag gaa gca ttt caa gca tct cat ttt gac agt       144
Ser Glu Gln Ser Thr Lys Glu Ala Phe Gln Ala Ser His Phe Asp Ser
        35                  40                  45

gat tca cag gtt cgt cta ggc aga tct tca gtt aat gat aat ctt cct       192
Asp Ser Gln Val Arg Leu Gly Arg Ser Ser Val Asn Asp Asn Leu Pro
    50                  55                  60

aat acc cgt cag ctt gac gag gag tgt gac atc aac gat ggg atg ata       240
Asn Thr Arg Gln Leu Asp Glu Glu Cys Asp Ile Asn Asp Gly Met Ile
65                  70                  75                  80

cga gtt cca ggt gat agg aca aat tat agt agt gat gcg tct gga gag       288
Arg Val Pro Gly Asp Arg Thr Asn Tyr Ser Ser Asp Ala Ser Gly Glu
                85                  90                  95

gtt gct gac cgt ggg ctt tct atc tct tct gcc cct caa agg gaa aat       336
Val Ala Asp Arg Gly Leu Ser Ile Ser Ser Ala Pro Gln Arg Glu Asn
            100                 105                 110

gta atc ctg cca aga ttg ggt cat gtc tgc atg gag gga cca ttt gtt       384
Val Ile Leu Pro Arg Leu Gly His Val Cys Met Glu Gly Pro Phe Val
        115                 120                 125

cag cgg caa aca tct gac aag gga ttc ccg aga ata att tcg tcg tta       432
Gln Arg Gln Thr Ser Asp Lys Gly Phe Pro Arg Ile Ile Ser Ser Leu

130                 135                 140

tcc atg gat gcc cgg gat gat ttc tct gcc atc gag aat cag gta cgc      480
Ser Met Asp Ala Arg Asp Asp Phe Ser Ala Ile Glu Asn Gln Val Arg
145                 150                 155                 160

gag cta atc aat gat ttg gga agt gat tcc ata gaa ggt cag aga tca      528
Glu Leu Ile Asn Asp Leu Gly Ser Asp Ser Ile Glu Gly Gln Arg Ser
                165                 170                 175

gca aca tca gag att cgc ctt cta gct aag cac aac atg gag aac agg      576
Ala Thr Ser Glu Ile Arg Leu Leu Ala Lys His Asn Met Glu Asn Arg
            180                 185                 190

att gcc att gct aat tgt ggg gct ata aac ttg ctg gtt ggc ctt ctt      624
Ile Ala Ile Ala Asn Cys Gly Ala Ile Asn Leu Leu Val Gly Leu Leu
        195                 200                 205

cat tca ccc gat gcc aaa atc caa gaa aat gca gtg aca gcc ctc ctt      672
His Ser Pro Asp Ala Lys Ile Gln Glu Asn Ala Val Thr Ala Leu Leu
    210                 215                 220

aat ttg tca ctc agt gat atc aat aag att gcc atc gtg aat gca gat      720
Asn Leu Ser Leu Ser Asp Ile Asn Lys Ile Ala Ile Val Asn Ala Asp
225                 230                 235                 240

gct att gat cct ctc atc cat gtc ctg gaa aca ggg aac cct gaa gct      768
Ala Ile Asp Pro Leu Ile His Val Leu Glu Thr Gly Asn Pro Glu Ala
                245                 250                 255

aaa gag aat tca gca gct act ttg ttc agt ctc tca att att gaa gaa      816
Lys Glu Asn Ser Ala Ala Thr Leu Phe Ser Leu Ser Ile Ile Glu Glu
            260                 265                 270

aac aga gtg agg ata ggg cga tct ggt gct gta aag cct ctc gtg gac      864
Asn Arg Val Arg Ile Gly Arg Ser Gly Ala Val Lys Pro Leu Val Asp
        275                 280                 285

ttg ctg gga aat ggg agc cca cga gga aag aaa gat gcg gtt act gca      912
Leu Leu Gly Asn Gly Ser Pro Arg Gly Lys Lys Asp Ala Val Thr Ala
    290                 295                 300

ttg ttt aat tta tcc ata ctt cat gag aac aag ggt cga att gtg caa      960
Leu Phe Asn Leu Ser Ile Leu His Glu Asn Lys Gly Arg Ile Val Gln
305                 310                 315                 320

gct gat gca ttg aag cac cta gtt gag ctt atg gac cct gct gct gga     1008
Ala Asp Ala Leu Lys His Leu Val Glu Leu Met Asp Pro Ala Ala Gly
                325                 330                 335

atg gtc gat aaa gct gta gct gtc ttg gca aat ctt gct acg ata cca     1056
Met Val Asp Lys Ala Val Ala Val Leu Ala Asn Leu Ala Thr Ile Pro
            340                 345                 350

gaa gga agg act gcg att ggg cag gcg cgt ggt att ccg gcc ctt gtt     1104
Glu Gly Arg Thr Ala Ile Gly Gln Ala Arg Gly Ile Pro Ala Leu Val
        355                 360                 365

gaa gtt gtc gaa ctg ggt tca gcg aaa gcg aag gaa aat gct acc gcg     1152
Glu Val Val Glu Leu Gly Ser Ala Lys Ala Lys Glu Asn Ala Thr Ala
    370                 375                 380

gca ttg ctt cag cta tgc aca aac agc agc agg ttt tgc aac ata gtt      1200
Ala Leu Leu Gln Leu Cys Thr Asn Ser Ser Arg Phe Cys Asn Ile Val
385                 390                 395                 400

ctt caa gag gat gcc gtg ccc cct tta gtc gca ctg tca cag tca gga      1248
Leu Gln Glu Asp Ala Val Pro Pro Leu Val Ala Leu Ser Gln Ser Gly
                405                 410                 415

aca cca cgc gca aga gaa aag gcg cag gtt ctc ctc agc tat ttc cgc      1296
Thr Pro Arg Ala Arg Glu Lys Ala Gln Val Leu Leu Ser Tyr Phe Arg
            420                 425                 430

agc caa aga cat ggg aac tcg gga agg aga tga                          1329
Ser Gln Arg His Gly Asn Ser Gly Arg Arg
        435                 440


<210>  2
<211>  442
<212>  PRT
<213>  Hordeum vulgare

<400>  2

Met Gln Met Ala Leu Leu Ala Arg Leu Ser Leu Ala Ser Ser Glu Gly
1               5                   10                  15


Arg Glu Ser Ser Leu Glu Glu Arg His Ala Gly Ser Asp Glu Gln Thr
            20                  25                  30


Ser Glu Gln Ser Thr Lys Glu Ala Phe Gln Ala Ser His Phe Asp Ser
        35                  40                  45


Asp Ser Gln Val Arg Leu Gly Arg Ser Ser Val Asn Asp Asn Leu Pro
    50                  55                  60


Asn Thr Arg Gln Leu Asp Glu Glu Cys Asp Ile Asn Asp Gly Met Ile
65                  70                  75                  80


Arg Val Pro Gly Asp Arg Thr Asn Tyr Ser Ser Asp Ala Ser Gly Glu
                85                  90                  95


Val Ala Asp Arg Gly Leu Ser Ile Ser Ser Ala Pro Gln Arg Glu Asn
            100                 105                 110


Val Ile Leu Pro Arg Leu Gly His Val Cys Met Glu Gly Pro Phe Val
        115                 120                 125


Gln Arg Gln Thr Ser Asp Lys Gly Phe Pro Arg Ile Ile Ser Ser Leu
    130                 135                 140

Ser Met Asp Ala Arg Asp Asp Phe Ser Ala Ile Glu Asn Gln Val Arg
145                 150                 155                 160

Glu Leu Ile Asn Asp Leu Gly Ser Asp Ser Ile Glu Gly Gln Arg Ser
                165                 170                 175

Ala Thr Ser Glu Ile Arg Leu Leu Ala Lys His Asn Met Glu Asn Arg
            180                 185                 190

Ile Ala Ile Ala Asn Cys Gly Ala Ile Asn Leu Leu Val Gly Leu Leu
        195                 200                 205

His Ser Pro Asp Ala Lys Ile Gln Glu Asn Ala Val Thr Ala Leu Leu
    210                 215                 220

Asn Leu Ser Leu Ser Asp Ile Asn Lys Ile Ala Ile Val Asn Ala Asp
225                 230                 235                 240

Ala Ile Asp Pro Leu Ile His Val Leu Glu Thr Gly Asn Pro Glu Ala
                245                 250                 255

Lys Glu Asn Ser Ala Ala Thr Leu Phe Ser Leu Ser Ile Ile Glu Glu
            260                 265                 270

Asn Arg Val Arg Ile Gly Arg Ser Gly Ala Val Lys Pro Leu Val Asp
        275                 280                 285

Leu Leu Gly Asn Gly Ser Pro Arg Gly Lys Lys Asp Ala Val Thr Ala
    290                 295                 300

Leu Phe Asn Leu Ser Ile Leu His Glu Asn Lys Gly Arg Ile Val Gln
305                 310                 315                 320

Ala Asp Ala Leu Lys His Leu Val Glu Leu Met Asp Pro Ala Ala Gly
                325                 330                 335

Met Val Asp Lys Ala Val Ala Val Leu Ala Asn Leu Ala Thr Ile Pro
            340                 345                 350

Glu Gly Arg Thr Ala Ile Gly Gln Ala Arg Gly Ile Pro Ala Leu Val
        355                 360                 365

Glu Val Val Glu Leu Gly Ser Ala Lys Ala Lys Glu Asn Ala Thr Ala
    370                 375                 380

Ala Leu Leu Gln Leu Cys Thr Asn Ser Ser Arg Phe Cys Asn Ile Val

385                 390                 395                 400

Leu Gln Glu Asp Ala Val Pro Pro Leu Val Ala Leu Ser Gln Ser Gly
                405                 410                 415

Thr Pro Arg Ala Arg Glu Lys Ala Gln Val Leu Leu Ser Tyr Phe Arg
            420                 425                 430

Ser Gln Arg His Gly Asn Ser Gly Arg Arg
        435                 440

<210>  3
<211>  2475
<212>  DNA
<213>  Oryza sativa

<220>
<221>  CDS
<222>  (1)..(2475)

<400>  3
atg gaa aat ttc tcc ccg aga acc ctg ctc aat agt atc ttg cgt atc      48
Met Glu Asn Phe Ser Pro Arg Thr Leu Leu Asn Ser Ile Leu Arg Ile
1               5                   10                  15

act gtc tta acc tcc gat ggc tct act gca agg ccc aag ccc att cag      96
Thr Val Leu Thr Ser Asp Gly Ser Thr Ala Arg Pro Lys Pro Ile Gln
            20                  25                  30

aag tac tgc caa aat gtg tgt gat atc tca agc att gtg agc cct ctc      144
Lys Tyr Cys Gln Asn Val Cys Asp Ile Ser Ser Ile Val Ser Pro Leu
        35                  40                  45

ata gag gat cta tgt gag tct cct gaa gag caa ctc aat gag gtg tta      192
Ile Glu Asp Leu Cys Glu Ser Pro Glu Glu Gln Leu Asn Glu Val Leu
    50                  55                  60

agg gag ctt ggc act gct att aac aga gct tca ggg ctt att ggg aac      240
Arg Glu Leu Gly Thr Ala Ile Asn Arg Ala Ser Gly Leu Ile Gly Asn
65                  70                  75                  80

tgg caa cag aca acc agc aaa ata tat ttt ata tgg cag att gaa tca      288
Trp Gln Gln Thr Thr Ser Lys Ile Tyr Phe Ile Trp Gln Ile Glu Ser
                85                  90                  95

gta atc tca gat atc cag gga tgt tct cta cag ctg tgc cag ctt gtt      336
Val Ile Ser Asp Ile Gln Gly Cys Ser Leu Gln Leu Cys Gln Leu Val
            100                 105                 110

aac tct cta tta cct tct ttg act ggc cgt gca tgc aca tgt att gag      384
Asn Ser Leu Leu Pro Ser Leu Thr Gly Arg Ala Cys Thr Cys Ile Glu
        115                 120                 125

aaa ctc caa gac ata aat tat gaa aac atg ttt gat ctg gta aag gag      432
Lys Leu Gln Asp Ile Asn Tyr Glu Asn Met Phe Asp Leu Val Lys Glu
    130                 135                 140

tct tca ttg gag cta gtt gag acg gac aca aca agt cct gag aat ctg 480
Ser Ser Leu Glu Leu Val Glu Thr Asp Thr Thr Ser Pro Glu Asn Leu
145                 150                 155                 160

tcg aga cta tct agt tca ttg agt ttg tca act aac ctg gaa ttt tac 528
Ser Arg Leu Ser Ser Ser Leu Ser Leu Ser Thr Asn Leu Glu Phe Tyr
                165                 170                 175

atg gaa gct gtt tcc ctt gag aat ctc aga gca agg gca atg cgg agt 576
Met Glu Ala Val Ser Leu Glu Asn Leu Arg Ala Arg Ala Met Arg Ser
            180                 185                 190

gag aac cgt gaa gaa atg gat ctg gct gac aag atg atc ccc ctg gtc 624
Glu Asn Arg Glu Glu Met Asp Leu Ala Asp Lys Met Ile Pro Leu Val
        195                 200                 205

aac tat atg cat gac cac ctt ctg agg gaa aca caa ctg ctt agc atc 672
Asn Tyr Met His Asp His Leu Leu Arg Glu Thr Gln Leu Leu Ser Ile
    210                 215                 220

aat ggg gtg ccc att cct gca gat ttt tgc tgc ccg ctg tcc cta gag 720
Asn Gly Val Pro Ile Pro Ala Asp Phe Cys Cys Pro Leu Ser Leu Glu
225                 230                 235                 240

ctg atg tca gat cct gtt att gta gca tct ggt cag aca tat gag cgg 768
Leu Met Ser Asp Pro Val Ile Val Ala Ser Gly Gln Thr Tyr Glu Arg
                245                 250                 255

gtt tat atc aag tta tgg ctt gat gag ggt ttt act atc tgc ccg aag 816
Val Tyr Ile Lys Leu Trp Leu Asp Glu Gly Phe Thr Ile Cys Pro Lys
            260                 265                 270

aca cgc caa aga ctt ggt cac tcc aat tta att cca aat tac acc gtg 864
Thr Arg Gln Arg Leu Gly His Ser Asn Leu Ile Pro Asn Tyr Thr Val
        275                 280                 285

aaa gct ttg ata gct aat tgg tgc gaa tca cac aac att agg ctt cct 912
Lys Ala Leu Ile Ala Asn Trp Cys Glu Ser His Asn Ile Arg Leu Pro
    290                 295                 300

gat cct atg aaa tcc ttg aaa ttg aac ttc cct ttg gct gcg tct gct 960
Asp Pro Met Lys Ser Leu Lys Leu Asn Phe Pro Leu Ala Ala Ser Ala
305                 310                 315                 320

ctc cag gat tcg agc acc aca gga agc agc cct cta cat cct act gtc 1008
Leu Gln Asp Ser Ser Thr Thr Gly Ser Ser Pro Leu His Pro Thr Val
                325                 330                 335

gct gct aag ggt aat att cct ggg tcc ccg gaa gct gac ctt tat atg 1056
Ala Ala Lys Gly Asn Ile Pro Gly Ser Pro Glu Ala Asp Leu Tyr Met
            340                 345                 350

aga agc ttg aat aga gca tct cct cca cac agt gta gtc cat cag aat 1104
Arg Ser Leu Asn Arg Ala Ser Pro Pro His Ser Val Val His Gln Asn
        355                 360                 365

tct cat gcg cat gtg aac cgt gct ggt cat gaa gcc tcc att aag caa 1152
Ser His Ala His Val Asn Arg Ala Gly His Glu Ala Ser Ile Lys Gln
    370                 375                 380

tct tca gaa aat gct aat ggt tct gca tca gat gtt tca agg tta tct     1200
Ser Ser Glu Asn Ala Asn Gly Ser Ala Ser Asp Val Ser Arg Leu Ser
385                 390                 395                 400

ctt gca ggt tct gaa aca aga gag tct agt ctg gaa gaa aga aat gct     1248
Leu Ala Gly Ser Glu Thr Arg Glu Ser Ser Leu Glu Glu Arg Asn Ala
                405                 410                 415

ggt tct atc ggt caa act tca gaa cag tca att gag gaa gca ttt caa     1296
Gly Ser Ile Gly Gln Thr Ser Glu Gln Ser Ile Glu Glu Ala Phe Gln
            420                 425                 430

gca tct aat ttg gac agg gat tca cat gac cat gtg ggt agt tct tcg     1344
Ala Ser Asn Leu Asp Arg Asp Ser His Asp His Val Gly Ser Ser Ser
        435                 440                 445

gtg aat ggt agc ctt cca aat agc ggt caa ctt gat gca gaa tgt gac     1392
Val Asn Gly Ser Leu Pro Asn Ser Gly Gln Leu Asp Ala Glu Cys Asp
    450                 455                 460

aat ggg cca agc gaa agg aca aat tac agt agt gat gca tct gga gaa     1440
Asn Gly Pro Ser Glu Arg Thr Asn Tyr Ser Ser Asp Ala Ser Gly Glu
465                 470                 475                 480

gtt aca gat ggg cct tca gca tct tct gct cct cag agg gag cat cta     1488
Val Thr Asp Gly Pro Ser Ala Ser Ser Ala Pro Gln Arg Glu His Leu
                485                 490                 495

atc cct tct aga ttg gct gat gtt cgt agt aga ggc caa ttt gtt cgg     1536
Ile Pro Ser Arg Leu Ala Asp Val Arg Ser Arg Gly Gln Phe Val Arg
            500                 505                 510

cga cca tct gaa agg ggt ttc ccc aga ata ata tct tcc tca tcc atg     1584
Arg Pro Ser Glu Arg Gly Phe Pro Arg Ile Ile Ser Ser Ser Ser Met
        515                 520                 525

gat aca cgg agt gat ctt tcc gcc atc gag aat cag gtc cgc aag tta     1632
Asp Thr Arg Ser Asp Leu Ser Ala Ile Glu Asn Gln Val Arg Lys Leu
    530                 535                 540

gtt gat gat tta aga agt gat tct gta gat gtt caa aga tca gcg aca     1680
Val Asp Asp Leu Arg Ser Asp Ser Val Asp Val Gln Arg Ser Ala Thr
545                 550                 555                 560

tca gat atc cgc ctt tta gct aag cac aac atg gag aac agg atc atc     1728
Ser Asp Ile Arg Leu Leu Ala Lys His Asn Met Glu Asn Arg Ile Ile
                565                 570                 575

att gca aac tgt gga gct ata aac ttg ctg gtt ggt ctt ctt cat tcg     1776
Ile Ala Asn Cys Gly Ala Ile Asn Leu Leu Val Gly Leu Leu His Ser
            580                 585                 590

cca gat tcc aaa acc caa gag cat gcc gtg aca gcc ctt ctg aat ttg     1824
Pro Asp Ser Lys Thr Gln Glu His Ala Val Thr Ala Leu Leu Asn Leu
        595                 600                 605

tca atc aat gat aat aat aag att gcc att gca aat gct gat gct gtt     1872
Ser Ile Asn Asp Asn Asn Lys Ile Ala Ile Ala Asn Ala Asp Ala Val
    610                 615                 620

gac ccc ctc atc cat gtc ctt gag act ggg aac cct gaa gcc aag gag     1920

Asp Pro Leu Ile His Val Leu Glu Thr Gly Asn Pro Glu Ala Lys Glu
625                 630                 635                 640

aat tca gcg gct aca tta ttc agt ctc tcg gtt att gaa gaa aac aaa      1968
Asn Ser Ala Ala Thr Leu Phe Ser Leu Ser Val Ile Glu Glu Asn Lys
                645                 650                 655

gtg agg att gga aga tcc ggt gcc atc aaa cct ctc gtc gac cta cta      2016
Val Arg Ile Gly Arg Ser Gly Ala Ile Lys Pro Leu Val Asp Leu Leu
            660                 665                 670

gga aat ggg acc cct cga gga aag aaa gat gca gct act gca ttg ttt      2064
Gly Asn Gly Thr Pro Arg Gly Lys Lys Asp Ala Ala Thr Ala Leu Phe
        675                 680                 685

aat tta tcc ata tta cat gag aac aag gcg cgt att gtg cag gct gac      2112
Asn Leu Ser Ile Leu His Glu Asn Lys Ala Arg Ile Val Gln Ala Asp
    690                 695                 700

gct gtg aag tac cta gtt gaa ctt atg gac cct gct gct gga atg gtt      2160
Ala Val Lys Tyr Leu Val Glu Leu Met Asp Pro Ala Ala Gly Met Val
705                 710                 715                 720

gac aaa gct gtg gct gtt ttg gca aac ctt gct acc ata cca gaa ggg      2208
Asp Lys Ala Val Ala Val Leu Ala Asn Leu Ala Thr Ile Pro Glu Gly
                725                 730                 735

agg aca gca att ggt caa gcg cgt ggt att cca gcc ctt gtt gaa gtt      2256
Arg Thr Ala Ile Gly Gln Ala Arg Gly Ile Pro Ala Leu Val Glu Val
            740                 745                 750

gtt gaa ctc ggt tca gca agg ggg aag gaa aat gcg gct gca gca ttg      2304
Val Glu Leu Gly Ser Ala Arg Gly Lys Glu Asn Ala Ala Ala Ala Leu
        755                 760                 765

ctt cag cta tgt aca aac agc agc aga ttt tgc agt ata gtt ctt caa      2352
Leu Gln Leu Cys Thr Asn Ser Ser Arg Phe Cys Ser Ile Val Leu Gln
    770                 775                 780

gag ggt gct gtg cct cct cta gtt gca ttg tca cag tca ggc acg cca      2400
Glu Gly Ala Val Pro Pro Leu Val Ala Leu Ser Gln Ser Gly Thr Pro
785                 790                 795                 800

cgg gca aga gag aag gca cag gct ctt ctc agc tac ttt cgc agc caa      2448
Arg Ala Arg Glu Lys Ala Gln Ala Leu Leu Ser Tyr Phe Arg Ser Gln
                805                 810                 815

agg cac ggg aat tca gca agg aga tga                                  2475
Arg His Gly Asn Ser Ala Arg Arg
            820

<210>  4
<211>  824
<212>  PRT
<213>  Oryza sativa

<400>  4

Met Glu Asn Phe Ser Pro Arg Thr Leu Leu Asn Ser Ile Leu Arg Ile
1               5                   10                  15

Thr Val Leu Thr Ser Asp Gly Ser Thr Ala Arg Pro Lys Pro Ile Gln
            20                  25                  30

Lys Tyr Cys Gln Asn Val Cys Asp Ile Ser Ser Ile Val Ser Pro Leu
        35                  40                  45

Ile Glu Asp Leu Cys Glu Ser Pro Glu Glu Gln Leu Asn Glu Val Leu
    50                  55                  60

Arg Glu Leu Gly Thr Ala Ile Asn Arg Ala Ser Gly Leu Ile Gly Asn
65                  70                  75                  80

Trp Gln Gln Thr Thr Ser Lys Ile Tyr Phe Ile Trp Gln Ile Glu Ser
                85                  90                  95

Val Ile Ser Asp Ile Gln Gly Cys Ser Leu Gln Leu Cys Gln Leu Val
            100                 105                 110

Asn Ser Leu Leu Pro Ser Leu Thr Gly Arg Ala Cys Thr Cys Ile Glu
        115                 120                 125

Lys Leu Gln Asp Ile Asn Tyr Glu Asn Met Phe Asp Leu Val Lys Glu
    130                 135                 140

Ser Ser Leu Glu Leu Val Glu Thr Asp Thr Thr Ser Pro Glu Asn Leu
145                 150                 155                 160

Ser Arg Leu Ser Ser Ser Leu Ser Leu Ser Thr Asn Leu Glu Phe Tyr
                165                 170                 175

Met Glu Ala Val Ser Leu Glu Asn Leu Arg Ala Arg Ala Met Arg Ser
            180                 185                 190

Glu Asn Arg Glu Glu Met Asp Leu Ala Asp Lys Met Ile Pro Leu Val
        195                 200                 205

Asn Tyr Met His Asp His Leu Leu Arg Glu Thr Gln Leu Leu Ser Ile
    210                 215                 220

Asn Gly Val Pro Ile Pro Ala Asp Phe Cys Cys Pro Leu Ser Leu Glu
225                 230                 235                 240

Leu Met Ser Asp Pro Val Ile Val Ala Ser Gly Gln Thr Tyr Glu Arg
                245                 250                 255

Val Tyr Ile Lys Leu Trp Leu Asp Glu Gly Phe Thr Ile Cys Pro Lys
            260                 265                 270

Thr Arg Gln Arg Leu Gly His Ser Asn Leu Ile Pro Asn Tyr Thr Val
        275                 280                 285

Lys Ala Leu Ile Ala Asn Trp Cys Glu Ser His Asn Ile Arg Leu Pro
    290                 295                 300

Asp Pro Met Lys Ser Leu Lys Leu Asn Phe Pro Leu Ala Ala Ser Ala
305                 310                 315                 320

Leu Gln Asp Ser Ser Thr Thr Gly Ser Ser Pro Leu His Pro Thr Val
                325                 330                 335

Ala Ala Lys Gly Asn Ile Pro Gly Ser Pro Glu Ala Asp Leu Tyr Met
            340                 345                 350

Arg Ser Leu Asn Arg Ala Ser Pro Pro His Ser Val Val His Gln Asn
        355                 360                 365

Ser His Ala His Val Asn Arg Ala Gly His Glu Ala Ser Ile Lys Gln
    370                 375                 380

Ser Ser Glu Asn Ala Asn Gly Ser Ala Ser Asp Val Ser Arg Leu Ser
385                 390                 395                 400

Leu Ala Gly Ser Glu Thr Arg Glu Ser Ser Leu Glu Glu Arg Asn Ala
                405                 410                 415

Gly Ser Ile Gly Gln Thr Ser Glu Gln Ser Ile Glu Glu Ala Phe Gln
            420                 425                 430

Ala Ser Asn Leu Asp Arg Asp Ser His Asp His Val Gly Ser Ser Ser
        435                 440                 445

Val Asn Gly Ser Leu Pro Asn Ser Gly Gln Leu Asp Ala Glu Cys Asp
    450                 455                 460

Asn Gly Pro Ser Glu Arg Thr Asn Tyr Ser Ser Asp Ala Ser Gly Glu
465                 470                 475                 480

Val Thr Asp Gly Pro Ser Ala Ser Ser Ala Pro Gln Arg Glu His Leu
                485                 490                 495

Ile Pro Ser Arg Leu Ala Asp Val Arg Ser Arg Gly Gln Phe Val Arg
        500                 505                 510

Arg Pro Ser Glu Arg Gly Phe Pro Arg Ile Ile Ser Ser Ser Met
    515                 520                 525

Asp Thr Arg Ser Asp Leu Ser Ala Ile Glu Asn Gln Val Arg Lys Leu
    530                 535                 540

Val Asp Asp Leu Arg Ser Asp Ser Val Asp Val Gln Arg Ser Ala Thr
545                 550                 555                 560

Ser Asp Ile Arg Leu Leu Ala Lys His Asn Met Glu Asn Arg Ile Ile
                565                 570                 575

Ile Ala Asn Cys Gly Ala Ile Asn Leu Leu Val Gly Leu Leu His Ser
        580                 585                 590

Pro Asp Ser Lys Thr Gln Glu His Ala Val Thr Ala Leu Leu Asn Leu
    595                 600                 605

Ser Ile Asn Asp Asn Asn Lys Ile Ala Ile Ala Asn Ala Asp Ala Val
    610                 615                 620

Asp Pro Leu Ile His Val Leu Glu Thr Gly Asn Pro Glu Ala Lys Glu
625                 630                 635                 640

Asn Ser Ala Ala Thr Leu Phe Ser Leu Ser Val Ile Glu Glu Asn Lys
                645                 650                 655

Val Arg Ile Gly Arg Ser Gly Ala Ile Lys Pro Leu Val Asp Leu Leu
        660                 665                 670

Gly Asn Gly Thr Pro Arg Gly Lys Lys Asp Ala Ala Thr Ala Leu Phe
    675                 680                 685

Asn Leu Ser Ile Leu His Glu Asn Lys Ala Arg Ile Val Gln Ala Asp
    690                 695                 700

Ala Val Lys Tyr Leu Val Glu Leu Met Asp Pro Ala Ala Gly Met Val
705                 710                 715                 720

Asp Lys Ala Val Ala Val Leu Ala Asn Leu Ala Thr Ile Pro Glu Gly
                725                 730                 735

Arg Thr Ala Ile Gly Gln Ala Arg Gly Ile Pro Ala Leu Val Glu Val

740                 745                 750

Val Glu Leu Gly Ser Ala Arg Gly Lys Glu Asn Ala Ala Ala Leu
        755                 760                 765

Leu Gln Leu Cys Thr Asn Ser Ser Arg Phe Cys Ser Ile Val Leu Gln
    770                 775                 780

Glu Gly Ala Val Pro Pro Leu Val Ala Leu Ser Gln Ser Gly Thr Pro
785                 790                 795                 800

Arg Ala Arg Glu Lys Ala Gln Ala Leu Leu Ser Tyr Phe Arg Ser Gln
                805                 810                 815

Arg His Gly Asn Ser Ala Arg Arg
            820

<210>  5
<211>  2370
<212>  DNA
<213>  Oryza sativa

<220>
<221>  CDS
<222>  (1)..(2370)

<400>  5
atg gcg ttt gtt tgt ggt ggt ggg caa gtg atg gat tca gtg tca ttg      48
Met Ala Phe Val Cys Gly Gly Gly Gln Val Met Asp Ser Val Ser Leu
1               5                   10                  15

tca cta ctc gat agt att tca aat ttc cgg gtg ctg tct tca agc aat      96
Ser Leu Leu Asp Ser Ile Ser Asn Phe Arg Val Leu Ser Ser Ser Asn
            20                  25                  30

gcc tcg aaa aca gag cta gtt aag aaa tat tgc caa acg atg gat ggc      144
Ala Ser Lys Thr Glu Leu Val Lys Lys Tyr Cys Gln Thr Met Asp Gly
        35                  40                  45

atc ctt gat cac ttg gag gtg gcc cta aac aga gct ttt cct cag att      192
Ile Leu Asp His Leu Glu Val Ala Leu Asn Arg Ala Phe Pro Gln Ile
    50                  55                  60

act cca gat ggt gaa cta agt aaa gtt att cag gct gat tca att att      240
Thr Pro Asp Gly Glu Leu Ser Lys Val Ile Gln Ala Asp Ser Ile Ile
65                  70                  75                  80

gcc aag atg cag ata tat gta ttc gaa tta tgc caa att gtc aat tct      288
Ala Lys Met Gln Ile Tyr Val Phe Glu Leu Cys Gln Ile Val Asn Ser
                85                  90                  95

ctc atg cag att gag tca atg cat ttg gag gat ctt gaa cac gat agc      336
Leu Met Gln Ile Glu Ser Met His Leu Glu Asp Leu Glu His Asp Ser
            100                 105                 110

tgt gga aaa att tca gat gtc att agg gag gct tcc agg gct tta gca 384
Cys Gly Lys Ile Ser Asp Val Ile Arg Glu Ala Ser Arg Ala Leu Ala
        115                 120                 125

ggg gaa gtt atg cca aat tca gag gaa ttt gga aag att caa act act 432
Gly Glu Val Met Pro Asn Ser Glu Glu Phe Gly Lys Ile Gln Thr Thr
    130                 135                 140

ttg agc tta tcc aca aat cag gag ttg ctg atg gaa tat gtt gca ctt 480
Leu Ser Leu Ser Thr Asn Gln Glu Leu Leu Met Glu Tyr Val Ala Leu
145                 150                 155                 160

gtt aag gtt aaa aca aaa ggt aat cat gaa gat aac aaa gaa atg gat 528
Val Lys Val Lys Thr Lys Gly Asn His Glu Asp Asn Lys Glu Met Asp
                165                 170                 175

gat att aac gat att gtt gaa tta gtc aac cat atg ctt gac aaa cat 576
Asp Ile Asn Asp Ile Val Glu Leu Val Asn His Met Leu Asp Lys His
            180                 185                 190

gtg gaa gaa aag caa aca cgt agc att aat gga gtg acc att cct gct 624
Val Glu Glu Lys Gln Thr Arg Ser Ile Asn Gly Val Thr Ile Pro Ala
        195                 200                 205

gat ttt tgt tgt cct ctt tcc ctt gaa cta atg tcg gat cca gtg att 672
Asp Phe Cys Cys Pro Leu Ser Leu Glu Leu Met Ser Asp Pro Val Ile
    210                 215                 220

gtg gca tct ggt caa acg tat gag cat gtt ttt atc aga aaa tgg ttt 720
Val Ala Ser Gly Gln Thr Tyr Glu His Val Phe Ile Arg Lys Trp Phe
225                 230                 235                 240

gat ctg gga tac aac att tgt cca aag aca cgc caa ata ttg gga cac 768
Asp Leu Gly Tyr Asn Ile Cys Pro Lys Thr Arg Gln Ile Leu Gly His
                245                 250                 255

acc aaa ttg att cct aac ttc act gtc aaa cag ttg att gaa aat tgg 816
Thr Lys Leu Ile Pro Asn Phe Thr Val Lys Gln Leu Ile Glu Asn Trp
            260                 265                 270

tgt gag gta cat ggt ata atg cta cca gat cct gtt aaa ctc ttg agt 864
Cys Glu Val His Gly Ile Met Leu Pro Asp Pro Val Lys Leu Leu Ser
        275                 280                 285

ttg tgc ttc cct gtt tcc ctc aac atc aca gat gga agt gca agt gca 912
Leu Cys Phe Pro Val Ser Leu Asn Ile Thr Asp Gly Ser Ala Ser Ala
    290                 295                 300

gac aag tct gga tca cca gaa cac tgc caa ttg gta gct gca ttg cat 960
Asp Lys Ser Gly Ser Pro Glu His Cys Gln Leu Val Ala Ala Leu His
305                 310                 315                 320

cca aaa gca cag tgc gca tcg gat gat agt cat cat tat aat ttg ata 1008
Pro Lys Ala Gln Cys Ala Ser Asp Asp Ser His His Tyr Asn Leu Ile
                325                 330                 335

cat gaa aac tct gat tca gat gat aga gtg tca tca ttt gga gac aca 1056
His Glu Asn Ser Asp Ser Asp Asp Arg Val Ser Ser Phe Gly Asp Thr
            340                 345                 350

gat gat tct gaa cct gat tct tta aga tta tca aca gaa act act gca 1104
Asp Asp Ser Glu Pro Asp Ser Leu Arg Leu Ser Thr Glu Thr Thr Ala
        355                 360                 365

gca aac aaa tct cta ctt gat gaa aaa act gat cgt tct gat ggt ctt 1152
Ala Asn Lys Ser Leu Leu Asp Glu Lys Thr Asp Arg Ser Asp Gly Leu
    370                 375                 380

aag caa ttg aga gac aat ggt ttt caa gtt tct gat gag gaa cag tat 1200
Lys Gln Leu Arg Asp Asn Gly Phe Gln Val Ser Asp Glu Glu Gln Tyr
385                 390                 395                 400

ctc gaa agg aat ggt aaa agt cat atc agc agc cat cat caa ctt gaa 1248
Leu Glu Arg Asn Gly Lys Ser His Ile Ser Ser His His Gln Leu Glu
                405                 410                 415

gtt gat gga gag aat gtc agg gta caa gca tca agt gac atc aat gca 1296
Val Asp Gly Glu Asn Val Arg Val Gln Ala Ser Ser Asp Ile Asn Ala
            420                 425                 430

tct gaa gtt atg caa gat gat ccg gtc acc aca tgt tca aag gta tca 1344
Ser Glu Val Met Gln Asp Asp Pro Val Thr Thr Cys Ser Lys Val Ser
        435                 440                 445

gat aac cct cct aga ttg ggt ggt gtt cgt tct cga aat cag cca aac 1392
Asp Asn Pro Pro Arg Leu Gly Gly Val Arg Ser Arg Asn Gln Pro Asn
    450                 455                 460

tgg tgg aga cag tct aat aaa act att cct agg atc gga ttg tca tct 1440
Trp Trp Arg Gln Ser Asn Lys Thr Ile Pro Arg Ile Gly Leu Ser Ser
465                 470                 475                 480

tcg aca gat tca aaa cca gat ttt tct ggc aat gat gct aaa gtg cgt 1488
Ser Thr Asp Ser Lys Pro Asp Phe Ser Gly Asn Asp Ala Lys Val Arg
                485                 490                 495

aat ctt atc gag gaa ctg aaa agt gat tct gct gag gtc caa agg tca 1536
Asn Leu Ile Glu Glu Leu Lys Ser Asp Ser Ala Glu Val Gln Arg Ser
            500                 505                 510

gca aca gga gag ctc cgc att ctt tct aga cac agc ttg gag aat aga 1584
Ala Thr Gly Glu Leu Arg Ile Leu Ser Arg His Ser Leu Glu Asn Arg
        515                 520                 525

att gcc atc gca aac tgc gga gca atc ccc ttc ttg gtg agt cta ctt 1632
Ile Ala Ile Ala Asn Cys Gly Ala Ile Pro Phe Leu Val Ser Leu Leu
    530                 535                 540

cat tct aca gac ccc agc aca caa gaa aat gct gtg aca att ctc ctg 1680
His Ser Thr Asp Pro Ser Thr Gln Glu Asn Ala Val Thr Ile Leu Leu
545                 550                 555                 560

aat ttg tca ttg gat gac aat aac aag att gcc ata gca agt gct gag 1728
Asn Leu Ser Leu Asp Asp Asn Asn Lys Ile Ala Ile Ala Ser Ala Glu
                565                 570                 575

gcc att gag cct ctc atc ttc gtt ctt cag gtg gga aac ccc gaa gcg 1776
Ala Ile Glu Pro Leu Ile Phe Val Leu Gln Val Gly Asn Pro Glu Ala
            580                 585                 590

aaa gcc aac tca gct gca act tta ttc agc ctc tca gtc att gaa gag 1824

Lys Ala Asn Ser Ala Ala Thr Leu Phe Ser Leu Ser Val Ile Glu Glu
        595                 600                 605

aac aag atc aag att gga cgt tcc ggt gcc atc gaa cca tta gta gat      1872
Asn Lys Ile Lys Ile Gly Arg Ser Gly Ala Ile Glu Pro Leu Val Asp
    610                 615                 620

tta ctg gga gaa ggt acc ccg caa ggg aag aag gat gca gct act gca      1920
Leu Leu Gly Glu Gly Thr Pro Gln Gly Lys Lys Asp Ala Ala Thr Ala
625                 630                 635                 640

ctc ttc aat ctg tcg ata ttt cat gaa cac aag acc cgc att gtt cag      1968
Leu Phe Asn Leu Ser Ile Phe His Glu His Lys Thr Arg Ile Val Gln
                645                 650                 655

gct ggg gct gtc aac cac ctg gtg gag ctg atg gat cca gct gct ggg      2016
Ala Gly Ala Val Asn His Leu Val Glu Leu Met Asp Pro Ala Ala Gly
            660                 665                 670

atg gtt gat aaa gct gtt gct gtt ctg gca aac ctt gcg act gtg cat      2064
Met Val Asp Lys Ala Val Ala Val Leu Ala Asn Leu Ala Thr Val His
        675                 680                 685

gat gga agg aat gcc att gct cag gca gga ggc atc cga gta ctg gtt      2112
Asp Gly Arg Asn Ala Ile Ala Gln Ala Gly Gly Ile Arg Val Leu Val
    690                 695                 700

gag gtt gtt gag ctg ggt tct gca cgt tca aag gag aat gcc gct gct      2160
Glu Val Val Glu Leu Gly Ser Ala Arg Ser Lys Glu Asn Ala Ala Ala
705                 710                 715                 720

gcc ctg cta caa ctc tgc aca aac agt aac agg ttt tgc acc ctg gtt      2208
Ala Leu Leu Gln Leu Cys Thr Asn Ser Asn Arg Phe Cys Thr Leu Val
                725                 730                 735

ctt caa gaa ggc gtc gtg cca cct ttg gtt gca ttg tcg caa tca ggc      2256
Leu Gln Glu Gly Val Val Pro Pro Leu Val Ala Leu Ser Gln Ser Gly
            740                 745                 750

aca gcc cgt gca aga gag aag gct cag gtt ctt cta agc tat ttt cgc      2304
Thr Ala Arg Ala Arg Glu Lys Ala Gln Val Leu Leu Ser Tyr Phe Arg
        755                 760                 765

aac cag cgc cac gtc agg gtt ggg aga ggg ctt agc ttg cta tta gag      2352
Asn Gln Arg His Val Arg Val Gly Arg Gly Leu Ser Leu Leu Leu Glu
    770                 775                 780

tta aaa cgg acc aca taa                                              2370
Leu Lys Arg Thr Thr
785

<210>  6
<211>  789
<212>  PRT
<213>  Oryza sativa

<400>  6

Met Ala Phe Val Cys Gly Gly Gly Gln Val Met Asp Ser Val Ser Leu
1               5                   10                  15

Ser Leu Leu Asp Ser Ile Ser Asn Phe Arg Val Leu Ser Ser Ser Asn
            20                  25                  30

Ala Ser Lys Thr Glu Leu Val Lys Lys Tyr Cys Gln Thr Met Asp Gly
        35                  40                  45

Ile Leu Asp His Leu Glu Val Ala Leu Asn Arg Ala Phe Pro Gln Ile
    50                  55                  60

Thr Pro Asp Gly Glu Leu Ser Lys Val Ile Gln Ala Asp Ser Ile Ile
65                  70                  75                  80

Ala Lys Met Gln Ile Tyr Val Phe Glu Leu Cys Gln Ile Val Asn Ser
                85                  90                  95

Leu Met Gln Ile Glu Ser Met His Leu Glu Asp Leu Glu His Asp Ser
            100                 105                 110

Cys Gly Lys Ile Ser Asp Val Ile Arg Glu Ala Ser Arg Ala Leu Ala
        115                 120                 125

Gly Glu Val Met Pro Asn Ser Glu Glu Phe Gly Lys Ile Gln Thr Thr
    130                 135                 140

Leu Ser Leu Ser Thr Asn Gln Glu Leu Leu Met Glu Tyr Val Ala Leu
145                 150                 155                 160

Val Lys Val Lys Thr Lys Gly Asn His Glu Asp Asn Lys Glu Met Asp
                165                 170                 175

Asp Ile Asn Asp Ile Val Glu Leu Val Asn His Met Leu Asp Lys His
            180                 185                 190

Val Glu Glu Lys Gln Thr Arg Ser Ile Asn Gly Val Thr Ile Pro Ala
        195                 200                 205

Asp Phe Cys Cys Pro Leu Ser Leu Glu Leu Met Ser Asp Pro Val Ile
    210                 215                 220

Val Ala Ser Gly Gln Thr Tyr Glu His Val Phe Ile Arg Lys Trp Phe
225                 230                 235                 240

Asp Leu Gly Tyr Asn Ile Cys Pro Lys Thr Arg Gln Ile Leu Gly His
                245                 250                 255

Thr Lys Leu Ile Pro Asn Phe Thr Val Lys Gln Leu Ile Glu Asn Trp
            260                 265                 270

Cys Glu Val His Gly Ile Met Leu Pro Asp Pro Val Lys Leu Leu Ser
        275                 280                 285

Leu Cys Phe Pro Val Ser Leu Asn Ile Thr Asp Gly Ser Ala Ser Ala
    290                 295                 300

Asp Lys Ser Gly Ser Pro Glu His Cys Gln Leu Val Ala Ala Leu His
305                 310                 315                 320

Pro Lys Ala Gln Cys Ala Ser Asp Asp Ser His His Tyr Asn Leu Ile
                325                 330                 335

His Glu Asn Ser Asp Ser Asp Asp Arg Val Ser Ser Phe Gly Asp Thr
            340                 345                 350

Asp Asp Ser Glu Pro Asp Ser Leu Arg Leu Ser Thr Glu Thr Thr Ala
        355                 360                 365

Ala Asn Lys Ser Leu Leu Asp Glu Lys Thr Asp Arg Ser Asp Gly Leu
    370                 375                 380

Lys Gln Leu Arg Asp Asn Gly Phe Gln Val Ser Asp Glu Glu Gln Tyr
385                 390                 395                 400

Leu Glu Arg Asn Gly Lys Ser His Ile Ser Ser His His Gln Leu Glu
                405                 410                 415

Val Asp Gly Glu Asn Val Arg Val Gln Ala Ser Ser Asp Ile Asn Ala
            420                 425                 430

Ser Glu Val Met Gln Asp Asp Pro Val Thr Thr Cys Ser Lys Val Ser
        435                 440                 445

Asp Asn Pro Pro Arg Leu Gly Gly Val Arg Ser Arg Asn Gln Pro Asn
    450                 455                 460

Trp Trp Arg Gln Ser Asn Lys Thr Ile Pro Arg Ile Gly Leu Ser Ser
465                 470                 475                 480

Ser Thr Asp Ser Lys Pro Asp Phe Ser Gly Asn Asp Ala Lys Val Arg
                485                 490                 495

Asn Leu Ile Glu Glu Leu Lys Ser Asp Ser Ala Glu Val Gln Arg Ser
            500                 505                 510

Ala Thr Gly Glu Leu Arg Ile Leu Ser Arg His Ser Leu Glu Asn Arg
        515                 520                 525

Ile Ala Ile Ala Asn Cys Gly Ala Ile Pro Phe Leu Val Ser Leu Leu
    530                 535                 540

His Ser Thr Asp Pro Ser Thr Gln Glu Asn Ala Val Thr Ile Leu Leu
545                 550                 555                 560

Asn Leu Ser Leu Asp Asp Asn Asn Lys Ile Ala Ile Ala Ser Ala Glu
                565                 570                 575

Ala Ile Glu Pro Leu Ile Phe Val Leu Gln Val Gly Asn Pro Glu Ala
            580                 585                 590

Lys Ala Asn Ser Ala Ala Thr Leu Phe Ser Leu Ser Val Ile Glu Glu
        595                 600                 605

Asn Lys Ile Lys Ile Gly Arg Ser Gly Ala Ile Glu Pro Leu Val Asp
    610                 615                 620

Leu Leu Gly Glu Gly Thr Pro Gln Gly Lys Lys Asp Ala Ala Thr Ala
625                 630                 635                 640

Leu Phe Asn Leu Ser Ile Phe His Glu His Lys Thr Arg Ile Val Gln
                645                 650                 655

Ala Gly Ala Val Asn His Leu Val Glu Leu Met Asp Pro Ala Ala Gly
            660                 665                 670

Met Val Asp Lys Ala Val Ala Val Leu Ala Asn Leu Ala Thr Val His
        675                 680                 685

Asp Gly Arg Asn Ala Ile Ala Gln Ala Gly Gly Ile Arg Val Leu Val
    690                 695                 700

Glu Val Val Glu Leu Gly Ser Ala Arg Ser Lys Glu Asn Ala Ala Ala
705                 710                 715                 720

Ala Leu Leu Gln Leu Cys Thr Asn Ser Asn Arg Phe Cys Thr Leu Val
                725                 730                 735

Leu Gln Glu Gly Val Val Pro Pro Leu Val Ala Leu Ser Gln Ser Gly

740                 745                 750

Thr Ala Arg Ala Arg Glu Lys Ala Gln Val Leu Leu Ser Tyr Phe Arg
        755                 760                 765

Asn Gln Arg His Val Arg Val Gly Arg Gly Leu Ser Leu Leu Leu Glu
    770                 775                 780

Leu Lys Arg Thr Thr
785

<210>  7
<211>  2391
<212>  DNA
<213>  Oryza sativa

<220>
<221>  CDS
<222>  (1)..(2391)

<400>  7
atg gat tca gtg tca ttg tca cta ctc gat agt att tca aat ttc cgg      48
Met Asp Ser Val Ser Leu Ser Leu Leu Asp Ser Ile Ser Asn Phe Arg
1               5                   10                  15

gtg ctg tct tca agc aat gcc tcg aaa aca gag cta gtt aag aaa tat      96
Val Leu Ser Ser Ser Asn Ala Ser Lys Thr Glu Leu Val Lys Lys Tyr
            20                  25                  30

tgc caa acg atg gat ggc atc ctt gat cac ttg gag gtg gcc cta aac      144
Cys Gln Thr Met Asp Gly Ile Leu Asp His Leu Glu Val Ala Leu Asn
        35                  40                  45

aga gct ttt cct cag att act cca gat ggt gaa cta agt aaa gtg ctt      192
Arg Ala Phe Pro Gln Ile Thr Pro Asp Gly Glu Leu Ser Lys Val Leu
    50                  55                  60

gaa gaa ctt ggc gct acc atc aat gaa gcg act gag cta gtt gga ggc      240
Glu Glu Leu Gly Ala Thr Ile Asn Glu Ala Thr Glu Leu Val Gly Gly
65                  70                  75                  80

tgg aat caa atg atg agc aag att tat ttt gtt att cag gct gat tca      288
Trp Asn Gln Met Met Ser Lys Ile Tyr Phe Val Ile Gln Ala Asp Ser
                85                  90                  95

att att gcc aag atg cag ata tat gta ttc gaa tta tgc caa att gtc      336
Ile Ile Ala Lys Met Gln Ile Tyr Val Phe Glu Leu Cys Gln Ile Val
            100                 105                 110

aat tct ctc atg cag att gag tca atg cat ttg gag gat ctt gaa cac      384
Asn Ser Leu Met Gln Ile Glu Ser Met His Leu Glu Asp Leu Glu His
        115                 120                 125

gat agc tgt gga aaa att tca gat gtc att agg gag gct tcc agg gct      432
Asp Ser Cys Gly Lys Ile Ser Asp Val Ile Arg Glu Ala Ser Arg Ala
    130                 135                 140

tta gca ggg gaa gtt atg cca aat tca gag gaa ttt gga aag att caa 480
Leu Ala Gly Glu Val Met Pro Asn Ser Glu Glu Phe Gly Lys Ile Gln
145                 150                 155                 160

act act ttg agc tta tcc aca aat cag gag ttg ctg atg gaa tat gtt 528
Thr Thr Leu Ser Leu Ser Thr Asn Gln Glu Leu Leu Met Glu Tyr Val
                165                 170                 175

gca ctt gtt aag gtt aaa aca aaa ggt aat cat gaa gat aac aaa gaa 576
Ala Leu Val Lys Val Lys Thr Lys Gly Asn His Glu Asp Asn Lys Glu
            180                 185                 190

atg gat gat att aac gat att gtt gaa tta gtc aac cat atg ctt gac 624
Met Asp Asp Ile Asn Asp Ile Val Glu Leu Val Asn His Met Leu Asp
        195                 200                 205

aaa cat gtg gaa gaa aag caa aca cgt agc att aat gga gtg acc att 672
Lys His Val Glu Glu Lys Gln Thr Arg Ser Ile Asn Gly Val Thr Ile
    210                 215                 220

cct gct gat ttt tgt tgt cct ctt tcc ctt gaa cta atg tcg gat cca 720
Pro Ala Asp Phe Cys Cys Pro Leu Ser Leu Glu Leu Met Ser Asp Pro
225                 230                 235                 240

gtg att gtg gca tct ggt caa acg tat gag cat gtt ttt atc aga aaa 768
Val Ile Val Ala Ser Gly Gln Thr Tyr Glu His Val Phe Ile Arg Lys
                245                 250                 255

tgg ttt gat ctg gga tac aac att tgt cca aag aca cgc caa ata ttg 816
Trp Phe Asp Leu Gly Tyr Asn Ile Cys Pro Lys Thr Arg Gln Ile Leu
            260                 265                 270

gga cac acc aaa ttg att cct aac ttc act gtc aaa cag ttg att gaa 864
Gly His Thr Lys Leu Ile Pro Asn Phe Thr Val Lys Gln Leu Ile Glu
        275                 280                 285

aat tgg tgt gag gta cat ggt ata atg cta cca gat cct gtt aaa ctc 912
Asn Trp Cys Glu Val His Gly Ile Met Leu Pro Asp Pro Val Lys Leu
    290                 295                 300

ttg agt ttg tgc ttc cct gtt tcc ctc aac atc aca gat gga agt gca 960
Leu Ser Leu Cys Phe Pro Val Ser Leu Asn Ile Thr Asp Gly Ser Ala
305                 310                 315                 320

agt gca gac aag tct gga tca cca gaa cac tgc caa ttg gta gct gca 1008
Ser Ala Asp Lys Ser Gly Ser Pro Glu His Cys Gln Leu Val Ala Ala
                325                 330                 335

ttg cat cca aaa gca cag tgc gca tcg gat gat agt cat cat tat aat 1056
Leu His Pro Lys Ala Gln Cys Ala Ser Asp Asp Ser His His Tyr Asn
            340                 345                 350

ttg ata cat gaa aac tct gat tca gat gat aga gtg tca tca ttt gga 1104
Leu Ile His Glu Asn Ser Asp Ser Asp Asp Arg Val Ser Ser Phe Gly
        355                 360                 365

gac aca gat gat tct gaa cct gat tct tta aga tta tca aca gaa act 1152
Asp Thr Asp Asp Ser Glu Pro Asp Ser Leu Arg Leu Ser Thr Glu Thr
    370                 375                 380

act gca gca aac aaa tct cta ctt gat gaa aaa act gat cgt tct gat     1200
Thr Ala Ala Asn Lys Ser Leu Leu Asp Glu Lys Thr Asp Arg Ser Asp
385                 390                 395                 400

ggt ctt aag caa ttg aga gac aat ggt ttt caa gtt tct gat gag gaa     1248
Gly Leu Lys Gln Leu Arg Asp Asn Gly Phe Gln Val Ser Asp Glu Glu
                405                 410                 415

cag tat ctc gaa agg aat ggt aaa agt cat atc agc agc cat cat caa     1296
Gln Tyr Leu Glu Arg Asn Gly Lys Ser His Ile Ser Ser His His Gln
            420                 425                 430

ctt gaa gtt gat gga gag aat gtc agg gta caa gca tca agt gac atc     1344
Leu Glu Val Asp Gly Glu Asn Val Arg Val Gln Ala Ser Ser Asp Ile
        435                 440                 445

aat gca tct gaa gtt atg caa gat gat ccg gtc acc aca tgt tca aag     1392
Asn Ala Ser Glu Val Met Gln Asp Asp Pro Val Thr Thr Cys Ser Lys
    450                 455                 460

gta tca gat aac cct cct aga ttg ggt ggt gtt cgt tct cga aat cag     1440
Val Ser Asp Asn Pro Pro Arg Leu Gly Gly Val Arg Ser Arg Asn Gln
465                 470                 475                 480

cca aac tgg tgg aga cag tct aat aaa act att cct agg atc gga ttg     1488
Pro Asn Trp Trp Arg Gln Ser Asn Lys Thr Ile Pro Arg Ile Gly Leu
                485                 490                 495

tca tct tcg aca gat tca aaa cca gat ttt tct ggc aat gat gct aaa     1536
Ser Ser Ser Thr Asp Ser Lys Pro Asp Phe Ser Gly Asn Asp Ala Lys
            500                 505                 510

gtg cgt aat ctt atc gag gaa ctg aaa agt gat tct gct gag gtc caa     1584
Val Arg Asn Leu Ile Glu Glu Leu Lys Ser Asp Ser Ala Glu Val Gln
        515                 520                 525

agg tca gca aca gga gag ctc cgc att ctt tct aga cac agc ttg gag     1632
Arg Ser Ala Thr Gly Glu Leu Arg Ile Leu Ser Arg His Ser Leu Glu
    530                 535                 540

aat aga att gcc atc gca aac tgc gga gca atc ccc ttc ttg gtg agt     1680
Asn Arg Ile Ala Ile Ala Asn Cys Gly Ala Ile Pro Phe Leu Val Ser
545                 550                 555                 560

cta ctt cat tct aca gac ccc agc aca caa gaa aat gct gtg aca att     1728
Leu Leu His Ser Thr Asp Pro Ser Thr Gln Glu Asn Ala Val Thr Ile
                565                 570                 575

ctc ctg aat ttg tca ttg gat gac aat aac aag att gcc ata gca agt     1776
Leu Leu Asn Leu Ser Leu Asp Asp Asn Asn Lys Ile Ala Ile Ala Ser
            580                 585                 590

gct gag gcc att gag cct ctc atc ttc gtt ctt cag gtg gga aac ccc     1824
Ala Glu Ala Ile Glu Pro Leu Ile Phe Val Leu Gln Val Gly Asn Pro
        595                 600                 605

gaa gcg aaa gcc aac tca gct gca act tta ttc agc ctc tca gtc att     1872
Glu Ala Lys Ala Asn Ser Ala Ala Thr Leu Phe Ser Leu Ser Val Ile
    610                 615                 620

gaa gag aac aag atc aag att gga cgt tcc ggt gcc atc gaa cca tta     1920

Glu Glu Asn Lys Ile Lys Ile Gly Arg Ser Gly Ala Ile Glu Pro Leu
625                 630                 635                 640

gta gat tta ctg gga gaa ggt acc ccg caa ggg aag aag gat gca gct      1968
Val Asp Leu Leu Gly Glu Gly Thr Pro Gln Gly Lys Lys Asp Ala Ala
                645                 650                 655

act gca ctc ttc aat ctg tcg ata ttt cat gaa cac aag acc cgc att      2016
Thr Ala Leu Phe Asn Leu Ser Ile Phe His Glu His Lys Thr Arg Ile
            660                 665                 670

gtt cag gct ggg gct gtc aac cac ctg gtg gag ctg atg gat cca gct      2064
Val Gln Ala Gly Ala Val Asn His Leu Val Glu Leu Met Asp Pro Ala
        675                 680                 685

gct ggg atg gtt gat aaa gct gtt gct gtt ctg gca aac ctt gcg act      2112
Ala Gly Met Val Asp Lys Ala Val Ala Val Leu Ala Asn Leu Ala Thr
    690                 695                 700

gtg cat gat gga agg aat gcc att gct cag gca gga ggc atc cga gta      2160
Val His Asp Gly Arg Asn Ala Ile Ala Gln Ala Gly Gly Ile Arg Val
705                 710                 715                 720

ctg gtt gag gtt gtt gag ctg ggt tct gca cgt tca aag gag aat gcc      2208
Leu Val Glu Val Val Glu Leu Gly Ser Ala Arg Ser Lys Glu Asn Ala
                725                 730                 735

gct gct gcc ctg cta caa ctc tgc aca aac agt aac agg ttt tgc acc      2256
Ala Ala Ala Leu Leu Gln Leu Cys Thr Asn Ser Asn Arg Phe Cys Thr
            740                 745                 750

ctg gtt ctt caa gaa ggc gtc gtg cca cct ttg gtt gca ttg tcg caa      2304
Leu Val Leu Gln Glu Gly Val Val Pro Pro Leu Val Ala Leu Ser Gln
        755                 760                 765

tca ggc aca gcc cgt gca aga gag aag gct cag gtt ctt cta agc tat      2352
Ser Gly Thr Ala Arg Ala Arg Glu Lys Ala Gln Val Leu Leu Ser Tyr
    770                 775                 780

ttt cgc aac cag cgc cac gtc agg gtt ggg aga ggg taa                  2391
Phe Arg Asn Gln Arg His Val Arg Val Gly Arg Gly
785                 790                 795

<210>  8
<211>  796
<212>  PRT
<213>  Oryza sativa

<400>  8

Met Asp Ser Val Ser Leu Ser Leu Leu Asp Ser Ile Ser Asn Phe Arg
1               5                   10                  15

Val Leu Ser Ser Ser Asn Ala Ser Lys Thr Glu Leu Val Lys Lys Tyr
            20                  25                  30

Cys Gln Thr Met Asp Gly Ile Leu Asp His Leu Glu Val Ala Leu Asn
        35                  40                  45

Arg Ala Phe Pro Gln Ile Thr Pro Asp Gly Glu Leu Ser Lys Val Leu
    50                  55                  60

Glu Glu Leu Gly Ala Thr Ile Asn Glu Ala Thr Glu Leu Val Gly Gly
65                  70                  75                  80

Trp Asn Gln Met Met Ser Lys Ile Tyr Phe Val Ile Gln Ala Asp Ser
                85                  90                  95

Ile Ile Ala Lys Met Gln Ile Tyr Val Phe Glu Leu Cys Gln Ile Val
            100                 105                 110

Asn Ser Leu Met Gln Ile Glu Ser Met His Leu Glu Asp Leu Glu His
        115                 120                 125

Asp Ser Cys Gly Lys Ile Ser Asp Val Ile Arg Glu Ala Ser Arg Ala
    130                 135                 140

Leu Ala Gly Glu Val Met Pro Asn Ser Glu Glu Phe Gly Lys Ile Gln
145                 150                 155                 160

Thr Thr Leu Ser Leu Ser Thr Asn Gln Glu Leu Leu Met Glu Tyr Val
                165                 170                 175

Ala Leu Val Lys Val Lys Thr Lys Gly Asn His Glu Asp Asn Lys Glu
            180                 185                 190

Met Asp Asp Ile Asn Asp Ile Val Glu Leu Val Asn His Met Leu Asp
        195                 200                 205

Lys His Val Glu Glu Lys Gln Thr Arg Ser Ile Asn Gly Val Thr Ile
    210                 215                 220

Pro Ala Asp Phe Cys Cys Pro Leu Ser Leu Glu Leu Met Ser Asp Pro
225                 230                 235                 240

Val Ile Val Ala Ser Gly Gln Thr Tyr Glu His Val Phe Ile Arg Lys
                245                 250                 255

Trp Phe Asp Leu Gly Tyr Asn Ile Cys Pro Lys Thr Arg Gln Ile Leu
            260                 265                 270

Gly His Thr Lys Leu Ile Pro Asn Phe Thr Val Lys Gln Leu Ile Glu
        275                 280                 285

Asn Trp Cys Glu Val His Gly Ile Met Leu Pro Asp Pro Val Lys Leu
    290                 295                 300

Leu Ser Leu Cys Phe Pro Val Ser Leu Asn Ile Thr Asp Gly Ser Ala
305                 310                 315                 320

Ser Ala Asp Lys Ser Gly Ser Pro Glu His Cys Gln Leu Val Ala Ala
                325                 330                 335

Leu His Pro Lys Ala Gln Cys Ala Ser Asp Asp Ser His His Tyr Asn
            340                 345                 350

Leu Ile His Glu Asn Ser Asp Ser Asp Asp Arg Val Ser Ser Phe Gly
        355                 360                 365

Asp Thr Asp Asp Ser Glu Pro Asp Ser Leu Arg Leu Ser Thr Glu Thr
    370                 375                 380

Thr Ala Ala Asn Lys Ser Leu Leu Asp Glu Lys Thr Asp Arg Ser Asp
385                 390                 395                 400

Gly Leu Lys Gln Leu Arg Asp Asn Gly Phe Gln Val Ser Asp Glu Glu
                405                 410                 415

Gln Tyr Leu Glu Arg Asn Gly Lys Ser His Ile Ser Ser His His Gln
            420                 425                 430

Leu Glu Val Asp Gly Glu Asn Val Arg Val Gln Ala Ser Ser Asp Ile
        435                 440                 445

Asn Ala Ser Glu Val Met Gln Asp Asp Pro Val Thr Thr Cys Ser Lys
    450                 455                 460

Val Ser Asp Asn Pro Pro Arg Leu Gly Gly Val Arg Ser Arg Asn Gln
465                 470                 475                 480

Pro Asn Trp Trp Arg Gln Ser Asn Lys Thr Ile Pro Arg Ile Gly Leu
                485                 490                 495

Ser Ser Ser Thr Asp Ser Lys Pro Asp Phe Ser Gly Asn Asp Ala Lys
            500                 505                 510

Val Arg Asn Leu Ile Glu Glu Leu Lys Ser Asp Ser Ala Glu Val Gln
        515                 520                 525

Arg Ser Ala Thr Gly Glu Leu Arg Ile Leu Ser Arg His Ser Leu Glu
    530                 535                 540

Asn Arg Ile Ala Ile Ala Asn Cys Gly Ala Ile Pro Phe Leu Val Ser
545                 550                 555                 560

Leu Leu His Ser Thr Asp Pro Ser Thr Gln Glu Asn Ala Val Thr Ile
                565                 570                 575

Leu Leu Asn Leu Ser Leu Asp Asp Asn Asn Lys Ile Ala Ile Ala Ser
            580                 585                 590

Ala Glu Ala Ile Glu Pro Leu Ile Phe Val Leu Gln Val Gly Asn Pro
        595                 600                 605

Glu Ala Lys Ala Asn Ser Ala Ala Thr Leu Phe Ser Leu Ser Val Ile
    610                 615                 620

Glu Glu Asn Lys Ile Lys Ile Gly Arg Ser Gly Ala Ile Glu Pro Leu
625                 630                 635                 640

Val Asp Leu Leu Gly Glu Gly Thr Pro Gln Gly Lys Lys Asp Ala Ala
                645                 650                 655

Thr Ala Leu Phe Asn Leu Ser Ile Phe His Glu His Lys Thr Arg Ile
            660                 665                 670

Val Gln Ala Gly Ala Val Asn His Leu Val Glu Leu Met Asp Pro Ala
        675                 680                 685

Ala Gly Met Val Asp Lys Ala Val Ala Val Leu Ala Asn Leu Ala Thr
    690                 695                 700

Val His Asp Gly Arg Asn Ala Ile Ala Gln Ala Gly Gly Ile Arg Val
705                 710                 715                 720

Leu Val Glu Val Val Glu Leu Gly Ser Ala Arg Ser Lys Glu Asn Ala
                725                 730                 735

Ala Ala Ala Leu Leu Gln Leu Cys Thr Asn Ser Asn Arg Phe Cys Thr
            740                 745                 750

Leu Val Leu Gln Glu Gly Val Val Pro Pro Leu Val Ala Leu Ser Gln
        755                 760                 765

Ser Gly Thr Ala Arg Ala Arg Glu Lys Ala Gln Val Leu Leu Ser Tyr

770                 775                 780

Phe Arg Asn Gln Arg His Val Arg Val Gly Arg Gly
785                 790                 795

<210>  9
<211>  1404
<212>  DNA
<213>  Oryza sativa

<220>
<221>  CDS
<222>  (1)..(1404)

<400>  9
atg gtg tcg cta gcc ggc tcc cag atc ccg tcg ccg ggg cag agt ccg      48
Met Val Ser Leu Ala Gly Ser Gln Ile Pro Ser Pro Gly Gln Ser Pro
1               5                   10                  15

tgc gcg gcg gcg cgg tcg cag cgc cgc ggc gcg ggg tac tcc atg cgg      96
Cys Ala Ala Ala Arg Ser Gln Arg Arg Gly Ala Gly Tyr Ser Met Arg
            20                  25                  30

acc atc cgg tcg gcg ctg ctg cag ccg gac tcc tgc ccg ggc tcg ccg      144
Thr Ile Arg Ser Ala Leu Leu Gln Pro Asp Ser Cys Pro Gly Ser Pro
        35                  40                  45

cat gtg gcg gcc gcg tac gac gcg gcg ggg gcg gac tcg gac atg gag      192
His Val Ala Ala Ala Tyr Asp Ala Ala Gly Ala Asp Ser Asp Met Glu
    50                  55                  60

aac ttg acg gac tcc gtg att gat ttc cat ctc agc gag ctg gcg gcc      240
Asn Leu Thr Asp Ser Val Ile Asp Phe His Leu Ser Glu Leu Ala Ala
65                  70                  75                  80

acc gcg ggg ccc gcg cac ccc gcg gcg gtg gcc aag tcg tcg tcg gcc      288
Thr Ala Gly Pro Ala His Pro Ala Ala Val Ala Lys Ser Ser Ser Ala
                85                  90                  95

aac gcg gcg gcc acg gag atg ctc gag ctc tcg cgg gac ttc agt gac      336
Asn Ala Ala Ala Thr Glu Met Leu Glu Leu Ser Arg Asp Phe Ser Asp
            100                 105                 110

tac tcg agc ttc aac tcg gat atc tcc ggc gag ctc gag cgg ctc gcg      384
Tyr Ser Ser Phe Asn Ser Asp Ile Ser Gly Glu Leu Glu Arg Leu Ala
        115                 120                 125

gcg gcg gcg gcg gcg gtg gtg acg ccc aga tcc gac gcg ccg cag gtg      432
Ala Ala Ala Ala Ala Val Val Thr Pro Arg Ser Asp Ala Pro Gln Val
    130                 135                 140

ggc gcc gtg gat ctg aat gag ctt gag tcg atg gat ctg tcc gtc gag      480
Gly Ala Val Asp Leu Asn Glu Leu Glu Ser Met Asp Leu Ser Val Glu
145                 150                 155                 160

gcg gcg ccg ctg gag cgc gtg gag ccg ttc gtg ctg gcg tgc gtg cgg      528
Ala Ala Pro Leu Glu Arg Val Glu Pro Phe Val Leu Ala Cys Val Arg
                165                 170                 175

gcg ctg ggg ccc gac gcc gcg cca gac gcg cgg cgc acc gcg gcg gcg     576
Ala Leu Gly Pro Asp Ala Ala Pro Asp Ala Arg Arg Thr Ala Ala Ala
            180                 185                 190

agg ata agg ctg ctg gcg aag cac agg tcg gac atc cgc gag ctg atc     624
Arg Ile Arg Leu Leu Ala Lys His Arg Ser Asp Ile Arg Glu Leu Ile
        195                 200                 205

ggc gtg tcc ggc gcc atc ccg gcg ctg gtg ccg ctg ctg cgg agc acc     672
Gly Val Ser Gly Ala Ile Pro Ala Leu Val Pro Leu Leu Arg Ser Thr
    210                 215                 220

gac ccg gtg gcg cag gag agc gcg gtg acg gcg ctg ctc aac ctc tcg     720
Asp Pro Val Ala Gln Glu Ser Ala Val Thr Ala Leu Leu Asn Leu Ser
225                 230                 235                 240

ctc gag gag cgg aac cgg tcg gcc atc acg gcg gcg ggg gcc atc aag     768
Leu Glu Glu Arg Asn Arg Ser Ala Ile Thr Ala Ala Gly Ala Ile Lys
                245                 250                 255

ccg ctc gtg tac gcg ctg cgg acg ggc acc gcg tcg gcc aag cag aac     816
Pro Leu Val Tyr Ala Leu Arg Thr Gly Thr Ala Ser Ala Lys Gln Asn
            260                 265                 270

gcc gcg tgc gcg ctg ctc agc ctc tcg ggc atc gag gag aac cgc gcc     864
Ala Ala Cys Ala Leu Leu Ser Leu Ser Gly Ile Glu Glu Asn Arg Ala
        275                 280                 285

acc atc ggc gcg tgc ggc gcc atc cct ccc ctc gtc gcg ctg ctc tcc     912
Thr Ile Gly Ala Cys Gly Ala Ile Pro Pro Leu Val Ala Leu Leu Ser
    290                 295                 300

gcg ggc tcc acc cgc ggc aag aag gac gcg ctc acc acg ctc tac cgg     960
Ala Gly Ser Thr Arg Gly Lys Lys Asp Ala Leu Thr Thr Leu Tyr Arg
305                 310                 315                 320

ctc tgc tcg gcg cgc cgg aac aag gag cgc gcg gtc agc gcc ggc gcc     1008
Leu Cys Ser Ala Arg Arg Asn Lys Glu Arg Ala Val Ser Ala Gly Ala
                325                 330                 335

gtc gtg ccg ctc atc cac ctc gtc ggc gag cgt ggc agc ggg acg tcg     1056
Val Val Pro Leu Ile His Leu Val Gly Glu Arg Gly Ser Gly Thr Ser
            340                 345                 350

gag aag gca atg gtg gtc ctc gcc agc ctc gcg ggc atc gtc gag ggc     1104
Glu Lys Ala Met Val Val Leu Ala Ser Leu Ala Gly Ile Val Glu Gly
        355                 360                 365

cgc gac gcc gtg gtg gag gct ggc ggg ata ccg gcg ctt gtc gag acc     1152
Arg Asp Ala Val Val Glu Ala Gly Gly Ile Pro Ala Leu Val Glu Thr
    370                 375                 380

atc gag gac ggc ccg gcg agg gag agg gag ttc gcc gtg gtg gcg ctg     1200
Ile Glu Asp Gly Pro Ala Arg Glu Arg Glu Phe Ala Val Val Ala Leu
385                 390                 395                 400

ctg cag ctc tgc tcc gag tgc ccc cgc aac cgc gcg ctt ctt gtc cgt     1248
Leu Gln Leu Cys Ser Glu Cys Pro Arg Asn Arg Ala Leu Leu Val Arg
                405                 410                 415

gag ggc gcc atc cca ccg ctt gtc gcg ctc tcg cag tcc ggc tct gcc     1296
Glu Gly Ala Ile Pro Pro Leu Val Ala Leu Ser Gln Ser Gly Ser Ala
            420                 425                 430

cgt gcc aag cac aag gct gaa act ttg ctt ggg tat ctc cgc gag caa     1344
Arg Ala Lys His Lys Ala Glu Thr Leu Leu Gly Tyr Leu Arg Glu Gln
        435                 440                 445

cgg caa gga ggt ggt ggc tgc agg gtt gaa ccc gtg gca gct tcg agc     1392
Arg Gln Gly Gly Gly Gly Cys Arg Val Glu Pro Val Ala Ala Ser Ser
    450                 455                 460

ttg gcc agg taa                                                     1404
Leu Ala Arg
465

<210>  10
<211>  467
<212>  PRT
<213>  Oryza sativa

<400>  10

Met Val Ser Leu Ala Gly Ser Gln Ile Pro Ser Pro Gly Gln Ser Pro
1               5                   10                  15

Cys Ala Ala Ala Arg Ser Gln Arg Arg Gly Ala Gly Tyr Ser Met Arg
            20                  25                  30

Thr Ile Arg Ser Ala Leu Leu Gln Pro Asp Ser Cys Pro Gly Ser Pro
        35                  40                  45

His Val Ala Ala Ala Tyr Asp Ala Ala Gly Ala Asp Ser Asp Met Glu
    50                  55                  60

Asn Leu Thr Asp Ser Val Ile Asp Phe His Leu Ser Glu Leu Ala Ala
65                  70                  75                  80

Thr Ala Gly Pro Ala His Pro Ala Ala Val Ala Lys Ser Ser Ser Ala
                85                  90                  95

Asn Ala Ala Ala Thr Glu Met Leu Glu Leu Ser Arg Asp Phe Ser Asp
            100                 105                 110

Tyr Ser Ser Phe Asn Ser Asp Ile Ser Gly Glu Leu Glu Arg Leu Ala
        115                 120                 125

Ala Ala Ala Ala Ala Val Val Thr Pro Arg Ser Asp Ala Pro Gln Val
    130                 135                 140

Gly Ala Val Asp Leu Asn Glu Leu Glu Ser Met Asp Leu Ser Val Glu

145                 150                 155                 160

Ala Ala Pro Leu Glu Arg Val Glu Pro Phe Val Leu Ala Cys Val Arg
                165                 170                 175

Ala Leu Gly Pro Asp Ala Ala Pro Asp Ala Arg Arg Thr Ala Ala Ala
            180                 185                 190

Arg Ile Arg Leu Leu Ala Lys His Arg Ser Asp Ile Arg Glu Leu Ile
        195                 200                 205

Gly Val Ser Gly Ala Ile Pro Ala Leu Val Pro Leu Leu Arg Ser Thr
    210                 215                 220

Asp Pro Val Ala Gln Glu Ser Ala Val Thr Ala Leu Leu Asn Leu Ser
225                 230                 235                 240

Leu Glu Glu Arg Asn Arg Ser Ala Ile Thr Ala Ala Gly Ala Ile Lys
                245                 250                 255

Pro Leu Val Tyr Ala Leu Arg Thr Gly Thr Ala Ser Ala Lys Gln Asn
            260                 265                 270

Ala Ala Cys Ala Leu Leu Ser Leu Ser Gly Ile Glu Glu Asn Arg Ala
        275                 280                 285

Thr Ile Gly Ala Cys Gly Ala Ile Pro Pro Leu Val Ala Leu Leu Ser
    290                 295                 300

Ala Gly Ser Thr Arg Gly Lys Lys Asp Ala Leu Thr Thr Leu Tyr Arg
305                 310                 315                 320

Leu Cys Ser Ala Arg Arg Asn Lys Glu Arg Ala Val Ser Ala Gly Ala
                325                 330                 335

Val Val Pro Leu Ile His Leu Val Gly Glu Arg Gly Ser Gly Thr Ser
            340                 345                 350

Glu Lys Ala Met Val Val Leu Ala Ser Leu Ala Gly Ile Val Glu Gly
        355                 360                 365

Arg Asp Ala Val Val Glu Ala Gly Gly Ile Pro Ala Leu Val Glu Thr
    370                 375                 380

Ile Glu Asp Gly Pro Ala Arg Glu Arg Glu Phe Ala Val Val Ala Leu
385                 390                 395                 400

Leu Gln Leu Cys Ser Glu Cys Pro Arg Asn Arg Ala Leu Leu Val Arg
                405                 410                 415

Glu Gly Ala Ile Pro Pro Leu Val Ala Leu Ser Gln Ser Gly Ser Ala
            420                 425                 430

Arg Ala Lys His Lys Ala Glu Thr Leu Leu Gly Tyr Leu Arg Glu Gln
        435                 440                 445

Arg Gln Gly Gly Gly Gly Cys Arg Val Glu Pro Val Ala Ala Ser Ser
    450                 455                 460

Leu Ala Arg
465

<210>  11
<211>  2373
<212>  DNA
<213>  Nicotiana tabacum

<220>
<221>  CDS
<222>  (1)..(2373)

<400>  11
atg gag ata tca ttg tta aaa gtg ctt ctc aac aat atc tcc tgt ttt      48
Met Glu Ile Ser Leu Leu Lys Val Leu Leu Asn Asn Ile Ser Cys Phe
1               5                   10                  15

tcc cat tta tca tca agt gat cac ata agt ggt gaa ctg gtt cgt aga      96
Ser His Leu Ser Ser Ser Asp His Ile Ser Gly Glu Leu Val Arg Arg
            20                  25                  30

tat tat tgt aag att gag gat ata ctg aag ctt gta aag ccg att ctt      144
Tyr Tyr Cys Lys Ile Glu Asp Ile Leu Lys Leu Val Lys Pro Ile Leu
        35                  40                  45

gac gcc atc gtt gat gtt gaa gct gct tct ggt gag ctg ctt ctg aaa      192
Asp Ala Ile Val Asp Val Glu Ala Ala Ser Gly Glu Leu Leu Leu Lys
    50                  55                  60

gcg ttt gct ggg ctg gct caa tgt gtt gat gaa ctg agg gag cta ttc      240
Ala Phe Ala Gly Leu Ala Gln Cys Val Asp Glu Leu Arg Glu Leu Phe
65                  70                  75                  80

gaa acc ttg gaa ccg ctg tgc agt aaa gtt tat ttt gtc ctg caa gct      288
Glu Thr Leu Glu Pro Leu Cys Ser Lys Val Tyr Phe Val Leu Gln Ala
                85                  90                  95

gaa cca ttg att ggg aaa att cga tca tgt agc ctg gaa ata ctt gag      336
Glu Pro Leu Ile Gly Lys Ile Arg Ser Cys Ser Leu Glu Ile Leu Glu
            100                 105                 110

ctt ctt aaa tct tct cat aaa agc ctt cca gct gat gta act ttg aca 384
Leu Leu Lys Ser Ser His Lys Ser Leu Pro Ala Asp Val Thr Leu Thr
        115                 120                 125

act ctc gag ctc tat ata ctg aaa att aag tat gta gat tat gaa atg 432
Thr Leu Glu Leu Tyr Ile Leu Lys Ile Lys Tyr Val Asp Tyr Glu Met
    130                 135                 140

ata tca gtg aca atc aca aag gtt att aaa gct caa gtg gaa ggc ttg 480
Ile Ser Val Thr Ile Thr Lys Val Ile Lys Ala Gln Val Glu Gly Leu
145                 150                 155                 160

gga acc agc tca gat agc ttt gcc aaa att gct gat tgc cta agc ttg 528
Gly Thr Ser Ser Asp Ser Phe Ala Lys Ile Ala Asp Cys Leu Ser Leu
                165                 170                 175

aac tca aac caa gag ctt ttg att gag ctt gtg gcc ctt gaa aaa ttg 576
Asn Ser Asn Gln Glu Leu Leu Ile Glu Leu Val Ala Leu Glu Lys Leu
            180                 185                 190

aaa gag aat gct gaa caa gct gaa aag agt gaa gtt gtt gaa tat att 624
Lys Glu Asn Ala Glu Gln Ala Glu Lys Ser Glu Val Val Glu Tyr Ile
        195                 200                 205

gag caa atg ata act ctt gtt tct cat atg cac gat tgc ttt gtt act 672
Glu Gln Met Ile Thr Leu Val Ser His Met His Asp Cys Phe Val Thr
    210                 215                 220

aca aaa cag tcc cag agt tgt acc gct gtg cca ata cct cct gat ttt 720
Thr Lys Gln Ser Gln Ser Cys Thr Ala Val Pro Ile Pro Pro Asp Phe
225                 230                 235                 240

tgc tgt cct ctt tca ctt gag ttg atg act gac cct gta att gtc gct 768
Cys Cys Pro Leu Ser Leu Glu Leu Met Thr Asp Pro Val Ile Val Ala
                245                 250                 255

tct ggt caa acc tat gag agg gct ttt att agg aga tgg att gat ctt 816
Ser Gly Gln Thr Tyr Glu Arg Ala Phe Ile Arg Arg Trp Ile Asp Leu
            260                 265                 270

ggc ctc act gtt tgc ccc aaa aca cgg caa act ctg gga cat aca aat 864
Gly Leu Thr Val Cys Pro Lys Thr Arg Gln Thr Leu Gly His Thr Asn
        275                 280                 285

ctc att cct aat tac act gtt aag gca ctg atc gca aac tgg tgc gaa 912
Leu Ile Pro Asn Tyr Thr Val Lys Ala Leu Ile Ala Asn Trp Cys Glu
    290                 295                 300

ata aac aat gta aag ctg cct gat ccc atg aag tct ttg agc ttg aac 960
Ile Asn Asn Val Lys Leu Pro Asp Pro Met Lys Ser Leu Ser Leu Asn
305                 310                 315                 320

cag cca tct ttg tca cca gac tcc acg caa tct tca ggt tct ccg aga 1008
Gln Pro Ser Leu Ser Pro Asp Ser Thr Gln Ser Ser Gly Ser Pro Arg
                325                 330                 335

aag agt ttg att tca tca act gta agc caa aga gaa gaa tca tct cca 1056
Lys Ser Leu Ile Ser Ser Thr Val Ser Gln Arg Glu Glu Ser Ser Pro
            340                 345                 350

tct cat ccc cgt tcc tct tca gag gaa tct tta cct gga gtt ggt ggt 1104

Ser His Pro Arg Ser Ser Ser Glu Glu Ser Leu Pro Gly Val Gly Gly
        355                 360                 365

aat att ctt gct ttt gat gtt gaa agg atg cgt att aag agt gaa gac      1152
Asn Ile Leu Ala Phe Asp Val Glu Arg Met Arg Ile Lys Ser Glu Asp
    370                 375                 380

cgg atg gcc cac tcc gga gag ata agt tca cat ggt cat agt aca tta      1200
Arg Met Ala His Ser Gly Glu Ile Ser Ser His Gly His Ser Thr Leu
385                 390                 395                 400

gta gct gat gac cag ttc cct ctg ggt cat aat cga aca acc tcg gca      1248
Val Ala Asp Asp Gln Phe Pro Leu Gly His Asn Arg Thr Thr Ser Ala
                405                 410                 415

cct agc acg ctt tct aat tca aac ttt tcc ccg gta att cct ggt gat      1296
Pro Ser Thr Leu Ser Asn Ser Asn Phe Ser Pro Val Ile Pro Gly Asp
            420                 425                 430

gga aac aag ttg tca gaa gat tct tct gtt gct tca ggg gat gtt ggg      1344
Gly Asn Lys Leu Ser Glu Asp Ser Ser Val Ala Ser Gly Asp Val Gly
        435                 440                 445

ttg gat tcc aag cct gct gct tct gtc ctt cca aag gag cca gaa ttt      1392
Leu Asp Ser Lys Pro Ala Ala Ser Val Leu Pro Lys Glu Pro Glu Phe
    450                 455                 460

cca tat aca cca gag atg aga cct cgt aat caa ctg atc tgg cgc aga      1440
Pro Tyr Thr Pro Glu Met Arg Pro Arg Asn Gln Leu Ile Trp Arg Arg
465                 470                 475                 480

cca acc gag agg ttt cca aga ata gtt tct tcc gct aca gtt gaa aga      1488
Pro Thr Glu Arg Phe Pro Arg Ile Val Ser Ser Ala Thr Val Glu Arg
                485                 490                 495

agg gct gat ctt tca gaa gtt gag gag caa gta aaa aag ttg att gag      1536
Arg Ala Asp Leu Ser Glu Val Glu Glu Gln Val Lys Lys Leu Ile Glu
            500                 505                 510

gag ttg aag agc act tcc ctt gat atg cag aga aat gct aca gct gaa      1584
Glu Leu Lys Ser Thr Ser Leu Asp Met Gln Arg Asn Ala Thr Ala Glu
        515                 520                 525

ctc cgg tta ctt gcc aag cat aat atg gat aac cgt atg gta att gca      1632
Leu Arg Leu Leu Ala Lys His Asn Met Asp Asn Arg Met Val Ile Ala
    530                 535                 540

aat tgt ggc gct atc agc tcg ttg gtt aac cta ctt cac tca aaa gac      1680
Asn Cys Gly Ala Ile Ser Ser Leu Val Asn Leu Leu His Ser Lys Asp
545                 550                 555                 560

atg aaa gta cag gaa gat gct gtt act gca ctt ctc aac ttg tca att      1728
Met Lys Val Gln Glu Asp Ala Val Thr Ala Leu Leu Asn Leu Ser Ile
                565                 570                 575

aat gac aac aac aag tgt gcc att gca aat gct gat gca atc gaa cct      1776
Asn Asp Asn Asn Lys Cys Ala Ile Ala Asn Ala Asp Ala Ile Glu Pro
            580                 585                 590

ctg att cat gtc ctc caa aca ggg agc gcc gag gcc aaa gaa aat tct      1824
Leu Ile His Val Leu Gln Thr Gly Ser Ala Glu Ala Lys Glu Asn Ser

595                 600                 605

gct gct act ctt ttt agc ctt tcc gtg atg gag gaa aac aag atg aag      1872
Ala Ala Thr Leu Phe Ser Leu Ser Val Met Glu Glu Asn Lys Met Lys
    610                 615                 620

att ggg agg tct gga gca atc aaa cct ctt gtt gat tta ctg gga aat      1920
Ile Gly Arg Ser Gly Ala Ile Lys Pro Leu Val Asp Leu Leu Gly Asn
625                 630                 635                 640

gga act cca agg ggc aag aaa gat gca gcg aca gct tta ttt aac ttg      1968
Gly Thr Pro Arg Gly Lys Lys Asp Ala Ala Thr Ala Leu Phe Asn Leu
                645                 650                 655

tca ata ctt cat gag aac aag tct cgt ata ata cag gct ggt gcg gta      2016
Ser Ile Leu His Glu Asn Lys Ser Arg Ile Ile Gln Ala Gly Ala Val
            660                 665                 670

aag tat ctc gta gag ttg atg gac cct gct act ggg atg gtt gac aag      2064
Lys Tyr Leu Val Glu Leu Met Asp Pro Ala Thr Gly Met Val Asp Lys
        675                 680                 685

gct gtt gca gtt ttg tcc aac ctt gct acc att ccc gag gga cga gca      2112
Ala Val Ala Val Leu Ser Asn Leu Ala Thr Ile Pro Glu Gly Arg Ala
    690                 695                 700

gaa atc ggt cag gaa gga ggg att cct ctt ctt gtt gag gtt gtt gag      2160
Glu Ile Gly Gln Glu Gly Gly Ile Pro Leu Leu Val Glu Val Val Glu
705                 710                 715                 720

ctg ggc tcc gca agg ggt aag gag aat gca gca gct gct ctc ttg caa      2208
Leu Gly Ser Ala Arg Gly Lys Glu Asn Ala Ala Ala Ala Leu Leu Gln
                725                 730                 735

cta tgc act aac agt agc agg ttc tgc aac atg gtt ctc cag gaa gga      2256
Leu Cys Thr Asn Ser Ser Arg Phe Cys Asn Met Val Leu Gln Glu Gly
            740                 745                 750

gct gta cct cca tta gtg gca ttg tca cag tcc ggc acc cca aga gca      2304
Ala Val Pro Pro Leu Val Ala Leu Ser Gln Ser Gly Thr Pro Arg Ala
        755                 760                 765

aga gaa aag gct caa caa cta ctt agc tac ttc cga aat caa cgc cat      2352
Arg Glu Lys Ala Gln Gln Leu Leu Ser Tyr Phe Arg Asn Gln Arg His
    770                 775                 780

ggt aat gca gga aga ggt tga                                          2373
Gly Asn Ala Gly Arg Gly
785                 790

<210>  12
<211>  790
<212>  PRT
<213>  Nicotiana tabacum

<400>  12

Met Glu Ile Ser Leu Leu Lys Val Leu Leu Asn Asn Ile Ser Cys Phe
1               5                   10                  15

Ser His Leu Ser Ser Ser Asp His Ile Ser Gly Glu Leu Val Arg Arg
            20                  25                  30

Tyr Tyr Cys Lys Ile Glu Asp Ile Leu Lys Leu Val Lys Pro Ile Leu
        35                  40                  45

Asp Ala Ile Val Asp Val Glu Ala Ala Ser Gly Glu Leu Leu Leu Lys
    50                  55                  60

Ala Phe Ala Gly Leu Ala Gln Cys Val Asp Glu Leu Arg Glu Leu Phe
65                  70                  75                  80

Glu Thr Leu Glu Pro Leu Cys Ser Lys Val Tyr Phe Val Leu Gln Ala
                85                  90                  95

Glu Pro Leu Ile Gly Lys Ile Arg Ser Cys Ser Leu Glu Ile Leu Glu
            100                 105                 110

Leu Leu Lys Ser Ser His Lys Ser Leu Pro Ala Asp Val Thr Leu Thr
        115                 120                 125

Thr Leu Glu Leu Tyr Ile Leu Lys Ile Lys Tyr Val Asp Tyr Glu Met
    130                 135                 140

Ile Ser Val Thr Ile Thr Lys Val Ile Lys Ala Gln Val Glu Gly Leu
145                 150                 155                 160

Gly Thr Ser Ser Asp Ser Phe Ala Lys Ile Ala Asp Cys Leu Ser Leu
                165                 170                 175

Asn Ser Asn Gln Glu Leu Leu Ile Glu Leu Val Ala Leu Glu Lys Leu
            180                 185                 190

Lys Glu Asn Ala Glu Gln Ala Glu Lys Ser Glu Val Val Glu Tyr Ile
        195                 200                 205

Glu Gln Met Ile Thr Leu Val Ser His Met His Asp Cys Phe Val Thr
    210                 215                 220

Thr Lys Gln Ser Gln Ser Cys Thr Ala Val Pro Ile Pro Pro Asp Phe
225                 230                 235                 240

Cys Cys Pro Leu Ser Leu Glu Leu Met Thr Asp Pro Val Ile Val Ala
                245                 250                 255

Ser Gly Gln Thr Tyr Glu Arg Ala Phe Ile Arg Arg Trp Ile Asp Leu
        260                 265                 270

Gly Leu Thr Val Cys Pro Lys Thr Arg Gln Thr Leu Gly His Thr Asn
        275                 280                 285

Leu Ile Pro Asn Tyr Thr Val Lys Ala Leu Ile Ala Asn Trp Cys Glu
    290                 295                 300

Ile Asn Asn Val Lys Leu Pro Asp Pro Met Lys Ser Leu Ser Leu Asn
305                 310                 315                 320

Gln Pro Ser Leu Ser Pro Asp Ser Thr Gln Ser Ser Gly Ser Pro Arg
                325                 330                 335

Lys Ser Leu Ile Ser Ser Thr Val Ser Gln Arg Glu Glu Ser Ser Pro
            340                 345                 350

Ser His Pro Arg Ser Ser Ser Glu Glu Ser Leu Pro Gly Val Gly Gly
        355                 360                 365

Asn Ile Leu Ala Phe Asp Val Glu Arg Met Arg Ile Lys Ser Glu Asp
    370                 375                 380

Arg Met Ala His Ser Gly Glu Ile Ser Ser His Gly His Ser Thr Leu
385                 390                 395                 400

Val Ala Asp Asp Gln Phe Pro Leu Gly His Asn Arg Thr Thr Ser Ala
                405                 410                 415

Pro Ser Thr Leu Ser Asn Ser Asn Phe Ser Pro Val Ile Pro Gly Asp
            420                 425                 430

Gly Asn Lys Leu Ser Glu Asp Ser Ser Val Ala Ser Gly Asp Val Gly
        435                 440                 445

Leu Asp Ser Lys Pro Ala Ala Ser Val Leu Pro Lys Glu Pro Glu Phe
    450                 455                 460

Pro Tyr Thr Pro Glu Met Arg Pro Arg Asn Gln Leu Ile Trp Arg Arg
465                 470                 475                 480

Pro Thr Glu Arg Phe Pro Arg Ile Val Ser Ser Ala Thr Val Glu Arg
                485                 490                 495

Arg Ala Asp Leu Ser Glu Val Glu Glu Gln Val Lys Lys Leu Ile Glu

500                 505                 510

Glu Leu Lys Ser Thr Ser Leu Asp Met Gln Arg Asn Ala Thr Ala Glu
        515                 520                 525

Leu Arg Leu Leu Ala Lys His Asn Met Asp Asn Arg Met Val Ile Ala
    530                 535                 540

Asn Cys Gly Ala Ile Ser Ser Leu Val Asn Leu Leu His Ser Lys Asp
545                 550                 555                 560

Met Lys Val Gln Glu Asp Ala Val Thr Ala Leu Leu Asn Leu Ser Ile
                565                 570                 575

Asn Asp Asn Asn Lys Cys Ala Ile Ala Asn Ala Asp Ala Ile Glu Pro
            580                 585                 590

Leu Ile His Val Leu Gln Thr Gly Ser Ala Glu Ala Lys Glu Asn Ser
        595                 600                 605

Ala Ala Thr Leu Phe Ser Leu Ser Val Met Glu Glu Asn Lys Met Lys
    610                 615                 620

Ile Gly Arg Ser Gly Ala Ile Lys Pro Leu Val Asp Leu Leu Gly Asn
625                 630                 635                 640

Gly Thr Pro Arg Gly Lys Lys Asp Ala Ala Thr Ala Leu Phe Asn Leu
                645                 650                 655

Ser Ile Leu His Glu Asn Lys Ser Arg Ile Ile Gln Ala Gly Ala Val
            660                 665                 670

Lys Tyr Leu Val Glu Leu Met Asp Pro Ala Thr Gly Met Val Asp Lys
        675                 680                 685

Ala Val Ala Val Leu Ser Asn Leu Ala Thr Ile Pro Glu Gly Arg Ala
    690                 695                 700

Glu Ile Gly Gln Glu Gly Gly Ile Pro Leu Leu Val Glu Val Val Glu
705                 710                 715                 720

Leu Gly Ser Ala Arg Gly Lys Glu Asn Ala Ala Ala Ala Leu Leu Gln
                725                 730                 735

Leu Cys Thr Asn Ser Ser Arg Phe Cys Asn Met Val Leu Gln Glu Gly
            740                 745                 750

Ala Val Pro Pro Leu Val Ala Leu Ser Gln Ser Gly Thr Pro Arg Ala
        755                 760                 765

Arg Glu Lys Ala Gln Gln Leu Leu Ser Tyr Phe Arg Asn Gln Arg His
    770                 775                 780

Gly Asn Ala Gly Arg Gly
785                 790

<210> 13
<211> 2436
<212> DNA
<213> Arabidopsis thaliana

<220>
<221> CDS
<222> (1)..(2436)

<400> 13
atg gaa gtt ctt ctc aga agt atc tcg tcg ttt cta aat ctg tca tct 48
Met Glu Val Leu Leu Arg Ser Ile Ser Ser Phe Leu Asn Leu Ser Ser
1               5                   10                  15

tct aaa cat att gat tta gac ccg ttt gag aag tac tat aag aga gtt 96
Ser Lys His Ile Asp Leu Asp Pro Phe Glu Lys Tyr Tyr Lys Arg Val
            20                  25                  30

gaa gag tta ttg aga gtg ttg aag cct ata gca gat gtt gtt gtt acc 144
Glu Glu Leu Leu Arg Val Leu Lys Pro Ile Ala Asp Val Val Val Thr
        35                  40                  45

tct gat ttt gtt ttt gat gag aaa ctt ggt aaa gca ttt gaa gaa ttg 192
Ser Asp Phe Val Phe Asp Glu Lys Leu Gly Lys Ala Phe Glu Glu Leu
    50                  55                  60

act cag gat gtt gat caa tcc att gat ctt ttc agg agt tgg caa gct 240
Thr Gln Asp Val Asp Gln Ser Ile Asp Leu Phe Arg Ser Trp Gln Ala
65                  70                  75                  80

ttc tct agt aaa gtc tat ttc gtt ctt caa att gaa tct ttg cta cca 288
Phe Ser Ser Lys Val Tyr Phe Val Leu Gln Ile Glu Ser Leu Leu Pro
                85                  90                  95

aag atg cgg gac acc att gtg gat act ttt cag ttt ctc atg tct tct 336
Lys Met Arg Asp Thr Ile Val Asp Thr Phe Gln Phe Leu Met Ser Ser
            100                 105                 110

aag aac cat cta cct gat gag cta agc cca gct tct ctt gag caa tgt 384
Lys Asn His Leu Pro Asp Glu Leu Ser Pro Ala Ser Leu Glu Gln Cys
        115                 120                 125

cta gag aag att aag cat ctt agt tat gaa gaa ata tct tct gtc att 432
Leu Glu Lys Ile Lys His Leu Ser Tyr Glu Glu Ile Ser Ser Val Ile
    130                 135                 140

gac ggt gct ttg agg gat cag aga gat ggt gtt gga cct agc cct gag     480
Asp Gly Ala Leu Arg Asp Gln Arg Asp Gly Val Gly Pro Ser Pro Glu
145                 150                 155                 160

atc ttg gtg aaa att gga gag aac act ggt ctt aga tca aac cag gag     528
Ile Leu Val Lys Ile Gly Glu Asn Thr Gly Leu Arg Ser Asn Gln Glu
                165                 170                 175

att ctg att gaa gct gtt gct cta gag agg cag aaa gag atg gct gag     576
Ile Leu Ile Glu Ala Val Ala Leu Glu Arg Gln Lys Glu Met Ala Glu
            180                 185                 190

cag tct gag aat aat gca gaa gtc gag ttc ctt gac caa ctg att gtt     624
Gln Ser Glu Asn Asn Ala Glu Val Glu Phe Leu Asp Gln Leu Ile Val
        195                 200                 205

att gta aac cgc atg cat gaa cgt ctt ctt ctg atc aaa cag act cag     672
Ile Val Asn Arg Met His Glu Arg Leu Leu Leu Ile Lys Gln Thr Gln
    210                 215                 220

act tct agt gtc gcc att ctt gcc gac ttc ttt tgc cct ctg tca ctt     720
Thr Ser Ser Val Ala Ile Leu Ala Asp Phe Phe Cys Pro Leu Ser Leu
225                 230                 235                 240

gaa gta atg act gat cca gtg att gtg tca tca gga caa aca tat gaa     768
Glu Val Met Thr Asp Pro Val Ile Val Ser Ser Gly Gln Thr Tyr Glu
                245                 250                 255

aag gcg ttt atc aag aga tgg att gat ttg ggt tta aaa gtg tgt ccc     816
Lys Ala Phe Ile Lys Arg Trp Ile Asp Leu Gly Leu Lys Val Cys Pro
            260                 265                 270

aag act cga cag acc ctg act cac act act cta ata ccc aat tac acc     864
Lys Thr Arg Gln Thr Leu Thr His Thr Thr Leu Ile Pro Asn Tyr Thr
        275                 280                 285

gtg aag gcc tta atc gct aac tgg tgt gag aca aac gat gtc aag ctg     912
Val Lys Ala Leu Ile Ala Asn Trp Cys Glu Thr Asn Asp Val Lys Leu
    290                 295                 300

cct gat ccc aat aaa tca aca agt tta aat gag ctt tct cct ctt tta     960
Pro Asp Pro Asn Lys Ser Thr Ser Leu Asn Glu Leu Ser Pro Leu Leu
305                 310                 315                 320

tca tgt aca gac tcc att cct agc acg ggt gct gat gtt tct gct cgt     1008
Ser Cys Thr Asp Ser Ile Pro Ser Thr Gly Ala Asp Val Ser Ala Arg
                325                 330                 335

aaa gtt agc aac aag tca cat gat tgg gat gct tct tca agt gaa acc     1056
Lys Val Ser Asn Lys Ser His Asp Trp Asp Ala Ser Ser Ser Glu Thr
            340                 345                 350

ggt aag ccc tcg ttc tca agc cga gca act gaa aga gaa ggt gct tct     1104
Gly Lys Pro Ser Phe Ser Ser Arg Ala Thr Glu Arg Glu Gly Ala Ser
        355                 360                 365

cct tca cgt cct gct tct gcc ttg ggt gct tct tca ccg ggt ata tct     1152
Pro Ser Arg Pro Ala Ser Ala Leu Gly Ala Ser Ser Pro Gly Ile Ser
    370                 375                 380

gga aat ggt tac ggt ttg gac gcc agg agg gga tca cta aat gat ttt     1200

Gly Asn Gly Tyr Gly Leu Asp Ala Arg Arg Gly Ser Leu Asn Asp Phe
385                 390                 395                 400

gaa gat aga tca aac gat tct cga gaa ctg agg aca gat gca cct ggt      1248
Glu Asp Arg Ser Asn Asp Ser Arg Glu Leu Arg Thr Asp Ala Pro Gly
                405                 410                 415

agg tca tct gta tct tca act aca cga ggc tca gta gaa aat gga caa      1296
Arg Ser Ser Val Ser Ser Thr Thr Arg Gly Ser Val Glu Asn Gly Gln
            420                 425                 430

aca tct gag aac cac cat cat agg tcc cct tct gct act agc act gtt      1344
Thr Ser Glu Asn His His His Arg Ser Pro Ser Ala Thr Ser Thr Val
        435                 440                 445

tcc aat gag gag ttt cca agg gca gat gcg aat gag aat tca gaa gaa      1392
Ser Asn Glu Glu Phe Pro Arg Ala Asp Ala Asn Glu Asn Ser Glu Glu
    450                 455                 460

tca gct cat gct aca cct tac agc agt gat gct tca gga gaa att aga      1440
Ser Ala His Ala Thr Pro Tyr Ser Ser Asp Ala Ser Gly Glu Ile Arg
465                 470                 475                 480

tca ggg cct ctt gct gca acc act tca gca gct act cgc cga gat ttg      1488
Ser Gly Pro Leu Ala Ala Thr Thr Ser Ala Ala Thr Arg Arg Asp Leu
                485                 490                 495

tct gat ttt tcc cca aaa ttc atg gat aga cgt acc cgt ggt caa ttt      1536
Ser Asp Phe Ser Pro Lys Phe Met Asp Arg Arg Thr Arg Gly Gln Phe
            500                 505                 510

tgg cga cgt cca tca gag aga ctc ggt tca agg att gtt tca gcg cct      1584
Trp Arg Arg Pro Ser Glu Arg Leu Gly Ser Arg Ile Val Ser Ala Pro
        515                 520                 525

tcg aat gag aca aga cgt gat ctt tct gag gtc gaa act caa gtt aag      1632
Ser Asn Glu Thr Arg Arg Asp Leu Ser Glu Val Glu Thr Gln Val Lys
    530                 535                 540

aag ttg gtg gag gag ttg aaa agc agc tca ttg gat act cag aga caa      1680
Lys Leu Val Glu Glu Leu Lys Ser Ser Ser Leu Asp Thr Gln Arg Gln
545                 550                 555                 560

gca acc gca gaa cta agg ttg cta gcc aag cac aac atg gat aat cgg      1728
Ala Thr Ala Glu Leu Arg Leu Leu Ala Lys His Asn Met Asp Asn Arg
                565                 570                 575

ata gtc att ggg aac tct gga gca atc gtc tta ttg gtg gaa cta ctt      1776
Ile Val Ile Gly Asn Ser Gly Ala Ile Val Leu Leu Val Glu Leu Leu
            580                 585                 590

tac tca act gac tca gct aca cag gaa aac gct gtt acc gca ctt ctc      1824
Tyr Ser Thr Asp Ser Ala Thr Gln Glu Asn Ala Val Thr Ala Leu Leu
        595                 600                 605

aac tta tct atc aat gac aac aac aaa aaa gca att gct gat gct ggt      1872
Asn Leu Ser Ile Asn Asp Asn Asn Lys Lys Ala Ile Ala Asp Ala Gly
    610                 615                 620

gca att gag ccg ctc att cac gtg ctt gaa aat ggg agc tct gaa gcc      1920
Ala Ile Glu Pro Leu Ile His Val Leu Glu Asn Gly Ser Ser Glu Ala

625                 630                 635                 640

aag gag aat tca gct gct act ctc ttc agc ctc tct gta ata gaa gaa      1968
Lys Glu Asn Ser Ala Ala Thr Leu Phe Ser Leu Ser Val Ile Glu Glu
                645                 650                 655

aac aag att aag att ggt cag tcg ggt gca atc ggg cct ctt gta gat      2016
Asn Lys Ile Lys Ile Gly Gln Ser Gly Ala Ile Gly Pro Leu Val Asp
            660                 665                 670

ctt ctc ggt aac ggt acc cct cgg ggt aag aaa gac gct gct act gcc      2064
Leu Leu Gly Asn Gly Thr Pro Arg Gly Lys Lys Asp Ala Ala Thr Ala
        675                 680                 685

ttg ttt aat cta tcg ata cat caa gaa aac aag gcg atg atc gtg caa      2112
Leu Phe Asn Leu Ser Ile His Gln Glu Asn Lys Ala Met Ile Val Gln
    690                 695                 700

tca ggt gct gtg aga tat ctt att gat ctg atg gac cca gca gct ggg      2160
Ser Gly Ala Val Arg Tyr Leu Ile Asp Leu Met Asp Pro Ala Ala Gly
705                 710                 715                 720

atg gtg gat aaa gca gtt gct gtt ttg gca aat cta gct aca att ccg      2208
Met Val Asp Lys Ala Val Ala Val Leu Ala Asn Leu Ala Thr Ile Pro
                725                 730                 735

gaa gga aga aac gcg att ggt caa gaa ggc gga atc cct ctt ctt gtt      2256
Glu Gly Arg Asn Ala Ile Gly Gln Glu Gly Gly Ile Pro Leu Leu Val
            740                 745                 750

gaa gtc gtt gag ttg ggt tca gct aga ggg aaa gaa aac gca gca gca      2304
Glu Val Val Glu Leu Gly Ser Ala Arg Gly Lys Glu Asn Ala Ala Ala
        755                 760                 765

gct ctt ctt caa ctt tca acc aac agt ggt cgg ttc tgc aac atg gtt      2352
Ala Leu Leu Gln Leu Ser Thr Asn Ser Gly Arg Phe Cys Asn Met Val
    770                 775                 780

ctt caa gaa ggc gcc gtt cct cca ctc gtc gct ctc tca cag tct ggt      2400
Leu Gln Glu Gly Ala Val Pro Pro Leu Val Ala Leu Ser Gln Ser Gly
785                 790                 795                 800

act cct aga gct aga gaa aag gta caa act tta taa                      2436
Thr Pro Arg Ala Arg Glu Lys Val Gln Thr Leu
                805                 810


<210>  14
<211>  811
<212>  PRT
<213>  Arabidopsis thaliana

<400>  14

Met Glu Val Leu Leu Arg Ser Ile Ser Ser Phe Leu Asn Leu Ser Ser
1               5                   10                  15


Ser Lys His Ile Asp Leu Asp Pro Phe Glu Lys Tyr Tyr Lys Arg Val
            20                  25                  30

Glu Glu Leu Leu Arg Val Leu Lys Pro Ile Ala Asp Val Val Val Thr
        35                  40                  45

Ser Asp Phe Val Phe Asp Glu Lys Leu Gly Lys Ala Phe Glu Glu Leu
    50                  55                  60

Thr Gln Asp Val Asp Gln Ser Ile Asp Leu Phe Arg Ser Trp Gln Ala
65                  70                  75                  80

Phe Ser Ser Lys Val Tyr Phe Val Leu Gln Ile Glu Ser Leu Leu Pro
                85                  90                  95

Lys Met Arg Asp Thr Ile Val Asp Thr Phe Gln Phe Leu Met Ser Ser
            100                 105                 110

Lys Asn His Leu Pro Asp Glu Leu Ser Pro Ala Ser Leu Glu Gln Cys
        115                 120                 125

Leu Glu Lys Ile Lys His Leu Ser Tyr Glu Glu Ile Ser Ser Val Ile
    130                 135                 140

Asp Gly Ala Leu Arg Asp Gln Arg Asp Gly Val Gly Pro Ser Pro Glu
145                 150                 155                 160

Ile Leu Val Lys Ile Gly Glu Asn Thr Gly Leu Arg Ser Asn Gln Glu
                165                 170                 175

Ile Leu Ile Glu Ala Val Ala Leu Glu Arg Gln Lys Glu Met Ala Glu
            180                 185                 190

Gln Ser Glu Asn Asn Ala Glu Val Glu Phe Leu Asp Gln Leu Ile Val
        195                 200                 205

Ile Val Asn Arg Met His Glu Arg Leu Leu Leu Ile Lys Gln Thr Gln
    210                 215                 220

Thr Ser Ser Val Ala Ile Leu Ala Asp Phe Phe Cys Pro Leu Ser Leu
225                 230                 235                 240

Glu Val Met Thr Asp Pro Val Ile Val Ser Ser Gly Gln Thr Tyr Glu
                245                 250                 255

Lys Ala Phe Ile Lys Arg Trp Ile Asp Leu Gly Leu Lys Val Cys Pro
            260                 265                 270

Lys Thr Arg Gln Thr Leu Thr His Thr Thr Leu Ile Pro Asn Tyr Thr
        275                 280                 285

Val Lys Ala Leu Ile Ala Asn Trp Cys Glu Thr Asn Asp Val Lys Leu
    290                 295                 300

Pro Asp Pro Asn Lys Ser Thr Ser Leu Asn Glu Leu Ser Pro Leu Leu
305                 310                 315                 320

Ser Cys Thr Asp Ser Ile Pro Ser Thr Gly Ala Asp Val Ser Ala Arg
                325                 330                 335

Lys Val Ser Asn Lys Ser His Asp Trp Asp Ala Ser Ser Ser Glu Thr
            340                 345                 350

Gly Lys Pro Ser Phe Ser Ser Arg Ala Thr Glu Arg Glu Gly Ala Ser
        355                 360                 365

Pro Ser Arg Pro Ala Ser Ala Leu Gly Ala Ser Ser Pro Gly Ile Ser
    370                 375                 380

Gly Asn Gly Tyr Gly Leu Asp Ala Arg Arg Gly Ser Leu Asn Asp Phe
385                 390                 395                 400

Glu Asp Arg Ser Asn Asp Ser Arg Glu Leu Arg Thr Asp Ala Pro Gly
                405                 410                 415

Arg Ser Ser Val Ser Ser Thr Thr Arg Gly Ser Val Glu Asn Gly Gln
            420                 425                 430

Thr Ser Glu Asn His His His Arg Ser Pro Ser Ala Thr Ser Thr Val
        435                 440                 445

Ser Asn Glu Glu Phe Pro Arg Ala Asp Ala Asn Glu Asn Ser Glu Glu
    450                 455                 460

Ser Ala His Ala Thr Pro Tyr Ser Ser Asp Ala Ser Gly Glu Ile Arg
465                 470                 475                 480

Ser Gly Pro Leu Ala Ala Thr Thr Ser Ala Ala Thr Arg Arg Asp Leu
                485                 490                 495

Ser Asp Phe Ser Pro Lys Phe Met Asp Arg Arg Thr Arg Gly Gln Phe
            500                 505                 510

Trp Arg Arg Pro Ser Glu Arg Leu Gly Ser Arg Ile Val Ser Ala Pro

515                 520                 525

Ser Asn Glu Thr Arg Arg Asp Leu Ser Glu Val Glu Thr Gln Val Lys
    530                 535                 540

Lys Leu Val Glu Glu Leu Lys Ser Ser Ser Leu Asp Thr Gln Arg Gln
545                 550                 555                 560

Ala Thr Ala Glu Leu Arg Leu Leu Ala Lys His Asn Met Asp Asn Arg
                565                 570                 575

Ile Val Ile Gly Asn Ser Gly Ala Ile Val Leu Leu Val Glu Leu Leu
            580                 585                 590

Tyr Ser Thr Asp Ser Ala Thr Gln Glu Asn Ala Val Thr Ala Leu Leu
        595                 600                 605

Asn Leu Ser Ile Asn Asp Asn Asn Lys Lys Ala Ile Ala Asp Ala Gly
    610                 615                 620

Ala Ile Glu Pro Leu Ile His Val Leu Glu Asn Gly Ser Ser Glu Ala
625                 630                 635                 640

Lys Glu Asn Ser Ala Ala Thr Leu Phe Ser Leu Ser Val Ile Glu Glu
                645                 650                 655

Asn Lys Ile Lys Ile Gly Gln Ser Gly Ala Ile Gly Pro Leu Val Asp
            660                 665                 670

Leu Leu Gly Asn Gly Thr Pro Arg Gly Lys Lys Asp Ala Ala Thr Ala
        675                 680                 685

Leu Phe Asn Leu Ser Ile His Gln Glu Asn Lys Ala Met Ile Val Gln
    690                 695                 700

Ser Gly Ala Val Arg Tyr Leu Ile Asp Leu Met Asp Pro Ala Ala Gly
705                 710                 715                 720

Met Val Asp Lys Ala Val Ala Val Leu Ala Asn Leu Ala Thr Ile Pro
                725                 730                 735

Glu Gly Arg Asn Ala Ile Gly Gln Glu Gly Gly Ile Pro Leu Leu Val
            740                 745                 750

Glu Val Val Glu Leu Gly Ser Ala Arg Gly Lys Glu Asn Ala Ala Ala
        755                 760                 765

Ala Leu Leu Gln Leu Ser Thr Asn Ser Gly Arg Phe Cys Asn Met Val
    770                 775                 780

Leu Gln Glu Gly Ala Val Pro Pro Leu Val Ala Leu Ser Gln Ser Gly
785                 790                 795                 800

Thr Pro Arg Ala Arg Glu Lys Val Gln Thr Leu
                805                 810

<210> 15
<211> 2775
<212> DNA
<213> Arabidopsis thaliana

<220>
<221> CDS
<222> (1)..(2775)

<400> 15
atg att ttg cgg ttt tgg cgg gaa aac att att ttg cgg ttt tgg cgg 48
Met Ile Leu Arg Phe Trp Arg Glu Asn Ile Ile Leu Arg Phe Trp Arg
1               5                   10                  15

aaa atc cat gat ttt gcg gtt ttg aaa ctc att cag atg tat cat cca 96
Lys Ile His Asp Phe Ala Val Leu Lys Leu Ile Gln Met Tyr His Pro
            20                  25                  30

gat gat cca tcc aaa tac ttg tta aat tat aaa aaa caa aca tca ttt 144
Asp Asp Pro Ser Lys Tyr Leu Leu Asn Tyr Lys Lys Gln Thr Ser Phe
        35                  40                  45

ttc atc tgc att tgg atg aat cat ttg gat gaa aaa caa aca agg tct 192
Phe Ile Cys Ile Trp Met Asn His Leu Asp Glu Lys Gln Thr Arg Ser
    50                  55                  60

gag tct gat ttc aca gtt tcc aaa aga gat ata aga agg gtg gaa atg 240
Glu Ser Asp Phe Thr Val Ser Lys Arg Asp Ile Arg Arg Val Glu Met
65                  70                  75                  80

gaa gtt ctt ctc aga agt atc tcg tcg ttt cta aat ctg tca tct tct 288
Glu Val Leu Leu Arg Ser Ile Ser Ser Phe Leu Asn Leu Ser Ser Ser
                85                  90                  95

aaa cat att gat tta gac ccg ttt gag aag tac tat aag aga gtt gaa 336
Lys His Ile Asp Leu Asp Pro Phe Glu Lys Tyr Tyr Lys Arg Val Glu
            100                 105                 110

gag tta ttg aga gtg ttg aag cct ata gca gat gtt gtt gtt acc tct 384
Glu Leu Leu Arg Val Leu Lys Pro Ile Ala Asp Val Val Val Thr Ser
        115                 120                 125

gat ttt gtt ttt gat gag aaa ctt ggt aaa gca ttt gaa gaa ttg act 432
Asp Phe Val Phe Asp Glu Lys Leu Gly Lys Ala Phe Glu Glu Leu Thr
    130                 135                 140

cag gat gtt gat caa tcc att gat ctt ttc agg agt tgg caa gct ttc 480
Gln Asp Val Asp Gln Ser Ile Asp Leu Phe Arg Ser Trp Gln Ala Phe
145                 150                 155                 160

tct agt aaa gtc tat ttc gtt ctt caa att gaa tct ttg cta cca aag 528
Ser Ser Lys Val Tyr Phe Val Leu Gln Ile Glu Ser Leu Leu Pro Lys
                165                 170                 175

atg cgg gac acc att gtg gat act ttt cag ttt ctc atg tct tct aag 576
Met Arg Asp Thr Ile Val Asp Thr Phe Gln Phe Leu Met Ser Ser Lys
            180                 185                 190

aac cat cta cct gat gag cta agc cca gct tct ctt gag caa tgt cta 624
Asn His Leu Pro Asp Glu Leu Ser Pro Ala Ser Leu Glu Gln Cys Leu
        195                 200                 205

gag aag att aag cat ctt agt tat gaa gaa ata tct tct gtc att gac 672
Glu Lys Ile Lys His Leu Ser Tyr Glu Glu Ile Ser Ser Val Ile Asp
    210                 215                 220

ggt gct ttg agg gat cag aga gat ggt gtt gga cct agc cct gag atc 720
Gly Ala Leu Arg Asp Gln Arg Asp Gly Val Gly Pro Ser Pro Glu Ile
225                 230                 235                 240

ttg gtg aaa att gga gag aac act ggt ctt aga tca aac cag gag att 768
Leu Val Lys Ile Gly Glu Asn Thr Gly Leu Arg Ser Asn Gln Glu Ile
                245                 250                 255

ctg att gaa gct gtt gct cta gag agg cag aaa gag atg gct gag cag 816
Leu Ile Glu Ala Val Ala Leu Glu Arg Gln Lys Glu Met Ala Glu Gln
            260                 265                 270

tct gag aat aat gca gaa gtc gag ttc ctt gac caa ctg att gtt att 864
Ser Glu Asn Asn Ala Glu Val Glu Phe Leu Asp Gln Leu Ile Val Ile
        275                 280                 285

gta aac cgc atg cat gaa cgt ctt ctt ctg atc aaa cag act cag act 912
Val Asn Arg Met His Glu Arg Leu Leu Leu Ile Lys Gln Thr Gln Thr
    290                 295                 300

tct agt gtc gcc att ctt gcc gac ttc ttt tgc cct ctg tca ctt gaa 960
Ser Ser Val Ala Ile Leu Ala Asp Phe Phe Cys Pro Leu Ser Leu Glu
305                 310                 315                 320

gta atg act gat cca gtg att gtg tca tca gga caa aca tat gaa aag 1008
Val Met Thr Asp Pro Val Ile Val Ser Ser Gly Gln Thr Tyr Glu Lys
                325                 330                 335

gcg ttt atc aag aga tgg att gat ttg ggt tta aaa gtg tgt ccc aag 1056
Ala Phe Ile Lys Arg Trp Ile Asp Leu Gly Leu Lys Val Cys Pro Lys
            340                 345                 350

act cga cag acc ctg act cac act act cta ata ccc aat tac acc gtg 1104
Thr Arg Gln Thr Leu Thr His Thr Thr Leu Ile Pro Asn Tyr Thr Val
        355                 360                 365

aag gcc tta atc gct aac tgg tgt gag aca aac gat gtc aag ctg cct 1152
Lys Ala Leu Ile Ala Asn Trp Cys Glu Thr Asn Asp Val Lys Leu Pro
    370                 375                 380

gat ccc aat aaa tca aca agt tta aat gag ctt tct cct ctt tta tca 1200

Asp Pro Asn Lys Ser Thr Ser Leu Asn Glu Leu Ser Pro Leu Leu Ser
385                 390                 395                 400

tgt aca gac tcc att cct agc acg ggt gct gat gtt tct gct cgt aaa      1248
Cys Thr Asp Ser Ile Pro Ser Thr Gly Ala Asp Val Ser Ala Arg Lys
                405                 410                 415

gtt agc aac aag tca cat gat tgg gat gct tct tca agt gaa acc ggt      1296
Val Ser Asn Lys Ser His Asp Trp Asp Ala Ser Ser Ser Glu Thr Gly
            420                 425                 430

aag ccc tcg ttc tca agc cga gca act gaa aga gaa ggt gct tct cct      1344
Lys Pro Ser Phe Ser Ser Arg Ala Thr Glu Arg Glu Gly Ala Ser Pro
        435                 440                 445

tca cgt cct gct tct gcc ttg ggt gct tct tca ccg ggt ata tct gga      1392
Ser Arg Pro Ala Ser Ala Leu Gly Ala Ser Ser Pro Gly Ile Ser Gly
    450                 455                 460

aat ggt tac ggt ttg gac gcc agg agg gga tca cta aat gat ttt gaa      1440
Asn Gly Tyr Gly Leu Asp Ala Arg Arg Gly Ser Leu Asn Asp Phe Glu
465                 470                 475                 480

gat aga tca aac gat tct cga gaa ctg agg aca gat gca cct ggt agg      1488
Asp Arg Ser Asn Asp Ser Arg Glu Leu Arg Thr Asp Ala Pro Gly Arg
                485                 490                 495

tca tct gta tct tca act aca cga ggc tca gta gaa aat gga caa aca      1536
Ser Ser Val Ser Ser Thr Thr Arg Gly Ser Val Glu Asn Gly Gln Thr
            500                 505                 510

tct gag aac cac cat cat agg tcc cct tct gct act agc act gtt tcc      1584
Ser Glu Asn His His His Arg Ser Pro Ser Ala Thr Ser Thr Val Ser
        515                 520                 525

aat gag gag ttt cca agg gca gat gcg aat gag aat tca gaa gaa tca      1632
Asn Glu Glu Phe Pro Arg Ala Asp Ala Asn Glu Asn Ser Glu Glu Ser
    530                 535                 540

gct cat gct aca cct tac agc agt gat gct tca gga gaa att aga tca      1680
Ala His Ala Thr Pro Tyr Ser Ser Asp Ala Ser Gly Glu Ile Arg Ser
545                 550                 555                 560

ggg cct ctt gct gca acc act tca gca gct act cgc cga gat ttg tct      1728
Gly Pro Leu Ala Ala Thr Thr Ser Ala Ala Thr Arg Arg Asp Leu Ser
                565                 570                 575

gat ttt tcc cca aaa ttc atg gat aga cgt acc cgt ggt caa ttt tgg      1776
Asp Phe Ser Pro Lys Phe Met Asp Arg Arg Thr Arg Gly Gln Phe Trp
            580                 585                 590

cga cgt cca tca gag aga ctc ggt tca agg att gtt tca gcg cct tcg      1824
Arg Arg Pro Ser Glu Arg Leu Gly Ser Arg Ile Val Ser Ala Pro Ser
        595                 600                 605

aat gag aca aga cgt gat ctt tct gag gtc gaa act caa gtt aag aag      1872
Asn Glu Thr Arg Arg Asp Leu Ser Glu Val Glu Thr Gln Val Lys Lys
    610                 615                 620

ttg gtg gag gag ttg aaa agc agc tca ttg gat act cag aga caa gca      1920
Leu Val Glu Glu Leu Lys Ser Ser Ser Leu Asp Thr Gln Arg Gln Ala

625                 630                 635                 640

acc gca gaa cta agg ttg cta gcc aag cac aac atg gat aat cgg ata      1968
Thr Ala Glu Leu Arg Leu Leu Ala Lys His Asn Met Asp Asn Arg Ile
                645                 650                 655

gtc att ggg aac tct gga gca atc gtc tta ttg gtg gaa cta ctt tac      2016
Val Ile Gly Asn Ser Gly Ala Ile Val Leu Leu Val Glu Leu Leu Tyr
            660                 665                 670

tca act gac tca gct aca cag gaa aac gct gtt acc gca ctt ctc aac      2064
Ser Thr Asp Ser Ala Thr Gln Glu Asn Ala Val Thr Ala Leu Leu Asn
        675                 680                 685

tta tct atc aat gac aac aac aaa aaa gca att gct gat gct ggt gca      2112
Leu Ser Ile Asn Asp Asn Asn Lys Lys Ala Ile Ala Asp Ala Gly Ala
    690                 695                 700

att gag ccg ctc att cac gtg ctt gaa aat ggg agc tct gaa gcc aag      2160
Ile Glu Pro Leu Ile His Val Leu Glu Asn Gly Ser Ser Glu Ala Lys
705                 710                 715                 720

gag aat tca gct gct act ctc ttc agc ctc tct gta ata gaa gaa aac      2208
Glu Asn Ser Ala Ala Thr Leu Phe Ser Leu Ser Val Ile Glu Glu Asn
                725                 730                 735

aag att aag att ggt cag tcg ggt gca atc ggg cct ctt gta gat ctt      2256
Lys Ile Lys Ile Gly Gln Ser Gly Ala Ile Gly Pro Leu Val Asp Leu
            740                 745                 750

ctc ggt aac ggt acc cct cgg ggt aag aaa gac gct gct act gcc ttg      2304
Leu Gly Asn Gly Thr Pro Arg Gly Lys Lys Asp Ala Ala Thr Ala Leu
        755                 760                 765

ttt aat cta tcg ata cat caa gaa aac aag gcg atg atc gtg caa tca      2352
Phe Asn Leu Ser Ile His Gln Glu Asn Lys Ala Met Ile Val Gln Ser
    770                 775                 780

ggt gct gtg aga tat ctt att gat ctg atg gac cca gca gct ggg atg      2400
Gly Ala Val Arg Tyr Leu Ile Asp Leu Met Asp Pro Ala Ala Gly Met
785                 790                 795                 800

gtg gat aaa gca gtt gct gtt ttg gca aat cta gct aca att ccg gaa      2448
Val Asp Lys Ala Val Ala Val Leu Ala Asn Leu Ala Thr Ile Pro Glu
                805                 810                 815

gga aga aac gcg att ggt caa gaa ggc gga atc cct ctt ctt gtt gaa      2496
Gly Arg Asn Ala Ile Gly Gln Glu Gly Gly Ile Pro Leu Leu Val Glu
            820                 825                 830

gtc gtt gag ttg ggt tca gct aga ggg aaa gaa aac gca gca gca gct      2544
Val Val Glu Leu Gly Ser Ala Arg Gly Lys Glu Asn Ala Ala Ala Ala
        835                 840                 845

ctt ctt caa ctt tca acc aac agt ggt cgg ttc tgc aac atg gtt ctt      2592
Leu Leu Gln Leu Ser Thr Asn Ser Gly Arg Phe Cys Asn Met Val Leu
    850                 855                 860

caa gaa ggc gcc gtt cct cca ctc gtc gct ctc tca cag tct ggt act      2640
Gln Glu Gly Ala Val Pro Pro Leu Val Ala Leu Ser Gln Ser Gly Thr
865                 870                 875                 880

cct aga gct aga gaa aag aaa cca acg gca tgg aaa cgc tgg gcg tgg     2688
Pro Arg Ala Arg Glu Lys Lys Pro Thr Ala Trp Lys Arg Trp Ala Trp
                885                 890                 895

ctg atg atg gat gat gat gat gat gat gat gtt gat gat gca cag att     2736
Leu Met Met Asp Asp Asp Asp Asp Asp Asp Val Asp Asp Ala Gln Ile
            900                 905                 910

ctg gtc tct cag tgc cta ttt tta tgt ttt gtc ttg tga                 2775
Leu Val Ser Gln Cys Leu Phe Leu Cys Phe Val Leu
        915                 920

<210>  16
<211>  924
<212>  PRT
<213>  Arabidopsis thaliana

<400>  16

Met Ile Leu Arg Phe Trp Arg Glu Asn Ile Ile Leu Arg Phe Trp Arg
1               5                   10                  15

Lys Ile His Asp Phe Ala Val Leu Lys Leu Ile Gln Met Tyr His Pro
            20                  25                  30

Asp Asp Pro Ser Lys Tyr Leu Leu Asn Tyr Lys Lys Gln Thr Ser Phe
        35                  40                  45

Phe Ile Cys Ile Trp Met Asn His Leu Asp Glu Lys Gln Thr Arg Ser
    50                  55                  60

Glu Ser Asp Phe Thr Val Ser Lys Arg Asp Ile Arg Arg Val Glu Met
65                  70                  75                  80

Glu Val Leu Leu Arg Ser Ile Ser Ser Phe Leu Asn Leu Ser Ser Ser
                85                  90                  95

Lys His Ile Asp Leu Asp Pro Phe Glu Lys Tyr Tyr Lys Arg Val Glu
            100                 105                 110

Glu Leu Leu Arg Val Leu Lys Pro Ile Ala Asp Val Val Val Thr Ser
        115                 120                 125

Asp Phe Val Phe Asp Glu Lys Leu Gly Lys Ala Phe Glu Glu Leu Thr
    130                 135                 140

Gln Asp Val Asp Gln Ser Ile Asp Leu Phe Arg Ser Trp Gln Ala Phe
145                 150                 155                 160

Ser Ser Lys Val Tyr Phe Val Leu Gln Ile Glu Ser Leu Leu Pro Lys
            165                 170                 175

Met Arg Asp Thr Ile Val Asp Thr Phe Gln Phe Leu Met Ser Ser Lys
        180                 185                 190

Asn His Leu Pro Asp Glu Leu Ser Pro Ala Ser Leu Glu Gln Cys Leu
    195                 200                 205

Glu Lys Ile Lys His Leu Ser Tyr Glu Glu Ile Ser Ser Val Ile Asp
    210                 215                 220

Gly Ala Leu Arg Asp Gln Arg Asp Gly Val Gly Pro Ser Pro Glu Ile
225                 230                 235                 240

Leu Val Lys Ile Gly Glu Asn Thr Gly Leu Arg Ser Asn Gln Glu Ile
            245                 250                 255

Leu Ile Glu Ala Val Ala Leu Glu Arg Gln Lys Glu Met Ala Glu Gln
        260                 265                 270

Ser Glu Asn Asn Ala Glu Val Glu Phe Leu Asp Gln Leu Ile Val Ile
    275                 280                 285

Val Asn Arg Met His Glu Arg Leu Leu Leu Ile Lys Gln Thr Gln Thr
    290                 295                 300

Ser Ser Val Ala Ile Leu Ala Asp Phe Phe Cys Pro Leu Ser Leu Glu
305                 310                 315                 320

Val Met Thr Asp Pro Val Ile Val Ser Ser Gly Gln Thr Tyr Glu Lys
            325                 330                 335

Ala Phe Ile Lys Arg Trp Ile Asp Leu Gly Leu Lys Val Cys Pro Lys
        340                 345                 350

Thr Arg Gln Thr Leu Thr His Thr Thr Leu Ile Pro Asn Tyr Thr Val
    355                 360                 365

Lys Ala Leu Ile Ala Asn Trp Cys Glu Thr Asn Asp Val Lys Leu Pro
    370                 375                 380

Asp Pro Asn Lys Ser Thr Ser Leu Asn Glu Leu Ser Pro Leu Leu Ser
385                 390                 395                 400

Cys Thr Asp Ser Ile Pro Ser Thr Gly Ala Asp Val Ser Ala Arg Lys

                405                 410                 415

Val Ser Asn Lys Ser His Asp Trp Asp Ala Ser Ser Ser Glu Thr Gly
            420                 425                 430

Lys Pro Ser Phe Ser Ser Arg Ala Thr Glu Arg Glu Gly Ala Ser Pro
        435                 440                 445

Ser Arg Pro Ala Ser Ala Leu Gly Ala Ser Ser Pro Gly Ile Ser Gly
    450                 455                 460

Asn Gly Tyr Gly Leu Asp Ala Arg Arg Gly Ser Leu Asn Asp Phe Glu
465                 470                 475                 480

Asp Arg Ser Asn Asp Ser Arg Glu Leu Arg Thr Asp Ala Pro Gly Arg
                485                 490                 495

Ser Ser Val Ser Ser Thr Thr Arg Gly Ser Val Glu Asn Gly Gln Thr
            500                 505                 510

Ser Glu Asn His His His Arg Ser Pro Ser Ala Thr Ser Thr Val Ser
        515                 520                 525

Asn Glu Glu Phe Pro Arg Ala Asp Ala Asn Glu Asn Ser Glu Glu Ser
    530                 535                 540

Ala His Ala Thr Pro Tyr Ser Ser Asp Ala Ser Gly Glu Ile Arg Ser
545                 550                 555                 560

Gly Pro Leu Ala Ala Thr Thr Ser Ala Ala Thr Arg Arg Asp Leu Ser
                565                 570                 575

Asp Phe Ser Pro Lys Phe Met Asp Arg Arg Thr Arg Gly Gln Phe Trp
            580                 585                 590

Arg Arg Pro Ser Glu Arg Leu Gly Ser Arg Ile Val Ser Ala Pro Ser
        595                 600                 605

Asn Glu Thr Arg Arg Asp Leu Ser Glu Val Glu Thr Gln Val Lys Lys
    610                 615                 620

Leu Val Glu Glu Leu Lys Ser Ser Ser Leu Asp Thr Gln Arg Gln Ala
625                 630                 635                 640

Thr Ala Glu Leu Arg Leu Leu Ala Lys His Asn Met Asp Asn Arg Ile
                645                 650                 655

Val Ile Gly Asn Ser Gly Ala Ile Val Leu Leu Val Glu Leu Leu Tyr
        660                 665                 670

Ser Thr Asp Ser Ala Thr Gln Glu Asn Ala Val Thr Ala Leu Leu Asn
            675                 680                 685

Leu Ser Ile Asn Asp Asn Asn Lys Lys Ala Ile Ala Asp Ala Gly Ala
    690                 695                 700

Ile Glu Pro Leu Ile His Val Leu Glu Asn Gly Ser Ser Glu Ala Lys
705                 710                 715                 720

Glu Asn Ser Ala Ala Thr Leu Phe Ser Leu Ser Val Ile Glu Glu Asn
                725                 730                 735

Lys Ile Lys Ile Gly Gln Ser Gly Ala Ile Gly Pro Leu Val Asp Leu
        740                 745                 750

Leu Gly Asn Gly Thr Pro Arg Gly Lys Lys Asp Ala Ala Thr Ala Leu
            755                 760                 765

Phe Asn Leu Ser Ile His Gln Glu Asn Lys Ala Met Ile Val Gln Ser
    770                 775                 780

Gly Ala Val Arg Tyr Leu Ile Asp Leu Met Asp Pro Ala Ala Gly Met
785                 790                 795                 800

Val Asp Lys Ala Val Ala Val Leu Ala Asn Leu Ala Thr Ile Pro Glu
                805                 810                 815

Gly Arg Asn Ala Ile Gly Gln Glu Gly Gly Ile Pro Leu Leu Val Glu
        820                 825                 830

Val Val Glu Leu Gly Ser Ala Arg Gly Lys Glu Asn Ala Ala Ala Ala
            835                 840                 845

Leu Leu Gln Leu Ser Thr Asn Ser Gly Arg Phe Cys Asn Met Val Leu
    850                 855                 860

Gln Glu Gly Ala Val Pro Pro Leu Val Ala Leu Ser Gln Ser Gly Thr
865                 870                 875                 880

Pro Arg Ala Arg Glu Lys Lys Pro Thr Ala Trp Lys Arg Trp Ala Trp
                885                 890                 895

Leu Met Met Asp Asp Asp Asp Asp Asp Asp Val Asp Asp Ala Gln Ile
            900                 905                 910

Leu Val Ser Gln Cys Leu Phe Leu Cys Phe Val Leu
        915                 920

<210> 17
<211> 2124
<212> DNA
<213> Arabidopsis thaliana

<220>
<221> CDS
<222> (1)..(2124)

<400> 17
atg atg gta cat atg gag gtg tct tgg tta aga gtt ctt cta gat aac 48
Met Met Val His Met Glu Val Ser Trp Leu Arg Val Leu Leu Asp Asn
1               5                   10                  15

atc tcc tcc tat cta agt tta tca tct atg gac gat tta tct tca aac 96
Ile Ser Ser Tyr Leu Ser Leu Ser Ser Met Asp Asp Leu Ser Ser Asn
            20                  25                  30

cct gct cat aag tac tac acc aga gga gaa gat ata gga aag ctt atc 144
Pro Ala His Lys Tyr Tyr Thr Arg Gly Glu Asp Ile Gly Lys Leu Ile
        35                  40                  45

aag cct gtt ctt gag aac ctc att gac tct gac gcg gct cct agc gag 192
Lys Pro Val Leu Glu Asn Leu Ile Asp Ser Asp Ala Ala Pro Ser Glu
    50                  55                  60

ttg ctt aac aat ggt ttt gaa gaa tta gct caa tac gtt gat gaa ctt 240
Leu Leu Asn Asn Gly Phe Glu Glu Leu Ala Gln Tyr Val Asp Glu Leu
65                  70                  75                  80

aga gaa cag ttt cag agt tgg caa cct ctt tca act aga atc ttt tat 288
Arg Glu Gln Phe Gln Ser Trp Gln Pro Leu Ser Thr Arg Ile Phe Tyr
                85                  90                  95

gtt ctt cga att gaa tca tta gca tca aag tta cga gaa tcc agt ttg 336
Val Leu Arg Ile Glu Ser Leu Ala Ser Lys Leu Arg Glu Ser Ser Leu
            100                 105                 110

gaa gtc ttt cag ctc ctc aaa cac tgc gaa caa cat ttg cct gct gac 384
Glu Val Phe Gln Leu Leu Lys His Cys Glu Gln His Leu Pro Ala Asp
        115                 120                 125

ttg atc tca cct tct ttt gag gag tgc att gaa ttg gtg aag tta gtg 432
Leu Ile Ser Pro Ser Phe Glu Glu Cys Ile Glu Leu Val Lys Leu Val
    130                 135                 140

gca aga gac gaa ata tcg tat act att gat caa gct cta aaa gat caa 480
Ala Arg Asp Glu Ile Ser Tyr Thr Ile Asp Gln Ala Leu Lys Asp Gln
145                 150                 155                 160

aag aaa ggt gtt gga cct act tca gag gtt ctg gtg aaa att gcc gag 528

Lys Lys Gly Val Gly Pro Thr Ser Glu Val Leu Val Lys Ile Ala Glu
                165                 170                 175

agt act ggt tta aga tcc aac cag gag att ctt gtt gaa ggt gtg gta      576
Ser Thr Gly Leu Arg Ser Asn Gln Glu Ile Leu Val Glu Gly Val Val
            180                 185                 190

ctt aca aac atg aag gag gat gct gag ctt acc gat aat gac acc gaa      624
Leu Thr Asn Met Lys Glu Asp Ala Glu Leu Thr Asp Asn Asp Thr Glu
        195                 200                 205

gcc gag tat cta gac gga ttg atc tct cta aca aca caa atg cat gag      672
Ala Glu Tyr Leu Asp Gly Leu Ile Ser Leu Thr Thr Gln Met His Glu
    210                 215                 220

tac ctt agc gac ata aag cag gct cag tta cgt tgt cca gta cgc gta      720
Tyr Leu Ser Asp Ile Lys Gln Ala Gln Leu Arg Cys Pro Val Arg Val
225                 230                 235                 240

cct tct gat ttc cgc tgc tct cta tct ctt gag ctt atg act gat cca      768
Pro Ser Asp Phe Arg Cys Ser Leu Ser Leu Glu Leu Met Thr Asp Pro
                245                 250                 255

gtc att gta gca tct ggt caa aca ttc gaa cgg gtt ttt atc cag aaa      816
Val Ile Val Ala Ser Gly Gln Thr Phe Glu Arg Val Phe Ile Gln Lys
            260                 265                 270

tgg atc gat atg gga ctc atg gtt tgt cca aag aca agg cag gct tta      864
Trp Ile Asp Met Gly Leu Met Val Cys Pro Lys Thr Arg Gln Ala Leu
        275                 280                 285

tct cat acc act ttg aca cct aat ttc att gtc aga gct ttt ctt gca      912
Ser His Thr Thr Leu Thr Pro Asn Phe Ile Val Arg Ala Phe Leu Ala
    290                 295                 300

agt tgg tgt gaa act aac aat gtc tat cct cct gat cca ttg gag ttg      960
Ser Trp Cys Glu Thr Asn Asn Val Tyr Pro Pro Asp Pro Leu Glu Leu
305                 310                 315                 320

att cac tca agt gag cca ttc cct ctt ctt gtt gaa tca gtg aga gct     1008
Ile His Ser Ser Glu Pro Phe Pro Leu Leu Val Glu Ser Val Arg Ala
                325                 330                 335

tca tca tca gag aat ggc cat tca gaa tct tta gat gca gag gaa ctg     1056
Ser Ser Ser Glu Asn Gly His Ser Glu Ser Leu Asp Ala Glu Glu Leu
            340                 345                 350

cgt cag gtc ttt agt agg tct gct tcg gcg cca ggc att gtc tct gaa     1104
Arg Gln Val Phe Ser Arg Ser Ala Ser Ala Pro Gly Ile Val Ser Glu
        355                 360                 365

gtg gtt tgc aaa acc aaa aga aac aac aat gct gct gca gat aga tca     1152
Val Val Cys Lys Thr Lys Arg Asn Asn Asn Ala Ala Ala Asp Arg Ser
    370                 375                 380

ctg aca cgg agt aat acc cct tgg aaa ttt cca gaa gag agg cat tgg     1200
Leu Thr Arg Ser Asn Thr Pro Trp Lys Phe Pro Glu Glu Arg His Trp
385                 390                 395                 400

cgt cac ccc ggg atc atc cca gcg acc gta aga gaa aca gga agc agt     1248
Arg His Pro Gly Ile Ile Pro Ala Thr Val Arg Glu Thr Gly Ser Ser

405                 410                 415

tca agt atc gaa acc gag gtg aag aaa ctc att gat gat ctc aag agt      1296
Ser Ser Ile Glu Thr Glu Val Lys Lys Leu Ile Asp Asp Leu Lys Ser
        420                 425                 430

tct tca ttg gat aca cag aga gag gcc aca gct aga atc agg ata cta      1344
Ser Ser Leu Asp Thr Gln Arg Glu Ala Thr Ala Arg Ile Arg Ile Leu
    435                 440                 445

gca aga aac agt aca gac aat cgc att gtc att gcg cgg tgc gaa gca      1392
Ala Arg Asn Ser Thr Asp Asn Arg Ile Val Ile Ala Arg Cys Glu Ala
    450                 455                 460

atc cct tcg tta gtc agt ctt ctt tac tca acg gat gag aga atc caa      1440
Ile Pro Ser Leu Val Ser Leu Leu Tyr Ser Thr Asp Glu Arg Ile Gln
465                 470                 475                 480

gca gac gca gtg act tgc tta cta aac tta tcc atc aac gac aac aac      1488
Ala Asp Ala Val Thr Cys Leu Leu Asn Leu Ser Ile Asn Asp Asn Asn
                485                 490                 495

aag tcc ctc atc gcg gaa agt gga gcc atc gta ccg ctt att cac gtt      1536
Lys Ser Leu Ile Ala Glu Ser Gly Ala Ile Val Pro Leu Ile His Val
            500                 505                 510

ctc aaa aca gga tac tta gaa gaa gct aaa gca aac tca gca gca act      1584
Leu Lys Thr Gly Tyr Leu Glu Glu Ala Lys Ala Asn Ser Ala Ala Thr
        515                 520                 525

cta ttc agc ttg tcg gtg atc gaa gag tac aag aca gag ata gga gaa      1632
Leu Phe Ser Leu Ser Val Ile Glu Glu Tyr Lys Thr Glu Ile Gly Glu
    530                 535                 540

gca gga gct ata gag cca ctt gtt gac ctc tta gga agt gga agt ctc      1680
Ala Gly Ala Ile Glu Pro Leu Val Asp Leu Leu Gly Ser Gly Ser Leu
545                 550                 555                 560

agt ggg aag aaa gat gca gcc acg gct tta ttc aac ctc tca ata cac      1728
Ser Gly Lys Lys Asp Ala Ala Thr Ala Leu Phe Asn Leu Ser Ile His
                565                 570                 575

cat gag aac aaa acg aaa gta atc gaa gct gga gca gtg aga tac tta      1776
His Glu Asn Lys Thr Lys Val Ile Glu Ala Gly Ala Val Arg Tyr Leu
            580                 585                 590

gtt gaa ctg atg gat cct gct ttt ggg atg gtg gag aaa gct gtg gtg      1824
Val Glu Leu Met Asp Pro Ala Phe Gly Met Val Glu Lys Ala Val Val
        595                 600                 605

gtg cta gcg aat ctt gca acg gtt aga gaa gga aag att gcg ata ggc      1872
Val Leu Ala Asn Leu Ala Thr Val Arg Glu Gly Lys Ile Ala Ile Gly
    610                 615                 620

gaa gaa gga gga ata ccg gta ttg gtg gaa gtt gtg gag tta ggt tca      1920
Glu Glu Gly Gly Ile Pro Val Leu Val Glu Val Val Glu Leu Gly Ser
625                 630                 635                 640

gca aga ggc aaa gag aat gca act gca gca cta ttg cag ctt tgt acg      1968
Ala Arg Gly Lys Glu Asn Ala Thr Ala Ala Leu Leu Gln Leu Cys Thr
                645                 650                 655

cat agc ccg aaa ttc tgc aac aat gtc ata aga gaa gga gtg att cca     2016
His Ser Pro Lys Phe Cys Asn Asn Val Ile Arg Glu Gly Val Ile Pro
            660                 665                 670

cct ctt gtg gca ctt act aaa tca gga aca gct aga ggc aaa gag aag     2064
Pro Leu Val Ala Leu Thr Lys Ser Gly Thr Ala Arg Gly Lys Glu Lys
        675                 680                 685

gca cag aat ctt ctg aag tac ttt aaa gca cac aga caa agc aat cag     2112
Ala Gln Asn Leu Leu Lys Tyr Phe Lys Ala His Arg Gln Ser Asn Gln
    690                 695                 700

agg aga ggc tga                                                     2124
Arg Arg Gly
705

<210>  18
<211>  707
<212>  PRT
<213>  Arabidopsis thaliana

<400>  18

Met Met Val His Met Glu Val Ser Trp Leu Arg Val Leu Leu Asp Asn
1               5                   10                  15

Ile Ser Ser Tyr Leu Ser Leu Ser Ser Met Asp Asp Leu Ser Ser Asn
            20                  25                  30

Pro Ala His Lys Tyr Tyr Thr Arg Gly Glu Asp Ile Gly Lys Leu Ile
        35                  40                  45

Lys Pro Val Leu Glu Asn Leu Ile Asp Ser Asp Ala Ala Pro Ser Glu
    50                  55                  60

Leu Leu Asn Asn Gly Phe Glu Glu Leu Ala Gln Tyr Val Asp Glu Leu
65                  70                  75                  80

Arg Glu Gln Phe Gln Ser Trp Gln Pro Leu Ser Thr Arg Ile Phe Tyr
                85                  90                  95

Val Leu Arg Ile Glu Ser Leu Ala Ser Lys Leu Arg Glu Ser Ser Leu
            100                 105                 110

Glu Val Phe Gln Leu Leu Lys His Cys Glu Gln His Leu Pro Ala Asp
        115                 120                 125

Leu Ile Ser Pro Ser Phe Glu Glu Cys Ile Glu Leu Val Lys Leu Val
    130                 135                 140

Ala Arg Asp Glu Ile Ser Tyr Thr Ile Asp Gln Ala Leu Lys Asp Gln
145                 150                 155                 160

Lys Lys Gly Val Gly Pro Thr Ser Glu Val Leu Val Lys Ile Ala Glu
                165                 170                 175

Ser Thr Gly Leu Arg Ser Asn Gln Glu Ile Leu Val Glu Gly Val Val
            180                 185                 190

Leu Thr Asn Met Lys Glu Asp Ala Glu Leu Thr Asp Asn Asp Thr Glu
        195                 200                 205

Ala Glu Tyr Leu Asp Gly Leu Ile Ser Leu Thr Thr Gln Met His Glu
    210                 215                 220

Tyr Leu Ser Asp Ile Lys Gln Ala Gln Leu Arg Cys Pro Val Arg Val
225                 230                 235                 240

Pro Ser Asp Phe Arg Cys Ser Leu Ser Leu Glu Leu Met Thr Asp Pro
                245                 250                 255

Val Ile Val Ala Ser Gly Gln Thr Phe Glu Arg Val Phe Ile Gln Lys
            260                 265                 270

Trp Ile Asp Met Gly Leu Met Val Cys Pro Lys Thr Arg Gln Ala Leu
        275                 280                 285

Ser His Thr Thr Leu Thr Pro Asn Phe Ile Val Arg Ala Phe Leu Ala
    290                 295                 300

Ser Trp Cys Glu Thr Asn Asn Val Tyr Pro Pro Asp Pro Leu Glu Leu
305                 310                 315                 320

Ile His Ser Ser Glu Pro Phe Pro Leu Leu Val Glu Ser Val Arg Ala
                325                 330                 335

Ser Ser Ser Glu Asn Gly His Ser Glu Ser Leu Asp Ala Glu Glu Leu
            340                 345                 350

Arg Gln Val Phe Ser Arg Ser Ala Ser Ala Pro Gly Ile Val Ser Glu
        355                 360                 365

Val Val Cys Lys Thr Lys Arg Asn Asn Asn Ala Ala Ala Asp Arg Ser
    370                 375                 380

Leu Thr Arg Ser Asn Thr Pro Trp Lys Phe Pro Glu Glu Arg His Trp

385                 390                 395                 400

Arg His Pro Gly Ile Ile Pro Ala Thr Val Arg Glu Thr Gly Ser Ser
                405                 410                 415

Ser Ser Ile Glu Thr Glu Val Lys Lys Leu Ile Asp Asp Leu Lys Ser
            420                 425                 430

Ser Ser Leu Asp Thr Gln Arg Glu Ala Thr Ala Arg Ile Arg Ile Leu
        435                 440                 445

Ala Arg Asn Ser Thr Asp Asn Arg Ile Val Ile Ala Arg Cys Glu Ala
    450                 455                 460

Ile Pro Ser Leu Val Ser Leu Leu Tyr Ser Thr Asp Glu Arg Ile Gln
465                 470                 475                 480

Ala Asp Ala Val Thr Cys Leu Leu Asn Leu Ser Ile Asn Asp Asn Asn
                485                 490                 495

Lys Ser Leu Ile Ala Glu Ser Gly Ala Ile Val Pro Leu Ile His Val
            500                 505                 510

Leu Lys Thr Gly Tyr Leu Glu Glu Ala Lys Ala Asn Ser Ala Ala Thr
        515                 520                 525

Leu Phe Ser Leu Ser Val Ile Glu Glu Tyr Lys Thr Glu Ile Gly Glu
    530                 535                 540

Ala Gly Ala Ile Glu Pro Leu Val Asp Leu Leu Gly Ser Gly Ser Leu
545                 550                 555                 560

Ser Gly Lys Lys Asp Ala Ala Thr Ala Leu Phe Asn Leu Ser Ile His
                565                 570                 575

His Glu Asn Lys Thr Lys Val Ile Glu Ala Gly Ala Val Arg Tyr Leu
            580                 585                 590

Val Glu Leu Met Asp Pro Ala Phe Gly Met Val Glu Lys Ala Val Val
        595                 600                 605

Val Leu Ala Asn Leu Ala Thr Val Arg Glu Gly Lys Ile Ala Ile Gly
    610                 615                 620

Glu Glu Gly Gly Ile Pro Val Leu Val Glu Val Val Glu Leu Gly Ser
625                 630                 635                 640

Ala Arg Gly Lys Glu Asn Ala Thr Ala Ala Leu Leu Gln Leu Cys Thr
                645                 650                 655

His Ser Pro Lys Phe Cys Asn Asn Val Ile Arg Glu Gly Val Ile Pro
            660                 665                 670

Pro Leu Val Ala Leu Thr Lys Ser Gly Thr Ala Arg Gly Lys Glu Lys
        675                 680                 685

Ala Gln Asn Leu Leu Lys Tyr Phe Lys Ala His Arg Gln Ser Asn Gln
    690                 695                 700

Arg Arg Gly
705

<210>  19
<211>  2097
<212>  DNA
<213>  Arabidopsis thaliana

<220>
<221>  CDS
<222>  (1)..(2097)

<400>  19
atg gag gtg tct tgg tta aga gtt ctt cta gat aac atc tcc tcc tat      48
Met Glu Val Ser Trp Leu Arg Val Leu Leu Asp Asn Ile Ser Ser Tyr
1               5                   10                  15

cta agt tta tca tct atg gac gat tta tct tca aac cct gct cat aag      96
Leu Ser Leu Ser Ser Met Asp Asp Leu Ser Ser Asn Pro Ala His Lys
            20                  25                  30

tac tac acc aga gga gaa gat ata gga aag ctt atc aag cct gtt ctt      144
Tyr Tyr Thr Arg Gly Glu Asp Ile Gly Lys Leu Ile Lys Pro Val Leu
        35                  40                  45

gag aac ctc att gac tct gac gcg gct cct agc gag ttg ctt aac aat      192
Glu Asn Leu Ile Asp Ser Asp Ala Ala Pro Ser Glu Leu Leu Asn Asn
    50                  55                  60

ggt ttt gaa gaa tta gct caa tac gtt gat gaa ctt aga gaa cag ttt      240
Gly Phe Glu Glu Leu Ala Gln Tyr Val Asp Glu Leu Arg Glu Gln Phe
65                  70                  75                  80

cag agt tgg caa cct ctt tca act aga atc ttt tat gtt ctt cga att      288
Gln Ser Trp Gln Pro Leu Ser Thr Arg Ile Phe Tyr Val Leu Arg Ile
                85                  90                  95

gaa tca tta gca tca aag tta cga gaa tcc agt ttg gaa gtc ttt cag      336
Glu Ser Leu Ala Ser Lys Leu Arg Glu Ser Ser Leu Glu Val Phe Gln
            100                 105                 110

ctc ctc aaa cac tgc gaa caa cat ttg cct gct gac ttg atc tca cct 384
Leu Leu Lys His Cys Glu Gln His Leu Pro Ala Asp Leu Ile Ser Pro
        115                 120                 125

tct ttt gag gag tgc att gaa ttg gtg aag tta gtg gca aga gac gaa 432
Ser Phe Glu Glu Cys Ile Glu Leu Val Lys Leu Val Ala Arg Asp Glu
    130                 135                 140

ata tcg tat act att gat caa gct cta aaa gat caa aag aaa ggt gtt 480
Ile Ser Tyr Thr Ile Asp Gln Ala Leu Lys Asp Gln Lys Lys Gly Val
145                 150                 155                 160

gga cct act tca gag gtt ctg gtg aaa att gcc gag agt act ggt tta 528
Gly Pro Thr Ser Glu Val Leu Val Lys Ile Ala Glu Ser Thr Gly Leu
                165                 170                 175

aga tcc aac cag gag att ctt gtt gaa ggt gtg gta ctt aca aac atg 576
Arg Ser Asn Gln Glu Ile Leu Val Glu Gly Val Val Leu Thr Asn Met
            180                 185                 190

aag gag gat gct gag ctt acc gat aat gac acc gaa gcc gag tat cta 624
Lys Glu Asp Ala Glu Leu Thr Asp Asn Asp Thr Glu Ala Glu Tyr Leu
        195                 200                 205

gac gga ttg atc tct cta aca aca caa atg cat gag tac ctt agc gac 672
Asp Gly Leu Ile Ser Leu Thr Thr Gln Met His Glu Tyr Leu Ser Asp
    210                 215                 220

ata aag cag gct cag tta cgt tgt cca gta cgc gta cct tct gat ttc 720
Ile Lys Gln Ala Gln Leu Arg Cys Pro Val Arg Val Pro Ser Asp Phe
225                 230                 235                 240

cgc tgc tct cta tct ctt gag ctt atg act gat cca gtc att gta gca 768
Arg Cys Ser Leu Ser Leu Glu Leu Met Thr Asp Pro Val Ile Val Ala
                245                 250                 255

tct ggt caa aca ttc gaa cgg gtt ttt atc cag aaa tgg atc gat atg 816
Ser Gly Gln Thr Phe Glu Arg Val Phe Ile Gln Lys Trp Ile Asp Met
            260                 265                 270

gga ctc atg gtt tgt cca aag aca agg cag gct tta tct cat acc act 864
Gly Leu Met Val Cys Pro Lys Thr Arg Gln Ala Leu Ser His Thr Thr
        275                 280                 285

ttg aca cct aat ttc att gtc aga gct ttt ctt gca agt tgg tgt gaa 912
Leu Thr Pro Asn Phe Ile Val Arg Ala Phe Leu Ala Ser Trp Cys Glu
    290                 295                 300

act aac aat gtc tat cct cct gat cca ttg gag ttg att cac tca agt 960
Thr Asn Asn Val Tyr Pro Pro Asp Pro Leu Glu Leu Ile His Ser Ser
305                 310                 315                 320

gag cca ttc cct ctt ctt gtt gaa tca gtg aga gct tca tca tca gag 1008
Glu Pro Phe Pro Leu Leu Val Glu Ser Val Arg Ala Ser Ser Ser Glu
                325                 330                 335

aat ggc cat tca gaa tct tta gat gca gag gaa ctg cgt cag gtc ttt 1056
Asn Gly His Ser Glu Ser Leu Asp Ala Glu Glu Leu Arg Gln Val Phe
            340                 345                 350

agt agg tct gct tcg gcg cca ggc att gtc tct gaa gtg gtt tgc aaa 1104

Ser Arg Ser Ala Ser Ala Pro Gly Ile Val Ser Glu Val Val Cys Lys
        355                 360                 365

acc aaa aga aac aac aat gct gct gca gat aga tca ctg aca cgg agt      1152
Thr Lys Arg Asn Asn Asn Ala Ala Ala Asp Arg Ser Leu Thr Arg Ser
    370                 375                 380

aat acc cct tgg aaa ttt cca gaa gag agg cat tgg cgt cac ccc ggg      1200
Asn Thr Pro Trp Lys Phe Pro Glu Glu Arg His Trp Arg His Pro Gly
385                 390                 395                 400

atc atc cca gcg acc gta aga gaa aca gga agc agt tca agt atc gaa      1248
Ile Ile Pro Ala Thr Val Arg Glu Thr Gly Ser Ser Ser Ser Ile Glu
                405                 410                 415

acc gag gtg aag aaa ctc att gat gat ctc aag agt tct tca ttg gat      1296
Thr Glu Val Lys Lys Leu Ile Asp Asp Leu Lys Ser Ser Ser Leu Asp
            420                 425                 430

aca cag aga gag gcc aca gct aga atc agg ata cta gca aga aac agt      1344
Thr Gln Arg Glu Ala Thr Ala Arg Ile Arg Ile Leu Ala Arg Asn Ser
        435                 440                 445

aca gac aat cgc att gtc att gcg cgg tgc gaa gca atc cct tcg tta      1392
Thr Asp Asn Arg Ile Val Ile Ala Arg Cys Glu Ala Ile Pro Ser Leu
    450                 455                 460

gtc agt ctt ctt tac tca acg gat gag aga atc caa gca gac gca gtg      1440
Val Ser Leu Leu Tyr Ser Thr Asp Glu Arg Ile Gln Ala Asp Ala Val
465                 470                 475                 480

act tgc tta cta aac tta tcc atc aac gac aac aac aag tcc ctc atc      1488
Thr Cys Leu Leu Asn Leu Ser Ile Asn Asp Asn Asn Lys Ser Leu Ile
                485                 490                 495

gcg gaa agt gga gcc atc gta ccg ctt att cac gtt ctc aaa aca gga      1536
Ala Glu Ser Gly Ala Ile Val Pro Leu Ile His Val Leu Lys Thr Gly
            500                 505                 510

tac tta gaa gaa gct aaa gca aac tca gca gca act cta ttc agc ttg      1584
Tyr Leu Glu Glu Ala Lys Ala Asn Ser Ala Ala Thr Leu Phe Ser Leu
        515                 520                 525

tcg gtg atc gaa gag tac aag aca gag ata gga gaa gca gga gct ata      1632
Ser Val Ile Glu Glu Tyr Lys Thr Glu Ile Gly Glu Ala Gly Ala Ile
    530                 535                 540

gag cca ctt gtt gac ctc tta gga agt gga agt ctc agt ggg aag aaa      1680
Glu Pro Leu Val Asp Leu Leu Gly Ser Gly Ser Leu Ser Gly Lys Lys
545                 550                 555                 560

gat gca gcc acg gct tta ttc aac ctc tca ata cac cat gag aac aaa      1728
Asp Ala Ala Thr Ala Leu Phe Asn Leu Ser Ile His His Glu Asn Lys
                565                 570                 575

acg aaa gta atc gaa gct gga gca gtg aga tac tta gtt gaa ctg atg      1776
Thr Lys Val Ile Glu Ala Gly Ala Val Arg Tyr Leu Val Glu Leu Met
            580                 585                 590

gat cct gct ttt ggg atg gtg gag aaa gct gtg gtg gtg cta gcg aat      1824
Asp Pro Ala Phe Gly Met Val Glu Lys Ala Val Val Val Leu Ala Asn

595                 600                 605

ctt gca acg gtt aga gaa gga aag att gcg ata ggc gaa gaa gga gga      1872
Leu Ala Thr Val Arg Glu Gly Lys Ile Ala Ile Gly Glu Glu Gly Gly
    610                 615                 620

ata ccg gta ttg gtg gaa gtt gtg gag tta ggt tca gca aga ggc aaa      1920
Ile Pro Val Leu Val Glu Val Val Glu Leu Gly Ser Ala Arg Gly Lys
625                 630                 635                 640

gag aat gca act gca gca cta ttg cag ctt tgt acg cat agc ccg aaa      1968
Glu Asn Ala Thr Ala Ala Leu Leu Gln Leu Cys Thr His Ser Pro Lys
                645                 650                 655

ttc tgc aac aat gtc ata aga gaa gga gtg att cca cct ctt gtg gca      2016
Phe Cys Asn Asn Val Ile Arg Glu Gly Val Ile Pro Pro Leu Val Ala
            660                 665                 670

ctt act aaa tca gga aca gct aga ggc aaa gag aag gtt ctt ttt ttg      2064
Leu Thr Lys Ser Gly Thr Ala Arg Gly Lys Glu Lys Val Leu Phe Leu
        675                 680                 685

ttt cct ctt ctt tgt ttg gta aat gtc tca tga                          2097
Phe Pro Leu Leu Cys Leu Val Asn Val Ser
    690                 695


<210>  20
<211>  698
<212>  PRT
<213>  Arabidopsis thaliana

<400>  20

Met Glu Val Ser Trp Leu Arg Val Leu Leu Asp Asn Ile Ser Ser Tyr
1               5                   10                  15


Leu Ser Leu Ser Ser Met Asp Asp Leu Ser Ser Asn Pro Ala His Lys
            20                  25                  30


Tyr Tyr Thr Arg Gly Glu Asp Ile Gly Lys Leu Ile Lys Pro Val Leu
        35                  40                  45


Glu Asn Leu Ile Asp Ser Asp Ala Ala Pro Ser Glu Leu Leu Asn Asn
    50                  55                  60


Gly Phe Glu Glu Leu Ala Gln Tyr Val Asp Glu Leu Arg Glu Gln Phe
65                  70                  75                  80


Gln Ser Trp Gln Pro Leu Ser Thr Arg Ile Phe Tyr Val Leu Arg Ile
                85                  90                  95


Glu Ser Leu Ala Ser Lys Leu Arg Glu Ser Ser Leu Glu Val Phe Gln
            100                 105                 110

Leu Leu Lys His Cys Glu Gln His Leu Pro Ala Asp Leu Ile Ser Pro
        115                 120                 125

Ser Phe Glu Glu Cys Ile Glu Leu Val Lys Leu Val Ala Arg Asp Glu
    130                 135                 140

Ile Ser Tyr Thr Ile Asp Gln Ala Leu Lys Asp Gln Lys Lys Gly Val
145                 150                 155                 160

Gly Pro Thr Ser Glu Val Leu Val Lys Ile Ala Glu Ser Thr Gly Leu
                165                 170                 175

Arg Ser Asn Gln Glu Ile Leu Val Glu Gly Val Val Leu Thr Asn Met
            180                 185                 190

Lys Glu Asp Ala Glu Leu Thr Asp Asn Asp Thr Glu Ala Glu Tyr Leu
        195                 200                 205

Asp Gly Leu Ile Ser Leu Thr Thr Gln Met His Glu Tyr Leu Ser Asp
    210                 215                 220

Ile Lys Gln Ala Gln Leu Arg Cys Pro Val Arg Val Pro Ser Asp Phe
225                 230                 235                 240

Arg Cys Ser Leu Ser Leu Glu Leu Met Thr Asp Pro Val Ile Val Ala
                245                 250                 255

Ser Gly Gln Thr Phe Glu Arg Val Phe Ile Gln Lys Trp Ile Asp Met
            260                 265                 270

Gly Leu Met Val Cys Pro Lys Thr Arg Gln Ala Leu Ser His Thr Thr
        275                 280                 285

Leu Thr Pro Asn Phe Ile Val Arg Ala Phe Leu Ala Ser Trp Cys Glu
    290                 295                 300

Thr Asn Asn Val Tyr Pro Pro Asp Pro Leu Glu Leu Ile His Ser Ser
305                 310                 315                 320

Glu Pro Phe Pro Leu Leu Val Glu Ser Val Arg Ala Ser Ser Ser Glu
                325                 330                 335

Asn Gly His Ser Glu Ser Leu Asp Ala Glu Glu Leu Arg Gln Val Phe
            340                 345                 350

Ser Arg Ser Ala Ser Ala Pro Gly Ile Val Ser Glu Val Val Cys Lys
        355                 360                 365

Thr Lys Arg Asn Asn Asn Ala Ala Ala Asp Arg Ser Leu Thr Arg Ser
    370                 375                 380

Asn Thr Pro Trp Lys Phe Pro Glu Glu Arg His Trp Arg His Pro Gly
385                 390                 395                 400

Ile Ile Pro Ala Thr Val Arg Glu Thr Gly Ser Ser Ser Ser Ile Glu
                405                 410                 415

Thr Glu Val Lys Lys Leu Ile Asp Asp Leu Lys Ser Ser Ser Leu Asp
            420                 425                 430

Thr Gln Arg Glu Ala Thr Ala Arg Ile Arg Ile Leu Ala Arg Asn Ser
        435                 440                 445

Thr Asp Asn Arg Ile Val Ile Ala Arg Cys Glu Ala Ile Pro Ser Leu
    450                 455                 460

Val Ser Leu Leu Tyr Ser Thr Asp Glu Arg Ile Gln Ala Asp Ala Val
465                 470                 475                 480

Thr Cys Leu Leu Asn Leu Ser Ile Asn Asp Asn Asn Lys Ser Leu Ile
                485                 490                 495

Ala Glu Ser Gly Ala Ile Val Pro Leu Ile His Val Leu Lys Thr Gly
            500                 505                 510

Tyr Leu Glu Glu Ala Lys Ala Asn Ser Ala Ala Thr Leu Phe Ser Leu
        515                 520                 525

Ser Val Ile Glu Glu Tyr Lys Thr Glu Ile Gly Glu Ala Gly Ala Ile
    530                 535                 540

Glu Pro Leu Val Asp Leu Leu Gly Ser Gly Ser Leu Ser Gly Lys Lys
545                 550                 555                 560

Asp Ala Ala Thr Ala Leu Phe Asn Leu Ser Ile His His Glu Asn Lys
                565                 570                 575

Thr Lys Val Ile Glu Ala Gly Ala Val Arg Tyr Leu Val Glu Leu Met
            580                 585                 590

Asp Pro Ala Phe Gly Met Val Glu Lys Ala Val Val Val Leu Ala Asn

595                 600                 605

Leu Ala Thr Val Arg Glu Gly Lys Ile Ala Ile Gly Glu Glu Gly Gly
    610                 615                 620

Ile Pro Val Leu Val Glu Val Val Glu Leu Gly Ser Ala Arg Gly Lys
625                 630                 635                 640

Glu Asn Ala Thr Ala Ala Leu Leu Gln Leu Cys Thr His Ser Pro Lys
                645                 650                 655

Phe Cys Asn Asn Val Ile Arg Glu Gly Val Ile Pro Pro Leu Val Ala
            660                 665                 670

Leu Thr Lys Ser Gly Thr Ala Arg Gly Lys Glu Lys Val Leu Phe Leu
        675                 680                 685

Phe Pro Leu Leu Cys Leu Val Asn Val Ser
    690                 695

<210>  21
<211>  2283
<212>  DNA
<213>  Arabidopsis thaliana

<220>
<221>  CDS
<222>  (1)..(2283)

<400>  21
atg gat cct gtt cct gtt cga tgt ctt ctt aac agt ata tct cgg tat      48
Met Asp Pro Val Pro Val Arg Cys Leu Leu Asn Ser Ile Ser Arg Tyr
1               5                   10                  15

ctt cat ctg gtt gcg tgc cag act ata aga ttt aat cct att caa aca      96
Leu His Leu Val Ala Cys Gln Thr Ile Arg Phe Asn Pro Ile Gln Thr
            20                  25                  30

tgt att gga aat atg gtt ctc ttg ttg aag ctc ttg aaa ccg ttg ctc      144
Cys Ile Gly Asn Met Val Leu Leu Leu Lys Leu Leu Lys Pro Leu Leu
        35                  40                  45

gat gaa gtt gtt gat tgc aag ata cct tct gat gac tgt tta tat aaa      192
Asp Glu Val Val Asp Cys Lys Ile Pro Ser Asp Asp Cys Leu Tyr Lys
    50                  55                  60

gga tgt gaa gac ctt gat tct gtt gtt aac cag gct cgg gag ttc tta      240
Gly Cys Glu Asp Leu Asp Ser Val Val Asn Gln Ala Arg Glu Phe Leu
65                  70                  75                  80

gag gac tgg tca cca aag ttg agc aag ttg ttt ggt gtg ttt caa tgc      288
Glu Asp Trp Ser Pro Lys Leu Ser Lys Leu Phe Gly Val Phe Gln Cys
                85                  90                  95

gag gtt ttg ttg gga aag gtc cag act tgt tcg ttg gag att agt cgc     336
Glu Val Leu Leu Gly Lys Val Gln Thr Cys Ser Leu Glu Ile Ser Arg
        100                 105                 110

ata ctt ctt cag tta tca cag tca agt ccg gtt act tca agc gta caa     384
Ile Leu Leu Gln Leu Ser Gln Ser Ser Pro Val Thr Ser Ser Val Gln
        115                 120                 125

agt gtt gag cgc tgc gtg cag gag act gag agt ttt aag caa gag ggg     432
Ser Val Glu Arg Cys Val Gln Glu Thr Glu Ser Phe Lys Gln Glu Gly
    130                 135                 140

aca tta atg gaa ctc atg gag aat gct tta cgg aat cag aaa gat gat     480
Thr Leu Met Glu Leu Met Glu Asn Ala Leu Arg Asn Gln Lys Asp Asp
145                 150                 155                 160

att acc tct ttg gat aac aat cat ctg gaa agc ata att caa atg ctt     528
Ile Thr Ser Leu Asp Asn Asn His Leu Glu Ser Ile Ile Gln Met Leu
                165                 170                 175

gga ttg ata tca aac caa gat ctc tta aag gaa agc att act gtg gag     576
Gly Leu Ile Ser Asn Gln Asp Leu Leu Lys Glu Ser Ile Thr Val Glu
        180                 185                 190

aaa gag agg ata aga tcc cag gcc agt aag tca gaa gaa gat atg gaa     624
Lys Glu Arg Ile Arg Ser Gln Ala Ser Lys Ser Glu Glu Asp Met Glu
        195                 200                 205

caa acc gaa cag ttg ata gaa ctc gtc ttg tgc atc cgt gaa cac atg     672
Gln Thr Glu Gln Leu Ile Glu Leu Val Leu Cys Ile Arg Glu His Met
    210                 215                 220

ctt aaa act gag ttt ctt gaa gtg gct aaa ggt atc tcg ata ccc ccg     720
Leu Lys Thr Glu Phe Leu Glu Val Ala Lys Gly Ile Ser Ile Pro Pro
225                 230                 235                 240

tat ttc cgg tgt cct ttg tca aca gaa ctc atg ctg gat ccg gta ata     768
Tyr Phe Arg Cys Pro Leu Ser Thr Glu Leu Met Leu Asp Pro Val Ile
                245                 250                 255

gta gct tca gga cag aca ttt gac aga aca tcc att aag aaa tgg ctt     816
Val Ala Ser Gly Gln Thr Phe Asp Arg Thr Ser Ile Lys Lys Trp Leu
            260                 265                 270

gat aac ggg tta gct gtt tgt cca agg acg cgg cag gtg ctg act cat     864
Asp Asn Gly Leu Ala Val Cys Pro Arg Thr Arg Gln Val Leu Thr His
        275                 280                 285

caa gaa ctc att ccc aat tac acg gtt aag gct atg ata gcg agt tgg     912
Gln Glu Leu Ile Pro Asn Tyr Thr Val Lys Ala Met Ile Ala Ser Trp
    290                 295                 300

ttg gag gca aac agg atc aac ctt gct act aac tct tgt cat cag tat     960
Leu Glu Ala Asn Arg Ile Asn Leu Ala Thr Asn Ser Cys His Gln Tyr
305                 310                 315                 320

gat ggt ggt gat gct tca tcc atg gct aat aat atg ggt tct caa gac    1008
Asp Gly Gly Asp Ala Ser Ser Met Ala Asn Asn Met Gly Ser Gln Asp
                325                 330                 335

ttt aac cgc acc gag agt ttt cgt ttt tct tta cgg agc agc agt tta 1056
Phe Asn Arg Thr Glu Ser Phe Arg Phe Ser Leu Arg Ser Ser Ser Leu
            340                 345                 350

acc tca aga tca tct ctt gaa act gga aat ggg ttt gag aaa ctg aag 1104
Thr Ser Arg Ser Ser Leu Glu Thr Gly Asn Gly Phe Glu Lys Leu Lys
        355                 360                 365

att aac gtg tct gcc agt tta tgc ggg gaa tct caa agc aag gat ctt 1152
Ile Asn Val Ser Ala Ser Leu Cys Gly Glu Ser Gln Ser Lys Asp Leu
    370                 375                 380

gaa ata ttc gag ctt ttg tct ccg ggg cag tct tac act cac agc agg 1200
Glu Ile Phe Glu Leu Leu Ser Pro Gly Gln Ser Tyr Thr His Ser Arg
385                 390                 395                 400

agt gaa tca gtt tgc agt gtt gtc tcg tct gtt gat tat gta cct tcg 1248
Ser Glu Ser Val Cys Ser Val Val Ser Ser Val Asp Tyr Val Pro Ser
                405                 410                 415

gtg aca cat gag aca gaa agt ata cta ggg aat cac caa agc tcc agt 1296
Val Thr His Glu Thr Glu Ser Ile Leu Gly Asn His Gln Ser Ser Ser
            420                 425                 430

gag atg tct ccc aag aaa aac tta gaa agt tca aac aat gta aat cat 1344
Glu Met Ser Pro Lys Lys Asn Leu Glu Ser Ser Asn Asn Val Asn His
        435                 440                 445

gag cat agc gca gca aag act tat gag tgt tct gta cat gat tta gat 1392
Glu His Ser Ala Ala Lys Thr Tyr Glu Cys Ser Val His Asp Leu Asp
    450                 455                 460

gat tca gga aca atg acg act tca cat acc ata aaa ttg gta gaa gat 1440
Asp Ser Gly Thr Met Thr Thr Ser His Thr Ile Lys Leu Val Glu Asp
465                 470                 475                 480

ctt aaa agc ggg tct aac aaa gtg aag act gct gct gca gct gaa ata 1488
Leu Lys Ser Gly Ser Asn Lys Val Lys Thr Ala Ala Ala Ala Glu Ile
                485                 490                 495

cgt cat ctc acc att aac agc att gaa aat cgt gtt cac atc ggg cgt 1536
Arg His Leu Thr Ile Asn Ser Ile Glu Asn Arg Val His Ile Gly Arg
            500                 505                 510

tgt ggt gct att act cca ctg ctg tca ctt tta tac tca gaa gaa aag 1584
Cys Gly Ala Ile Thr Pro Leu Leu Ser Leu Leu Tyr Ser Glu Glu Lys
        515                 520                 525

cta act caa gaa cac gca gtc acg gct ctt ttg aat ctt tcc atc agt 1632
Leu Thr Gln Glu His Ala Val Thr Ala Leu Leu Asn Leu Ser Ile Ser
    530                 535                 540

gaa cta aac aaa gcc atg att gtg gaa gtc ggg gcg ata gaa ccg ctt 1680
Glu Leu Asn Lys Ala Met Ile Val Glu Val Gly Ala Ile Glu Pro Leu
545                 550                 555                 560

gtt cat gtt ttg aac aca gga aat gac aga gcc aaa gag aat tca gca 1728
Val His Val Leu Asn Thr Gly Asn Asp Arg Ala Lys Glu Asn Ser Ala
                565                 570                 575

gca tca ttg ttc agt ctg tct gtt ctg cag gtc aac aga gaa cga ata 1776

Ala Ser Leu Phe Ser Leu Ser Val Leu Gln Val Asn Arg Glu Arg Ile
            580                 585                 590

ggc cag tct aac gca gcg ata caa gct ctg gtg aat ctt ctt ggt aaa      1824
Gly Gln Ser Asn Ala Ala Ile Gln Ala Leu Val Asn Leu Leu Gly Lys
        595                 600                 605

gga aca ttt aga gga aag aaa gac gcc gcc tct gct ttg ttc aat cta      1872
Gly Thr Phe Arg Gly Lys Lys Asp Ala Ala Ser Ala Leu Phe Asn Leu
    610                 615                 620

tcg att act cat gat aac aag gcc cgt atc gtg caa gct aag gcg gtt      1920
Ser Ile Thr His Asp Asn Lys Ala Arg Ile Val Gln Ala Lys Ala Val
625                 630                 635                 640

aag tac ctt gtg gag ctg tta gac cca gat tta gag atg gtt gat aaa      1968
Lys Tyr Leu Val Glu Leu Leu Asp Pro Asp Leu Glu Met Val Asp Lys
                645                 650                 655

gca gtt gct ctt ctt gca aat ctt tct gca gtt gga gaa ggg cgt caa      2016
Ala Val Ala Leu Leu Ala Asn Leu Ser Ala Val Gly Glu Gly Arg Gln
            660                 665                 670

gcc atc gtg agg gaa ggt ggg att cca tta ctt gtt gaa act gtt gac      2064
Ala Ile Val Arg Glu Gly Gly Ile Pro Leu Leu Val Glu Thr Val Asp
        675                 680                 685

tta gga tct cag aga ggg aaa gag aat gca gct tct gtg ctg ctt cag      2112
Leu Gly Ser Gln Arg Gly Lys Glu Asn Ala Ala Ser Val Leu Leu Gln
    690                 695                 700

ttg tgt ctg aac agt ccc aag ttt tgc act ctg gtc ttg caa gaa ggc      2160
Leu Cys Leu Asn Ser Pro Lys Phe Cys Thr Leu Val Leu Gln Glu Gly
705                 710                 715                 720

gcc ata cct ccg ctt gtt gcc ttg tct cag tct ggt aca cag aga gca      2208
Ala Ile Pro Pro Leu Val Ala Leu Ser Gln Ser Gly Thr Gln Arg Ala
                725                 730                 735

aaa gag aag gca cag caa ctt ctt agc cac ttc cga aac cag aga gat      2256
Lys Glu Lys Ala Gln Gln Leu Leu Ser His Phe Arg Asn Gln Arg Asp
            740                 745                 750

gca agg atg aag aaa ggt aga tca tga                                  2283
Ala Arg Met Lys Lys Gly Arg Ser
        755                 760

<210>  22
<211>  760
<212>  PRT
<213>  Arabidopsis thaliana

<400>  22

Met Asp Pro Val Pro Val Arg Cys Leu Leu Asn Ser Ile Ser Arg Tyr
1               5                   10                  15

Leu His Leu Val Ala Cys Gln Thr Ile Arg Phe Asn Pro Ile Gln Thr
            20                  25                  30

Cys Ile Gly Asn Met Val Leu Leu Leu Lys Leu Leu Lys Pro Leu Leu
        35                  40                  45

Asp Glu Val Val Asp Cys Lys Ile Pro Ser Asp Asp Cys Leu Tyr Lys
    50                  55                  60

Gly Cys Glu Asp Leu Asp Ser Val Val Asn Gln Ala Arg Glu Phe Leu
65                  70                  75                  80

Glu Asp Trp Ser Pro Lys Leu Ser Lys Leu Phe Gly Val Phe Gln Cys
                85                  90                  95

Glu Val Leu Leu Gly Lys Val Gln Thr Cys Ser Leu Glu Ile Ser Arg
            100                 105                 110

Ile Leu Leu Gln Leu Ser Gln Ser Ser Pro Val Thr Ser Ser Val Gln
        115                 120                 125

Ser Val Glu Arg Cys Val Gln Glu Thr Glu Ser Phe Lys Gln Glu Gly
    130                 135                 140

Thr Leu Met Glu Leu Met Glu Asn Ala Leu Arg Asn Gln Lys Asp Asp
145                 150                 155                 160

Ile Thr Ser Leu Asp Asn Asn His Leu Glu Ser Ile Ile Gln Met Leu
                165                 170                 175

Gly Leu Ile Ser Asn Gln Asp Leu Leu Lys Glu Ser Ile Thr Val Glu
            180                 185                 190

Lys Glu Arg Ile Arg Ser Gln Ala Ser Lys Ser Glu Glu Asp Met Glu
        195                 200                 205

Gln Thr Glu Gln Leu Ile Glu Leu Val Leu Cys Ile Arg Glu His Met
    210                 215                 220

Leu Lys Thr Glu Phe Leu Glu Val Ala Lys Gly Ile Ser Ile Pro Pro
225                 230                 235                 240

Tyr Phe Arg Cys Pro Leu Ser Thr Glu Leu Met Leu Asp Pro Val Ile
                245                 250                 255

Val Ala Ser Gly Gln Thr Phe Asp Arg Thr Ser Ile Lys Lys Trp Leu
            260                 265                 270

Asp Asn Gly Leu Ala Val Cys Pro Arg Thr Arg Gln Val Leu Thr His
        275                 280                 285

Gln Glu Leu Ile Pro Asn Tyr Thr Val Lys Ala Met Ile Ala Ser Trp
    290                 295                 300

Leu Glu Ala Asn Arg Ile Asn Leu Ala Thr Asn Ser Cys His Gln Tyr
305                 310                 315                 320

Asp Gly Gly Asp Ala Ser Ser Met Ala Asn Asn Met Gly Ser Gln Asp
                325                 330                 335

Phe Asn Arg Thr Glu Ser Phe Arg Phe Ser Leu Arg Ser Ser Ser Leu
            340                 345                 350

Thr Ser Arg Ser Ser Leu Glu Thr Gly Asn Gly Phe Glu Lys Leu Lys
        355                 360                 365

Ile Asn Val Ser Ala Ser Leu Cys Gly Glu Ser Gln Ser Lys Asp Leu
    370                 375                 380

Glu Ile Phe Glu Leu Leu Ser Pro Gly Gln Ser Tyr Thr His Ser Arg
385                 390                 395                 400

Ser Glu Ser Val Cys Ser Val Val Ser Ser Val Asp Tyr Val Pro Ser
                405                 410                 415

Val Thr His Glu Thr Glu Ser Ile Leu Gly Asn His Gln Ser Ser Ser
            420                 425                 430

Glu Met Ser Pro Lys Lys Asn Leu Glu Ser Ser Asn Asn Val Asn His
        435                 440                 445

Glu His Ser Ala Ala Lys Thr Tyr Glu Cys Ser Val His Asp Leu Asp
    450                 455                 460

Asp Ser Gly Thr Met Thr Thr Ser His Thr Ile Lys Leu Val Glu Asp
465                 470                 475                 480

Leu Lys Ser Gly Ser Asn Lys Val Lys Thr Ala Ala Ala Ala Glu Ile
                485                 490                 495

Arg His Leu Thr Ile Asn Ser Ile Glu Asn Arg Val His Ile Gly Arg
            500                 505                 510

Cys Gly Ala Ile Thr Pro Leu Leu Ser Leu Leu Tyr Ser Glu Glu Lys
        515                 520                 525

Leu Thr Gln Glu His Ala Val Thr Ala Leu Leu Asn Leu Ser Ile Ser
    530                 535                 540

Glu Leu Asn Lys Ala Met Ile Val Glu Val Gly Ala Ile Glu Pro Leu
545                 550                 555                 560

Val His Val Leu Asn Thr Gly Asn Asp Arg Ala Lys Glu Asn Ser Ala
                565                 570                 575

Ala Ser Leu Phe Ser Leu Ser Val Leu Gln Val Asn Arg Glu Arg Ile
            580                 585                 590

Gly Gln Ser Asn Ala Ala Ile Gln Ala Leu Val Asn Leu Leu Gly Lys
        595                 600                 605

Gly Thr Phe Arg Gly Lys Lys Asp Ala Ala Ser Ala Leu Phe Asn Leu
    610                 615                 620

Ser Ile Thr His Asp Asn Lys Ala Arg Ile Val Gln Ala Lys Ala Val
625                 630                 635                 640

Lys Tyr Leu Val Glu Leu Leu Asp Pro Asp Leu Glu Met Val Asp Lys
                645                 650                 655

Ala Val Ala Leu Leu Ala Asn Leu Ser Ala Val Gly Glu Gly Arg Gln
            660                 665                 670

Ala Ile Val Arg Glu Gly Gly Ile Pro Leu Leu Val Glu Thr Val Asp
        675                 680                 685

Leu Gly Ser Gln Arg Gly Lys Glu Asn Ala Ala Ser Val Leu Leu Gln
    690                 695                 700

Leu Cys Leu Asn Ser Pro Lys Phe Cys Thr Leu Val Leu Gln Glu Gly
705                 710                 715                 720

Ala Ile Pro Pro Leu Val Ala Leu Ser Gln Ser Gly Thr Gln Arg Ala
                725                 730                 735

Lys Glu Lys Ala Gln Gln Leu Leu Ser His Phe Arg Asn Gln Arg Asp
            740                 745                 750

Ala Arg Met Lys Lys Gly Arg Ser

755                 760

<210>  23
<211>  2283
<212>  DNA
<213>  Arabidopsis thaliana

<220>
<221>  CDS
<222>  (1)..(2283)

<400>  23
atg gat cct gtt cct gtt cga tgt ctt ctt aac agt ata tct cgg tat      48
Met Asp Pro Val Pro Val Arg Cys Leu Leu Asn Ser Ile Ser Arg Tyr
1               5                   10                  15

ctt cat ctg gtt gcg tgc cag act ata aga ttt aat cct att caa aca      96
Leu His Leu Val Ala Cys Gln Thr Ile Arg Phe Asn Pro Ile Gln Thr
            20                  25                  30

tgt att gga aat atg gtt ctc ttg ttg aag ctc ttg aaa ccg ttg ctc      144
Cys Ile Gly Asn Met Val Leu Leu Leu Lys Leu Leu Lys Pro Leu Leu
        35                  40                  45

gat gaa gtt gtt gat tgc aag ata cct tct gat gac tgt tta tat aaa      192
Asp Glu Val Val Asp Cys Lys Ile Pro Ser Asp Asp Cys Leu Tyr Lys
    50                  55                  60

gga cgt gaa gac ctt gat tct gtt gtt aac cag gct cgg gag ttc tta      240
Gly Arg Glu Asp Leu Asp Ser Val Val Asn Gln Ala Arg Glu Phe Leu
65                  70                  75                  80

gag gac tgg tca cca aag ttg agc aag ttg ttt ggt gtg ttt caa tgc      288
Glu Asp Trp Ser Pro Lys Leu Ser Lys Leu Phe Gly Val Phe Gln Cys
                85                  90                  95

gag gtt ttg ttg gga aag gtc cag act tgt tcg ttg gag att agt cgc      336
Glu Val Leu Leu Gly Lys Val Gln Thr Cys Ser Leu Glu Ile Ser Arg
            100                 105                 110

ata ctt ctt cag tta tca cag tca agt ccg gtt act tca agc gta caa      384
Ile Leu Leu Gln Leu Ser Gln Ser Ser Pro Val Thr Ser Ser Val Gln
        115                 120                 125

agt gtt gag cgc tgc gtg cag gag act gag agt ttt aag caa gag ggg      432
Ser Val Glu Arg Cys Val Gln Glu Thr Glu Ser Phe Lys Gln Glu Gly
    130                 135                 140

aca tta atg gaa ctc atg gag aat gct tta cgg aat cag aaa gat gat      480
Thr Leu Met Glu Leu Met Glu Asn Ala Leu Arg Asn Gln Lys Asp Asp
145                 150                 155                 160

att acc tct ttg gat aac aat cat ctg gaa agc ata att caa atg ctt      528
Ile Thr Ser Leu Asp Asn Asn His Leu Glu Ser Ile Ile Gln Met Leu
                165                 170                 175

gga ttg ata tca aac caa gat ctc tta aag gaa agc att act gtg gag      576
Gly Leu Ile Ser Asn Gln Asp Leu Leu Lys Glu Ser Ile Thr Val Glu
            180                 185                 190

aaa gag agg ata aga tcc cag gcc agt aag tca gaa gaa gat atg gaa 624
Lys Glu Arg Ile Arg Ser Gln Ala Ser Lys Ser Glu Glu Asp Met Glu
        195                 200                 205

caa acc gaa cag ttg ata gaa ctc gtc ttg tgc atc cgt gaa cac atg 672
Gln Thr Glu Gln Leu Ile Glu Leu Val Leu Cys Ile Arg Glu His Met
    210                 215                 220

ctt aaa act gag ttt ctt gaa gtg gct aaa ggt atc tcg ata ccc ccg 720
Leu Lys Thr Glu Phe Leu Glu Val Ala Lys Gly Ile Ser Ile Pro Pro
225                 230                 235                 240

tat ttc cgg tgt cct ttg tca aca gaa ctc atg ctg gat ccg gta ata 768
Tyr Phe Arg Cys Pro Leu Ser Thr Glu Leu Met Leu Asp Pro Val Ile
                245                 250                 255

gta gct tca gga cag aca ttt gac aga aca tcc att aag aaa tgg ctt 816
Val Ala Ser Gly Gln Thr Phe Asp Arg Thr Ser Ile Lys Lys Trp Leu
            260                 265                 270

gat aac ggg tta gct gtt tgt cca agg acg cgg cag gtg ctg act cat 864
Asp Asn Gly Leu Ala Val Cys Pro Arg Thr Arg Gln Val Leu Thr His
        275                 280                 285

caa gaa ctc att ccc aat tac acg gtt aag gct atg ata gcg agt tgg 912
Gln Glu Leu Ile Pro Asn Tyr Thr Val Lys Ala Met Ile Ala Ser Trp
    290                 295                 300

ttg gag gca aac agg atc aac ctt gct act aac tct tgt cat cag tat 960
Leu Glu Ala Asn Arg Ile Asn Leu Ala Thr Asn Ser Cys His Gln Tyr
305                 310                 315                 320

gat ggt ggt gat gct tca tcc atg gct aat aat atg ggt tct caa gac 1008
Asp Gly Gly Asp Ala Ser Ser Met Ala Asn Asn Met Gly Ser Gln Asp
                325                 330                 335

ttt aac cgc acc gag agt ttt cgt ttt tct tta cgg agc agc agt tta 1056
Phe Asn Arg Thr Glu Ser Phe Arg Phe Ser Leu Arg Ser Ser Ser Leu
            340                 345                 350

acc tca aga tca tct ctt gaa act gga aat ggg ttt gag aaa ctg aag 1104
Thr Ser Arg Ser Ser Leu Glu Thr Gly Asn Gly Phe Glu Lys Leu Lys
        355                 360                 365

att aac gtg tct gcc agt tta tgc ggg gaa tct caa agc aag gat ctt 1152
Ile Asn Val Ser Ala Ser Leu Cys Gly Glu Ser Gln Ser Lys Asp Leu
    370                 375                 380

gaa ata ttc gag ctt ttg tct ccg ggg cag tct tac act cac agc agg 1200
Glu Ile Phe Glu Leu Leu Ser Pro Gly Gln Ser Tyr Thr His Ser Arg
385                 390                 395                 400

agt gaa tca gtt tgc agt gtt gtc tcg tct gtt gat tat gta cct tcg 1248
Ser Glu Ser Val Cys Ser Val Val Ser Ser Val Asp Tyr Val Pro Ser
                405                 410                 415

gtg aca cat gag aca gaa agt ata cta ggg aat cac caa agc tcc agt 1296
Val Thr His Glu Thr Glu Ser Ile Leu Gly Asn His Gln Ser Ser Ser
            420                 425                 430

gag atg tct ccc aag aaa aac tta gaa agt tca aac aat gta aat cat     1344
Glu Met Ser Pro Lys Lys Asn Leu Glu Ser Ser Asn Asn Val Asn His
        435                 440                 445

gag cat agc gca gca aag act tat gag tgt tct gta cat gat tta gat     1392
Glu His Ser Ala Ala Lys Thr Tyr Glu Cys Ser Val His Asp Leu Asp
    450                 455                 460

gat tca gga aca atg acg act tca cat acc ata aaa ttg gta gaa gat     1440
Asp Ser Gly Thr Met Thr Thr Ser His Thr Ile Lys Leu Val Glu Asp
465                 470                 475                 480

ctt aaa agc ggg tct aac aaa gtg aag act gct gct gca gct gaa ata     1488
Leu Lys Ser Gly Ser Asn Lys Val Lys Thr Ala Ala Ala Ala Glu Ile
                485                 490                 495

cgt cat ctc acc att aac agc att gaa aat cgt gtt cac atc ggg cgt     1536
Arg His Leu Thr Ile Asn Ser Ile Glu Asn Arg Val His Ile Gly Arg
            500                 505                 510

tgt ggt gct att act cca ctg ctg tca ctt tta tac tca gaa gaa aag     1584
Cys Gly Ala Ile Thr Pro Leu Leu Ser Leu Leu Tyr Ser Glu Glu Lys
        515                 520                 525

cta act caa gaa cac gca gtc acg gct ctt ttg aat ctt tcc atc agt     1632
Leu Thr Gln Glu His Ala Val Thr Ala Leu Leu Asn Leu Ser Ile Ser
    530                 535                 540

gaa cta aac aaa gcc atg att gtg gaa gtc ggg gcg gta gaa ccg ctt     1680
Glu Leu Asn Lys Ala Met Ile Val Glu Val Gly Ala Val Glu Pro Leu
545                 550                 555                 560

gtt cat gtt ttg aac aca gga aat gac aga gcc aaa gag aat tca gca     1728
Val His Val Leu Asn Thr Gly Asn Asp Arg Ala Lys Glu Asn Ser Ala
                565                 570                 575

gca tca ttg ttc agt ctg tct gtt ctg cag gtc aac aga gaa cga ata     1776
Ala Ser Leu Phe Ser Leu Ser Val Leu Gln Val Asn Arg Glu Arg Ile
            580                 585                 590

ggc cag tct aac gca gcg ata caa gct ctg gtg aat ctt ctt ggt aaa     1824
Gly Gln Ser Asn Ala Ala Ile Gln Ala Leu Val Asn Leu Leu Gly Lys
        595                 600                 605

gga aca ttt aga gga aag aaa gac gcc gcc tct gct ttg ttc aat cta     1872
Gly Thr Phe Arg Gly Lys Lys Asp Ala Ala Ser Ala Leu Phe Asn Leu
    610                 615                 620

tcg att act cat gat aac aag gcc cgt atc gtg caa gct aag gcg gtt     1920
Ser Ile Thr His Asp Asn Lys Ala Arg Ile Val Gln Ala Lys Ala Val
625                 630                 635                 640

aag tac ctt gtg gag ctg tta gac cca gat tta gag atg gtt gat aaa     1968
Lys Tyr Leu Val Glu Leu Leu Asp Pro Asp Leu Glu Met Val Asp Lys
                645                 650                 655

gca gtt gct ctt ctt gca aat ctt tct gca gtt gga gaa ggg cgt caa     2016
Ala Val Ala Leu Leu Ala Asn Leu Ser Ala Val Gly Glu Gly Arg Gln
            660                 665                 670

gcc atc gtg agg gaa ggt ggg att cca tta ctt gtt gaa act gtt gac     2064

Ala Ile Val Arg Glu Gly Gly Ile Pro Leu Leu Val Glu Thr Val Asp
        675                 680                 685

tta gga tct cag aga ggg aaa gag aat gca gct tct gtg ctg ctt cag      2112
Leu Gly Ser Gln Arg Gly Lys Glu Asn Ala Ala Ser Val Leu Leu Gln
    690                 695                 700

ttg tgt ctg aac agt ccc aag ttt tgc act ctg gtc ttg caa gaa ggc      2160
Leu Cys Leu Asn Ser Pro Lys Phe Cys Thr Leu Val Leu Gln Glu Gly
705                 710                 715                 720

gcc ata cct ccg ctt gtt gcc ttg tct cag tct ggt aca cag aga gca      2208
Ala Ile Pro Pro Leu Val Ala Leu Ser Gln Ser Gly Thr Gln Arg Ala
                725                 730                 735

aaa gag aag gca cag caa ctt ctt agc cac ttc cga aac cag aga gat      2256
Lys Glu Lys Ala Gln Gln Leu Leu Ser His Phe Arg Asn Gln Arg Asp
            740                 745                 750

gca agg atg aag aaa ggt aga tca tga                                  2283
Ala Arg Met Lys Lys Gly Arg Ser
        755                 760

<210>  24
<211>  760
<212>  PRT
<213>  Arabidopsis thaliana

<400>  24

Met Asp Pro Val Pro Val Arg Cys Leu Leu Asn Ser Ile Ser Arg Tyr
1               5                   10                  15

Leu His Leu Val Ala Cys Gln Thr Ile Arg Phe Asn Pro Ile Gln Thr
            20                  25                  30

Cys Ile Gly Asn Met Val Leu Leu Leu Lys Leu Leu Lys Pro Leu Leu
        35                  40                  45

Asp Glu Val Val Asp Cys Lys Ile Pro Ser Asp Asp Cys Leu Tyr Lys
    50                  55                  60

Gly Arg Glu Asp Leu Asp Ser Val Val Asn Gln Ala Arg Glu Phe Leu
65                  70                  75                  80

Glu Asp Trp Ser Pro Lys Leu Ser Lys Leu Phe Gly Val Phe Gln Cys
                85                  90                  95

Glu Val Leu Leu Gly Lys Val Gln Thr Cys Ser Leu Glu Ile Ser Arg
            100                 105                 110

Ile Leu Leu Gln Leu Ser Gln Ser Ser Pro Val Thr Ser Ser Val Gln
        115                 120                 125

Ser Val Glu Arg Cys Val Gln Glu Thr Glu Ser Phe Lys Gln Glu Gly
    130                 135                 140

Thr Leu Met Glu Leu Met Glu Asn Ala Leu Arg Asn Gln Lys Asp Asp
145                 150                 155                 160

Ile Thr Ser Leu Asp Asn Asn His Leu Glu Ser Ile Ile Gln Met Leu
                165                 170                 175

Gly Leu Ile Ser Asn Gln Asp Leu Leu Lys Glu Ser Ile Thr Val Glu
            180                 185                 190

Lys Glu Arg Ile Arg Ser Gln Ala Ser Lys Ser Glu Glu Asp Met Glu
        195                 200                 205

Gln Thr Glu Gln Leu Ile Glu Leu Val Leu Cys Ile Arg Glu His Met
    210                 215                 220

Leu Lys Thr Glu Phe Leu Glu Val Ala Lys Gly Ile Ser Ile Pro Pro
225                 230                 235                 240

Tyr Phe Arg Cys Pro Leu Ser Thr Glu Leu Met Leu Asp Pro Val Ile
                245                 250                 255

Val Ala Ser Gly Gln Thr Phe Asp Arg Thr Ser Ile Lys Lys Trp Leu
            260                 265                 270

Asp Asn Gly Leu Ala Val Cys Pro Arg Thr Arg Gln Val Leu Thr His
        275                 280                 285

Gln Glu Leu Ile Pro Asn Tyr Thr Val Lys Ala Met Ile Ala Ser Trp
    290                 295                 300

Leu Glu Ala Asn Arg Ile Asn Leu Ala Thr Asn Ser Cys His Gln Tyr
305                 310                 315                 320

Asp Gly Gly Asp Ala Ser Ser Met Ala Asn Asn Met Gly Ser Gln Asp
                325                 330                 335

Phe Asn Arg Thr Glu Ser Phe Arg Phe Ser Leu Arg Ser Ser Ser Leu
            340                 345                 350

Thr Ser Arg Ser Ser Leu Glu Thr Gly Asn Gly Phe Glu Lys Leu Lys
        355                 360                 365

Ile Asn Val Ser Ala Ser Leu Cys Gly Glu Ser Gln Ser Lys Asp Leu
    370                 375                 380

Glu Ile Phe Glu Leu Leu Ser Pro Gly Gln Ser Tyr Thr His Ser Arg
385                 390                 395                 400

Ser Glu Ser Val Cys Ser Val Val Ser Ser Val Asp Tyr Val Pro Ser
                405                 410                 415

Val Thr His Glu Thr Glu Ser Ile Leu Gly Asn His Gln Ser Ser Ser
            420                 425                 430

Glu Met Ser Pro Lys Lys Asn Leu Glu Ser Ser Asn Asn Val Asn His
        435                 440                 445

Glu His Ser Ala Ala Lys Thr Tyr Glu Cys Ser Val His Asp Leu Asp
    450                 455                 460

Asp Ser Gly Thr Met Thr Thr Ser His Thr Ile Lys Leu Val Glu Asp
465                 470                 475                 480

Leu Lys Ser Gly Ser Asn Lys Val Lys Thr Ala Ala Ala Ala Glu Ile
                485                 490                 495

Arg His Leu Thr Ile Asn Ser Ile Glu Asn Arg Val His Ile Gly Arg
            500                 505                 510

Cys Gly Ala Ile Thr Pro Leu Leu Ser Leu Leu Tyr Ser Glu Glu Lys
        515                 520                 525

Leu Thr Gln Glu His Ala Val Thr Ala Leu Leu Asn Leu Ser Ile Ser
    530                 535                 540

Glu Leu Asn Lys Ala Met Ile Val Glu Val Gly Ala Val Glu Pro Leu
545                 550                 555                 560

Val His Val Leu Asn Thr Gly Asn Asp Arg Ala Lys Glu Asn Ser Ala
                565                 570                 575

Ala Ser Leu Phe Ser Leu Ser Val Leu Gln Val Asn Arg Glu Arg Ile
            580                 585                 590

Gly Gln Ser Asn Ala Ala Ile Gln Ala Leu Val Asn Leu Leu Gly Lys
        595                 600                 605

Gly Thr Phe Arg Gly Lys Lys Asp Ala Ala Ser Ala Leu Phe Asn Leu
    610                 615                 620

Ser Ile Thr His Asp Asn Lys Ala Arg Ile Val Gln Ala Lys Ala Val
625                 630                 635                 640

Lys Tyr Leu Val Glu Leu Leu Asp Pro Asp Leu Glu Met Val Asp Lys
                645                 650                 655

Ala Val Ala Leu Leu Ala Asn Leu Ser Ala Val Gly Glu Gly Arg Gln
            660                 665                 670

Ala Ile Val Arg Glu Gly Gly Ile Pro Leu Leu Val Glu Thr Val Asp
        675                 680                 685

Leu Gly Ser Gln Arg Gly Lys Glu Asn Ala Ala Ser Val Leu Leu Gln
    690                 695                 700

Leu Cys Leu Asn Ser Pro Lys Phe Cys Thr Leu Val Leu Gln Glu Gly
705                 710                 715                 720

Ala Ile Pro Pro Leu Val Ala Leu Ser Gln Ser Gly Thr Gln Arg Ala
                725                 730                 735

Lys Glu Lys Ala Gln Gln Leu Leu Ser His Phe Arg Asn Gln Arg Asp
            740                 745                 750

Ala Arg Met Lys Lys Gly Arg Ser
        755                 760

<210>  25
<211>  2184
<212>  DNA
<213>  Arabidopsis thaliana

<220>
<221>  CDS
<222>  (1)..(2184)

<400>  25
atg gtt ctc ttg ttg aag ctc ttg aaa ccg ttg ctc gat gaa gtt gtt      48
Met Val Leu Leu Leu Lys Leu Leu Lys Pro Leu Leu Asp Glu Val Val
1               5                   10                  15

gat tgc aag ata cct tct gat gac tgt tta tat aaa gga tgt gaa gac      96
Asp Cys Lys Ile Pro Ser Asp Asp Cys Leu Tyr Lys Gly Cys Glu Asp
            20                  25                  30

ctt gat tct gtt gtt aac cag gct cgg gag ttc tta gag gac tgg tca      144
Leu Asp Ser Val Val Asn Gln Ala Arg Glu Phe Leu Glu Asp Trp Ser

35                  40                  45

cca aag ttg agc aag ttg ttt ggt gtg ttt caa tgc gag gtt ttg ttg      192
Pro Lys Leu Ser Lys Leu Phe Gly Val Phe Gln Cys Glu Val Leu Leu
    50                  55                  60

gga aag gtc cag act tgt tcg ttg gag att agt cgc ata ctt ctt cag      240
Gly Lys Val Gln Thr Cys Ser Leu Glu Ile Ser Arg Ile Leu Leu Gln
65                  70                  75                  80

tta tca cag tca agt ccg gtt act tca agc gta caa agt gtt gag cgc      288
Leu Ser Gln Ser Ser Pro Val Thr Ser Ser Val Gln Ser Val Glu Arg
                85                  90                  95

tgc gtg cag gag act gag agt ttt aag caa gag ggg aca tta atg gaa      336
Cys Val Gln Glu Thr Glu Ser Phe Lys Gln Glu Gly Thr Leu Met Glu
            100                 105                 110

ctc atg gag aat gct tta cgg aat cag aaa gat gat att acc tct ttg      384
Leu Met Glu Asn Ala Leu Arg Asn Gln Lys Asp Asp Ile Thr Ser Leu
        115                 120                 125

gat aac aat cat ctg gaa agc ata att caa atg ctt gga ttg ata tca      432
Asp Asn Asn His Leu Glu Ser Ile Ile Gln Met Leu Gly Leu Ile Ser
    130                 135                 140

aac caa gat ctc tta aag gaa agc att act gtg gag aaa gag agg ata      480
Asn Gln Asp Leu Leu Lys Glu Ser Ile Thr Val Glu Lys Glu Arg Ile
145                 150                 155                 160

aga tcc cag gcc agt aag tca gaa gaa gat atg gaa caa acc gaa cag      528
Arg Ser Gln Ala Ser Lys Ser Glu Glu Asp Met Glu Gln Thr Glu Gln
                165                 170                 175

ttg ata gaa ctc gtc ttg tgc atc cgt gaa cac atg ctt aaa act gag      576
Leu Ile Glu Leu Val Leu Cys Ile Arg Glu His Met Leu Lys Thr Glu
            180                 185                 190

ttt ctt gaa gtg gct aaa ggt atc tcg ata ccc ccg tat ttc cgg tgt      624
Phe Leu Glu Val Ala Lys Gly Ile Ser Ile Pro Pro Tyr Phe Arg Cys
        195                 200                 205

cct ttg tca aca gaa ctc atg ctg gat ccg gta ata gta gct tca gga      672
Pro Leu Ser Thr Glu Leu Met Leu Asp Pro Val Ile Val Ala Ser Gly
    210                 215                 220

cag aca ttt gac aga aca tcc att aag aaa tgg ctt gat aac ggg tta      720
Gln Thr Phe Asp Arg Thr Ser Ile Lys Lys Trp Leu Asp Asn Gly Leu
225                 230                 235                 240

gct gtt tgt cca agg acg cgg cag gtg ctg act cat caa gaa ctc att      768
Ala Val Cys Pro Arg Thr Arg Gln Val Leu Thr His Gln Glu Leu Ile
                245                 250                 255

ccc aat tac acg gtt aag gct atg ata gcg agt tgg ttg gag gca aac      816
Pro Asn Tyr Thr Val Lys Ala Met Ile Ala Ser Trp Leu Glu Ala Asn
            260                 265                 270

agg atc aac ctt gct act aac tct tgt cat cag tat gat ggt ggt gat      864
Arg Ile Asn Leu Ala Thr Asn Ser Cys His Gln Tyr Asp Gly Gly Asp
        275                 280                 285

gct tca tcc atg gct aat aat atg ggt tct caa gac ttt aac cgc acc 912
Ala Ser Ser Met Ala Asn Asn Met Gly Ser Gln Asp Phe Asn Arg Thr
    290                 295                 300

gag agt ttt cgt ttt tct tta cgg agc agc agt tta acc tca aga tca 960
Glu Ser Phe Arg Phe Ser Leu Arg Ser Ser Ser Leu Thr Ser Arg Ser
305                 310                 315                 320

tct ctt gaa act gga aat ggg ttt gag aaa ctg aag att aac gtg tct 1008
Ser Leu Glu Thr Gly Asn Gly Phe Glu Lys Leu Lys Ile Asn Val Ser
                325                 330                 335

gcc agt tta tgc ggg gaa tct caa agc aag gat ctt gaa ata ttc gag 1056
Ala Ser Leu Cys Gly Glu Ser Gln Ser Lys Asp Leu Glu Ile Phe Glu
            340                 345                 350

ctt ttg tct ccg ggg cag tct tac act cac agc agg agt gaa tca gtt 1104
Leu Leu Ser Pro Gly Gln Ser Tyr Thr His Ser Arg Ser Glu Ser Val
        355                 360                 365

tgc agt gtt gtc tcg tct gtt gat tat gta cct tcg gtg aca cat gag 1152
Cys Ser Val Val Ser Ser Val Asp Tyr Val Pro Ser Val Thr His Glu
    370                 375                 380

aca gaa agt ata cta ggg aat cac caa agc tcc agt gag atg tct ccc 1200
Thr Glu Ser Ile Leu Gly Asn His Gln Ser Ser Ser Glu Met Ser Pro
385                 390                 395                 400

aag aaa aac tta gaa agt tca aac aat gta aat cat gag cat agc gca 1248
Lys Lys Asn Leu Glu Ser Ser Asn Asn Val Asn His Glu His Ser Ala
                405                 410                 415

gca aag act tat gag tgt tct gta cat gat tta gat gat tca gga aca 1296
Ala Lys Thr Tyr Glu Cys Ser Val His Asp Leu Asp Asp Ser Gly Thr
            420                 425                 430

atg acg act tca cat acc ata aaa ttg gta gaa gat ctt aaa agc ggg 1344
Met Thr Thr Ser His Thr Ile Lys Leu Val Glu Asp Leu Lys Ser Gly
        435                 440                 445

tct aac aaa gtg aag act gct gct gca gct gaa ata cgt cat ctc acc 1392
Ser Asn Lys Val Lys Thr Ala Ala Ala Ala Glu Ile Arg His Leu Thr
    450                 455                 460

att aac agc att gaa aat cgt gtt cac atc ggg cgt tgt ggt gct att 1440
Ile Asn Ser Ile Glu Asn Arg Val His Ile Gly Arg Cys Gly Ala Ile
465                 470                 475                 480

act cca ctg ctg tca ctt tta tac tca gaa gaa aag cta act caa gaa 1488
Thr Pro Leu Leu Ser Leu Leu Tyr Ser Glu Glu Lys Leu Thr Gln Glu
                485                 490                 495

cac gca gtc acg gct ctt ttg aat ctt tcc atc agt gaa cta aac aaa 1536
His Ala Val Thr Ala Leu Leu Asn Leu Ser Ile Ser Glu Leu Asn Lys
            500                 505                 510

gcc atg att gtg gaa gtc ggg gcg ata gaa ccg ctt gtt cat gtt ttg 1584
Ala Met Ile Val Glu Val Gly Ala Ile Glu Pro Leu Val His Val Leu
        515                 520                 525

aac aca gga aat gac aga gcc aaa gag aat tca gca gca tca ttg ttc 1632
Asn Thr Gly Asn Asp Arg Ala Lys Glu Asn Ser Ala Ala Ser Leu Phe
    530                 535                 540

agt ctg tct gtt ctg cag gtc aac aga gaa cga ata ggc cag tct aac 1680
Ser Leu Ser Val Leu Gln Val Asn Arg Glu Arg Ile Gly Gln Ser Asn
545                 550                 555                 560

gca gcg ata caa gct ctg gtg aat ctt ctt ggt aaa gga aca ttt aga 1728
Ala Ala Ile Gln Ala Leu Val Asn Leu Leu Gly Lys Gly Thr Phe Arg
                565                 570                 575

gga aag aaa gac gcc gcc tct gct ttg ttc aat cta tcg att act cat 1776
Gly Lys Lys Asp Ala Ala Ser Ala Leu Phe Asn Leu Ser Ile Thr His
            580                 585                 590

gat aac aag gcc cgt atc gtg caa gct aag gcg gtt aag tac ctt gtg 1824
Asp Asn Lys Ala Arg Ile Val Gln Ala Lys Ala Val Lys Tyr Leu Val
        595                 600                 605

gag ctg tta gac cca gat tta gag atg gtt gat aaa gca gtt gct ctt 1872
Glu Leu Leu Asp Pro Asp Leu Glu Met Val Asp Lys Ala Val Ala Leu
    610                 615                 620

ctt gca aat ctt tct gca gtt gga gaa ggg cgt caa gcc atc gtg agg 1920
Leu Ala Asn Leu Ser Ala Val Gly Glu Gly Arg Gln Ala Ile Val Arg
625                 630                 635                 640

gaa ggt ggg att cca tta ctt gtt gaa act gtt gac tta gga tct cag 1968
Glu Gly Gly Ile Pro Leu Leu Val Glu Thr Val Asp Leu Gly Ser Gln
                645                 650                 655

aga ggg aaa gag aat gca gct tct gtg ctg ctt cag ttg tgt ctg aac 2016
Arg Gly Lys Glu Asn Ala Ala Ser Val Leu Leu Gln Leu Cys Leu Asn
            660                 665                 670

agt ccc aag ttt tgc act ctg gtc ttg caa gaa ggc gcc ata cct ccg 2064
Ser Pro Lys Phe Cys Thr Leu Val Leu Gln Glu Gly Ala Ile Pro Pro
        675                 680                 685

ctt gtt gcc ttg tct cag tct ggt aca cag aga gca aaa gag aag gta 2112
Leu Val Ala Leu Ser Gln Ser Gly Thr Gln Arg Ala Lys Glu Lys Val
    690                 695                 700

tat act ata ttc ttc ttc tgc ggt tac acg aaa aca cac caa gtt cag 2160
Tyr Thr Ile Phe Phe Phe Cys Gly Tyr Thr Lys Thr His Gln Val Gln
705                 710                 715                 720

ttt ctt att gat cga gat atc tga 2184
Phe Leu Ile Asp Arg Asp Ile
                725

<210> 26
<211> 727
<212> PRT
<213> Arabidopsis thaliana

<400> 26

Met Val Leu Leu Leu Lys Leu Leu Lys Pro Leu Leu Asp Glu Val Val

1               5                   10                  15

Asp Cys Lys Ile Pro Ser Asp Asp Cys Leu Tyr Lys Gly Cys Glu Asp
            20                  25                  30

Leu Asp Ser Val Val Asn Gln Ala Arg Glu Phe Leu Glu Asp Trp Ser
        35                  40                  45

Pro Lys Leu Ser Lys Leu Phe Gly Val Phe Gln Cys Glu Val Leu Leu
    50                  55                  60

Gly Lys Val Gln Thr Cys Ser Leu Glu Ile Ser Arg Ile Leu Leu Gln
65                  70                  75                  80

Leu Ser Gln Ser Ser Pro Val Thr Ser Ser Val Gln Ser Val Glu Arg
                85                  90                  95

Cys Val Gln Glu Thr Glu Ser Phe Lys Gln Glu Gly Thr Leu Met Glu
            100                 105                 110

Leu Met Glu Asn Ala Leu Arg Asn Gln Lys Asp Asp Ile Thr Ser Leu
        115                 120                 125

Asp Asn Asn His Leu Glu Ser Ile Ile Gln Met Leu Gly Leu Ile Ser
    130                 135                 140

Asn Gln Asp Leu Leu Lys Glu Ser Ile Thr Val Glu Lys Glu Arg Ile
145                 150                 155                 160

Arg Ser Gln Ala Ser Lys Ser Glu Glu Asp Met Glu Gln Thr Glu Gln
                165                 170                 175

Leu Ile Glu Leu Val Leu Cys Ile Arg Glu His Met Leu Lys Thr Glu
            180                 185                 190

Phe Leu Glu Val Ala Lys Gly Ile Ser Ile Pro Pro Tyr Phe Arg Cys
        195                 200                 205

Pro Leu Ser Thr Glu Leu Met Leu Asp Pro Val Ile Val Ala Ser Gly
    210                 215                 220

Gln Thr Phe Asp Arg Thr Ser Ile Lys Lys Trp Leu Asp Asn Gly Leu
225                 230                 235                 240

Ala Val Cys Pro Arg Thr Arg Gln Val Leu Thr His Gln Glu Leu Ile
                245                 250                 255

Pro Asn Tyr Thr Val Lys Ala Met Ile Ala Ser Trp Leu Glu Ala Asn
            260                 265                 270

Arg Ile Asn Leu Ala Thr Asn Ser Cys His Gln Tyr Asp Gly Gly Asp
        275                 280                 285

Ala Ser Ser Met Ala Asn Asn Met Gly Ser Gln Asp Phe Asn Arg Thr
    290                 295                 300

Glu Ser Phe Arg Phe Ser Leu Arg Ser Ser Ser Leu Thr Ser Arg Ser
305                 310                 315                 320

Ser Leu Glu Thr Gly Asn Gly Phe Glu Lys Leu Lys Ile Asn Val Ser
                325                 330                 335

Ala Ser Leu Cys Gly Glu Ser Gln Ser Lys Asp Leu Glu Ile Phe Glu
            340                 345                 350

Leu Leu Ser Pro Gly Gln Ser Tyr Thr His Ser Arg Ser Glu Ser Val
        355                 360                 365

Cys Ser Val Val Ser Ser Val Asp Tyr Val Pro Ser Val Thr His Glu
    370                 375                 380

Thr Glu Ser Ile Leu Gly Asn His Gln Ser Ser Ser Glu Met Ser Pro
385                 390                 395                 400

Lys Lys Asn Leu Glu Ser Ser Asn Asn Val Asn His Glu His Ser Ala
                405                 410                 415

Ala Lys Thr Tyr Glu Cys Ser Val His Asp Leu Asp Asp Ser Gly Thr
            420                 425                 430

Met Thr Thr Ser His Thr Ile Lys Leu Val Glu Asp Leu Lys Ser Gly
        435                 440                 445

Ser Asn Lys Val Lys Thr Ala Ala Ala Ala Glu Ile Arg His Leu Thr
    450                 455                 460

Ile Asn Ser Ile Glu Asn Arg Val His Ile Gly Arg Cys Gly Ala Ile
465                 470                 475                 480

Thr Pro Leu Leu Ser Leu Leu Tyr Ser Glu Glu Lys Leu Thr Gln Glu
                485                 490                 495

His Ala Val Thr Ala Leu Leu Asn Leu Ser Ile Ser Glu Leu Asn Lys
            500                 505                 510

Ala Met Ile Val Glu Val Gly Ala Ile Glu Pro Leu Val His Val Leu
        515                 520                 525

Asn Thr Gly Asn Asp Arg Ala Lys Glu Asn Ser Ala Ala Ser Leu Phe
    530                 535                 540

Ser Leu Ser Val Leu Gln Val Asn Arg Glu Arg Ile Gly Gln Ser Asn
545                 550                 555                 560

Ala Ala Ile Gln Ala Leu Val Asn Leu Leu Gly Lys Gly Thr Phe Arg
                565                 570                 575

Gly Lys Lys Asp Ala Ala Ser Ala Leu Phe Asn Leu Ser Ile Thr His
            580                 585                 590

Asp Asn Lys Ala Arg Ile Val Gln Ala Lys Ala Val Lys Tyr Leu Val
        595                 600                 605

Glu Leu Leu Asp Pro Asp Leu Glu Met Val Asp Lys Ala Val Ala Leu
    610                 615                 620

Leu Ala Asn Leu Ser Ala Val Gly Glu Gly Arg Gln Ala Ile Val Arg
625                 630                 635                 640

Glu Gly Gly Ile Pro Leu Leu Val Glu Thr Val Asp Leu Gly Ser Gln
                645                 650                 655

Arg Gly Lys Glu Asn Ala Ala Ser Val Leu Leu Gln Leu Cys Leu Asn
            660                 665                 670

Ser Pro Lys Phe Cys Thr Leu Val Leu Gln Glu Gly Ala Ile Pro Pro
        675                 680                 685

Leu Val Ala Leu Ser Gln Ser Gly Thr Gln Arg Ala Lys Glu Lys Val
    690                 695                 700

Tyr Thr Ile Phe Phe Phe Cys Gly Tyr Thr Lys Thr His Gln Val Gln
705                 710                 715                 720

Phe Leu Ile Asp Arg Asp Ile
                725

<210> 27
<211> 1983
<212> DNA
<213> Arabidopsis thaliana

<220>
<221> CDS
<222> (1)..(1983)

<400> 27
atg gag gaa gag aaa gct tct gct gca cag agc tta atc gat gta gtt 48
Met Glu Glu Glu Lys Ala Ser Ala Ala Gln Ser Leu Ile Asp Val Val
1               5                   10                  15

aac gag att gct gcg att tct gat tat cgt ata aca gtg aag aag ctt 96
Asn Glu Ile Ala Ala Ile Ser Asp Tyr Arg Ile Thr Val Lys Lys Leu
            20                  25                  30

tgt tat aat cta gcg agg aga tta aag ctg ctt gtt cct atg ttt gag 144
Cys Tyr Asn Leu Ala Arg Arg Leu Lys Leu Leu Val Pro Met Phe Glu
        35                  40                  45

gaa att aga gaa agt aac gaa ccg atc agc gaa gat acg ttg aag act 192
Glu Ile Arg Glu Ser Asn Glu Pro Ile Ser Glu Asp Thr Leu Lys Thr
    50                  55                  60

ttg atg aat ttg aag gaa gct atg tgt tca gcg aag gat tat ctc aaa 240
Leu Met Asn Leu Lys Glu Ala Met Cys Ser Ala Lys Asp Tyr Leu Lys
65                  70                  75                  80

ttt tgt agc caa ggg agc aag att tat ctg gtg atg gag agg gaa caa 288
Phe Cys Ser Gln Gly Ser Lys Ile Tyr Leu Val Met Glu Arg Glu Gln
                85                  90                  95

gtg aca agt aaa ttg atg gag gtg tct gtt aag tta gaa caa tct tta 336
Val Thr Ser Lys Leu Met Glu Val Ser Val Lys Leu Glu Gln Ser Leu
            100                 105                 110

agc cag att cca tat gaa gaa ctc gat ata tcg gat gaa gtt aga gaa 384
Ser Gln Ile Pro Tyr Glu Glu Leu Asp Ile Ser Asp Glu Val Arg Glu
        115                 120                 125

cag gtt gag ctg gtt ctt agt cag ttt cgg cga gct aaa gga aga gta 432
Gln Val Glu Leu Val Leu Ser Gln Phe Arg Arg Ala Lys Gly Arg Val
    130                 135                 140

gat gta tca gat gat gag cta tat gaa gat ctt cag tcg ctt tgc aac 480
Asp Val Ser Asp Asp Glu Leu Tyr Glu Asp Leu Gln Ser Leu Cys Asn
145                 150                 155                 160

aaa agt agt gat gta gat gct tat cag cct gtg cta gag cgg gtt gcg 528
Lys Ser Ser Asp Val Asp Ala Tyr Gln Pro Val Leu Glu Arg Val Ala
                165                 170                 175

aag aag tta cat ttg atg gag att cct gac cta gct caa gaa tca gtg 576
Lys Lys Leu His Leu Met Glu Ile Pro Asp Leu Ala Gln Glu Ser Val
            180                 185                 190

gct ctg cat gaa atg gtt gct tca agc ggt gga gat gtt ggt gaa aat 624
Ala Leu His Glu Met Val Ala Ser Ser Gly Gly Asp Val Gly Glu Asn

        195                 200                 205

att gag gag atg gca atg gta tta aag atg att aag gat ttt gtg cag 672
Ile Glu Glu Met Ala Met Val Leu Lys Met Ile Lys Asp Phe Val Gln
    210                 215                 220

acg gag gat gat aat ggc gag gag cag aaa gta gga gtt aac tct aga 720
Thr Glu Asp Asp Asn Gly Glu Glu Gln Lys Val Gly Val Asn Ser Arg
225                 230                 235                 240

agc aat gga cag act tct acg gca gcg agt cag aag ata cct gtg att 768
Ser Asn Gly Gln Thr Ser Thr Ala Ala Ser Gln Lys Ile Pro Val Ile
                245                 250                 255

cct gat gat ttt cgc tgt ccg att tcg ctg gaa atg atg aga gat cca 816
Pro Asp Asp Phe Arg Cys Pro Ile Ser Leu Glu Met Met Arg Asp Pro
            260                 265                 270

gtt att gtt tca tca ggg cag aca tac gaa cgc aca tgt att gag aaa 864
Val Ile Val Ser Ser Gly Gln Thr Tyr Glu Arg Thr Cys Ile Glu Lys
        275                 280                 285

tgg ata gaa ggt gga cac tcg aca tgt cca aaa aca cag cag gcg cta 912
Trp Ile Glu Gly Gly His Ser Thr Cys Pro Lys Thr Gln Gln Ala Leu
    290                 295                 300

aca agc aca acc ctc aca cca aac tat gtt ctc cgt agt ctc ata gct 960
Thr Ser Thr Thr Leu Thr Pro Asn Tyr Val Leu Arg Ser Leu Ile Ala
305                 310                 315                 320

cag tgg tgc gag gcc aac gat att gag cct cca aag cct ccg agc agt 1008
Gln Trp Cys Glu Ala Asn Asp Ile Glu Pro Pro Lys Pro Pro Ser Ser
                325                 330                 335

tta aga ccc aga aaa gta tcg tcc ttc tca tct ccc gca gaa gcg aac 1056
Leu Arg Pro Arg Lys Val Ser Ser Phe Ser Ser Pro Ala Glu Ala Asn
            340                 345                 350

aag att gaa gat ctt atg tgg aga ctt gcg tac gga aac ccc gag gac 1104
Lys Ile Glu Asp Leu Met Trp Arg Leu Ala Tyr Gly Asn Pro Glu Asp
        355                 360                 365

caa cga tct gca gct ggg gaa atc cgc ctt ctt gca aaa cga aat gca 1152
Gln Arg Ser Ala Ala Gly Glu Ile Arg Leu Leu Ala Lys Arg Asn Ala
    370                 375                 380

gac aac cgc gtg gcc ata gcc gaa gct gga gcc ata cct ctt ctc gta 1200
Asp Asn Arg Val Ala Ile Ala Glu Ala Gly Ala Ile Pro Leu Leu Val
385                 390                 395                 400

ggt ctc ctc tca act cct gat tct cgt att caa gaa cat tcg gta aca 1248
Gly Leu Leu Ser Thr Pro Asp Ser Arg Ile Gln Glu His Ser Val Thr
                405                 410                 415

gct ctt cta aac ctc tcc ata tgt gag aac aac aaa gga gcc att gtt 1296
Ala Leu Leu Asn Leu Ser Ile Cys Glu Asn Asn Lys Gly Ala Ile Val
            420                 425                 430

tca gct gga gct att cct ggt ata gtt caa gtg ctt aag aaa gga agc 1344
Ser Ala Gly Ala Ile Pro Gly Ile Val Gln Val Leu Lys Lys Gly Ser
        435                 440                 445

atg gag gcc aga gag aat gcg gcg gct aca ctt ttc agt cta tca gtg 1392
Met Glu Ala Arg Glu Asn Ala Ala Ala Thr Leu Phe Ser Leu Ser Val
    450                 455                 460

atc gat gaa aat aaa gtg act atc ggt gcc tta gga gca att ccg cca 1440
Ile Asp Glu Asn Lys Val Thr Ile Gly Ala Leu Gly Ala Ile Pro Pro
465                 470                 475                 480

ctc gtt gta tta ctt aat gaa ggt aca caa aga ggc aag aaa gat gct 1488
Leu Val Val Leu Leu Asn Glu Gly Thr Gln Arg Gly Lys Lys Asp Ala
                485                 490                 495

gct act gca ctc ttt aac ctc tgt ata tac caa gga aac aaa gga aaa 1536
Ala Thr Ala Leu Phe Asn Leu Cys Ile Tyr Gln Gly Asn Lys Gly Lys
            500                 505                 510

gct ata cgt gca gga gtg att ccc acg ttg act aga ctc ttg aca gag 1584
Ala Ile Arg Ala Gly Val Ile Pro Thr Leu Thr Arg Leu Leu Thr Glu
        515                 520                 525

ccc gga agc gga atg gtc gat gag gca ctc gcg att ttg gcg att ctc 1632
Pro Gly Ser Gly Met Val Asp Glu Ala Leu Ala Ile Leu Ala Ile Leu
    530                 535                 540

tct agc cac ccc gaa gga aaa gca atc ata gga tcc tct gat gca gtc 1680
Ser Ser His Pro Glu Gly Lys Ala Ile Ile Gly Ser Ser Asp Ala Val
545                 550                 555                 560

cca agt ttg gtt gag ttt atc aga act ggc tcg cct aga aac aga gaa 1728
Pro Ser Leu Val Glu Phe Ile Arg Thr Gly Ser Pro Arg Asn Arg Glu
                565                 570                 575

aac gca gct gct gtt cta gtc cac ctc tgt tct gga gac cca caa cat 1776
Asn Ala Ala Ala Val Leu Val His Leu Cys Ser Gly Asp Pro Gln His
            580                 585                 590

ctt gtc gaa gcg cag aaa ctc ggc ctt atg ggt cca ttg ata gat tta 1824
Leu Val Glu Ala Gln Lys Leu Gly Leu Met Gly Pro Leu Ile Asp Leu
        595                 600                 605

gct gga aat ggg acg gat aga ggg aaa cga aaa gca gcg cag ttg ctt 1872
Ala Gly Asn Gly Thr Asp Arg Gly Lys Arg Lys Ala Ala Gln Leu Leu
    610                 615                 620

gaa cgc atc agc cgt ctc gct gaa cag cag aag gaa acg gct gtg tca 1920
Glu Arg Ile Ser Arg Leu Ala Glu Gln Gln Lys Glu Thr Ala Val Ser
625                 630                 635                 640

caa ccg gaa gaa gaa gct gaa cca aca cat cca gaa tcc acc aca gaa 1968
Gln Pro Glu Glu Glu Ala Glu Pro Thr His Pro Glu Ser Thr Thr Glu
                645                 650                 655

gct gca gat act taa 1983
Ala Ala Asp Thr
            660

<210> 28
<211> 660
<212> PRT

<213>  Arabidopsis thaliana

<400>  28

Met Glu Glu Glu Lys Ala Ser Ala Ala Gln Ser Leu Ile Asp Val Val
1               5                   10                  15

Asn Glu Ile Ala Ala Ile Ser Asp Tyr Arg Ile Thr Val Lys Lys Leu
            20                  25                  30

Cys Tyr Asn Leu Ala Arg Arg Leu Lys Leu Leu Val Pro Met Phe Glu
        35                  40                  45

Glu Ile Arg Glu Ser Asn Glu Pro Ile Ser Glu Asp Thr Leu Lys Thr
    50                  55                  60

Leu Met Asn Leu Lys Glu Ala Met Cys Ser Ala Lys Asp Tyr Leu Lys
65                  70                  75                  80

Phe Cys Ser Gln Gly Ser Lys Ile Tyr Leu Val Met Glu Arg Glu Gln
                85                  90                  95

Val Thr Ser Lys Leu Met Glu Val Ser Val Lys Leu Glu Gln Ser Leu
            100                 105                 110

Ser Gln Ile Pro Tyr Glu Glu Leu Asp Ile Ser Asp Glu Val Arg Glu
        115                 120                 125

Gln Val Glu Leu Val Leu Ser Gln Phe Arg Arg Ala Lys Gly Arg Val
    130                 135                 140

Asp Val Ser Asp Asp Glu Leu Tyr Glu Asp Leu Gln Ser Leu Cys Asn
145                 150                 155                 160

Lys Ser Ser Asp Val Asp Ala Tyr Gln Pro Val Leu Glu Arg Val Ala
                165                 170                 175

Lys Lys Leu His Leu Met Glu Ile Pro Asp Leu Ala Gln Glu Ser Val
            180                 185                 190

Ala Leu His Glu Met Val Ala Ser Ser Gly Gly Asp Val Gly Glu Asn
        195                 200                 205

Ile Glu Glu Met Ala Met Val Leu Lys Met Ile Lys Asp Phe Val Gln
    210                 215                 220

Thr Glu Asp Asp Asn Gly Glu Glu Gln Lys Val Gly Val Asn Ser Arg

225                 230                 235                 240

Ser Asn Gly Gln Thr Ser Thr Ala Ala Ser Gln Lys Ile Pro Val Ile
                245                 250                 255

Pro Asp Asp Phe Arg Cys Pro Ile Ser Leu Glu Met Met Arg Asp Pro
            260                 265                 270

Val Ile Val Ser Ser Gly Gln Thr Tyr Glu Arg Thr Cys Ile Glu Lys
        275                 280                 285

Trp Ile Glu Gly Gly His Ser Thr Cys Pro Lys Thr Gln Gln Ala Leu
    290                 295                 300

Thr Ser Thr Thr Leu Thr Pro Asn Tyr Val Leu Arg Ser Leu Ile Ala
305                 310                 315                 320

Gln Trp Cys Glu Ala Asn Asp Ile Glu Pro Pro Lys Pro Pro Ser Ser
                325                 330                 335

Leu Arg Pro Arg Lys Val Ser Ser Phe Ser Ser Pro Ala Glu Ala Asn
            340                 345                 350

Lys Ile Glu Asp Leu Met Trp Arg Leu Ala Tyr Gly Asn Pro Glu Asp
        355                 360                 365

Gln Arg Ser Ala Ala Gly Glu Ile Arg Leu Leu Ala Lys Arg Asn Ala
    370                 375                 380

Asp Asn Arg Val Ala Ile Ala Glu Ala Gly Ala Ile Pro Leu Leu Val
385                 390                 395                 400

Gly Leu Leu Ser Thr Pro Asp Ser Arg Ile Gln Glu His Ser Val Thr
                405                 410                 415

Ala Leu Leu Asn Leu Ser Ile Cys Glu Asn Asn Lys Gly Ala Ile Val
            420                 425                 430

Ser Ala Gly Ala Ile Pro Gly Ile Val Gln Val Leu Lys Lys Gly Ser
        435                 440                 445

Met Glu Ala Arg Glu Asn Ala Ala Ala Thr Leu Phe Ser Leu Ser Val
    450                 455                 460

Ile Asp Glu Asn Lys Val Thr Ile Gly Ala Leu Gly Ala Ile Pro Pro
465                 470                 475                 480

Leu Val Val Leu Leu Asn Glu Gly Thr Gln Arg Gly Lys Lys Asp Ala
                485                 490                 495

Ala Thr Ala Leu Phe Asn Leu Cys Ile Tyr Gln Gly Asn Lys Gly Lys
            500                 505                 510

Ala Ile Arg Ala Gly Val Ile Pro Thr Leu Thr Arg Leu Leu Thr Glu
        515                 520                 525

Pro Gly Ser Gly Met Val Asp Glu Ala Leu Ala Ile Leu Ala Ile Leu
    530                 535                 540

Ser Ser His Pro Glu Gly Lys Ala Ile Ile Gly Ser Ser Asp Ala Val
545                 550                 555                 560

Pro Ser Leu Val Glu Phe Ile Arg Thr Gly Ser Pro Arg Asn Arg Glu
                565                 570                 575

Asn Ala Ala Ala Val Leu Val His Leu Cys Ser Gly Asp Pro Gln His
            580                 585                 590

Leu Val Glu Ala Gln Lys Leu Gly Leu Met Gly Pro Leu Ile Asp Leu
        595                 600                 605

Ala Gly Asn Gly Thr Asp Arg Gly Lys Arg Lys Ala Ala Gln Leu Leu
    610                 615                 620

Glu Arg Ile Ser Arg Leu Ala Glu Gln Gln Lys Glu Thr Ala Val Ser
625                 630                 635                 640

Gln Pro Glu Glu Glu Ala Glu Pro Thr His Pro Glu Ser Thr Thr Glu
                645                 650                 655

Ala Ala Asp Thr
            660

<210>  29
<211>  1068
<212>  DNA
<213>  Arabidopsis thaliana

<220>
<221>  CDS
<222>  (1)..(1068)

<400>  29

atg gag atg gag aat cac cgc ccc ggc agt ttc acc tac atg ggc cgc     48
Met Glu Met Glu Asn His Arg Pro Gly Ser Phe Thr Tyr Met Gly Arg
1               5                   10                  15

aaa ttc agc gat tta agt ctc aac gat gac tcc tct gct ttc agc gat     96
Lys Phe Ser Asp Leu Ser Leu Asn Asp Asp Ser Ser Ala Phe Ser Asp
            20                  25                  30

tgt aac agc gac aga tcc ggc gaa ttc ccc act gct tcc tcc gag agc     144
Cys Asn Ser Asp Arg Ser Gly Glu Phe Pro Thr Ala Ser Ser Glu Ser
        35                  40                  45

cgt cgt ctc ctc ctc tct tgc gcc tct gag aat tcc gat gat ctc atc     192
Arg Arg Leu Leu Leu Ser Cys Ala Ser Glu Asn Ser Asp Asp Leu Ile
    50                  55                  60

aat cat ctc gtg tcg cat ctt gat tcc tcc tat tcg atc gat gag cag     240
Asn His Leu Val Ser His Leu Asp Ser Ser Tyr Ser Ile Asp Glu Gln
65                  70                  75                  80

aag caa gct gct atg gag atc agg ctc tta tcc aag aac aaa cct gag     288
Lys Gln Ala Ala Met Glu Ile Arg Leu Leu Ser Lys Asn Lys Pro Glu
                85                  90                  95

aat cgg atc aaa atc gcc aag gcc ggt gcg att aag ccg ttg att tct     336
Asn Arg Ile Lys Ile Ala Lys Ala Gly Ala Ile Lys Pro Leu Ile Ser
            100                 105                 110

ctg atc tct tct tcg gat ctt cag ctt cag gag tat ggt gtc act gca     384
Leu Ile Ser Ser Ser Asp Leu Gln Leu Gln Glu Tyr Gly Val Thr Ala
        115                 120                 125

atc ttg aat cta tct ctc tgc gac gag aac aaa gag tcg att gct tct     432
Ile Leu Asn Leu Ser Leu Cys Asp Glu Asn Lys Glu Ser Ile Ala Ser
    130                 135                 140

tcc ggt gcg att aag ccg ctt gtc agg gct ttg aaa atg gga aca ccg     480
Ser Gly Ala Ile Lys Pro Leu Val Arg Ala Leu Lys Met Gly Thr Pro
145                 150                 155                 160

act gct aaa gag aac gct gct tgt gct ctg ctc cgt cta tcg cag atc     528
Thr Ala Lys Glu Asn Ala Ala Cys Ala Leu Leu Arg Leu Ser Gln Ile
                165                 170                 175

gag gag aac aaa gtc gcc atc ggg aga tcc gga gcg att cct ctg ttg     576
Glu Glu Asn Lys Val Ala Ile Gly Arg Ser Gly Ala Ile Pro Leu Leu
            180                 185                 190

gtg aac ctt cta gaa aca ggc gga ttc aga gcg aag aag gac gcg tcg     624
Val Asn Leu Leu Glu Thr Gly Gly Phe Arg Ala Lys Lys Asp Ala Ser
        195                 200                 205

acg gct ctg tac tcg ttg tgc tca gct aaa gag aac aaa atc aga gcc     672
Thr Ala Leu Tyr Ser Leu Cys Ser Ala Lys Glu Asn Lys Ile Arg Ala
    210                 215                 220

gtg caa tcg gga att atg aag ccg ctt gtt gaa ttg atg gcg gat ttc     720
Val Gln Ser Gly Ile Met Lys Pro Leu Val Glu Leu Met Ala Asp Phe
225                 230                 235                 240

gga tca aac atg gtg gat aaa tcg gcg ttt gtg atg agt ctg tta atg     768

Gly Ser Asn Met Val Asp Lys Ser Ala Phe Val Met Ser Leu Leu Met
                245                 250                 255

tcg gtg ccg gaa tcg aaa ccg gcg att gtg gag gaa gga gga gtt ccg      816
Ser Val Pro Glu Ser Lys Pro Ala Ile Val Glu Glu Gly Gly Val Pro
            260                 265                 270

gtg ctg gtg gag ata gta gag gtg gga aca cag aga cag aaa gag atg      864
Val Leu Val Glu Ile Val Glu Val Gly Thr Gln Arg Gln Lys Glu Met
        275                 280                 285

gct gtg tcg ata ttg cta cag ctt tgt gag gag agt gtt gtg tat aga      912
Ala Val Ser Ile Leu Leu Gln Leu Cys Glu Glu Ser Val Val Tyr Arg
    290                 295                 300

aca atg gtg gct aga gaa gga gcg ata cct ccg cta gtg gct ctg tcg      960
Thr Met Val Ala Arg Glu Gly Ala Ile Pro Pro Leu Val Ala Leu Ser
305                 310                 315                 320

cag gca gga aca agt cga gct aag caa aag gct gag gcg ttg att gag     1008
Gln Ala Gly Thr Ser Arg Ala Lys Gln Lys Ala Glu Ala Leu Ile Glu
                325                 330                 335

ctt cta agg caa cca aga tcc att agt aat ggt ggt gct aga tca tcg     1056
Leu Leu Arg Gln Pro Arg Ser Ile Ser Asn Gly Gly Ala Arg Ser Ser
            340                 345                 350

tcc caa ctc tga                                                     1068
Ser Gln Leu
        355


<210>  30
<211>  355
<212>  PRT
<213>  Arabidopsis thaliana

<400>  30

Met Glu Met Glu Asn His Arg Pro Gly Ser Phe Thr Tyr Met Gly Arg
1               5                   10                  15


Lys Phe Ser Asp Leu Ser Leu Asn Asp Asp Ser Ser Ala Phe Ser Asp
            20                  25                  30


Cys Asn Ser Asp Arg Ser Gly Glu Phe Pro Thr Ala Ser Ser Glu Ser
        35                  40                  45


Arg Arg Leu Leu Leu Ser Cys Ala Ser Glu Asn Ser Asp Asp Leu Ile
    50                  55                  60


Asn His Leu Val Ser His Leu Asp Ser Ser Tyr Ser Ile Asp Glu Gln
65                  70                  75                  80


Lys Gln Ala Ala Met Glu Ile Arg Leu Leu Ser Lys Asn Lys Pro Glu
                85                  90                  95

Asn Arg Ile Lys Ile Ala Lys Ala Gly Ala Ile Lys Pro Leu Ile Ser
            100                 105                 110

Leu Ile Ser Ser Ser Asp Leu Gln Leu Gln Glu Tyr Gly Val Thr Ala
        115                 120                 125

Ile Leu Asn Leu Ser Leu Cys Asp Glu Asn Lys Glu Ser Ile Ala Ser
    130                 135                 140

Ser Gly Ala Ile Lys Pro Leu Val Arg Ala Leu Lys Met Gly Thr Pro
145                 150                 155                 160

Thr Ala Lys Glu Asn Ala Ala Cys Ala Leu Leu Arg Leu Ser Gln Ile
                165                 170                 175

Glu Glu Asn Lys Val Ala Ile Gly Arg Ser Gly Ala Ile Pro Leu Leu
            180                 185                 190

Val Asn Leu Leu Glu Thr Gly Gly Phe Arg Ala Lys Lys Asp Ala Ser
        195                 200                 205

Thr Ala Leu Tyr Ser Leu Cys Ser Ala Lys Glu Asn Lys Ile Arg Ala
    210                 215                 220

Val Gln Ser Gly Ile Met Lys Pro Leu Val Glu Leu Met Ala Asp Phe
225                 230                 235                 240

Gly Ser Asn Met Val Asp Lys Ser Ala Phe Val Met Ser Leu Leu Met
                245                 250                 255

Ser Val Pro Glu Ser Lys Pro Ala Ile Val Glu Glu Gly Gly Val Pro
            260                 265                 270

Val Leu Val Glu Ile Val Glu Val Gly Thr Gln Arg Gln Lys Glu Met
        275                 280                 285

Ala Val Ser Ile Leu Leu Gln Leu Cys Glu Glu Ser Val Val Tyr Arg
    290                 295                 300

Thr Met Val Ala Arg Glu Gly Ala Ile Pro Pro Leu Val Ala Leu Ser
305                 310                 315                 320

Gln Ala Gly Thr Ser Arg Ala Lys Gln Lys Ala Glu Ala Leu Ile Glu
                325                 330                 335

Leu Leu Arg Gln Pro Arg Ser Ile Ser Asn Gly Gly Ala Arg Ser Ser
            340                 345                 350

Ser Gln Leu
        355

<210>  31
<211>  1068
<212>  DNA
<213>  Arabidopsis thaliana

<220>
<221>  CDS
<222>  (1)..(1068)

<400>  31
atg gag atg gag aat cac cgc ccc ggc agt ttc acc tac atg ggc cgc      48
Met Glu Met Glu Asn His Arg Pro Gly Ser Phe Thr Tyr Met Gly Arg
1               5                   10                  15

aaa ttc agc gat tta agt ctc aac gat gac tcc tct gct ttc agc gat      96
Lys Phe Ser Asp Leu Ser Leu Asn Asp Asp Ser Ser Ala Phe Ser Asp
            20                  25                  30

tgt aac agc gac aga tcc ggc gaa ttc ccc act gct tcc tcc gag agc      144
Cys Asn Ser Asp Arg Ser Gly Glu Phe Pro Thr Ala Ser Ser Glu Ser
        35                  40                  45

cgt cgt ctc ctc ctc tct tgc gcc tct gag aat tcc gat gat ctc atc      192
Arg Arg Leu Leu Leu Ser Cys Ala Ser Glu Asn Ser Asp Asp Leu Ile
    50                  55                  60

aat cat ctc gtg tcg cat ctt gat tcc tcc tat tcg atc gat gag cag      240
Asn His Leu Val Ser His Leu Asp Ser Ser Tyr Ser Ile Asp Glu Gln
65                  70                  75                  80

aag caa gct gct atg gag atc agg ctc tta tcc aag aac aaa cct gag      288
Lys Gln Ala Ala Met Glu Ile Arg Leu Leu Ser Lys Asn Lys Pro Glu
                85                  90                  95

aat cgg atc aaa atc gcc aag gcc ggt gcg att aag ccg ttg att tct      336
Asn Arg Ile Lys Ile Ala Lys Ala Gly Ala Ile Lys Pro Leu Ile Ser
            100                 105                 110

ctg atc tct tct tcg gat ctt cag ctt cag gag tat ggt gtc act gca      384
Leu Ile Ser Ser Ser Asp Leu Gln Leu Gln Glu Tyr Gly Val Thr Ala
        115                 120                 125

atc ttg aat cta tct ctc tgc gac gag aac aaa gag tcg att gct tct      432
Ile Leu Asn Leu Ser Leu Cys Asp Glu Asn Lys Glu Ser Ile Ala Ser
    130                 135                 140

tcc ggt gcg att aag ccg ctt gtc agg gct ttg aaa atg gga aca ccg      480
Ser Gly Ala Ile Lys Pro Leu Val Arg Ala Leu Lys Met Gly Thr Pro
145                 150                 155                 160

act gct aaa gag aac gct gct tgt gct ctg ctc cgt cta tcg cag atc      528

Thr Ala Lys Glu Asn Ala Ala Cys Ala Leu Leu Arg Leu Ser Gln Ile
                165                 170                 175

gag gag aac aaa gtc gcc atc ggg aga tcc gga gcg att cct ctg ttg      576
Glu Glu Asn Lys Val Ala Ile Gly Arg Ser Gly Ala Ile Pro Leu Leu
            180                 185                 190

gtg aac ctt cta gaa aca ggc gga ttc aga gcg aag aag gac gcg tcg      624
Val Asn Leu Leu Glu Thr Gly Gly Phe Arg Ala Lys Lys Asp Ala Ser
        195                 200                 205

acg gct ctg tac tcg ttg tgc tca gct aaa gag aac aaa atc aga gcc      672
Thr Ala Leu Tyr Ser Leu Cys Ser Ala Lys Glu Asn Lys Ile Arg Ala
    210                 215                 220

gtg caa tcg gga att atg aag ccg ctt gtt gaa ttg atg gcg gat ttc      720
Val Gln Ser Gly Ile Met Lys Pro Leu Val Glu Leu Met Ala Asp Phe
225                 230                 235                 240

gga tca aac atg gtg gat aaa tcg gcg ttt gtg atg agt ctg tta atg      768
Gly Ser Asn Met Val Asp Lys Ser Ala Phe Val Met Ser Leu Leu Met
                245                 250                 255

tcg gtg ccg gaa tcg aaa ccg gcg att gtg gag gaa gga gga gtt ccg      816
Ser Val Pro Glu Ser Lys Pro Ala Ile Val Glu Glu Gly Gly Val Pro
            260                 265                 270

gtg ctg gtg gag ata gta gag gtg gga aca cag aga cag aaa gag atg      864
Val Leu Val Glu Ile Val Glu Val Gly Thr Gln Arg Gln Lys Glu Met
        275                 280                 285

gct gtg tcg ata ttg cta cag ctt tgt gag gag agt gtt gtg tat aga      912
Ala Val Ser Ile Leu Leu Gln Leu Cys Glu Glu Ser Val Val Tyr Arg
    290                 295                 300

aca atg gtg gct aga gaa gga gcg ata cct ccg cta gtg gct ctg tcg      960
Thr Met Val Ala Arg Glu Gly Ala Ile Pro Pro Leu Val Ala Leu Ser
305                 310                 315                 320

cag gca gga aca agt cga gct aag caa aag gct gag gcg ttg att gag     1008
Gln Ala Gly Thr Ser Arg Ala Lys Gln Lys Ala Glu Ala Leu Ile Glu
                325                 330                 335

ctt cta agg caa cta aga tcc att agt aat ggt ggt gct aga tca tcg     1056
Leu Leu Arg Gln Leu Arg Ser Ile Ser Asn Gly Gly Ala Arg Ser Ser
            340                 345                 350

tcc caa ctc tga                                                     1068
Ser Gln Leu
        355


<210>  32
<211>  355
<212>  PRT
<213>  Arabidopsis thaliana

<400>  32

Met Glu Met Glu Asn His Arg Pro Gly Ser Phe Thr Tyr Met Gly Arg
1               5                   10                  15

Lys Phe Ser Asp Leu Ser Leu Asn Asp Asp Ser Ser Ala Phe Ser Asp
            20                  25                  30

Cys Asn Ser Asp Arg Ser Gly Glu Phe Pro Thr Ala Ser Ser Glu Ser
        35                  40                  45

Arg Arg Leu Leu Leu Ser Cys Ala Ser Glu Asn Ser Asp Asp Leu Ile
    50                  55                  60

Asn His Leu Val Ser His Leu Asp Ser Ser Tyr Ser Ile Asp Glu Gln
65                  70                  75                  80

Lys Gln Ala Ala Met Glu Ile Arg Leu Leu Ser Lys Asn Lys Pro Glu
                85                  90                  95

Asn Arg Ile Lys Ile Ala Lys Ala Gly Ala Ile Lys Pro Leu Ile Ser
            100                 105                 110

Leu Ile Ser Ser Ser Asp Leu Gln Leu Gln Glu Tyr Gly Val Thr Ala
        115                 120                 125

Ile Leu Asn Leu Ser Leu Cys Asp Glu Asn Lys Glu Ser Ile Ala Ser
    130                 135                 140

Ser Gly Ala Ile Lys Pro Leu Val Arg Ala Leu Lys Met Gly Thr Pro
145                 150                 155                 160

Thr Ala Lys Glu Asn Ala Ala Cys Ala Leu Leu Arg Leu Ser Gln Ile
                165                 170                 175

Glu Glu Asn Lys Val Ala Ile Gly Arg Ser Gly Ala Ile Pro Leu Leu
            180                 185                 190

Val Asn Leu Leu Glu Thr Gly Gly Phe Arg Ala Lys Lys Asp Ala Ser
        195                 200                 205

Thr Ala Leu Tyr Ser Leu Cys Ser Ala Lys Glu Asn Lys Ile Arg Ala
    210                 215                 220

Val Gln Ser Gly Ile Met Lys Pro Leu Val Glu Leu Met Ala Asp Phe
225                 230                 235                 240

Gly Ser Asn Met Val Asp Lys Ser Ala Phe Val Met Ser Leu Leu Met
                245                 250                 255

Ser Val Pro Glu Ser Lys Pro Ala Ile Val Glu Glu Gly Gly Val Pro
            260                 265                 270

Val Leu Val Glu Ile Val Glu Val Gly Thr Gln Arg Gln Lys Glu Met
        275                 280                 285

Ala Val Ser Ile Leu Leu Gln Leu Cys Glu Glu Ser Val Val Tyr Arg
    290                 295                 300

Thr Met Val Ala Arg Glu Gly Ala Ile Pro Pro Leu Val Ala Leu Ser
305                 310                 315                 320

Gln Ala Gly Thr Ser Arg Ala Lys Gln Lys Ala Glu Ala Leu Ile Glu
                325                 330                 335

Leu Leu Arg Gln Leu Arg Ser Ile Ser Asn Gly Gly Ala Arg Ser Ser
            340                 345                 350

Ser Gln Leu
        355

<210>  33
<211>  1068
<212>  DNA
<213>  Arabidopsis thaliana

<220>
<221>  CDS
<222>  (1)..(1068)

<400>  33
atg gag atg gag aat cac cgc ccc ggc agt ttc acc tac atg ggc cgc      48
Met Glu Met Glu Asn His Arg Pro Gly Ser Phe Thr Tyr Met Gly Arg
1               5                   10                  15

aaa ttc agc gat tta agt ctc aac gat gac tcc tct gct ttc agc gat      96
Lys Phe Ser Asp Leu Ser Leu Asn Asp Asp Ser Ser Ala Phe Ser Asp
            20                  25                  30

tgt aac agc gac aga tcc ggc gaa ttc ccc act gct tcc tcc gag agc      144
Cys Asn Ser Asp Arg Ser Gly Glu Phe Pro Thr Ala Ser Ser Glu Ser
        35                  40                  45

cgt cgt ctc ctc ctc tct tgc gcc tct gag aat tcc gat gat ctc atc      192
Arg Arg Leu Leu Leu Ser Cys Ala Ser Glu Asn Ser Asp Asp Leu Ile
    50                  55                  60

aat cat ctc gtg tcg cat ctt gat tcc tcc tat tcg atc gat gag cag      240
Asn His Leu Val Ser His Leu Asp Ser Ser Tyr Ser Ile Asp Glu Gln
65                  70                  75                  80

aag caa gct gct atg gag atc agg ctc tta tcc aag aac aaa cct gag      288

Lys Gln Ala Ala Met Glu Ile Arg Leu Leu Ser Lys Asn Lys Pro Glu
                85                  90                  95

aat cgg atc aaa atc gcc aag gcc ggt gcg att aag ccg ttg att tct 336
Asn Arg Ile Lys Ile Ala Lys Ala Gly Ala Ile Lys Pro Leu Ile Ser
            100                 105                 110

ctg atc tct tct tcg gat ctt cag ctt cag gag tat ggt gtc act gcw 384
Leu Ile Ser Ser Ser Asp Leu Gln Leu Gln Glu Tyr Gly Val Thr Ala
        115                 120                 125

atc ttg aat cta tct ctc tgc gac gag aac aaa gag tcg att gct tct 432
Ile Leu Asn Leu Ser Leu Cys Asp Glu Asn Lys Glu Ser Ile Ala Ser
    130                 135                 140

tcc ggt gcg att aag ccg ctt gtc agg gct ttg aaa atg gga aca ccg 480
Ser Gly Ala Ile Lys Pro Leu Val Arg Ala Leu Lys Met Gly Thr Pro
145                 150                 155                 160

act gct aaa gat aac gct gct tgt gct ctg ctc cgt cta tcg cag atc 528
Thr Ala Lys Asp Asn Ala Ala Cys Ala Leu Leu Arg Leu Ser Gln Ile
                165                 170                 175

gag gag aac aaa gtc gcc atc ggg aga tcc gga gcg att cct ctg ttg 576
Glu Glu Asn Lys Val Ala Ile Gly Arg Ser Gly Ala Ile Pro Leu Leu
            180                 185                 190

gtg aac ctt cta gaa aca ggc gga ttc aga gcg aag aag gac gcg tcg 624
Val Asn Leu Leu Glu Thr Gly Gly Phe Arg Ala Lys Lys Asp Ala Ser
        195                 200                 205

acg gct ctg tac tcg ttg tgc tca gct aaa gag aac aaa atc aga gcc 672
Thr Ala Leu Tyr Ser Leu Cys Ser Ala Lys Glu Asn Lys Ile Arg Ala
    210                 215                 220

gtg caa tcg gga att atg aag ccg ctt gtt gaa ttg atg gcg gat ttc 720
Val Gln Ser Gly Ile Met Lys Pro Leu Val Glu Leu Met Ala Asp Phe
225                 230                 235                 240

gga tca aac atg gtg gat aaa tcg gcg ttt gtg atg agt ctg tta atg 768
Gly Ser Asn Met Val Asp Lys Ser Ala Phe Val Met Ser Leu Leu Met
                245                 250                 255

tcg gtg ccg gaa tcg aaa ccg gcg att gtg gag gaa gga gga gtt ccg 816
Ser Val Pro Glu Ser Lys Pro Ala Ile Val Glu Glu Gly Gly Val Pro
            260                 265                 270

gtg ctg gtg gag ata gta gag gtg gga aca cag aga cag aaa gag atg 864
Val Leu Val Glu Ile Val Glu Val Gly Thr Gln Arg Gln Lys Glu Met
        275                 280                 285

gct gtg tcg ata ttg cta cag ctt tgt gag gag agt gtt gtg tat aga 912
Ala Val Ser Ile Leu Leu Gln Leu Cys Glu Glu Ser Val Val Tyr Arg
    290                 295                 300

aca atg gtg gct aga gaa gga gcg ata cct ccg cta gtg gct ctg tcg 960
Thr Met Val Ala Arg Glu Gly Ala Ile Pro Pro Leu Val Ala Leu Ser
305                 310                 315                 320

cag gca gga aca agt cga gct aag caa aag gct gag gcg ttg att gag 1008
Gln Ala Gly Thr Ser Arg Ala Lys Gln Lys Ala Glu Ala Leu Ile Glu

325                 330                 335

ctt cta agg caa cca aga tcc att agt aat ggt ggt gct aga tca tcg      1056
Leu Leu Arg Gln Pro Arg Ser Ile Ser Asn Gly Gly Ala Arg Ser Ser
        340                 345                 350

tcc caa ctc tga                                                      1068
Ser Gln Leu
        355

<210>  34
<211>  355
<212>  PRT
<213>  Arabidopsis thaliana

<400>  34

Met Glu Met Glu Asn His Arg Pro Gly Ser Phe Thr Tyr Met Gly Arg
1               5                   10                  15

Lys Phe Ser Asp Leu Ser Leu Asn Asp Asp Ser Ser Ala Phe Ser Asp
            20                  25                  30

Cys Asn Ser Asp Arg Ser Gly Glu Phe Pro Thr Ala Ser Ser Glu Ser
        35                  40                  45

Arg Arg Leu Leu Leu Ser Cys Ala Ser Glu Asn Ser Asp Asp Leu Ile
    50                  55                  60

Asn His Leu Val Ser His Leu Asp Ser Ser Tyr Ser Ile Asp Glu Gln
65                  70                  75                  80

Lys Gln Ala Ala Met Glu Ile Arg Leu Leu Ser Lys Asn Lys Pro Glu
                85                  90                  95

Asn Arg Ile Lys Ile Ala Lys Ala Gly Ala Ile Lys Pro Leu Ile Ser
            100                 105                 110

Leu Ile Ser Ser Ser Asp Leu Gln Leu Gln Glu Tyr Gly Val Thr Ala
        115                 120                 125

Ile Leu Asn Leu Ser Leu Cys Asp Glu Asn Lys Glu Ser Ile Ala Ser
    130                 135                 140

Ser Gly Ala Ile Lys Pro Leu Val Arg Ala Leu Lys Met Gly Thr Pro
145                 150                 155                 160

Thr Ala Lys Asp Asn Ala Ala Cys Ala Leu Leu Arg Leu Ser Gln Ile
                165                 170                 175

Glu Glu Asn Lys Val Ala Ile Gly Arg Ser Gly Ala Ile Pro Leu Leu
            180                 185                 190

Val Asn Leu Leu Glu Thr Gly Gly Phe Arg Ala Lys Lys Asp Ala Ser
        195                 200                 205

Thr Ala Leu Tyr Ser Leu Cys Ser Ala Lys Glu Asn Lys Ile Arg Ala
    210                 215                 220

Val Gln Ser Gly Ile Met Lys Pro Leu Val Glu Leu Met Ala Asp Phe
225                 230                 235                 240

Gly Ser Asn Met Val Asp Lys Ser Ala Phe Val Met Ser Leu Leu Met
                245                 250                 255

Ser Val Pro Glu Ser Lys Pro Ala Ile Val Glu Glu Gly Gly Val Pro
            260                 265                 270

Val Leu Val Glu Ile Val Glu Val Gly Thr Gln Arg Gln Lys Glu Met
        275                 280                 285

Ala Val Ser Ile Leu Leu Gln Leu Cys Glu Glu Ser Val Val Tyr Arg
    290                 295                 300

Thr Met Val Ala Arg Glu Gly Ala Ile Pro Pro Leu Val Ala Leu Ser
305                 310                 315                 320

Gln Ala Gly Thr Ser Arg Ala Lys Gln Lys Ala Glu Ala Leu Ile Glu
                325                 330                 335

Leu Leu Arg Gln Pro Arg Ser Ile Ser Asn Gly Gly Ala Arg Ser Ser
            340                 345                 350

Ser Gln Leu
        355

<210>  35
<211>  1971
<212>  DNA
<213>  Arabidopsis thaliana

<220>
<221>  CDS
<222>  (1)..(1971)

<400>  35
atg gat aca gat gaa gaa gcc aca gga gat gca gag aac cgt gat gaa        48

Met Asp Thr Asp Glu Glu Ala Thr Gly Asp Ala Glu Asn Arg Asp Glu
1               5                   10                  15

gaa gtt acc gca gaa gaa ccg att cac gat gag gtt gtg gat gcg gtg      96
Glu Val Thr Ala Glu Glu Pro Ile His Asp Glu Val Val Asp Ala Val
            20                  25                  30

gag att cat gag gaa gaa gtg aaa gaa gat gat gat gat tgt gaa gga      144
Glu Ile His Glu Glu Glu Val Lys Glu Asp Asp Asp Asp Cys Glu Gly
        35                  40                  45

ttg gtg agc gat atc gta tcg att gtc gag ttt ttg gat cag att aac      192
Leu Val Ser Asp Ile Val Ser Ile Val Glu Phe Leu Asp Gln Ile Asn
    50                  55                  60

ggt tat cga aga aca caa caa aaa gaa tgt ttt aat ctc gtt aga cga      240
Gly Tyr Arg Arg Thr Gln Gln Lys Glu Cys Phe Asn Leu Val Arg Arg
65                  70                  75                  80

ttg aag att ctt att cca ttt ttg gat gag att cga ggt ttt gaa tca      288
Leu Lys Ile Leu Ile Pro Phe Leu Asp Glu Ile Arg Gly Phe Glu Ser
                85                  90                  95

cca agt tgc aag cat ttt tta aat cgt ttg agg aaa gtg ttt ctt gct      336
Pro Ser Cys Lys His Phe Leu Asn Arg Leu Arg Lys Val Phe Leu Ala
            100                 105                 110

gcc aag aaa tta tta gaa act tgc agc aat ggc agt aaa atc tat atg      384
Ala Lys Lys Leu Leu Glu Thr Cys Ser Asn Gly Ser Lys Ile Tyr Met
        115                 120                 125

gca ttg gat ggc gaa aca atg atg acg aga ttt cat tcg att tac gaa      432
Ala Leu Asp Gly Glu Thr Met Met Thr Arg Phe His Ser Ile Tyr Glu
    130                 135                 140

aag ttg aat cgt gtt ctt gtt aaa gct cct ttt gat gaa tta atg att      480
Lys Leu Asn Arg Val Leu Val Lys Ala Pro Phe Asp Glu Leu Met Ile
145                 150                 155                 160

tct ggt gat gcg aaa gac gag att gat tca ttg tgt aaa caa ctg aaa      528
Ser Gly Asp Ala Lys Asp Glu Ile Asp Ser Leu Cys Lys Gln Leu Lys
                165                 170                 175

aaa gca aaa aga aga aca gat aca caa gac ata gag cta gca gta gac      576
Lys Ala Lys Arg Arg Thr Asp Thr Gln Asp Ile Glu Leu Ala Val Asp
            180                 185                 190

atg atg gtg gta ttc tca aaa acc gat cct cga aac gca gat agc gcg      624
Met Met Val Val Phe Ser Lys Thr Asp Pro Arg Asn Ala Asp Ser Ala
        195                 200                 205

ata ata gag agg cta gcg aaa aag ctt gag cta caa aca att gat gat      672
Ile Ile Glu Arg Leu Ala Lys Lys Leu Glu Leu Gln Thr Ile Asp Asp
    210                 215                 220

tta aag aca gaa act ata gcc ata caa agc tta atc caa gac aaa gga      720
Leu Lys Thr Glu Thr Ile Ala Ile Gln Ser Leu Ile Gln Asp Lys Gly
225                 230                 235                 240

ggt ttg aac ata gag act aaa caa cat atc att gag ctt ctt aac aag      768
Gly Leu Asn Ile Glu Thr Lys Gln His Ile Ile Glu Leu Leu Asn Lys

245                 250                 255

ttc aag aag ctt caa ggt ctt gaa gct acc gac att ctc tac caa ccc      816
Phe Lys Lys Leu Gln Gly Leu Glu Ala Thr Asp Ile Leu Tyr Gln Pro
            260                 265                 270

gtc atc aat aaa gca atc acc aag tca acg tct cta ata tta cct cat      864
Val Ile Asn Lys Ala Ile Thr Lys Ser Thr Ser Leu Ile Leu Pro His
        275                 280                 285

gag ttt ttg tgt cct ata aca ctc gaa ata atg ctt gac ccg gtt atc      912
Glu Phe Leu Cys Pro Ile Thr Leu Glu Ile Met Leu Asp Pro Val Ile
    290                 295                 300

atc gcc act gga cag aca tat gag aag gag agt ata cag aaa tgg ttt      960
Ile Ala Thr Gly Gln Thr Tyr Glu Lys Glu Ser Ile Gln Lys Trp Phe
305                 310                 315                 320

gac gca gga cat aag act tgt cct aaa aca aga cag gag tta gat cat     1008
Asp Ala Gly His Lys Thr Cys Pro Lys Thr Arg Gln Glu Leu Asp His
                325                 330                 335

ctc tct ctt gca cct aac ttc gct tta aag aac ttg att atg cag tgg     1056
Leu Ser Leu Ala Pro Asn Phe Ala Leu Lys Asn Leu Ile Met Gln Trp
            340                 345                 350

tgt gag aag aac aat ttc aag att cca gag aaa gaa gta agt cct gac     1104
Cys Glu Lys Asn Asn Phe Lys Ile Pro Glu Lys Glu Val Ser Pro Asp
        355                 360                 365

tca caa aat gag cag aaa gat gag gtc tct ttg ctg gtg gaa gcg tta     1152
Ser Gln Asn Glu Gln Lys Asp Glu Val Ser Leu Leu Val Glu Ala Leu
    370                 375                 380

tcg tca agc caa ctg gaa gaa caa cga aga tca gtg aag cag atg cgt     1200
Ser Ser Ser Gln Leu Glu Glu Gln Arg Arg Ser Val Lys Gln Met Arg
385                 390                 395                 400

ttg cta gcc aga gaa aat ccc gag aac cgc gtt tta ata gcg aat gca     1248
Leu Leu Ala Arg Glu Asn Pro Glu Asn Arg Val Leu Ile Ala Asn Ala
                405                 410                 415

gga gcg att cct ttg tta gtt caa ctc ctt tct tac cct gat tca gga     1296
Gly Ala Ile Pro Leu Leu Val Gln Leu Leu Ser Tyr Pro Asp Ser Gly
            420                 425                 430

atc caa gaa aac gcg gta acg aca ttg ttg aat cta tct atc gac gag     1344
Ile Gln Glu Asn Ala Val Thr Thr Leu Leu Asn Leu Ser Ile Asp Glu
        435                 440                 445

gtc aac aag aaa ctc att tca aat gaa gga gct att cca aac att att     1392
Val Asn Lys Lys Leu Ile Ser Asn Glu Gly Ala Ile Pro Asn Ile Ile
    450                 455                 460

gaa atc ctt gaa aat gga aac aga gag gca aga gag aac tct gct gca     1440
Glu Ile Leu Glu Asn Gly Asn Arg Glu Ala Arg Glu Asn Ser Ala Ala
465                 470                 475                 480

gct ttg ttt agt tta tcg atg ctc gat gag aac aaa gta act atc gga     1488
Ala Leu Phe Ser Leu Ser Met Leu Asp Glu Asn Lys Val Thr Ile Gly
                485                 490                 495

tta tcg aat ggg ata ccg cct tta gtc gat tta cta caa cat ggg aca     1536
Leu Ser Asn Gly Ile Pro Pro Leu Val Asp Leu Leu Gln His Gly Thr
            500                 505                 510

tta aga ggg aag aaa gat gct ctc act gca ctc ttt aac ttg tct ctt     1584
Leu Arg Gly Lys Lys Asp Ala Leu Thr Ala Leu Phe Asn Leu Ser Leu
        515                 520                 525

aac tca gct aat aaa gga aga gct atc gat gct ggt att gtt caa cct     1632
Asn Ser Ala Asn Lys Gly Arg Ala Ile Asp Ala Gly Ile Val Gln Pro
    530                 535                 540

ttg ctt aac ctt ctt aaa gat aaa aac tta ggg atg atc gat gaa gcg     1680
Leu Leu Asn Leu Leu Lys Asp Lys Asn Leu Gly Met Ile Asp Glu Ala
545                 550                 555                 560

ctt tcg att ctg ttg ctg ctt gca tca cac cct gaa gga cgt caa gcc     1728
Leu Ser Ile Leu Leu Leu Leu Ala Ser His Pro Glu Gly Arg Gln Ala
                565                 570                 575

att gga caa ctc tcc ttc att gaa aca ctt gtg gaa ttc atc aga caa     1776
Ile Gly Gln Leu Ser Phe Ile Glu Thr Leu Val Glu Phe Ile Arg Gln
            580                 585                 590

ggc acc ccg aaa aac aaa gag tgt gcg acc tcg gtg ctg ctt gaa cta     1824
Gly Thr Pro Lys Asn Lys Glu Cys Ala Thr Ser Val Leu Leu Glu Leu
        595                 600                 605

ggc tct aac aac tcg tct ttt atc ctc gca gcg ctt caa ttc gga gtt     1872
Gly Ser Asn Asn Ser Ser Phe Ile Leu Ala Ala Leu Gln Phe Gly Val
    610                 615                 620

tat gaa tat ctg gta gaa ata acc acc tct gga aca aac aga gct cag     1920
Tyr Glu Tyr Leu Val Glu Ile Thr Thr Ser Gly Thr Asn Arg Ala Gln
625                 630                 635                 640

aga aaa gca aat gct ctt ata caa ctc ata agc aaa tct gaa caa att     1968
Arg Lys Ala Asn Ala Leu Ile Gln Leu Ile Ser Lys Ser Glu Gln Ile
                645                 650                 655

tag                                                                 1971


<210>  36
<211>  656
<212>  PRT
<213>  Arabidopsis thaliana

<400>  36

Met Asp Thr Asp Glu Glu Ala Thr Gly Asp Ala Glu Asn Arg Asp Glu
1               5                   10                  15


Glu Val Thr Ala Glu Glu Pro Ile His Asp Glu Val Val Asp Ala Val
            20                  25                  30


Glu Ile His Glu Glu Glu Val Lys Glu Asp Asp Asp Asp Cys Glu Gly
        35                  40                  45

Leu Val Ser Asp Ile Val Ser Ile Val Glu Phe Leu Asp Gln Ile Asn
    50                  55                  60

Gly Tyr Arg Arg Thr Gln Gln Lys Glu Cys Phe Asn Leu Val Arg Arg
65                  70                  75                  80

Leu Lys Ile Leu Ile Pro Phe Leu Asp Glu Ile Arg Gly Phe Glu Ser
                85                  90                  95

Pro Ser Cys Lys His Phe Leu Asn Arg Leu Arg Lys Val Phe Leu Ala
            100                 105                 110

Ala Lys Lys Leu Leu Glu Thr Cys Ser Asn Gly Ser Lys Ile Tyr Met
        115                 120                 125

Ala Leu Asp Gly Glu Thr Met Met Thr Arg Phe His Ser Ile Tyr Glu
    130                 135                 140

Lys Leu Asn Arg Val Leu Val Lys Ala Pro Phe Asp Glu Leu Met Ile
145                 150                 155                 160

Ser Gly Asp Ala Lys Asp Glu Ile Asp Ser Leu Cys Lys Gln Leu Lys
                165                 170                 175

Lys Ala Lys Arg Arg Thr Asp Thr Gln Asp Ile Glu Leu Ala Val Asp
            180                 185                 190

Met Met Val Val Phe Ser Lys Thr Asp Pro Arg Asn Ala Asp Ser Ala
        195                 200                 205

Ile Ile Glu Arg Leu Ala Lys Lys Leu Glu Leu Gln Thr Ile Asp Asp
    210                 215                 220

Leu Lys Thr Glu Thr Ile Ala Ile Gln Ser Leu Ile Gln Asp Lys Gly
225                 230                 235                 240

Gly Leu Asn Ile Glu Thr Lys Gln His Ile Ile Glu Leu Leu Asn Lys
                245                 250                 255

Phe Lys Lys Leu Gln Gly Leu Glu Ala Thr Asp Ile Leu Tyr Gln Pro
            260                 265                 270

Val Ile Asn Lys Ala Ile Thr Lys Ser Thr Ser Leu Ile Leu Pro His
        275                 280                 285

Glu Phe Leu Cys Pro Ile Thr Leu Glu Ile Met Leu Asp Pro Val Ile
    290                 295                 300

Ile Ala Thr Gly Gln Thr Tyr Glu Lys Glu Ser Ile Gln Lys Trp Phe
305                 310                 315                 320

Asp Ala Gly His Lys Thr Cys Pro Lys Thr Arg Gln Glu Leu Asp His
                325                 330                 335

Leu Ser Leu Ala Pro Asn Phe Ala Leu Lys Asn Leu Ile Met Gln Trp
            340                 345                 350

Cys Glu Lys Asn Asn Phe Lys Ile Pro Glu Lys Glu Val Ser Pro Asp
        355                 360                 365

Ser Gln Asn Glu Gln Lys Asp Glu Val Ser Leu Leu Val Glu Ala Leu
    370                 375                 380

Ser Ser Ser Gln Leu Glu Glu Gln Arg Arg Ser Val Lys Gln Met Arg
385                 390                 395                 400

Leu Leu Ala Arg Glu Asn Pro Glu Asn Arg Val Leu Ile Ala Asn Ala
                405                 410                 415

Gly Ala Ile Pro Leu Leu Val Gln Leu Leu Ser Tyr Pro Asp Ser Gly
            420                 425                 430

Ile Gln Glu Asn Ala Val Thr Thr Leu Leu Asn Leu Ser Ile Asp Glu
        435                 440                 445

Val Asn Lys Lys Leu Ile Ser Asn Glu Gly Ala Ile Pro Asn Ile Ile
    450                 455                 460

Glu Ile Leu Glu Asn Gly Asn Arg Glu Ala Arg Glu Asn Ser Ala Ala
465                 470                 475                 480

Ala Leu Phe Ser Leu Ser Met Leu Asp Glu Asn Lys Val Thr Ile Gly
                485                 490                 495

Leu Ser Asn Gly Ile Pro Pro Leu Val Asp Leu Leu Gln His Gly Thr
            500                 505                 510

Leu Arg Gly Lys Lys Asp Ala Leu Thr Ala Leu Phe Asn Leu Ser Leu
        515                 520                 525

Asn Ser Ala Asn Lys Gly Arg Ala Ile Asp Ala Gly Ile Val Gln Pro
    530                 535                 540

Leu Leu Asn Leu Leu Lys Asp Lys Asn Leu Gly Met Ile Asp Glu Ala
545                 550                 555                 560

Leu Ser Ile Leu Leu Leu Leu Ala Ser His Pro Glu Gly Arg Gln Ala
                565                 570                 575

Ile Gly Gln Leu Ser Phe Ile Glu Thr Leu Val Glu Phe Ile Arg Gln
            580                 585                 590

Gly Thr Pro Lys Asn Lys Glu Cys Ala Thr Ser Val Leu Leu Glu Leu
        595                 600                 605

Gly Ser Asn Asn Ser Ser Phe Ile Leu Ala Ala Leu Gln Phe Gly Val
    610                 615                 620

Tyr Glu Tyr Leu Val Glu Ile Thr Thr Ser Gly Thr Asn Arg Ala Gln
625                 630                 635                 640

Arg Lys Ala Asn Ala Leu Ile Gln Leu Ile Ser Lys Ser Glu Gln Ile
                645                 650                 655

<210>  37
<211>  1983
<212>  DNA
<213>  Arabidopsis thaliana

<220>
<221>  CDS
<222>  (1)..(1983)

<400>  37
atg gtc gat gtg atg gat aca gat gaa gaa gcc aca gga gat gca gag      48
Met Val Asp Val Met Asp Thr Asp Glu Glu Ala Thr Gly Asp Ala Glu
1               5                   10                  15

agc cgt gat gaa gaa gtt acc gca gaa gaa ccg att cac gat gag gtt      96
Ser Arg Asp Glu Glu Val Thr Ala Glu Glu Pro Ile His Asp Glu Val
            20                  25                  30

gtg gat gcg gtg gag att cat gag gaa gaa gtg aaa gaa gat gat gat      144
Val Asp Ala Val Glu Ile His Glu Glu Glu Val Lys Glu Asp Asp Asp
        35                  40                  45

gat tgt gaa gga ttg gtg agc gat atc gta tcg att gtc gag ttt ttg      192
Asp Cys Glu Gly Leu Val Ser Asp Ile Val Ser Ile Val Glu Phe Leu
    50                  55                  60

gat cag att aac ggt tat cga aga aca caa caa aaa gaa tgt ttt aat      240
Asp Gln Ile Asn Gly Tyr Arg Arg Thr Gln Gln Lys Glu Cys Phe Asn

65                  70                  75                  80

ctc gtt aga cga ttg aag att ctt att cca ttt ttg gat gag att cga      288
Leu Val Arg Arg Leu Lys Ile Leu Ile Pro Phe Leu Asp Glu Ile Arg
                85                  90                  95

ggt ttt gaa tca cca agt tgc aag cat ttt tta aat cgt ttg agg aaa      336
Gly Phe Glu Ser Pro Ser Cys Lys His Phe Leu Asn Arg Leu Arg Lys
            100                 105                 110

gtg ttt ctt gct gcc aag aaa tta tta gaa act tgc agc aat ggc agt      384
Val Phe Leu Ala Ala Lys Lys Leu Leu Glu Thr Cys Ser Asn Gly Ser
        115                 120                 125

aaa atc tat atg gca ttg gat ggc gaa aca atg atg acg aga ttt cat      432
Lys Ile Tyr Met Ala Leu Asp Gly Glu Thr Met Met Thr Arg Phe His
    130                 135                 140

tcg att tac gaa aag ttg aat cgt gtt ctt gtt aaa gct cct ttt gat      480
Ser Ile Tyr Glu Lys Leu Asn Arg Val Leu Val Lys Ala Pro Phe Asp
145                 150                 155                 160

gaa tta atg att tct ggt gat gcg aaa gac gag att gat tca ttg tgt      528
Glu Leu Met Ile Ser Gly Asp Ala Lys Asp Glu Ile Asp Ser Leu Cys
                165                 170                 175

aaa caa ctg aaa aaa gca aaa aga aga aca gat aca caa gac ata gag      576
Lys Gln Leu Lys Lys Ala Lys Arg Arg Thr Asp Thr Gln Asp Ile Glu
            180                 185                 190

cta gca gta gac atg atg gtg gta ttc tca aaa acc gat cct cga aac      624
Leu Ala Val Asp Met Met Val Val Phe Ser Lys Thr Asp Pro Arg Asn
        195                 200                 205

gca gat agc gcg ata ata gag agg cta gcg aaa aag ctt gag cta caa      672
Ala Asp Ser Ala Ile Ile Glu Arg Leu Ala Lys Lys Leu Glu Leu Gln
    210                 215                 220

aca att gat gat tta aag aca gaa act ata gcc ata caa agc tta atc      720
Thr Ile Asp Asp Leu Lys Thr Glu Thr Ile Ala Ile Gln Ser Leu Ile
225                 230                 235                 240

caa gac aaa gga ggt ttg aac ata gag act aaa caa cat atc att gag      768
Gln Asp Lys Gly Gly Leu Asn Ile Glu Thr Lys Gln His Ile Ile Glu
                245                 250                 255

ctt ctt aac aag ttc aag aag ctt caa ggt ctt gaa gct acc gac att      816
Leu Leu Asn Lys Phe Lys Lys Leu Gln Gly Leu Glu Ala Thr Asp Ile
            260                 265                 270

ctc tac caa ccc gtc atc aat aaa gca atc acc aag tca acg tct cta      864
Leu Tyr Gln Pro Val Ile Asn Lys Ala Ile Thr Lys Ser Thr Ser Leu
        275                 280                 285

ata tta cct cat gag ttt ttg tgt cct ata aca ctc gga ata atg ctt      912
Ile Leu Pro His Glu Phe Leu Cys Pro Ile Thr Leu Gly Ile Met Leu
    290                 295                 300

gac ccg gtt atc atc gcc act gga cag aca tat gag aag gag agt ata      960
Asp Pro Val Ile Ile Ala Thr Gly Gln Thr Tyr Glu Lys Glu Ser Ile
305                 310                 315                 320

cag aaa tgg ttt gac gca gga cat aag act tgt cct aaa aca aga cag 1008
Gln Lys Trp Phe Asp Ala Gly His Lys Thr Cys Pro Lys Thr Arg Gln
                325                 330                 335

gag tta gat cat ctc tct ctt gca cct aac ttc gct tta aag aac ttg 1056
Glu Leu Asp His Leu Ser Leu Ala Pro Asn Phe Ala Leu Lys Asn Leu
            340                 345                 350

att atg cag tgg tgt gag aag aac aat ttc aag att cca gag aaa gaa 1104
Ile Met Gln Trp Cys Glu Lys Asn Asn Phe Lys Ile Pro Glu Lys Glu
        355                 360                 365

gta agt cct gac tca caa aat gag cag aaa gat gag gtc tct ttg ctg 1152
Val Ser Pro Asp Ser Gln Asn Glu Gln Lys Asp Glu Val Ser Leu Leu
    370                 375                 380

gtg gaa gcg tta tcg tca agc caa ctg gaa gaa caa cga aga tca gtg 1200
Val Glu Ala Leu Ser Ser Ser Gln Leu Glu Glu Gln Arg Arg Ser Val
385                 390                 395                 400

aag cag atg cgt ttg cta gcc aga gaa aat ccc gag aac cgc gtt tta 1248
Lys Gln Met Arg Leu Leu Ala Arg Glu Asn Pro Glu Asn Arg Val Leu
                405                 410                 415

ata gcg aat gca gga gcg att cct ttg tta gtt caa ctc ctt tct tac 1296
Ile Ala Asn Ala Gly Ala Ile Pro Leu Leu Val Gln Leu Leu Ser Tyr
            420                 425                 430

cct gat tca gga atc caa gaa aac gcg gta acg aca ttg ttg aat cta 1344
Pro Asp Ser Gly Ile Gln Glu Asn Ala Val Thr Thr Leu Leu Asn Leu
        435                 440                 445

tct atc gac gag gtc aac aag aaa ctc att tca aat gaa gga gct att 1392
Ser Ile Asp Glu Val Asn Lys Lys Leu Ile Ser Asn Glu Gly Ala Ile
    450                 455                 460

cca aac att att gaa atc ctt gaa aat gga aac aga gag gca aga gag 1440
Pro Asn Ile Ile Glu Ile Leu Glu Asn Gly Asn Arg Glu Ala Arg Glu
465                 470                 475                 480

aac tct gct gca gct ttg ttt agt tta tcg atg ctc gat gag aac aaa 1488
Asn Ser Ala Ala Ala Leu Phe Ser Leu Ser Met Leu Asp Glu Asn Lys
                485                 490                 495

gta act atc gga tta tcg aat ggg ata ccg cct tta gtc gat tta cta 1536
Val Thr Ile Gly Leu Ser Asn Gly Ile Pro Pro Leu Val Asp Leu Leu
            500                 505                 510

caa cat ggg aca tta aga ggg aag aaa gat gct ctc act gca ctc ttt 1584
Gln His Gly Thr Leu Arg Gly Lys Lys Asp Ala Leu Thr Ala Leu Phe
        515                 520                 525

aac ttg tct ctt aac tca gct aat aaa gga aga gct atc gat gct ggt 1632
Asn Leu Ser Leu Asn Ser Ala Asn Lys Gly Arg Ala Ile Asp Ala Gly
    530                 535                 540

att gtt caa cct ttg ctt aac ctt ctt aaa gat aaa aac tta ggg atg 1680
Ile Val Gln Pro Leu Leu Asn Leu Leu Lys Asp Lys Asn Leu Gly Met
545                 550                 555                 560

atc gat gaa gcg ctt tcg att ctg ttg ctg ctt gca tca cac cct gaa     1728
Ile Asp Glu Ala Leu Ser Ile Leu Leu Leu Leu Ala Ser His Pro Glu
                565                 570                 575

gga cgt caa gcc att gga caa ctc tcc ttc att gaa aca ctt gtg gaa     1776
Gly Arg Gln Ala Ile Gly Gln Leu Ser Phe Ile Glu Thr Leu Val Glu
            580                 585                 590

ttc atc aga caa ggc acc ccg aaa aac aaa gag tgt gcg acc tcg gtg     1824
Phe Ile Arg Gln Gly Thr Pro Lys Asn Lys Glu Cys Ala Thr Ser Val
        595                 600                 605

ctg ctt gaa cta ggc tct aac aac tcg tct ttt atc ctc gca gcg ctt     1872
Leu Leu Glu Leu Gly Ser Asn Asn Ser Ser Phe Ile Leu Ala Ala Leu
    610                 615                 620

caa ttc gga gtt tat gaa tat ctg gta gaa ata acc acc tct gga aca     1920
Gln Phe Gly Val Tyr Glu Tyr Leu Val Glu Ile Thr Thr Ser Gly Thr
625                 630                 635                 640

aac aga gct cag aga aaa gca aat gct ctt ata caa ctc ata agc aaa     1968
Asn Arg Ala Gln Arg Lys Ala Asn Ala Leu Ile Gln Leu Ile Ser Lys
                645                 650                 655

tct gaa caa att tag                                                 1983
Ser Glu Gln Ile
            660


<210>  38
<211>  660
<212>  PRT
<213>  Arabidopsis thaliana

<400>  38

Met Val Asp Val Met Asp Thr Asp Glu Glu Ala Thr Gly Asp Ala Glu
1               5                   10                  15


Ser Arg Asp Glu Glu Val Thr Ala Glu Glu Pro Ile His Asp Glu Val
            20                  25                  30


Val Asp Ala Val Glu Ile His Glu Glu Glu Val Lys Glu Asp Asp Asp
        35                  40                  45


Asp Cys Glu Gly Leu Val Ser Asp Ile Val Ser Ile Val Glu Phe Leu
    50                  55                  60


Asp Gln Ile Asn Gly Tyr Arg Arg Thr Gln Gln Lys Glu Cys Phe Asn
65                  70                  75                  80


Leu Val Arg Arg Leu Lys Ile Leu Ile Pro Phe Leu Asp Glu Ile Arg
                85                  90                  95


Gly Phe Glu Ser Pro Ser Cys Lys His Phe Leu Asn Arg Leu Arg Lys

100                 105                 110

Val Phe Leu Ala Ala Lys Lys Leu Leu Glu Thr Cys Ser Asn Gly Ser
        115                 120                 125

Lys Ile Tyr Met Ala Leu Asp Gly Glu Thr Met Met Thr Arg Phe His
    130                 135                 140

Ser Ile Tyr Glu Lys Leu Asn Arg Val Leu Val Lys Ala Pro Phe Asp
145                 150                 155                 160

Glu Leu Met Ile Ser Gly Asp Ala Lys Asp Glu Ile Asp Ser Leu Cys
                165                 170                 175

Lys Gln Leu Lys Lys Ala Lys Arg Arg Thr Asp Thr Gln Asp Ile Glu
            180                 185                 190

Leu Ala Val Asp Met Met Val Val Phe Ser Lys Thr Asp Pro Arg Asn
        195                 200                 205

Ala Asp Ser Ala Ile Ile Glu Arg Leu Ala Lys Lys Leu Glu Leu Gln
    210                 215                 220

Thr Ile Asp Asp Leu Lys Thr Glu Thr Ile Ala Ile Gln Ser Leu Ile
225                 230                 235                 240

Gln Asp Lys Gly Gly Leu Asn Ile Glu Thr Lys Gln His Ile Ile Glu
                245                 250                 255

Leu Leu Asn Lys Phe Lys Lys Leu Gln Gly Leu Glu Ala Thr Asp Ile
            260                 265                 270

Leu Tyr Gln Pro Val Ile Asn Lys Ala Ile Thr Lys Ser Thr Ser Leu
        275                 280                 285

Ile Leu Pro His Glu Phe Leu Cys Pro Ile Thr Leu Gly Ile Met Leu
    290                 295                 300

Asp Pro Val Ile Ile Ala Thr Gly Gln Thr Tyr Glu Lys Glu Ser Ile
305                 310                 315                 320

Gln Lys Trp Phe Asp Ala Gly His Lys Thr Cys Pro Lys Thr Arg Gln
                325                 330                 335

Glu Leu Asp His Leu Ser Leu Ala Pro Asn Phe Ala Leu Lys Asn Leu
            340                 345                 350

Ile Met Gln Trp Cys Glu Lys Asn Asn Phe Lys Ile Pro Glu Lys Glu
        355                 360                 365

Val Ser Pro Asp Ser Gln Asn Glu Gln Lys Asp Glu Val Ser Leu Leu
    370                 375                 380

Val Glu Ala Leu Ser Ser Ser Gln Leu Glu Glu Gln Arg Arg Ser Val
385                 390                 395                 400

Lys Gln Met Arg Leu Leu Ala Arg Glu Asn Pro Glu Asn Arg Val Leu
                405                 410                 415

Ile Ala Asn Ala Gly Ala Ile Pro Leu Leu Val Gln Leu Leu Ser Tyr
            420                 425                 430

Pro Asp Ser Gly Ile Gln Glu Asn Ala Val Thr Thr Leu Leu Asn Leu
        435                 440                 445

Ser Ile Asp Glu Val Asn Lys Lys Leu Ile Ser Asn Glu Gly Ala Ile
    450                 455                 460

Pro Asn Ile Ile Glu Ile Leu Glu Asn Gly Asn Arg Glu Ala Arg Glu
465                 470                 475                 480

Asn Ser Ala Ala Ala Leu Phe Ser Leu Ser Met Leu Asp Glu Asn Lys
                485                 490                 495

Val Thr Ile Gly Leu Ser Asn Gly Ile Pro Pro Leu Val Asp Leu Leu
            500                 505                 510

Gln His Gly Thr Leu Arg Gly Lys Lys Asp Ala Leu Thr Ala Leu Phe
        515                 520                 525

Asn Leu Ser Leu Asn Ser Ala Asn Lys Gly Arg Ala Ile Asp Ala Gly
    530                 535                 540

Ile Val Gln Pro Leu Leu Asn Leu Leu Lys Asp Lys Asn Leu Gly Met
545                 550                 555                 560

Ile Asp Glu Ala Leu Ser Ile Leu Leu Leu Leu Ala Ser His Pro Glu
                565                 570                 575

Gly Arg Gln Ala Ile Gly Gln Leu Ser Phe Ile Glu Thr Leu Val Glu
            580                 585                 590

Phe Ile Arg Gln Gly Thr Pro Lys Asn Lys Glu Cys Ala Thr Ser Val
        595                 600                 605

Leu Leu Glu Leu Gly Ser Asn Asn Ser Ser Phe Ile Leu Ala Ala Leu
    610                 615                 620

Gln Phe Gly Val Tyr Glu Tyr Leu Val Glu Ile Thr Thr Ser Gly Thr
625                 630                 635                 640

Asn Arg Ala Gln Arg Lys Ala Asn Ala Leu Ile Gln Leu Ile Ser Lys
                645                 650                 655

Ser Glu Gln Ile
            660

<210>  39
<211>  1920
<212>  DNA
<213>  Arabidopsis thaliana

<220>
<221>  CDS
<222>  (1)..(1920)

<400>  39
atg gga tta acg aat tgt tgt tcc cac gag gag cta atg agt cga ctc      48
Met Gly Leu Thr Asn Cys Cys Ser His Glu Glu Leu Met Ser Arg Leu
1               5                   10                  15

gtt gac tcc gtt aaa gaa ata tca ggg ttt tca tct tca agg ggt ttt      96
Val Asp Ser Val Lys Glu Ile Ser Gly Phe Ser Ser Ser Arg Gly Phe
            20                  25                  30

att ggg aag atc caa ggc gat ctt gtt cgt agg atc acg ctt ctc agc      144
Ile Gly Lys Ile Gln Gly Asp Leu Val Arg Arg Ile Thr Leu Leu Ser
        35                  40                  45

cct ttc ttc gag gaa ttg att gac gtc aat gtt gaa ttg aaa aag gat      192
Pro Phe Phe Glu Glu Leu Ile Asp Val Asn Val Glu Leu Lys Lys Asp
    50                  55                  60

cag att aca ggg ttt gag gct atg aga atc gct ctt gat tca agt ctt      240
Gln Ile Thr Gly Phe Glu Ala Met Arg Ile Ala Leu Asp Ser Ser Leu
65                  70                  75                  80

gag ctt ttt cga tcg gtt aat gga gga agc aag ctt ttt cag ctt ttc      288
Glu Leu Phe Arg Ser Val Asn Gly Gly Ser Lys Leu Phe Gln Leu Phe
                85                  90                  95

gat aga gat tct ctt gtg gag aag ttc cgt gac atg aca gtg gag ata      336
Asp Arg Asp Ser Leu Val Glu Lys Phe Arg Asp Met Thr Val Glu Ile
            100                 105                 110

gaa gca gcg tta agt cag att cct tat gag aag att gag gta tca gag      384

Glu Ala Ala Leu Ser Gln Ile Pro Tyr Glu Lys Ile Glu Val Ser Glu
        115                 120                 125

gaa gtc aga gaa cag gtt cag ctt ctg cat ttt cag ttc aag aga gca      432
Glu Val Arg Glu Gln Val Gln Leu Leu His Phe Gln Phe Lys Arg Ala
    130                 135                 140

aaa gaa aga tgg gag gag tct gat cta cag ctt agc cat gat cta gct      480
Lys Glu Arg Trp Glu Glu Ser Asp Leu Gln Leu Ser His Asp Leu Ala
145                 150                 155                 160

atg gca gag aat gtg atg gat cct gac cct ata atc ctc aaa aga ctt      528
Met Ala Glu Asn Val Met Asp Pro Asp Pro Ile Ile Leu Lys Arg Leu
                165                 170                 175

tca caa gag ctc caa ctt act acc att gat gag ctg aag aaa gaa tcg      576
Ser Gln Glu Leu Gln Leu Thr Thr Ile Asp Glu Leu Lys Lys Glu Ser
            180                 185                 190

cat gcg ata cat gag tat ttt ctt tca tat gat gga gat cct gat gac      624
His Ala Ile His Glu Tyr Phe Leu Ser Tyr Asp Gly Asp Pro Asp Asp
        195                 200                 205

tgt ttc gag agg atg tct tca ctt ctt aaa aac ctg gta gac ttt gta      672
Cys Phe Glu Arg Met Ser Ser Leu Leu Lys Asn Leu Val Asp Phe Val
    210                 215                 220

aca atg gaa agt tca gac cct gat cca tcc act ggc agc aga atc gtt      720
Thr Met Glu Ser Ser Asp Pro Asp Pro Ser Thr Gly Ser Arg Ile Val
225                 230                 235                 240

tcg aga cat cgt tct cct gtt ata cca gag tat ttt cgg tgt ccg ata      768
Ser Arg His Arg Ser Pro Val Ile Pro Glu Tyr Phe Arg Cys Pro Ile
                245                 250                 255

tca ctt gaa ctg atg aag gat cct gtt atc gtc tcc act gga cag ctg      816
Ser Leu Glu Leu Met Lys Asp Pro Val Ile Val Ser Thr Gly Gln Leu
            260                 265                 270

aat ttt tcg acc ttg cag aca tat gaa aga tca tca att cag aag tgg      864
Asn Phe Ser Thr Leu Gln Thr Tyr Glu Arg Ser Ser Ile Gln Lys Trp
        275                 280                 285

ctt gat gct ggt cat aaa aca tgt ccg aaa tct cag gag aca ctt tta      912
Leu Asp Ala Gly His Lys Thr Cys Pro Lys Ser Gln Glu Thr Leu Leu
    290                 295                 300

cat gct gga tta acc cct aat tat gtg tta aag agt ctc att gct ttg      960
His Ala Gly Leu Thr Pro Asn Tyr Val Leu Lys Ser Leu Ile Ala Leu
305                 310                 315                 320

tgg tgt gaa agc aac ggc att gag cta ccg caa aat caa ggg agc tgt     1008
Trp Cys Glu Ser Asn Gly Ile Glu Leu Pro Gln Asn Gln Gly Ser Cys
                325                 330                 335

aga acc aca aaa ata gga gga agc agc tct tca gat tgt gat cga aca     1056
Arg Thr Thr Lys Ile Gly Gly Ser Ser Ser Ser Asp Cys Asp Arg Thr
            340                 345                 350

ttt gtc ctt tcc ttg tta gag aaa ttg gcc aac ggt act aca gaa cag     1104
Phe Val Leu Ser Leu Leu Glu Lys Leu Ala Asn Gly Thr Thr Glu Gln

355                 360                 365

caa aga gct gca gct gga gaa tta agg tta cta gcc aag agg aac gtg      1152
Gln Arg Ala Ala Ala Gly Glu Leu Arg Leu Leu Ala Lys Arg Asn Val
    370                 375                 380

gat aac aga gtt tgt atc gct gag gct gga gcc ata cca ctc ctt gta      1200
Asp Asn Arg Val Cys Ile Ala Glu Ala Gly Ala Ile Pro Leu Leu Val
385                 390                 395                 400

gag ctt cta tcc tca cca gat cct cgg act cag gaa cat tct gtg aca      1248
Glu Leu Leu Ser Ser Pro Asp Pro Arg Thr Gln Glu His Ser Val Thr
                405                 410                 415

gct ctt ctg aat ctt tcc ata aat gaa ggg aac aaa gga gcc att gtt      1296
Ala Leu Leu Asn Leu Ser Ile Asn Glu Gly Asn Lys Gly Ala Ile Val
            420                 425                 430

gat gca gga gcc ata acg gat ata gta gaa gtc cta aag aac gga agc      1344
Asp Ala Gly Ala Ile Thr Asp Ile Val Glu Val Leu Lys Asn Gly Ser
        435                 440                 445

atg gaa gct aga gag aac gct gct gca acc ctt ttc agt tta tct gtt      1392
Met Glu Ala Arg Glu Asn Ala Ala Ala Thr Leu Phe Ser Leu Ser Val
    450                 455                 460

ata gat gaa aac aaa gtg gca ata ggt gct gct gga gct atc caa gca      1440
Ile Asp Glu Asn Lys Val Ala Ile Gly Ala Ala Gly Ala Ile Gln Ala
465                 470                 475                 480

ctt ata agc ttg ctt gag gaa gga acc cga aga ggc aaa aaa gat gct      1488
Leu Ile Ser Leu Leu Glu Glu Gly Thr Arg Arg Gly Lys Lys Asp Ala
                485                 490                 495

gct aca gcg att ttc aac tta tgc ata tac cag ggg aac aaa tca agg      1536
Ala Thr Ala Ile Phe Asn Leu Cys Ile Tyr Gln Gly Asn Lys Ser Arg
            500                 505                 510

gcg gtt aaa ggc ggt att gtt gac cct ctg acc aga tta ctg aaa gat      1584
Ala Val Lys Gly Gly Ile Val Asp Pro Leu Thr Arg Leu Leu Lys Asp
        515                 520                 525

gca ggt ggc gga atg gtg gat gag gct ctg gcc att tta gca att ctt      1632
Ala Gly Gly Gly Met Val Asp Glu Ala Leu Ala Ile Leu Ala Ile Leu
    530                 535                 540

tca act aac caa gaa ggg aaa aca gcg ata gct gaa gca gaa tct atc      1680
Ser Thr Asn Gln Glu Gly Lys Thr Ala Ile Ala Glu Ala Glu Ser Ile
545                 550                 555                 560

ccg gtt ttg gtt gag att ata agg aca ggg tca cca agg aac cgg gaa      1728
Pro Val Leu Val Glu Ile Ile Arg Thr Gly Ser Pro Arg Asn Arg Glu
                565                 570                 575

aat gct gca gca ata ctt tgg tat cta tgt att ggg aat ata gaa agg      1776
Asn Ala Ala Ala Ile Leu Trp Tyr Leu Cys Ile Gly Asn Ile Glu Arg
            580                 585                 590

cta aat gta gca aga gag gtt ggt gca gat gtg gcc ttg aag gaa ctt      1824
Leu Asn Val Ala Arg Glu Val Gly Ala Asp Val Ala Leu Lys Glu Leu
        595                 600                 605

act gag aat ggc act gat aga gca aag agg aaa gct gcg agc ttg ttg      1872
Thr Glu Asn Gly Thr Asp Arg Ala Lys Arg Lys Ala Ala Ser Leu Leu
    610                 615                 620

gag ctt att cag caa acc gaa ggt gtt gca gta act act gtt cca tga      1920
Glu Leu Ile Gln Gln Thr Glu Gly Val Ala Val Thr Thr Val Pro
625                 630                 635

<210>  40
<211>  639
<212>  PRT
<213>  Arabidopsis thaliana

<400>  40

Met Gly Leu Thr Asn Cys Cys Ser His Glu Glu Leu Met Ser Arg Leu
1               5                   10                  15

Val Asp Ser Val Lys Glu Ile Ser Gly Phe Ser Ser Ser Arg Gly Phe
            20                  25                  30

Ile Gly Lys Ile Gln Gly Asp Leu Val Arg Arg Ile Thr Leu Leu Ser
        35                  40                  45

Pro Phe Phe Glu Glu Leu Ile Asp Val Asn Val Glu Leu Lys Lys Asp
    50                  55                  60

Gln Ile Thr Gly Phe Glu Ala Met Arg Ile Ala Leu Asp Ser Ser Leu
65                  70                  75                  80

Glu Leu Phe Arg Ser Val Asn Gly Gly Ser Lys Leu Phe Gln Leu Phe
                85                  90                  95

Asp Arg Asp Ser Leu Val Glu Lys Phe Arg Asp Met Thr Val Glu Ile
            100                 105                 110

Glu Ala Ala Leu Ser Gln Ile Pro Tyr Glu Lys Ile Glu Val Ser Glu
        115                 120                 125

Glu Val Arg Glu Gln Val Gln Leu Leu His Phe Gln Phe Lys Arg Ala
    130                 135                 140

Lys Glu Arg Trp Glu Glu Ser Asp Leu Gln Leu Ser His Asp Leu Ala
145                 150                 155                 160

Met Ala Glu Asn Val Met Asp Pro Asp Pro Ile Ile Leu Lys Arg Leu
                165                 170                 175

Ser Gln Glu Leu Gln Leu Thr Thr Ile Asp Glu Leu Lys Lys Glu Ser
        180                 185                 190

His Ala Ile His Glu Tyr Phe Leu Ser Tyr Asp Gly Asp Pro Asp Asp
    195                 200                 205

Cys Phe Glu Arg Met Ser Ser Leu Leu Lys Asn Leu Val Asp Phe Val
    210                 215                 220

Thr Met Glu Ser Ser Asp Pro Asp Pro Ser Thr Gly Ser Arg Ile Val
225                 230                 235                 240

Ser Arg His Arg Ser Pro Val Ile Pro Glu Tyr Phe Arg Cys Pro Ile
                245                 250                 255

Ser Leu Glu Leu Met Lys Asp Pro Val Ile Val Ser Thr Gly Gln Leu
        260                 265                 270

Asn Phe Ser Thr Leu Gln Thr Tyr Glu Arg Ser Ser Ile Gln Lys Trp
    275                 280                 285

Leu Asp Ala Gly His Lys Thr Cys Pro Lys Ser Gln Glu Thr Leu Leu
    290                 295                 300

His Ala Gly Leu Thr Pro Asn Tyr Val Leu Lys Ser Leu Ile Ala Leu
305                 310                 315                 320

Trp Cys Glu Ser Asn Gly Ile Glu Leu Pro Gln Asn Gln Gly Ser Cys
                325                 330                 335

Arg Thr Thr Lys Ile Gly Gly Ser Ser Ser Ser Asp Cys Asp Arg Thr
        340                 345                 350

Phe Val Leu Ser Leu Leu Glu Lys Leu Ala Asn Gly Thr Thr Glu Gln
    355                 360                 365

Gln Arg Ala Ala Ala Gly Glu Leu Arg Leu Leu Ala Lys Arg Asn Val
    370                 375                 380

Asp Asn Arg Val Cys Ile Ala Glu Ala Gly Ala Ile Pro Leu Leu Val
385                 390                 395                 400

Glu Leu Leu Ser Ser Pro Asp Pro Arg Thr Gln Glu His Ser Val Thr
                405                 410                 415

Ala Leu Leu Asn Leu Ser Ile Asn Glu Gly Asn Lys Gly Ala Ile Val

420                 425                 430

Asp Ala Gly Ala Ile Thr Asp Ile Val Glu Val Leu Lys Asn Gly Ser
        435                 440                 445

Met Glu Ala Arg Glu Asn Ala Ala Ala Thr Leu Phe Ser Leu Ser Val
    450                 455                 460

Ile Asp Glu Asn Lys Val Ala Ile Gly Ala Ala Gly Ala Ile Gln Ala
465                 470                 475                 480

Leu Ile Ser Leu Leu Glu Glu Gly Thr Arg Arg Gly Lys Lys Asp Ala
                485                 490                 495

Ala Thr Ala Ile Phe Asn Leu Cys Ile Tyr Gln Gly Asn Lys Ser Arg
            500                 505                 510

Ala Val Lys Gly Gly Ile Val Asp Pro Leu Thr Arg Leu Leu Lys Asp
        515                 520                 525

Ala Gly Gly Gly Met Val Asp Glu Ala Leu Ala Ile Leu Ala Ile Leu
    530                 535                 540

Ser Thr Asn Gln Glu Gly Lys Thr Ala Ile Ala Glu Ala Glu Ser Ile
545                 550                 555                 560

Pro Val Leu Val Glu Ile Ile Arg Thr Gly Ser Pro Arg Asn Arg Glu
                565                 570                 575

Asn Ala Ala Ala Ile Leu Trp Tyr Leu Cys Ile Gly Asn Ile Glu Arg
            580                 585                 590

Leu Asn Val Ala Arg Glu Val Gly Ala Asp Val Ala Leu Lys Glu Leu
        595                 600                 605

Thr Glu Asn Gly Thr Asp Arg Ala Lys Arg Lys Ala Ala Ser Leu Leu
    610                 615                 620

Glu Leu Ile Gln Gln Thr Glu Gly Val Ala Val Thr Thr Val Pro
625                 630                 635

<210>  41
<211>  1899
<212>  DNA
<213>  Arabidopsis thaliana

<220>
<221>  CDS
<222>  (1)..(1899)

<400>  41
atg gga tta acg aat tgt tgt tcc cac gag gag cta atg agt cga ctc      48
Met Gly Leu Thr Asn Cys Cys Ser His Glu Glu Leu Met Ser Arg Leu
1               5                   10                  15

gtt gac tcc gtt aaa gaa ata tca ggg ttt tca tct tca agg ggt ttt      96
Val Asp Ser Val Lys Glu Ile Ser Gly Phe Ser Ser Ser Arg Gly Phe
            20                  25                  30

att ggg aag atc caa ggc gat ctt gtt cgt agg atc acg ctt ctc agc      144
Ile Gly Lys Ile Gln Gly Asp Leu Val Arg Arg Ile Thr Leu Leu Ser
        35                  40                  45

cct ttc ttc gag gaa ttg att gac gtc aat gtt gaa ttg aaa aag gat      192
Pro Phe Phe Glu Glu Leu Ile Asp Val Asn Val Glu Leu Lys Lys Asp
    50                  55                  60

cag att aca ggg ttt gag gct atg aga atc gct ctt gat tca agt ctt      240
Gln Ile Thr Gly Phe Glu Ala Met Arg Ile Ala Leu Asp Ser Ser Leu
65                  70                  75                  80

gag ctt ttt cga tcg gtt aat gga gga agc aag ctt ttt cag ctt ttc      288
Glu Leu Phe Arg Ser Val Asn Gly Gly Ser Lys Leu Phe Gln Leu Phe
                85                  90                  95

gat aga gat tct ctt gtg gag aag ttc cgt gac atg aca gtg gag ata      336
Asp Arg Asp Ser Leu Val Glu Lys Phe Arg Asp Met Thr Val Glu Ile
            100                 105                 110

gaa gca gcg tta agt cag att cct tat gag aag att gag gta tca gag      384
Glu Ala Ala Leu Ser Gln Ile Pro Tyr Glu Lys Ile Glu Val Ser Glu
        115                 120                 125

gaa gtc aga gaa cag gtt cag ctt ctg cat ttt cag ttc aag aga gca      432
Glu Val Arg Glu Gln Val Gln Leu Leu His Phe Gln Phe Lys Arg Ala
    130                 135                 140

aaa gaa aga tgg gag gag tct gat cta cag ctt agc cat gat cta gct      480
Lys Glu Arg Trp Glu Glu Ser Asp Leu Gln Leu Ser His Asp Leu Ala
145                 150                 155                 160

atg gca gag aat gtg atg gat cct gac cct ata atc ctc aaa aga ctt      528
Met Ala Glu Asn Val Met Asp Pro Asp Pro Ile Ile Leu Lys Arg Leu
                165                 170                 175

tca caa gag ctc caa ctt act acc att gat gag ctg aag aaa gaa tcg      576
Ser Gln Glu Leu Gln Leu Thr Thr Ile Asp Glu Leu Lys Lys Glu Ser
            180                 185                 190

cat gcg ata cat gag tat ttt ctt tca tat gat gga gat cct gat gac      624
His Ala Ile His Glu Tyr Phe Leu Ser Tyr Asp Gly Asp Pro Asp Asp
        195                 200                 205

tgt ttc gag agg atg tct tca ctt ctt aaa aac ctg gta gac ttt gta      672
Cys Phe Glu Arg Met Ser Ser Leu Leu Lys Asn Leu Val Asp Phe Val
    210                 215                 220

aca atg gaa agt tca gac cct gat cca tcc act ggc agc aga atc gtt     720
Thr Met Glu Ser Ser Asp Pro Asp Pro Ser Thr Gly Ser Arg Ile Val
225                 230                 235                 240

tcg aga cat cgt tct cct gtt ata cca gag tat ttt cgg tgt ccg ata     768
Ser Arg His Arg Ser Pro Val Ile Pro Glu Tyr Phe Arg Cys Pro Ile
                245                 250                 255

tca ctt gaa ctg atg aag gat cct gtt atc gtc tcc act gga cag aca     816
Ser Leu Glu Leu Met Lys Asp Pro Val Ile Val Ser Thr Gly Gln Thr
            260                 265                 270

tat gaa aga tca tca att cag aag tgg ctt gat gct ggt cat aaa aca     864
Tyr Glu Arg Ser Ser Ile Gln Lys Trp Leu Asp Ala Gly His Lys Thr
        275                 280                 285

tgt ccg aaa tct cag gag aca ctt tta cat gct gga tta acc cct aat     912
Cys Pro Lys Ser Gln Glu Thr Leu Leu His Ala Gly Leu Thr Pro Asn
    290                 295                 300

tat gtg tta aag agt ctc att gct ttg tgg tgt gaa agc aac ggc att     960
Tyr Val Leu Lys Ser Leu Ile Ala Leu Trp Cys Glu Ser Asn Gly Ile
305                 310                 315                 320

gag cta ccg caa aat caa ggg agc tgt aga acc aca aaa ata gga gga     1008
Glu Leu Pro Gln Asn Gln Gly Ser Cys Arg Thr Thr Lys Ile Gly Gly
                325                 330                 335

agc agc tct tca gat tgt gat cga aca ttt gtc ctt tcc ttg tta gag     1056
Ser Ser Ser Ser Asp Cys Asp Arg Thr Phe Val Leu Ser Leu Leu Glu
            340                 345                 350

aaa ttg gcc aac ggt act aca gaa cag caa aga gct gca gct gga gaa     1104
Lys Leu Ala Asn Gly Thr Thr Glu Gln Gln Arg Ala Ala Ala Gly Glu
        355                 360                 365

tta agg tta cta gcc aag agg aac gtg gat aac aga gtt tgt atc gct     1152
Leu Arg Leu Leu Ala Lys Arg Asn Val Asp Asn Arg Val Cys Ile Ala
    370                 375                 380

gag gct gga gcc ata cca ctc ctt gta gag ctt cta tcc tca cca gat     1200
Glu Ala Gly Ala Ile Pro Leu Leu Val Glu Leu Leu Ser Ser Pro Asp
385                 390                 395                 400

cct cgg act cag gaa cat tct gtg aca gct ctt ctg aat ctt tcc ata     1248
Pro Arg Thr Gln Glu His Ser Val Thr Ala Leu Leu Asn Leu Ser Ile
                405                 410                 415

aat gaa ggg aac aaa gga gcc att gtt gat gca gga gcc ata acg gat     1296
Asn Glu Gly Asn Lys Gly Ala Ile Val Asp Ala Gly Ala Ile Thr Asp
            420                 425                 430

ata gta gaa gtc cta aag aac gga agc atg gaa gct aga gag aac gct     1344
Ile Val Glu Val Leu Lys Asn Gly Ser Met Glu Ala Arg Glu Asn Ala
        435                 440                 445

gct gca acc ctt ttc agt tta tct gtt ata gat gaa aac aaa gtg gca     1392
Ala Ala Thr Leu Phe Ser Leu Ser Val Ile Asp Glu Asn Lys Val Ala
    450                 455                 460

ata ggt gct gct gga gct atc caa gca ctt ata agc ttg ctt gag gaa     1440

Ile Gly Ala Ala Gly Ala Ile Gln Ala Leu Ile Ser Leu Leu Glu Glu
465                 470                 475                 480

gga acc cga aga ggc aaa aaa gat gct gct aca gcg att ttc aac tta      1488
Gly Thr Arg Arg Gly Lys Lys Asp Ala Ala Thr Ala Ile Phe Asn Leu
                485                 490                 495

tgc ata tac cag ggg aac aaa tca agg gcg gtt aaa ggc ggt att gtt      1536
Cys Ile Tyr Gln Gly Asn Lys Ser Arg Ala Val Lys Gly Gly Ile Val
            500                 505                 510

gac cct ctg acc aga tta ctg aaa gat gca ggt ggc gga atg gtg gat      1584
Asp Pro Leu Thr Arg Leu Leu Lys Asp Ala Gly Gly Gly Met Val Asp
        515                 520                 525

gag gct ctg gcc att tta gca att ctt tca act aac caa gaa ggg aaa      1632
Glu Ala Leu Ala Ile Leu Ala Ile Leu Ser Thr Asn Gln Glu Gly Lys
    530                 535                 540

aca gcg ata gct gaa gca gaa tct atc ccg gtt ttg gtt gag att ata      1680
Thr Ala Ile Ala Glu Ala Glu Ser Ile Pro Val Leu Val Glu Ile Ile
545                 550                 555                 560

agg aca ggg tca cca agg aac cgg gaa aat gct gca gca ata ctt tgg      1728
Arg Thr Gly Ser Pro Arg Asn Arg Glu Asn Ala Ala Ala Ile Leu Trp
                565                 570                 575

tat cta tgt att ggg aat ata gaa agg cta aat gta gca aga gag gtt      1776
Tyr Leu Cys Ile Gly Asn Ile Glu Arg Leu Asn Val Ala Arg Glu Val
            580                 585                 590

ggt gca gat gtg gcc ttg aag gaa ctt act gag aat ggc act gat aga      1824
Gly Ala Asp Val Ala Leu Lys Glu Leu Thr Glu Asn Gly Thr Asp Arg
        595                 600                 605

gca aag agg aaa gct gcg agc ttg ttg gag ctt att cag caa acc gaa      1872
Ala Lys Arg Lys Ala Ala Ser Leu Leu Glu Leu Ile Gln Gln Thr Glu
    610                 615                 620

ggt gtt gca gta act act gtt cca tga                                  1899
Gly Val Ala Val Thr Thr Val Pro
625                 630

<210>  42
<211>  632
<212>  PRT
<213>  Arabidopsis thaliana

<400>  42

Met Gly Leu Thr Asn Cys Cys Ser His Glu Glu Leu Met Ser Arg Leu
1               5                   10                  15

Val Asp Ser Val Lys Glu Ile Ser Gly Phe Ser Ser Ser Arg Gly Phe
            20                  25                  30

Ile Gly Lys Ile Gln Gly Asp Leu Val Arg Arg Ile Thr Leu Leu Ser
        35                  40                  45

Pro Phe Phe Glu Glu Leu Ile Asp Val Asn Val Glu Leu Lys Lys Asp
    50                  55                  60

Gln Ile Thr Gly Phe Glu Ala Met Arg Ile Ala Leu Asp Ser Ser Leu
65                  70                  75                  80

Glu Leu Phe Arg Ser Val Asn Gly Gly Ser Lys Leu Phe Gln Leu Phe
                85                  90                  95

Asp Arg Asp Ser Leu Val Glu Lys Phe Arg Asp Met Thr Val Glu Ile
            100                 105                 110

Glu Ala Ala Leu Ser Gln Ile Pro Tyr Glu Lys Ile Glu Val Ser Glu
        115                 120                 125

Glu Val Arg Glu Gln Val Gln Leu Leu His Phe Gln Phe Lys Arg Ala
    130                 135                 140

Lys Glu Arg Trp Glu Glu Ser Asp Leu Gln Leu Ser His Asp Leu Ala
145                 150                 155                 160

Met Ala Glu Asn Val Met Asp Pro Asp Pro Ile Ile Leu Lys Arg Leu
                165                 170                 175

Ser Gln Glu Leu Gln Leu Thr Thr Ile Asp Glu Leu Lys Lys Glu Ser
            180                 185                 190

His Ala Ile His Glu Tyr Phe Leu Ser Tyr Asp Gly Asp Pro Asp Asp
        195                 200                 205

Cys Phe Glu Arg Met Ser Ser Leu Leu Lys Asn Leu Val Asp Phe Val
    210                 215                 220

Thr Met Glu Ser Ser Asp Pro Asp Pro Ser Thr Gly Ser Arg Ile Val
225                 230                 235                 240

Ser Arg His Arg Ser Pro Val Ile Pro Glu Tyr Phe Arg Cys Pro Ile
                245                 250                 255

Ser Leu Glu Leu Met Lys Asp Pro Val Ile Val Ser Thr Gly Gln Thr
            260                 265                 270

Tyr Glu Arg Ser Ser Ile Gln Lys Trp Leu Asp Ala Gly His Lys Thr
        275                 280                 285

Cys Pro Lys Ser Gln Glu Thr Leu Leu His Ala Gly Leu Thr Pro Asn
    290                 295                 300

Tyr Val Leu Lys Ser Leu Ile Ala Leu Trp Cys Glu Ser Asn Gly Ile
305                 310                 315                 320

Glu Leu Pro Gln Asn Gln Gly Ser Cys Arg Thr Thr Lys Ile Gly Gly
                325                 330                 335

Ser Ser Ser Ser Asp Cys Asp Arg Thr Phe Val Leu Ser Leu Leu Glu
            340                 345                 350

Lys Leu Ala Asn Gly Thr Thr Glu Gln Gln Arg Ala Ala Ala Gly Glu
        355                 360                 365

Leu Arg Leu Leu Ala Lys Arg Asn Val Asp Asn Arg Val Cys Ile Ala
    370                 375                 380

Glu Ala Gly Ala Ile Pro Leu Leu Val Glu Leu Leu Ser Ser Pro Asp
385                 390                 395                 400

Pro Arg Thr Gln Glu His Ser Val Thr Ala Leu Leu Asn Leu Ser Ile
                405                 410                 415

Asn Glu Gly Asn Lys Gly Ala Ile Val Asp Ala Gly Ala Ile Thr Asp
            420                 425                 430

Ile Val Glu Val Leu Lys Asn Gly Ser Met Glu Ala Arg Glu Asn Ala
        435                 440                 445

Ala Ala Thr Leu Phe Ser Leu Ser Val Ile Asp Glu Asn Lys Val Ala
    450                 455                 460

Ile Gly Ala Ala Gly Ala Ile Gln Ala Leu Ile Ser Leu Leu Glu Glu
465                 470                 475                 480

Gly Thr Arg Arg Gly Lys Lys Asp Ala Ala Thr Ala Ile Phe Asn Leu
                485                 490                 495

Cys Ile Tyr Gln Gly Asn Lys Ser Arg Ala Val Lys Gly Gly Ile Val
            500                 505                 510

Asp Pro Leu Thr Arg Leu Leu Lys Asp Ala Gly Gly Gly Met Val Asp
        515                 520                 525

Glu Ala Leu Ala Ile Leu Ala Ile Leu Ser Thr Asn Gln Glu Gly Lys
    530                 535                 540

Thr Ala Ile Ala Glu Ala Glu Ser Ile Pro Val Leu Val Glu Ile Ile
545                 550                 555                 560

Arg Thr Gly Ser Pro Arg Asn Arg Glu Asn Ala Ala Ala Ile Leu Trp
                565                 570                 575

Tyr Leu Cys Ile Gly Asn Ile Glu Arg Leu Asn Val Ala Arg Glu Val
            580                 585                 590

Gly Ala Asp Val Ala Leu Lys Glu Leu Thr Glu Asn Gly Thr Asp Arg
        595                 600                 605

Ala Lys Arg Lys Ala Ala Ser Leu Leu Glu Leu Ile Gln Gln Thr Glu
    610                 615                 620

Gly Val Ala Val Thr Thr Val Pro
625                 630

<210>  43
<211>  1419
<212>  DNA
<213>  Arabidopsis thaliana

<220>
<221>  CDS
<222>  (1)..(1419)

<400>  43
atg gta tcg gtg gag gaa cct tta tct cat tcc aat tcc act cgc ttt      48
Met Val Ser Val Glu Glu Pro Leu Ser His Ser Asn Ser Thr Arg Phe
1               5                   10                  15

ccg tta aca acc gat ttc tac ggt tca tca tcg ccg tcg gcg gcg agg      96
Pro Leu Thr Thr Asp Phe Tyr Gly Ser Ser Ser Pro Ser Ala Ala Arg
            20                  25                  30

tta cac cgt caa gct ggc cgg tcg atg aga aca gtg aga tct aac ttc      144
Leu His Arg Gln Ala Gly Arg Ser Met Arg Thr Val Arg Ser Asn Phe
        35                  40                  45

tat caa agc gga gat caa tct tgc tca ttc gtc ggc tca atc ggc gat      192
Tyr Gln Ser Gly Asp Gln Ser Cys Ser Phe Val Gly Ser Ile Gly Asp
    50                  55                  60

aaa tca gag tat gcg tcg gag ttt ctc tcg gat tcc gtc atc gac atg      240
Lys Ser Glu Tyr Ala Ser Glu Phe Leu Ser Asp Ser Val Ile Asp Met
65                  70                  75                  80

aga ctc ggc gag ctt gct ttg aaa aac agt aat tct ctc aat tca aac      288
Arg Leu Gly Glu Leu Ala Leu Lys Asn Ser Asn Ser Leu Asn Ser Asn

                85                  90                  95

gct tcc tca atg aaa gag gaa gcg ttt ctc gac att tct cag gcg ttt 336
Ala Ser Ser Met Lys Glu Glu Ala Phe Leu Asp Ile Ser Gln Ala Phe
            100                 105                 110

agt gat ttt tcc gct tgt agt agt gat atc tcc ggc gag tta cag cgt 384
Ser Asp Phe Ser Ala Cys Ser Ser Asp Ile Ser Gly Glu Leu Gln Arg
        115                 120                 125

ctt gct tgc ttg ccg tcg ccg gag gct gat aga aat gag agc ggc gga 432
Leu Ala Cys Leu Pro Ser Pro Glu Ala Asp Arg Asn Glu Ser Gly Gly
    130                 135                 140

gat aac gaa gcg gag cat gat cca gag tta gag aga gag cct tgt cta 480
Asp Asn Glu Ala Glu His Asp Pro Glu Leu Glu Arg Glu Pro Cys Leu
145                 150                 155                 160

ggg ttt cta cag aga gaa aac ttc tct aca gag att atc gag tgt att 528
Gly Phe Leu Gln Arg Glu Asn Phe Ser Thr Glu Ile Ile Glu Cys Ile
                165                 170                 175

tcg ccg gaa gat ctg cag cca act gtg aaa cta tgc atc gac gga ctt 576
Ser Pro Glu Asp Leu Gln Pro Thr Val Lys Leu Cys Ile Asp Gly Leu
            180                 185                 190

cgt tcc tct tcg gtg gcg ata aag cga tct gct gcg gcg aag cta cgg 624
Arg Ser Ser Ser Val Ala Ile Lys Arg Ser Ala Ala Ala Lys Leu Arg
        195                 200                 205

cta ttg gcg aag aat cga gcg gat aat cgt gtg ttg att ggg gaa tct 672
Leu Leu Ala Lys Asn Arg Ala Asp Asn Arg Val Leu Ile Gly Glu Ser
    210                 215                 220

gga gct att caa gct ttg att cca ctt ctt cgt tgt aac gat cca tgg 720
Gly Ala Ile Gln Ala Leu Ile Pro Leu Leu Arg Cys Asn Asp Pro Trp
225                 230                 235                 240

acg caa gag cgc gca gtt aca gct ctg tta aac ctc tcg tta cac gac 768
Thr Gln Glu Arg Ala Val Thr Ala Leu Leu Asn Leu Ser Leu His Asp
                245                 250                 255

cag aac aaa gct gta atc gcc gca gga gga gcg att aaa tca cta gtg 816
Gln Asn Lys Ala Val Ile Ala Ala Gly Gly Ala Ile Lys Ser Leu Val
            260                 265                 270

tgg gta ctc aaa acg ggg acg gag act tca aag cag aac gct gca tgt 864
Trp Val Leu Lys Thr Gly Thr Glu Thr Ser Lys Gln Asn Ala Ala Cys
        275                 280                 285

gct ttg ctt agt ttg gcg cta ttg gag gag aac aaa ggc tca atc gga 912
Ala Leu Leu Ser Leu Ala Leu Leu Glu Glu Asn Lys Gly Ser Ile Gly
    290                 295                 300

gct tgc ggt gct att ccg ccg ctg gtt tct ctt ctg ttg aac gga tct 960
Ala Cys Gly Ala Ile Pro Pro Leu Val Ser Leu Leu Leu Asn Gly Ser
305                 310                 315                 320

tgc agg gga aag aag gat gcg ttg acg gcg ctc tac aag ctg tgt acg 1008
Cys Arg Gly Lys Lys Asp Ala Leu Thr Ala Leu Tyr Lys Leu Cys Thr
                325                 330                 335

ctt cag caa aac aag gag aga gcg gtc act gct gga gcg gtg aag ccg     1056
Leu Gln Gln Asn Lys Glu Arg Ala Val Thr Ala Gly Ala Val Lys Pro
            340                 345                 350

ttg gtg gac ctt gtg gct gag gaa ggg act ggt atg gcg gag aaa gct     1104
Leu Val Asp Leu Val Ala Glu Glu Gly Thr Gly Met Ala Glu Lys Ala
        355                 360                 365

atg gtg gtt ctg agt agc ctt gca gcg ata gat gat ggc aaa gag gct     1152
Met Val Val Leu Ser Ser Leu Ala Ala Ile Asp Asp Gly Lys Glu Ala
    370                 375                 380

att gtc gag gaa gga ggg atc gca gcg ctt gtt gag gcc atc gag gat     1200
Ile Val Glu Glu Gly Gly Ile Ala Ala Leu Val Glu Ala Ile Glu Asp
385                 390                 395                 400

gga tct gtg aaa ggg aaa gaa ttt gcg atc ttg acg ctg ttg cag ctt     1248
Gly Ser Val Lys Gly Lys Glu Phe Ala Ile Leu Thr Leu Leu Gln Leu
                405                 410                 415

tgt tct gat agc gtt aga aac cgt ggg ttg ctt gtg agg gaa ggc gcg     1296
Cys Ser Asp Ser Val Arg Asn Arg Gly Leu Leu Val Arg Glu Gly Ala
            420                 425                 430

att cct ccg ctt gtg ggc ctc tct cag agc ggc tcc gtc agt gtt aga     1344
Ile Pro Pro Leu Val Gly Leu Ser Gln Ser Gly Ser Val Ser Val Arg
        435                 440                 445

gct aag cgc aag gca gaa aga ctt ctg ggg tat ctt cgg gag cca agg     1392
Ala Lys Arg Lys Ala Glu Arg Leu Leu Gly Tyr Leu Arg Glu Pro Arg
    450                 455                 460

aag gag gca agt tca tca agc cca tga                                 1419
Lys Glu Ala Ser Ser Ser Ser Pro
465                 470


<210>  44
<211>  472
<212>  PRT
<213>  Arabidopsis thaliana

<400>  44

Met Val Ser Val Glu Glu Pro Leu Ser His Ser Asn Ser Thr Arg Phe
1               5                   10                  15


Pro Leu Thr Thr Asp Phe Tyr Gly Ser Ser Ser Pro Ser Ala Ala Arg
            20                  25                  30


Leu His Arg Gln Ala Gly Arg Ser Met Arg Thr Val Arg Ser Asn Phe
        35                  40                  45


Tyr Gln Ser Gly Asp Gln Ser Cys Ser Phe Val Gly Ser Ile Gly Asp
    50                  55                  60

Lys Ser Glu Tyr Ala Ser Glu Phe Leu Ser Asp Ser Val Ile Asp Met
65                  70                  75                  80

Arg Leu Gly Glu Leu Ala Leu Lys Asn Ser Asn Ser Leu Asn Ser Asn
                85                  90                  95

Ala Ser Ser Met Lys Glu Glu Ala Phe Leu Asp Ile Ser Gln Ala Phe
            100                 105                 110

Ser Asp Phe Ser Ala Cys Ser Ser Asp Ile Ser Gly Glu Leu Gln Arg
        115                 120                 125

Leu Ala Cys Leu Pro Ser Pro Glu Ala Asp Arg Asn Glu Ser Gly Gly
    130                 135                 140

Asp Asn Glu Ala Glu His Asp Pro Glu Leu Glu Arg Glu Pro Cys Leu
145                 150                 155                 160

Gly Phe Leu Gln Arg Glu Asn Phe Ser Thr Glu Ile Ile Glu Cys Ile
                165                 170                 175

Ser Pro Glu Asp Leu Gln Pro Thr Val Lys Leu Cys Ile Asp Gly Leu
            180                 185                 190

Arg Ser Ser Ser Val Ala Ile Lys Arg Ser Ala Ala Ala Lys Leu Arg
        195                 200                 205

Leu Leu Ala Lys Asn Arg Ala Asp Asn Arg Val Leu Ile Gly Glu Ser
    210                 215                 220

Gly Ala Ile Gln Ala Leu Ile Pro Leu Leu Arg Cys Asn Asp Pro Trp
225                 230                 235                 240

Thr Gln Glu Arg Ala Val Thr Ala Leu Leu Asn Leu Ser Leu His Asp
                245                 250                 255

Gln Asn Lys Ala Val Ile Ala Ala Gly Gly Ala Ile Lys Ser Leu Val
            260                 265                 270

Trp Val Leu Lys Thr Gly Thr Glu Thr Ser Lys Gln Asn Ala Ala Cys
        275                 280                 285

Ala Leu Leu Ser Leu Ala Leu Leu Glu Glu Asn Lys Gly Ser Ile Gly
    290                 295                 300

Ala Cys Gly Ala Ile Pro Pro Leu Val Ser Leu Leu Leu Asn Gly Ser

305                 310                 315                 320

Cys Arg Gly Lys Lys Asp Ala Leu Thr Ala Leu Tyr Lys Leu Cys Thr
                325                 330                 335

Leu Gln Gln Asn Lys Glu Arg Ala Val Thr Ala Gly Ala Val Lys Pro
            340                 345                 350

Leu Val Asp Leu Val Ala Glu Glu Gly Thr Gly Met Ala Glu Lys Ala
        355                 360                 365

Met Val Val Leu Ser Ser Leu Ala Ala Ile Asp Asp Gly Lys Glu Ala
    370                 375                 380

Ile Val Glu Glu Gly Gly Ile Ala Ala Leu Val Glu Ala Ile Glu Asp
385                 390                 395                 400

Gly Ser Val Lys Gly Lys Glu Phe Ala Ile Leu Thr Leu Leu Gln Leu
                405                 410                 415

Cys Ser Asp Ser Val Arg Asn Arg Gly Leu Leu Val Arg Glu Gly Ala
            420                 425                 430

Ile Pro Pro Leu Val Gly Leu Ser Gln Ser Gly Ser Val Ser Val Arg
        435                 440                 445

Ala Lys Arg Lys Ala Glu Arg Leu Leu Gly Tyr Leu Arg Glu Pro Arg
    450                 455                 460

Lys Glu Ala Ser Ser Ser Ser Pro
465                 470

<210>  45
<211>  54
<212>  DNA
<213>  Artificial Sequence

<220>
<223>  GeneRacer Oligo dT Primer

<400>  45
gctgtcaacg atacgctacg taacggcatg acagtgtttt tttttttttt tttt          54

<210>  46
<211>  25
<212>  DNA
<213>  Artificial Sequence

<220>

<223>  Primer MWG 1

<400>  46
gcagacatga cccaatcttg gcagg                                          25


<210>  47
<211>  23
<212>  DNA
<213>  Artificial Sequence

<220>
<223>  GeneRacer 5´Primer (Invitrogen)

<400>  47
cgactggagc acgaggacac tga                                            23


<210>  48
<211>  25
<212>  DNA
<213>  Artificial Sequence

<220>
<223>  Primer MWG 2

<400>  48
ccacggtcag caacctctcc agacg                                          25


<210>  49
<211>  26
<212>  DNA
<213>  Artificial Sequence

<220>
<223>  GeneRacer 5´nested Primer (Invitrogen)

<400>  49
ggacactgac atggactgaa ggagta                                         26


<210>  50
<211>  27
<212>  DNA
<213>  Artificial Sequence

<220>
<223>  Primer MWG 3

<400>  50
cagatgatag ttattgttgt tgactgg                                        27


<210>  51
<211>  25
<212>  DNA
<213>  Artificial Sequence

<220>
<223>  GeneRacer 3´Primer (Invitrogen)

<400>  51
gctgtcaacg atacgctacg taacg                                          25


<210>  52
<211>  24
<212>  DNA
<213>  Artificial Sequence

<220>
<223>  Primer MWG 4

<400>  52
ctcatcttct caagctactg gtgg                                           24


<210>  53
<211>  23
<212>  DNA
<213>  Artificial Sequence

<220>
<223>  GeneRacer 3´nested Primer (Invitrogen)

<400>  53
cgctacgtaa cggcatgaca gtg                                            23


<210>  54
<211>  21
<212>  DNA
<213>  Artificial Sequence

<220>
<223>  Primer MWG 29

<400>  54
atatgcaaat ggctctgcta g                                              21


<210>  55
<211>  21
<212>  DNA
<213>  Artificial Sequence

<220>
<223>  Primer MWG 30

<400>  55
tatcatctcc ttcccgagtt c                                              21


<210>  56
<211>  27
<212>  DNA
<213>  Artificial Sequence

<220>
<223>  Primer MWG 31

<400>  56
cccgggatga ttttgcggtt ttggcgg                                          27


<210>  57
<211>  31
<212>  DNA
<213>  Artificial Sequence

<220>
<223>  Primer MWG 32

<400>  57
cccgggtcac aagacaaaac ataaaaatag g                                     31


<210>  58
<211>  21
<212>  DNA
<213>  Artificial Sequence

<220>
<223>  Primer MWG 32b

<400>  58
gactcacact actctaatac c                                                21


<210>  59
<211>  20
<212>  DNA
<213>  Artificial Sequence

<220>
<223>  Primer MWG 33

<400>  59
gacatcgttt gtctcacacc                                                  20


<210>  60
<211>  249
<212>  PRT
<213>  Artificial Sequence

<220>
<223>  Consensus Sequence


<220>
<221>  misc_feature
<222>  (2)..(2)
<223>  Xaa can be any naturally occurring amino acid

<220>
<221>  misc_feature
<222>  (4)..(10)
<223>  Xaa can be any naturally occurring amino acid

<220>
<221>  misc_feature

<222>  (12)..(13)
<223>  Xaa can be any naturally occurring amino acid

<220>
<221>  misc_feature
<222>  (15)..(18)
<223>  Xaa can be any naturally occurring amino acid

<220>
<221>  misc_feature
<222>  (21)..(22)
<223>  Xaa can be any naturally occurring amino acid

<220>
<221>  misc_feature
<222>  (24)..(25)
<223>  Xaa can be any naturally occurring amino acid

<220>
<221>  misc_feature
<222>  (27)..(34)
<223>  Xaa can be any naturally occurring amino acid

<220>
<221>  misc_feature
<222>  (36)..(38)
<223>  Xaa can be any naturally occurring amino acid

<220>
<221>  misc_feature
<222>  (41)..(42)
<223>  Xaa can be any naturally occurring amino acid

<220>
<221>  misc_feature
<222>  (47)..(50)
<223>  Xaa can be any naturally occurring amino acid

<220>
<221>  misc_feature
<222>  (52)..(54)
<223>  Xaa can be any naturally occurring amino acid

<220>
<221>  misc_feature
<222>  (56)..(59)
<223>  Xaa can be any naturally occurring amino acid

<220>
<221>  misc_feature
<222>  (62)..(67)
<223>  Xaa can be any naturally occurring amino acid

<220>
<221>  misc_feature
<222>  (69)..(70)
<223>  Xaa can be any naturally occurring amino acid

<220>
<221>  MISC_FEATURE
<222>  (72)..(72)

<223>  Xaa can be no or any amino acid

<220>
<221>  misc_feature
<222>  (73)..(78)
<223>  Xaa can be any naturally occurring amino acid

<220>
<221>  misc_feature
<222>  (80)..(80)
<223>  Xaa can be any naturally occurring amino acid

<220>
<221>  misc_feature
<222>  (82)..(83)
<223>  Xaa can be any naturally occurring amino acid

<220>
<221>  misc_feature
<222>  (85)..(86)
<223>  Xaa can be any naturally occurring amino acid

<220>
<221>  misc_feature
<222>  (88)..(96)
<223>  Xaa can be any naturally occurring amino acid

<220>
<221>  misc_feature
<222>  (99)..(100)
<223>  Xaa can be any naturally occurring amino acid

<220>
<221>  MISC_FEATURE
<222>  (101)..(101)
<223>  Xaa can be no or any amino acid

<220>
<221>  misc_feature
<222>  (102)..(106)
<223>  Xaa can be any naturally occurring amino acid

<220>
<221>  misc_feature
<222>  (108)..(109)
<223>  Xaa can be any naturally occurring amino acid

<220>
<221>  misc_feature
<222>  (112)..(113)
<223>  Xaa can be any naturally occurring amino acid

<220>
<221>  misc_feature
<222>  (115)..(118)
<223>  Xaa can be any naturally occurring amino acid

<220>
<221>  misc_feature
<222>  (123)..(128)
<223>  Xaa can be any naturally occurring amino acid

<220>
<221> misc_feature
<222> (130)..(135)
<223> Xaa can be any naturally occurring amino acid

<220>
<221> misc_feature
<222> (137)..(147)
<223> Xaa can be any naturally occurring amino acid

<220>
<221> misc_feature
<222> (149)..(150)
<223> Xaa can be any naturally occurring amino acid

<220>
<221> misc_feature
<222> (152)..(152)
<223> Xaa can be any naturally occurring amino acid

<220>
<221> MISC_FEATURE
<222> (153)..(153)
<223> Xaa can be no or any amino acid

<220>
<221> misc_feature
<222> (154)..(169)
<223> Xaa can be any naturally occurring amino acid

<220>
<221> misc_feature
<222> (171)..(188)
<223> Xaa can be any naturally occurring amino acid

<220>
<221> misc_feature
<222> (192)..(195)
<223> Xaa can be any naturally occurring amino acid

<220>
<221> misc_feature
<222> (197)..(201)
<223> Xaa can be any naturally occurring amino acid

<220>
<221> misc_feature
<222> (203)..(203)
<223> Xaa can be any naturally occurring amino acid

<220>
<221> misc_feature
<222> (205)..(207)
<223> Xaa can be any naturally occurring amino acid

<220>
<221> misc_feature
<222> (209)..(210)
<223> Xaa can be any naturally occurring amino acid

<220>
<221>  misc_feature
<222>  (212)..(230)
<223>  Xaa can be any naturally occurring amino acid

<220>
<221>  misc_feature
<222>  (232)..(237)
<223>  Xaa can be any naturally occurring amino acid

<220>
<221>  MISC_FEATURE
<222>  (239)..(239)
<223>  Xaa can be no or any amino acid

<220>
<221>  MISC_FEATURE
<222>  (240)..(240)
<223>  Xaa can be no or any amino acid

<220>
<221>  misc_feature
<222>  (241)..(242)
<223>  Xaa can be any naturally occurring amino acid

<220>
<221>  misc_feature
<222>  (244)..(246)
<223>  Xaa can be any naturally occurring amino acid

<220>
<221>  misc_feature
<222>  (248)..(249)
<223>  Xaa can be any naturally occurring amino acid

<400>  60

Arg Xaa Leu Xaa Xaa Xaa Xaa Xaa Xaa Xaa Arg Xaa Xaa Ile Xaa Xaa
1               5                   10                  15

Xaa Xaa Ala Ile Xaa Xaa Leu Xaa Xaa Leu Xaa Xaa Xaa Xaa Xaa Xaa
            20                  25                  30

Xaa Xaa Gln Xaa Xaa Xaa Val Thr Xaa Xaa Leu Asn Leu Ser Xaa Xaa
        35                  40                  45

Xaa Xaa Asn Xaa Xaa Xaa Ile Xaa Xaa Xaa Xaa Ala Ile Xaa Xaa Xaa
    50                  55                  60

Xaa Xaa Xaa Leu Xaa Xaa Gly Xaa Xaa Xaa Xaa Xaa Xaa Xaa Asn Xaa
65                  70                  75                  80

Ala Xaa Xaa Leu Xaa Xaa Leu Xaa Xaa Xaa Xaa Xaa Xaa Xaa Xaa Xaa
                85                  90                  95

Ile Gly Xaa Xaa Xaa Xaa Xaa Xaa Xaa Xaa Leu Xaa Xaa Leu Leu Xaa
            100                 105                 110

Xaa Gly Xaa Xaa Xaa Xaa Lys Lys Asp Ala Xaa Xaa Xaa Xaa Xaa Xaa
        115                 120                 125

Leu Xaa Xaa Xaa Xaa Xaa Xaa Lys Xaa Xaa Xaa Xaa Xaa Xaa Xaa Xaa
    130                 135                 140

Xaa Xaa Xaa Leu Xaa Xaa Leu Xaa Xaa Xaa Xaa Xaa Xaa Xaa Xaa Xaa
145                 150                 155                 160

Xaa Xaa Xaa Xaa Xaa Xaa Xaa Xaa Xaa Leu Xaa Xaa Xaa Xaa Xaa Xaa
                165                 170                 175

Xaa Xaa Xaa Xaa Xaa Xaa Xaa Xaa Xaa Xaa Xaa Xaa Leu Val Glu Xaa
            180                 185                 190

Xaa Xaa Xaa Gly Xaa Xaa Xaa Xaa Xaa Glu Xaa Ala Xaa Xaa Xaa Leu
        195                 200                 205

Xaa Xaa Leu Xaa Xaa Xaa Xaa Xaa Xaa Xaa Xaa Xaa Xaa Xaa Xaa Xaa
    210                 215                 220

Xaa Xaa Xaa Xaa Xaa Xaa Leu Xaa Xaa Xaa Xaa Xaa Xaa Gly Xaa Xaa
225                 230                 235                 240

Xaa Xaa Arg Xaa Xaa Xaa Lys Xaa Xaa
                245

<210>  61
<211>  249
<212>  PRT
<213>  Artificial

<220>
<223>  Consensus Sequence


<220>
<221>  misc_feature
<222>  (6)..(8)
<223>  Xaa can be any naturally occurring amino acid

<220>
<221>  misc_feature
<222>  (13)..(13)
<223>  Xaa can be any naturally occurring amino acid

<220>
<221>  misc_feature

<222>  (16)..(17)
<223>  Xaa can be any naturally occurring amino acid

<220>
<221>  misc_feature
<222>  (21)..(22)
<223>  Xaa can be any naturally occurring amino acid

<220>
<221>  misc_feature
<222>  (25)..(25)
<223>  Xaa can be any naturally occurring amino acid

<220>
<221>  misc_feature
<222>  (28)..(28)
<223>  Xaa can be any naturally occurring amino acid

<220>
<221>  misc_feature
<222>  (30)..(30)
<223>  Xaa can be any naturally occurring amino acid

<220>
<221>  misc_feature
<222>  (32)..(33)
<223>  Xaa can be any naturally occurring amino acid

<220>
<221>  misc_feature
<222>  (37)..(37)
<223>  Xaa can be any naturally occurring amino acid

<220>
<221>  misc_feature
<222>  (48)..(48)
<223>  Xaa can be any naturally occurring amino acid

<220>
<221>  misc_feature
<222>  (50)..(50)
<223>  Xaa can be any naturally occurring amino acid

<220>
<221>  misc_feature
<222>  (53)..(53)
<223>  Xaa can be any naturally occurring amino acid

<220>
<221>  misc_feature
<222>  (57)..(57)
<223>  Xaa can be any naturally occurring amino acid

<220>
<221>  misc_feature
<222>  (62)..(62)
<223>  Xaa can be any naturally occurring amino acid

<220>
<221>  misc_feature
<222>  (65)..(66)

<223> Xaa can be any naturally occurring amino acid

<220>
<221> misc_feature
<222> (69)..(70)
<223> Xaa can be any naturally occurring amino acid

<220>
<221> MISC_FEATURE
<222> (72)..(72)
<223> Xaa can be no or any amino acid

<220>
<221> misc_feature
<222> (73)..(74)
<223> Xaa can be any naturally occurring amino acid

<220>
<221> misc_feature
<222> (95)..(96)
<223> Xaa can be any naturally occurring amino acid

<220>
<221> misc_feature
<222> (99)..(99)
<223> Xaa can be any naturally occurring amino acid

<220>
<221> MISC_FEATURE
<222> (101)..(101)
<223> Xaa can be no or any amino acid

<220>
<221> misc_feature
<222> (105)..(105)
<223> Xaa can be any naturally occurring amino acid

<220>
<221> misc_feature
<222> (113)..(113)
<223> Xaa can be any naturally occurring amino acid

<220>
<221> misc_feature
<222> (116)..(116)
<223> Xaa can be any naturally occurring amino acid

<220>
<221> misc_feature
<222> (132)..(133)
<223> Xaa can be any naturally occurring amino acid

<220>
<221> misc_feature
<222> (137)..(137)
<223> Xaa can be any naturally occurring amino acid

<220>
<221> misc_feature
<222> (139)..(139)
<223> Xaa can be any naturally occurring amino acid

<220>
<221> misc_feature
<222> (146)..(147)
<223> Xaa can be any naturally occurring amino acid

<220>
<221> MISC_FEATURE
<222> (153)..(153)
<223> Xaa can be no or any amino acid

<220>
<221> misc_feature
<222> (156)..(157)
<223> Xaa can be any naturally occurring amino acid

<220>
<221> misc_feature
<222> (173)..(173)
<223> Xaa can be any naturally occurring amino acid

<220>
<221> misc_feature
<222> (178)..(178)
<223> Xaa can be any naturally occurring amino acid

<220>
<221> misc_feature
<222> (181)..(182)
<223> Xaa can be any naturally occurring amino acid

<220>
<221> misc_feature
<222> (188)..(188)
<223> Xaa can be any naturally occurring amino acid

<220>
<221> misc_feature
<222> (192)..(192)
<223> Xaa can be any naturally occurring amino acid

<220>
<221> misc_feature
<222> (198)..(198)
<223> Xaa can be any naturally occurring amino acid

<220>
<221> misc_feature
<222> (200)..(200)
<223> Xaa can be any naturally occurring amino acid

<220>
<221> misc_feature
<222> (207)..(207)
<223> Xaa can be any naturally occurring amino acid

<220>
<221> misc_feature
<222> (213)..(213)
<223> Xaa can be any naturally occurring amino acid

<220>
<221>  misc_feature
<222>  (216)..(217)
<223>  Xaa can be any naturally occurring amino acid

<220>
<221>  misc_feature
<222>  (220)..(221)
<223>  Xaa can be any naturally occurring amino acid

<220>
<221>  misc_feature
<222>  (228)..(228)
<223>  Xaa can be any naturally occurring amino acid

<220>
<221>  MISC_FEATURE
<222>  (239)..(239)
<223>  Xaa can be no or any amino acid

<220>
<221>  MISC_FEATURE
<222>  (240)..(240)
<223>  Xaa can be no or any amino acid

<220>
<221>  misc_feature
<222>  (242)..(242)
<223>  Xaa can be any naturally occurring amino acid

<220>
<221>  misc_feature
<222>  (249)..(249)
<223>  Xaa can be any naturally occurring amino acid

<400>  61

Arg Leu Leu Ala Lys Xaa Xaa Xaa Glu Asn Arg Ile Xaa Ile Ala Xaa
1               5                   10                  15

Xaa Gly Ala Ile Xaa Xaa Leu Val Xaa Leu Leu Xaa Ser Xaa Asp Xaa
            20                  25                  30

Xaa Thr Gln Glu Xaa Ala Val Thr Ala Leu Leu Asn Leu Ser Ile Xaa
        35                  40                  45

Asp Xaa Asn Lys Xaa Ala Ile Ala Xaa Ala Gly Ala Ile Xaa Pro Leu
    50                  55                  60

Xaa Xaa Val Leu Xaa Xaa Gly Xaa Xaa Xaa Glu Ala Lys Glu Asn Ser
65                  70                  75                  80

Ala Ala Thr Leu Phe Ser Leu Ser Val Ile Glu Glu Asn Lys Xaa Xaa
                85                  90                  95

Ile Gly Xaa Ser Xaa Gly Ala Ile Xaa Pro Leu Val Asp Leu Leu Gly
            100                 105                 110

Xaa Gly Thr Xaa Arg Gly Lys Lys Asp Ala Ala Thr Ala Leu Phe Asn
        115                 120                 125

Leu Ser Ile Xaa Xaa Glu Asn Lys Xaa Arg Xaa Val Gln Ala Gly Ala
    130                 135                 140

Val Xaa Xaa Leu Val Glu Leu Met Xaa Asp Pro Xaa Xaa Gly Met Val
145                 150                 155                 160

Asp Lys Ala Val Ala Val Leu Ala Asn Leu Ala Thr Xaa Pro Glu Gly
                165                 170                 175

Arg Xaa Ala Ile Xaa Xaa Glu Gly Gly Ile Pro Xaa Leu Val Glu Xaa
            180                 185                 190

Val Glu Leu Gly Ser Xaa Arg Xaa Lys Glu Asn Ala Ala Ala Xaa Leu
        195                 200                 205

Leu Gln Leu Cys Xaa Asn Ser Xaa Xaa Phe Cys Xaa Xaa Val Leu Gln
    210                 215                 220

Glu Gly Ala Xaa Pro Pro Leu Val Ala Leu Ser Gln Ser Gly Xaa Xaa
225                 230                 235                 240

Thr Xaa Arg Ala Lys Glu Lys Ala Xaa
                245

<210>  62
<211>  249
<212>  PRT
<213>  Artificial Sequence

<220>
<223>  Consensus Sequence


<220>
<221>  misc_feature
<222>  (6)..(7)
<223>  Xaa can be any naturally occurring amino acid

<220>
<221>  misc_feature
<222>  (13)..(13)
<223>  Xaa can be any naturally occurring amino acid

<220>
<221>  misc_feature

<222>  (16)..(16)
<223>  Xaa can be any naturally occurring amino acid

<220>
<221>  misc_feature
<222>  (21)..(22)
<223>  Xaa can be any naturally occurring amino acid

<220>
<221>  misc_feature
<222>  (25)..(25)
<223>  Xaa can be any naturally occurring amino acid

<220>
<221>  misc_feature
<222>  (30)..(30)
<223>  Xaa can be any naturally occurring amino acid

<220>
<221>  misc_feature
<222>  (32)..(33)
<223>  Xaa can be any naturally occurring amino acid

<220>
<221>  misc_feature
<222>  (37)..(37)
<223>  Xaa can be any naturally occurring amino acid

<220>
<221>  misc_feature
<222>  (48)..(48)
<223>  Xaa can be any naturally occurring amino acid

<220>
<221>  misc_feature
<222>  (50)..(50)
<223>  Xaa can be any naturally occurring amino acid

<220>
<221>  misc_feature
<222>  (53)..(53)
<223>  Xaa can be any naturally occurring amino acid

<220>
<221>  misc_feature
<222>  (57)..(57)
<223>  Xaa can be any naturally occurring amino acid

<220>
<221>  misc_feature
<222>  (62)..(62)
<223>  Xaa can be any naturally occurring amino acid

<220>
<221>  misc_feature
<222>  (69)..(70)
<223>  Xaa can be any naturally occurring amino acid

<220>
<221>  MISC_FEATURE
<222>  (72)..(72)

<223>  Xaa can be no or any amino acid

<220>
<221>  misc_feature
<222>  (74)..(74)
<223>  Xaa can be any naturally occurring amino acid

<220>
<221>  misc_feature
<222>  (96)..(96)
<223>  Xaa can be any naturally occurring amino acid

<220>
<221>  misc_feature
<222>  (99)..(99)
<223>  Xaa can be any naturally occurring amino acid

<220>
<221>  MISC_FEATURE
<222>  (101)..(101)
<223>  Xaa can be no or any amino acid

<220>
<221>  misc_feature
<222>  (105)..(105)
<223>  Xaa can be any naturally occurring amino acid

<220>
<221>  misc_feature
<222>  (113)..(113)
<223>  Xaa can be any naturally occurring amino acid

<220>
<221>  misc_feature
<222>  (116)..(116)
<223>  Xaa can be any naturally occurring amino acid

<220>
<221>  misc_feature
<222>  (132)..(133)
<223>  Xaa can be any naturally occurring amino acid

<220>
<221>  misc_feature
<222>  (137)..(137)
<223>  Xaa can be any naturally occurring amino acid

<220>
<221>  MISC_FEATURE
<222>  (153)..(153)
<223>  Xaa can be no or any amino acid

<220>
<221>  misc_feature
<222>  (178)..(178)
<223>  Xaa can be any naturally occurring amino acid

<220>
<221>  misc_feature
<222>  (182)..(182)
<223>  Xaa can be any naturally occurring amino acid

<220>
<221> misc_feature
<222> (198)..(198)
<223> Xaa can be any naturally occurring amino acid

<220>
<221> misc_feature
<222> (216)..(216)
<223> Xaa can be any naturally occurring amino acid

<220>
<221> misc_feature
<222> (220)..(220)
<223> Xaa can be any naturally occurring amino acid

<220>
<221> MISC_FEATURE
<222> (239)..(239)
<223> Xaa can be no or any amino acid

<220>
<221> MISC_FEATURE
<222> (240)..(240)
<223> Xaa can be no or any amino acid

<220>
<221> misc_feature
<222> (242)..(242)
<223> Xaa can be any naturally occurring amino acid

<400> 62

Arg Leu Leu Ala Lys Xaa Xaa Met Glu Asn Arg Ile Xaa Ile Ala Xaa
1               5                   10                  15

Ala Gly Ala Ile Xaa Xaa Leu Val Xaa Leu Leu Tyr Ser Xaa Asp Xaa
            20                  25                  30

Xaa Thr Gln Glu Xaa Ala Val Thr Ala Leu Leu Asn Leu Ser Ile Xaa
        35                  40                  45

Asp Xaa Asn Lys Xaa Ala Ile Ala Xaa Ala Gly Ala Ile Xaa Pro Leu
    50                  55                  60

Ile His Val Leu Xaa Xaa Gly Xaa Ser Xaa Glu Ala Lys Glu Asn Ser
65                  70                  75                  80

Ala Ala Thr Leu Phe Ser Leu Ser Val Ile Glu Glu Asn Lys Val Xaa
                85                  90                  95

Ile Gly Xaa Ser Xaa Gly Ala Ile Xaa Pro Leu Val Asp Leu Leu Gly
            100                 105                 110

Xaa Gly Thr Xaa Arg Gly Lys Lys Asp Ala Ala Thr Ala Leu Phe Asn
        115                 120                 125

Leu Ser Ile Xaa Xaa Glu Asn Lys Xaa Arg Ile Val Gln Ala Gly Ala
    130                 135                 140

Val Lys Tyr Leu Val Glu Leu Met Xaa Asp Pro Ala Ala Gly Met Val
145                 150                 155                 160

Asp Lys Ala Val Ala Val Leu Ala Asn Leu Ala Thr Val Pro Glu Gly
                165                 170                 175

Arg Xaa Ala Ile Gly Xaa Glu Gly Gly Ile Pro Val Leu Val Glu Val
            180                 185                 190

Val Glu Leu Gly Ser Xaa Arg Gly Lys Glu Asn Ala Ala Ala Val Leu
        195                 200                 205

Leu Gln Leu Cys Thr Asn Ser Xaa Arg Phe Cys Xaa Leu Val Leu Gln
    210                 215                 220

Glu Gly Ala Ile Pro Pro Leu Val Ala Leu Ser Gln Ser Gly Xaa Xaa
225                 230                 235                 240

Thr Xaa Arg Ala Lys Glu Lys Ala Gln
                245

<210>  63
<211>  2023
<212>  DNA
<213>  Hordeum vulgare

<220>
<221>  Unsure
<222>  (1)..(2023)

<400>  63
gaaagtgcat ttccttgttg cttccccagg ctgaccgacc agccacacca acccaaacca      60

acgcggcaaa accttggccc ccgcctgtct agcgctgccg ccgtgccgtg ccgtgccgtg     120

ccgtgcctca gcgccagcga ccgccgacga ctcaatcccg taccacgccc caccgccgcc     180

tcccacgccc ctcgtgccca tcgccgatcc cgttccccct caccgcagca tcgcgctccc     240

gcgtatccgc cccttccgca accgtcggcc attggtttct gagagcctca agatttgagc     300

accacaggca acagcctcct acatcctgtg tcggtacttg gtagggtaat cttccctgga     360

ccccgggagc tgacatgtat agagaagctt gaacagagca tgtgaaccat cctgaaatct     420

gatgccatgt gaaccatcct ggtcatgagg cctcatcgat cagtctttag atatgcaaat 480

ggctctgcta gcaaggcttt ctcttgcaag ttctgaagga agagagtcta gtttggaaga 540

aagacatgct ggttctgatg aacaacttca gaacaatcaa cgaaggaagc atttcaagca 600

tctcattttg cagtgattca caggttcgtc taggcagatc ttcagttaat gataatcttc 660

ctaatacccg tcagcttgac gaggagtgtg acatcaagat gggatgatac gagttccagg 720

tgataggaca aattatagta ggatgcgtct ggagaggttg ctgaccgtgg gctttctatc 780

tcttctgccc ctcaaaggga aaatgtaatc ctgccagatt gggtcatgtc tgcatggagg 840

gaccatttgt tcagcggcaa catctgacaa gggattcccg agaataattt cgtcgttatc 900

catggatgcc cgggatgatt tctctgccat cgagaatcag gtacgcgagt aatcaatgat 960

ttgggaagtg attccataga aggtcagaga tcagcacatc agagattcgc cttctagcta 1020

agcacaacat ggagaacaga ttgccattgc taattgtggg gctataactt gctggttggc 1080

cttcttcatt cacccgatgc caaaatccaa gaaaatgcag tgacagccct ccttaatttg 1140

tcactcagtg atatcaataa gattgccatc gtgaatgcag atgctattga tcctctcatc 1200

atgtcctgga aacagggaac cctgaagcta aagagaattc agcagtactt tgttcagtct 1260

ctcaattatt gaagaaaaca gagtgaggat agggcgatct ggtgctgtaa agcctctcgt 1320

ggacttgctg ggaatgggag cccacgagga aagaaagatg cggttactgc attgttaatt 1380

tatccatact tcatgagaac aagggtcgaa ttgtgcaagc tgatgcattg aagcacctag 1440

ttgagcttat ggaccctgct gctgaatggt cgataaagct gtagctgtct tggcaaatct 1500

tgctacgata cagaaggaag gactgcgatt gggcaggcgc gtggtattcc ggcccttgtt 1560

gaagttgtcg aactgggttc agcgaaagga aggaaaatgc taccgcggca ttgcttcagc 1620

tatgcacaaa cagcacaggt tttgcaacat agttcttcaa gaggatgccg tgcccccttt 1680

agtcgcactg tcacagtcag gaacaccacg cgcaagagaa aaggcgcagg ttctcctcag 1740

ctatttccgc agccaaagac atgggaactc ggaaggagat gaggacgatg gtcctacgat 1800

atatttttct agttacgtcg agtatttccc tgaatttctc agatgatagt tattgttgtt 1860

gactggcgct gtgtactgct tatagtcact gtgagattgt gccatcttct caagctactg 1920

gtggattagt tgctgtgttt ggactggtcg ttgttgttgt tgagatggtg tattcttcgg 1980

gtttatattt ttttacatct tgtctattgg tatctaaaaa aaa 2023

<210> 64
<211> 480
<212> DNA
<213> Hordeum vulgare

<220>
<221>  5'UTR
<222>  (1)..(480)

<400>  64
gaaagtgcat ttccttgttg cttccccagg ctgaccgacc agccaccacc aacccaaacc      60

aacgcggcaa aaccttggcc cccgcctgtc ctagcgctgc cgccgtgccg tgccgtgccg     120

tgccgtgcct cagcgccagc gaccgccgac gactcaatcc cgtaccacgc ccccaccgcc     180

gcctcccacg cccctcgtgc ccatcgccga tcccgttccc cctcaccgca gcatcgcgct     240

cccgcggtat ccgccccttc cgcaaccgtc ggccattggt ttctgagagc cttcaagatt     300

tgagcaccac aggcaacagc ctcctacatc ctgtgtcggt acttggtagg gtaatcttcc     360

ctggaccccg ggagctgaca tgtatatgag aagcttgaac agagcatgtg aaccatcctg     420

aaatctgatg cacatgtgaa ccatcctggt catgaggcct catcgatcag tctttagatt     480